(19) Europäisches Patentamt
European Patent Office
Office européen des brevets

(11) EP 4 549 437 A1

(12) EUROPEAN PATENT APPLICATION
published in accordance with Art. 153(4) EPC

(43) Date of publication:
**07.05.2025 Bulletin 2025/19**

(21) Application number: **23830492.7**

(22) Date of filing: **30.06.2023**

(51) International Patent Classification (IPC):
***C07D 417/12*** (2006.01) ***C07D 401/04*** (2006.01)
***C07D 401/14*** (2006.01) ***C07D 413/14*** (2006.01)
***A61K 31/517*** (2006.01) ***A61K 31/5377*** (2006.01)
***A61P 35/00*** (2006.01)

(52) Cooperative Patent Classification (CPC):
**A61K 31/517; A61K 31/5377; A61P 35/00; C07D 401/04; C07D 401/14; C07D 413/14; C07D 417/12**

(86) International application number:
**PCT/CN2023/104529**

(87) International publication number:
**WO 2024/002328 (04.01.2024 Gazette 2024/01)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **30.06.2022 CN 202210773035**
**16.01.2023 CN 202310066234**
**21.06.2023 CN 202310739407**

(71) Applicant: **Suzhou Genhouse Bio Co., Ltd.**
**Suzhou,Jiangsu 215123 (CN)**

(72) Inventors:
- **ZHANG, Guiping**
**Suzhou, Jiangsu 215123 (CN)**
- **WANG, Kuifeng**
**Suzhou, Jiangsu 215123 (CN)**
- **LI, Jiapeng**
**Suzhou, Jiangsu 215123 (CN)**
- **ZHENG, Jiyue**
**Suzhou, Jiangsu 215123 (CN)**
- **DUAN, Shuangshuang**
**Suzhou, Jiangsu 215123 (CN)**
- **ZHANG, Tao**
**Suzhou, Jiangsu 215123 (CN)**

(74) Representative: **Cabinet Becker et Associés**
**25, rue Louis le Grand**
**75002 Paris (FR)**

(54) **NITROGEN-CONTAINING COMPOUND AND USE THEREOF**

(57) Provided are a nitrogen-containing compound and use thereof. Specifically, provided is a nitrogen-containing compound represented by formula I, a pharmaceutically acceptable salt thereof, a stereoisomer thereof, a tautomer thereof, a metabolite thereof, a prodrug thereof, an isotopically labeled derivative thereof, a solvate thereof, or a solvate of the pharmaceutically acceptable salt thereof. The above nitrogen-containing compound can effectively inhibit the overexpression of KIF18A protein in some human tumor cells.

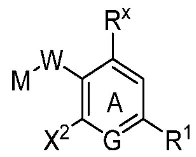

I

EP 4 549 437 A1

## Description

The present application claims priority to the Chinese Patent Application No. 2022107730353 filed on June 30, 2022, the Chinese Patent Application No. 202310066234.5 filed on January 16, 2023, and the Chinese Patent Application No. 2023107394075 filed on June 21, 2023, which are incorporated herein by reference in their entirety.

TECHNICAL FIELD

The present disclosure relates to a nitrogen-containing compound and use thereof.

BACKGROUND

There are 46 chromosomes in humans (two sets of 23), but in cancer, this number varies because during cell division, chromosome segregation occurs, leading to a phenomenon known as aneuploidy. Aneuploidy, which refers to the presence of an abnormal number of chromosomes in a cell, not only leads to common genetic diseases, but is also a hallmark of cancer cells. Not all cancers exhibit aneuploidy, but about 90% of solid tumors and 75% of blood cancers exhibit some degree of aneuploidy.

KIF18A, a member of the kinesin-8 family of kinesins, is a mitotic kinesin that can utilize the energy released from ATP hydrolysis to move along microtubules toward the plus end within a cell. Meanwhile, KIF18A localizes at the plus ends of microtubules, regulating the dynamic instability of the microtubules and exhibiting activity similar to that of a microtubule depolymerase. During mitosis, KIF18A can regulate spindle microtubule dynamics and chromosome oscillation, playing a crucial role in timely completion of chromosome alignment in mitosis, maintenance of genome stability, and successful completion of mitosis.

The KIF18A protein is overexpressed in some human tumor cells (e.g., breast cancer, ovarian cancer, and colorectal cancer, etc.), and is associated with tumor invasion and metastasis. After inhibition of the KIF18A protein, tumor cells with chromosomal instability exhibit mitotic delay, multipolar spindles, and increased cell death. Many anti-mitotic drugs are already used clinically for cancer treatment, including tubulin inhibitors that can stabilize tubulin or prevent tubulin assembly. Since tubulin is the main component of the mitotic spindle, disruption of tubulin dynamics by these drugs results in mitotic arrest and inhibition of tumor cell growth. However, because tubulin inhibitors also have inhibitory effects on normal cells, such drugs have relatively significant toxic and side effects. Aneuploid cancer cells with chromosomal instability (CIN) characteristics rely on the KIF18A protein for proliferation, whereas diploid cells do not. As a result, inhibiting the activity of the KIF18A protein has less impact on normal cells, while the proliferation of tumor cells is selectively inhibited. Therefore, KIF18A inhibitors can lead to a relatively good therapeutic window due to their selectivity between normal cells and tumor cells and thus have relatively good clinical application prospects for tumor treatment.

CONTENT OF THE PRESENT INVENTION

The present disclosure aims to solve the technical problem of the structural uniformity of KIF18A inhibitors in the prior art, and thus provides a nitrogen-containing compound and use thereof. The nitrogen-containing compound of the present disclosure can effectively inhibit the overexpression of the KIF18A protein in some human tumor cells.

The present disclosure provides a nitrogen-containing compound represented by formula I, a pharmaceutically acceptable salt thereof, a stereoisomer thereof, a tautomer thereof, a metabolite thereof, a prodrug thereof, an isotopically labeled derivative thereof, a solvate thereof, or a solvate of the pharmaceutically acceptable salt thereof:

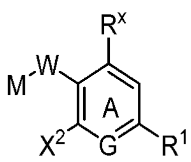

I

wherein $R^x$ is 3- to 6-membered monocyclic cycloalkyl, 5- to 6-membered monocyclic heterocycloalkyl, 6- to 9-membered bridged heterocycloalkyl, 7- to 9-membered spiro heterocycloalkyl, 5-to 6-membered monocyclic heterocycloalkyl substituted with one or more $R^{x-1}$, 6- to 9-membered bridged heterocycloalkyl substituted with one or more $R^{x-2}$, 7- to 9-membered spiro heterocycloalkyl substituted with one or more $R^{x-3}$, or 3- to 6-membered monocyclic cycloalkyl substituted with one or more $R^{x-4}$; in the "5-to 6-membered monocyclic heterocycloalkyl", the "6- to 9-membered bridged heterocycloalkyl", the "7- to 9-membered spiro heterocycloalkyl", the "5- to 6-membered monocyclic heterocycloalkyl substituted with one or more $R^{x-1}$", the "6- to 9-membered bridged heterocycloalkyl substituted

with one or more $R^{x-2}$", and the "7- to 9-membered spiro heterocycloalkyl substituted with one or more $R^{x-3}$", the heteroatom(s) independently consist(s) of 1 N atom and 0, 1, or 2 X atoms, the X atom being independently selected from 1 or 2 of N, O, and S; the "5- to 6-membered monocyclic heterocycloalkyl", the "6- to 9-membered bridged heterocycloalkyl", the "7- to 9-membered spiro heterocycloalkyl", the "5- to 6-membered monocyclic heterocycloalkyl substituted with one or more $R^{x-1}$", the "6- to 9-membered bridged heterocycloalkyl substituted with one or more $R^{x-2}$", and the "7- to 9-membered spiro heterocycloalkyl substituted with one or more $R^{x-3}$" are connected to ring A through the N atom;

each of $R^{x-1}$, $R^{x-2}$, $R^{x-3}$, and $R^{x-4}$ is independently halogen, $R^{N-10k,}$ $R^{N-10l}$, $C_{1-6}$ alkyl, $C_{1-6}$ alkyl substituted with one or more halogens, -$OR^{N-a}$, $C_{1-6}$ alkoxy substituted with one or more halogens, CN, - $NR^{N-a}R^{N-a}$, or oxo;

$R^1$ is -L-$R^{1-1}$;

L is *-$NHSO_2$-, -NH-, *-NHC(O)-, -NHC(O)NH-, *-$SO_2$NH-, or -$SO_2$-; * represents the end connected to ring A;

$R^{1-1}$ is $C_{1-6}$ alkyl, 3- to 6-membered monocyclic cycloalkyl, 4- to 8-membered monocyclic heterocycloalkyl, $C_{1-6}$ alkyl substituted with one or more $R^a$, or 3- to 6-membered monocyclic cycloalkyl substituted with one or more $R^b$; in the 4- to 8-membered monocyclic heterocycloalkyl, the heteroatom is independently selected from 1, 2, or 3 of N, O, and S, and the number of the heteroatom is independently 1, 2, 3, or 4;

each $R^a$ is independently hydroxyl, halogen, or -NH($R^{a-1}$);

$R^{a-1}$ is $C_{1-6}$ alkyl;

each $R^b$ is independently $C_{1-6}$ alkyl or $C_{1-6}$ alkyl substituted with one or more hydroxyl;

G is C($R^{10}$) or N; $R^{10}$ is H, halogen, $C_{1-6}$ alkyl, $C_{1-6}$ alkyl substituted with one or more halogens, -OH, -O-$R^{N-8a}$, or -O-$R^{N-8b}$;

M is phenyl, naphthyl, 5-membered heteroaryl, 6-membered heteroaryl, 9- to 10-membered heteroaryl, 6-membered heterocycloalkenyl, 6-membered heteroaryl substituted with one or more $R^2$, 9- to 10-membered heteroaryl substituted with one or more $R^3$, 6-membered heterocycloalkenyl substituted with one or more $R^4$, phenyl substituted with one or more $R^5$, naphthyl substituted with one or more $R^5$, or 5-membered heteroaryl substituted with one or more $R^6$; in the "5-membered heteroaryl", "6-membered heteroaryl", "9- to 10-membered heteroaryl", "6-membered heterocycloalkenyl", "6-membered heteroaryl substituted with one or more $R^2$", "9- to 10-membered heteroaryl substituted with one or more $R^3$", "6-membered heterocycloalkenyl substituted with one or more $R^4$", and "5-membered heteroaryl substituted with one or more $R^6$", the heteroatom is independently selected from 1, 2, or 3 of N, O, and S, and the number of the heteroatom is independently 1, 2, 3, or 4;

each of $R^2$, $R^3$, $R^4$, $R^5$, and $R^6$ is independently oxo, halogen, $R^{N-4a}$, $R^{N-4b}$, $C_{1-6}$ alkyl, $C_{1-6}$ alkyl substituted with one or more halogens, cyano, $C_{1-6}$ alkoxy, -O-$R^{N-6a}$, or -Y-$R^{N-13}$;

$R^{N-6a}$ is a saturated or partially saturated 3-, 4-, 5-, or 6-membered monocyclic ring comprising 0, 1, 2, or 3 N atoms and 0, 1, or 2 atoms selected from O and S;

Y is -$C_{1-6}$ alkyl-, -N($C_{1-6}$ alkyl)-$C_{1-6}$ alkyl-, -C(=O)$NR^{N-a}R^{N-a}$($C_{1-6}$ alkyl), -O-, -O-$C_{1-6}$ alkyl-, S, S=O, $S(=O)_2$, -S(=O)(=N-$R^{N-13}$)-, or -S(=O)(=NH)-;

$R^{N-13}$ is $R^{N-13a}$ or $R^{N-13b}$;

each of $R^{N-4a}$, $R^{N-8a}$, $R^{N-10k}$, and $R^{N-13a}$ is independently selected from the group consisting of: a saturated, partially saturated, or unsaturated 3-, 4-, 5-, 6-, or 7-membered monocyclic ring or 4-, 5-, 6-, 7-, 8-, 9-, 10-, 11-, or 12-membered bicyclic ring comprising 0, 1, 2, or 3 N atoms and 0, 1, or 2 atoms selected from O and S, wherein the monocyclic or bicyclic ring is substituted with 0, 1, 2, or 3 groups selected from: halogen, $C_{1-6}$ alkyl, $C_{1-6}$ alkyl substituted with one or more halogens, -$OR^{N-a}$, $C_{1-6}$ alkoxy substituted with one or more halogens, CN, -C(=O)$R^{N-b}$, -C(=O)$OR^{N-a}$, -C(=O)$NR^{N-a}R^{N-a}$, -C(=$NR^{N-a}$)$NR^{N-a}R^{N-a}$, - OC(=O)$R^{N-b}$, -OC(=O)$NR^{N-a}R^{N-a}$, -$OC_{1-6}$ alkyl $NR^{N-a}R^{N-a}$, -$OC_{1-6}$ alkyl $OR^{N-a}$, -$SR^{N-a}$, -S(=O)$R^{N-b}$, -$S(=O)_2R^{N-b}$, -$S(=O)_2NR^{N-a}$

$R^{N-a}$, -$NR^{N-a}R^{N-a}$, -N($R^{N-a}$)C(=O)$R^{N-b}$, -N($R^{N-a}$)C(=O)$OR^{N-b}$, -N($R^{N-a}$)C(=O)$NR^{N-a}R^{N-a}$, -N($R^{N-a}$)C(=$NR^{N-a}$) $NR^{N-a}R^{N-a}$, -N($R^{N-a}$)$S(=O)_2R^{N-b}$, -N($R^{N-a}$)$S(=O)_2NR^{N-a}R^{N-a}$, -$NR^{N-a}C_{1-6}$ alkyl $NR^{N-a}R^{N-a}$, -$NR^{N-a}C_{1-6}$ alkyl $OR^{N-a}$, - $C_{1-6}$ alkyl $NR^{N-a}R^{N-a}$, -$C_{1-6}$ alkyl $OR^{N-a}$, -$C_{1-6}$ alkyl N($R^{N-a}$)C(=O)$R^{N-b}$, -$C_{1-6}$ alkyl OC(=O)$R^{N-b}$, -$C_{1-6}$ alkyl C(=O)$NR^{N-a}R^{N-a}$, -$C_{1-6}$ alkyl C(=O)$OR^{N-a}$, $R^{N-14}$, and oxo;

each of $R^{N-4b}$, $R^{N-8b}$, $R^{N-10l}$, and $R^{N-13b}$ is independently $C_{1-6}$ alkyl substituted with 0, 1, 2, 3, 4, or 5 groups selected from halogen, -$OR^{N-a}$, $C_{1-6}$ alkoxy substituted with one or more halogens, and CN;

each $R^{N-14}$ is independently selected from the group consisting of: a saturated, partially saturated, or unsaturated 3-, 4-, 5-, 6-, or 7-membered monocyclic ring or 4-, 5-, 6-, 7-, 8-, 9-, 10-, 11-, or 12-membered bicyclic ring comprising 0, 1, 2, or 3 N atoms and 0 or 1 atom selected from O and S, wherein the monocyclic or bicyclic ring is substituted with 0, 1, 2, or 3 groups selected from: halogen, $C_{1-6}$ alkyl, $C_{1-6}$ alkyl substituted with one or more halogens, -$OR^{N-a}$, $C_{1-6}$ alkoxy substituted with one or more halogens, CN, -C(=O)$R^{N-b}$, - C(=O)$OR^{N-a}$, -C(=O)$NR^{N-a}R^{N-a}$, -C(=$NR^{N-a}$)$NR^{N-a}R^{N-a}$, -OC(=O)$R^{N-b}$, -OC(=O)$NR^{N-a}R^{N-a}$, -$OC_{1-6}$ alkyl $NR^{N-a}R^{N-a}$, -$OC_{1-6}$ alkyl $OR^{N-a}$, -$SR^{N-a}$, -S(=O)$R^{N-b}$, -$S(=O)_2R^{N-b}$, -$S(=O)_2NR^{N-a}R^{N-a}$, -$NR^{N-a}R^{N-a}$, -N($R^{N-a}$)C(=O)$R^{N-b}$, -N($R^{N-a}$)C(=O)$OR^{N-b}$, -N($R^{N-a}$)C(=O)$NR^{N-a}R^{N-a}$, -N($R^{N-a}$)C(=$NR^{N-a}$)$NR^{N-a}R^{N-a}$, -N($R^{N-a}$)$S(=O)_2R^{N-b}$, -N($R^{N-a}$)S(=O)2$NR^{N-a}R^{N-a}$, -$NR^{N-a}C_{1-6}$ alkyl $NR^{N-a}R^{N-a}$, -$NR^{N-a}C_{1-6}$ alkyl $OR^{N-a}$, -$C_{1-6}$ alkyl $NR^{N-a}R^{N-a}$, -$C_{1-6}$ alkyl $OR^{N-a}$, -$C_{1-6}$ alkyl N($R^{N-a}$)C(=O)$R^{N-b}$, -$C_{1-6}$ alkyl OC(=O)$R^{N-b}$, -$C_{1-6}$

alkyl C(=O)NR$^{N-a}$R$^{N-a}$, -$C_{1-6}$ alkyl C(=O)OR$^{N-a}$, and oxo;

each $R^{N-a}$ is independently H or $R^{N-b}$;

each $R^{N-b}$ is independently $C_{1-6}$ alkyl, phenyl, or benzyl, wherein the $C_{1-6}$ alkyl is substituted with 0, 1, 2, or 3 substituents selected from: halogen, -OH, -O$C_{1-6}$ alkyl, -$NH_2$, -NH$C_{1-6}$ alkyl, -OC(=O)$C_{1-6}$ alkyl, or -N($C_{1-6}$ alkyl)$C_{1-6}$ alkyl; and the phenyl or benzyl is substituted with 0, 1, 2, or 3 substituents selected from: halogen, $C_{1-6}$ alkyl, $C_{1-6}$ alkyl substituted with one or more halogens, -OH, -O$C_{1-6}$ alkyl, -$NH_2$, -NH$C_{1-6}$ alkyl, -OC(=O)$C_{1-6}$ alkyl, or -N($C_{1-6}$ alkyl) $C_{1-6}$ alkyl;

W and $X^2$ are as defined in any one of the following schemes:

scheme 1: W is *-CONR$^W$-; $X^2$ is $R^{W-1}$; * represents the end connected to ring A;

scheme 2: W is *-NR$^W$CO-; $X^2$ is $R^{W-1}$; * represents the end connected to ring A; and

scheme 3: W is *-C(=$X^1$)NR$^W$-; $X^1$, $X^2$, and the C atoms therebetween together form 6-membered heteroaryl; in the 6-membered heteroaryl, the heteroatom is selected from 1, 2, or 3 of N, O, and S, and the number of the heteroatom is 1, 2, or 3; * represents the end connected to ring A;

$R^W$ is H, $C_{1-6}$ alkyl, or $C_{1-6}$ alkyl substituted with one or more halogens;

$R^{W-1}$ is H, halogen, $C_{1-6}$ alkyl, or $C_{1-6}$ alkyl substituted with one or more halogens; and

the compound represented by formula I satisfies one, two, or three of the following conditions:

condition 1: $R^x$ is 6- to 9-membered bridged heterocycloalkyl or 6- to 9-membered bridged heterocycloalkyl substituted with one or more $R^{x-2}$;

condition 2: M is naphthyl, 5-membered heteroaryl, 9- to 10-membered heteroaryl, 6-membered heterocycloalkenyl, naphthyl substituted with one or more $R^5$, 5-membered heteroaryl substituted with one or more $R^6$, 9- to 10-membered heteroaryl substituted with one or more $R^3$, or 6-membered heterocycloalkenyl substituted with one or more $R^4$; and

condition 3: W is *-C(=$X^1$)NR$^W$-; $X^1$, $X^2$, and the C atoms therebetween together form 6-membered heteroaryl.

The present disclosure provides a nitrogen-containing compound represented by formula II, a pharmaceutically acceptable salt thereof, a stereoisomer thereof, a tautomer thereof, a metabolite thereof, a prodrug thereof, an isotopically labeled derivative thereof, a solvate thereof, or a solvate of the pharmaceutically acceptable salt thereof:

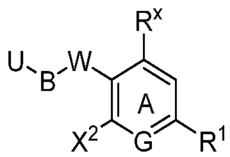

II

wherein $R^x$ is 3- to 6-membered monocyclic cycloalkyl, 5- to 6-membered monocyclic heterocycloalkyl, 6- to 9-membered bridged heterocycloalkyl, 7- to 9-membered spiro heterocycloalkyl, 5-to 6-membered monocyclic heterocycloalkyl substituted with one or more $R^{x-1}$, 6- to 9-membered bridged heterocycloalkyl substituted with one or more $R^{x-2}$, 7- to 9-membered spiro heterocycloalkyl substituted with one or more $R^{x-3}$, or 3- to 6-membered monocyclic cycloalkyl substituted with one or more $R^{x-4}$; in the "5-to 6-membered monocyclic heterocycloalkyl", the "6- to 9-membered bridged heterocycloalkyl", the "7- to 9-membered spiro heterocycloalkyl", the "5- to 6-membered monocyclic heterocycloalkyl substituted with one or more $R^{x-1}$", the "6- to 9-membered bridged heterocycloalkyl substituted with one or more $R^{x-2}$", and the "7- to 9-membered spiro heterocycloalkyl substituted with one or more $R^{x-3}$", the heteroatom(s) independently consist(s) of 1 N atom and 0, 1, or 2 X atoms, the X atom being independently selected from 1 or 2 of N, O, and S; the "5- to 6-membered monocyclic heterocycloalkyl", the "6- to 9-membered bridged heterocycloalkyl", the "7- to 9-membered spiro heterocycloalkyl", the "5- to 6-membered monocyclic heterocycloalkyl substituted with one or more $R^{x-1}$", the "6- to 9-membered bridged heterocycloalkyl substituted with one or more $R^{x-2}$", and the "7- to 9-membered spiro heterocycloalkyl substituted with one or more $R^{x-3}$" are connected to ring A through the N atom;

each of $R^{x-1}$, $R^{x-2}$, $R^{x-3}$, and $R^{x-4}$ is independently halogen, $C_{1-6}$ alkyl, $C_{1-6}$ alkoxy, $C_{1-6}$ alkyl substituted with one or more halogens, or $C_{1-6}$ alkoxy substituted with one or more halogens;

$R^1$ is -L-$R^{1-1}$;

L is *-NH$SO_2$-, -NH-, *-NHC(O)-, -NHC(O)NH-, *-$SO_2$NH-, or -$SO_2$-; * represents the end connected to ring A;

$R^{1-1}$ is $C_{1-6}$ alkyl, 3- to 6-membered monocyclic cycloalkyl, 4- to 8-membered monocyclic heterocycloalkyl, $C_{1-6}$ alkyl substituted with one or more $R^a$, or 3- to 6-membered monocyclic cycloalkyl substituted with one or more $R^b$; in the 4- to 8-membered monocyclic heterocycloalkyl, the heteroatom is independently selected from 1, 2, or 3 of N, O, and S, and the number of the heteroatom is independently 1, 2, 3, or 4;

each $R^a$ is independently hydroxyl, halogen, or -NH($R^{a-1}$);

$R^{a-1}$ is $C_{1-6}$ alkyl;

each $R^b$ is independently $C_{1-6}$ alkyl or $C_{1-6}$ alkyl substituted with one or more hydroxyl;
B is phenyl, naphthyl, 5-membered heteroaryl, 6-membered heteroaryl, 9- to 10-membered heteroaryl, 6-membered heterocycloalkenyl, 6-membered heteroaryl substituted with one or more $R^2$, 9- to 10-membered heteroaryl substituted with one or more $R^3$, 6-membered heterocycloalkenyl substituted with one or more $R^4$, phenyl substituted with one or more $R^5$, naphthyl substituted with one or more $R^5$, or 5-membered heteroaryl substituted with one or more $R^6$; in the "5-membered heteroaryl", "6-membered heteroaryl", "9- to 10-membered heteroaryl", "6-membered heterocycloalkenyl", "6-membered heteroaryl substituted with one or more $R^2$", "9- to 10-membered heteroaryl substituted with one or more $R^3$", "6-membered heterocycloalkenyl substituted with one or more $R^4$", and "5-membered heteroaryl substituted with one or more $R^6$", the heteroatom is independently selected from 1, 2, or 3 of N, O, and S, and the number of the heteroatom is independently 1, 2, 3, or 4;
each of $R^2$, $R^3$, $R^4$, $R^5$, and $R^6$ is independently halogen, cyano, oxo (=O), $C_{1-6}$ alkyl, or $C_{1-6}$ alkyl substituted with one or more halogens;
U is 3- to 10-membered cycloalkyl, 4- to 10-membered heterocycloalkyl, 6- to 10-membered aryl, 5- to 10-membered heteroaryl, 3- to 10-membered cycloalkyl substituted with one or more $R^{2-1}$, 4- to 10-membered heterocycloalkyl substituted with one or more $R^{2-2}$, or 5- to 10-membered heteroaryl substituted with one or more $R^{2-3}$; in the "5- to 10-membered heteroaryl", "4- to 10-membered heterocycloalkyl", "4- to 10-membered heterocycloalkyl substituted with one or more $R^{2-2}$", and "5- to 10-membered heteroaryl substituted with one or more $R^{2-3}$", the heteroatom is independently selected from 1, 2, or 3 of N, O, and S, and the number of the heteroatom is independently 1, 2, 3, or 4;
each of $R^{2-1}$, $R^{2-2}$, and $R^{2-3}$ is independently halogen, $C_{1-6}$ alkyl, oxo, CN, hydroxyl, 3- to 6-membered monocyclic cycloalkyl, $C_{1-6}$ alkyl substituted with one or more halogens, $C_{1-6}$ alkoxy substituted with one or more halogens, 3- to 6-membered monocyclic cycloalkyl substituted with one or more halogens, or $C_{1-6}$ alkoxy;
W and $X^2$ are as defined in any one of the following schemes:

(1) W is *-CONR$^W$-; $X^2$ is $R^{W-1}$; * represents the end connected to ring A;
(2) W is *-NR$^W$CO-; $X^2$ is $R^{W-1}$; * represents the end connected to ring A; and
(3) W is *-C(=$X^1$)NR$^W$-; $X^1$, $X^2$, and the C atoms therebetween together form 6-membered heteroaryl; in the 6-membered heteroaryl, the heteroatom is selected from 1, 2, or 3 of N, O, and S, and the number of the heteroatom is 1, 2, or 3; * represents the end connected to ring A;

$R^W$ is H, $C_{1-6}$ alkyl, or $C_{1-6}$ alkyl substituted with one or more halogens;
$R^{W-1}$ is H, halogen, $C_{1-6}$ alkyl, or $C_{1-6}$ alkyl substituted with one or more halogens;
G is C($R^{10}$) or N;
$R^{10}$ is H, halogen, $C_{1-6}$ alkyl, hydroxyl, or $C_{1-6}$ alkyl substituted with one or more halogens; and
the compound represented by formula II satisfies one, two, or three of the following conditions:

(1)$R^x$ is 6- to 9-membered bridged heterocycloalkyl or 6- to 9-membered bridged heterocycloalkyl substituted with one or more $R^{x-2}$;
(2)B is naphthyl, 5-membered heteroaryl, 9- to 10-membered heteroaryl, 6-membered heterocycloalkenyl, naphthyl substituted with one or more $R^5$, 5-membered heteroaryl substituted with one or more $R^6$, 9- to 10-membered heteroaryl substituted with one or more $R^3$, or 6-membered heterocycloalkenyl substituted with one or more $R^4$; and
(3)W is *-C(=$X^1$)NR$^W$-; $X^1$, $X^2$, and the C atoms therebetween together form 6-membered heteroaryl.

In a certain embodiment, in the nitrogen-containing compound represented by formula **II**, the pharmaceutically acceptable salt thereof, the stereoisomer thereof, the tautomer thereof, the metabolite thereof, the prodrug thereof, the isotopically labeled derivative thereof, the solvate thereof, or the solvate of the pharmaceutically acceptable salt thereof, certain groups in the nitrogen-containing compound represented by formula **II** are defined as follows, and the remaining groups are defined as described in any one of the other embodiments (hereinafter referred to as "in a certain embodiment"):

$R^x$ is 6- to 9-membered bridged heterocycloalkyl, 7- to 9-membered spiro heterocycloalkyl, or 6-to 9-membered bridged heterocycloalkyl substituted with one or more $R^{x-2}$; in the "6- to 9-membered bridged heterocycloalkyl", the "7- to 9-membered spiro heterocycloalkyl", and the "6- to 9-membered bridged heterocycloalkyl substituted with one or more $R^{x-2}$", the heteroatom(s) independently consist(s) of 1 N atom and 0, 1, or 2 X atoms, the X atom being independently selected from 1 or 2 of N, O, and S; the "6- to 9-membered bridged heterocycloalkyl", the "7- to 9-membered spiro heterocycloalkyl", and the "6- to 9-membered bridged heterocycloalkyl substituted with one or more $R^{x-2}$" are connected to ring A through the N atom;

each $R^{x-2}$ is independently $C_{1-6}$ alkyl;
$R^1$ is -L-$R^{1-1}$;
L is *-$NHSO_2$-; * represents the end connected to ring A;
$R^{1-1}$ is $C_{1-6}$ alkyl, $C_{1-6}$ alkyl substituted with one or more $R^a$, or 3- to 6-membered monocyclic cycloalkyl substituted with one or more $R^b$;
each $R^a$ is independently hydroxyl;
each $R^b$ is independently $C_{1-6}$ alkyl;
B is 5-membered heteroaryl, 9- to 10-membered heteroaryl, 6-membered heteroaryl substituted with one or more $R^2$, or 9- to 10-membered heteroaryl substituted with one or more $R^3$; in the "5-membered heteroaryl", "9- to 10-membered heteroaryl", "6-membered heteroaryl substituted with one or more $R^2$", and "9- to 10-membered heteroaryl substituted with one or more $R^3$", the heteroatom is independently selected from 1, 2, or 3 of N, O, and S, and the number of the heteroatom is independently 1, 2, 3, or 4;
each $R^2$ is independently $C_{1-6}$ alkyl;
each $R^3$ is independently halogen, $C_{1-6}$ alkyl, or $C_{1-6}$ alkyl substituted with one or more halogens;
U is 3- to 10-membered cycloalkyl, 4- to 10-membered heterocycloalkyl, 3- to 10-membered cycloalkyl substituted with one or more $R^{2-1}$, or 4- to 10-membered heterocycloalkyl substituted with one or more $R^{2-2}$; in the "4- to 10-membered heterocycloalkyl" or "4- to 10-membered heterocycloalkyl substituted with one or more $R^{2-2}$", the heteroatom is independently selected from 1, 2, or 3 of N, O, and S, and the number of the heteroatom is independently 1, 2, 3, or 4;
each of $R^{2-1}$, $R^{2-2}$, and $R^{2-3}$ is independently halogen;
W and $X^2$ are as defined in any one of the following schemes:

(1) W is *-CON$R^W$-; $X^2$ is $R^{W-1}$; * represents the end connected to ring A; and
(2) W is *-C(=$X^1$)N$R^W$-; $X^1$, $X^2$, and the C atoms therebetween together form 6-membered heteroaryl; in the 6-membered heteroaryl, the heteroatom is selected from 1, 2, or 3 of N, O, and S, and the number of the heteroatom is 1, 2, or 3; * represents the end connected to ring A;

$R^W$ is H; $R^{W-1}$ is H;
G is C($R^{10}$) or N;
$R^{10}$ is H; and
the compound represented by formula II satisfies one, two, or three of the following conditions:

(1) $R^x$ is 6- to 9-membered bridged heterocycloalkyl or 6- to 9-membered bridged heterocycloalkyl substituted with one or more $R^{x-2}$;
(2) B is naphthyl, 5-membered heteroaryl, 9- to 10-membered heteroaryl, 6-membered heterocycloalkenyl, naphthyl substituted with one or more $R^5$, 5-membered heteroaryl substituted with one or more $R^6$, 9- to 10-membered heteroaryl substituted with one or more $R^3$, or 6-membered heterocycloalkenyl substituted with one or more $R^4$; and
(3) W is *-C(=$X^1$)N$R^W$-; $X^1$, $X^2$, and the C atoms therebetween together form 6-membered heteroaryl.

In a certain embodiment, $R^x$ is 6- to 9-membered bridged heterocycloalkyl, 7- to 9-membered spiro heterocycloalkyl, or 6- to 9-membered bridged heterocycloalkyl substituted with one or more $R^{x-2}$; in the "6-to 9-membered bridged heterocycloalkyl", the "7- to 9-membered spiro heterocycloalkyl", and the "6- to 9-membered bridged heterocycloalkyl substituted with one or more $R^{x-2}$", the heteroatom(s) independently consist(s) of 1 N atom and 0, 1, or 2 X atoms, the X atom being independently selected from 1 or 2 of N, O, and S; the "6- to 9-membered bridged heterocycloalkyl", the "7- to 9-membered spiro heterocycloalkyl", and the "6- to 9-membered bridged heterocycloalkyl substituted with one or more $R^{x-2}$" are connected to ring A through the N atom;

each $R^{x-2}$ is independently $C_{1-6}$ alkyl;
$R^1$ is -L-$R^{1-1}$;
L is *-$NHSO_2$-; * represents the end connected to ring A;
$R^{1-1}$ is $C_{1-6}$ alkyl, $C_{1-6}$ alkyl substituted with one or more $R^a$, or 3- to 6-membered monocyclic cycloalkyl substituted with one or more $R^b$;
each $R^a$ is independently hydroxyl;
each $R^b$ is independently $C_{1-6}$ alkyl;
B is 9- to 10-membered heteroaryl, 6-membered heteroaryl substituted with one or more $R^2$, or 9-to 10-membered heteroaryl substituted with one or more $R^3$; in the "5-membered heteroaryl", "9- to 10-membered heteroaryl", "6-membered heteroaryl substituted with one or more $R^2$", and "9- to 10-membered heteroaryl substituted with one or more

more $R^3$", the heteroatom is independently selected from 1, 2, or 3 of N, O, and S, and the number of the heteroatom is independently 1, 2, 3, or 4; when the 9- to 10-membered heteroaryl is 9-membered heteroaryl with 2 heteroatoms, the heteroatoms are independently selected from 1 or 2 of N and O; when the 9- to 10-membered heteroaryl is 9-membered heteroaryl with 3 heteroatoms, the heteroatoms are N; when the 9- to 10-membered heteroaryl is 10-membered heteroaryl with 2 heteroatoms of N, the heteroatoms are located on different rings;
when B is 6-membered heteroaryl substituted with one or more $R^2$, $R^x$ is 8- to 9-membered bridged heterocycloalkyl; in the "8- to 9-membered bridged heterocycloalkyl", the heteroatom(s) independently consist(s) of 1 N atom and 0, 1, or 2 X atoms, the X atom being independently selected from 1 or 2 of N, O, and S; the "8- to 9-membered bridged heterocycloalkyl" is connected to ring A through the N atom;
each $R^2$ is independently $C_{1-6}$ alkyl;
each $R^3$ is independently halogen or $C_{1-6}$ alkyl;
U is 4- to 10-membered heterocycloalkyl or 4- to 10-membered heterocycloalkyl substituted with one or more $R^{2-2}$; in the "4- to 10-membered heterocycloalkyl" or "4- to 10-membered heterocycloalkyl substituted with one or more $R^{2-2}$", the heteroatoms are independently selected from 1, 2, or 3 of N, O, and S, and the number of the heteroatoms is independently 2, 3, or 4;
each $R^{2-2}$ is independently halogen;
W is *-CONR$^W$-; $X^2$ is $R^{W-1}$; * represents the end connected to ring A;
$R^W$ is H; $R^{W-1}$ is H;
G is C($R^{10}$) or N;
$R^{10}$ is H; and
the compound represented by formula II satisfies one or two of the following conditions:

(1) $R^x$ is 6- to 9-membered bridged heterocycloalkyl or 6- to 9-membered bridged heterocycloalkyl substituted with one or more $R^{x-2}$; and
(2) B is 9- to 10-membered heteroaryl or 9- to 10-membered heteroaryl substituted with one or more $R^3$.

In a certain embodiment, $R^x$ is 6- to 9-membered bridged heterocycloalkyl, 6- to 9-membered bridged heterocycloalkyl substituted with one or more $R^{x-2}$, or 7- to 9-membered spiro heterocycloalkyl;

each $R^{x-2}$; is independently $C_{1-6}$ alkyl;
$R^1$ is -L-$R^{1-1}$;
L is *-$NHSO_2$-;
$R^{1-1}$ is $C_{1-6}$ alkyl, $C_{1-6}$ alkyl substituted with one or more $R^a$, or 3- to 6-membered monocyclic cycloalkyl substituted with one or more $R^b$;
$R^a$ is hydroxyl;
each $R^b$ is independently $C_{1-6}$ alkyl;
B is 5-membered heteroaryl, 9- to 10-membered heteroaryl, 6-membered heteroaryl substituted with one or more $R^2$, or 9- to 10-membered heteroaryl substituted with one or more $R^3$;
each $R^2$ is independently $C_{1-6}$ alkyl;
each $R^3$ is independently $C_{1-6}$ alkyl;
U is 4- to 10-membered heterocycloalkyl, 3- to 10-membered cycloalkyl substituted with one or more $R^{2-1}$, or 4- to 10-membered heterocycloalkyl substituted with one or more $R^{2-2}$;
each $R^{2-1}$ is independently halogen;
each $R^{2-2}$ is independently halogen;
W is *-CONH-; $X^2$ is H; and
the compound represented by formula II satisfies one or two of the following conditions:

(1) $R^x$ is 6- to 9-membered bridged heterocycloalkyl; and
(2) B is 5-membered heteroaryl, naphthyl, 9- to 10-membered heteroaryl, naphthyl substituted with one or more $R^5$, or 9- to 10-membered heteroaryl substituted with one or more $R^3$.

In a certain embodiment, $R^x$ is 6- to 9-membered bridged heterocycloalkyl, 6- to 9-membered bridged heterocycloalkyl substituted with one or more $R^{x-2}$, or 7- to 9-membered spiro heterocycloalkyl;

each $R^{x-2}$ is independently $C_{1-6}$ alkyl;
$R^1$ is -L-$R^{1-1}$;
L is *-$NHSO_2$-;
$R^{1-1}$ is $C_{1-6}$ alkyl, $C_{1-6}$ alkyl substituted with one or more $R^a$, or 3- to 6-membered monocyclic cycloalkyl substituted

with one or more $R^b$;
$R^a$ is hydroxyl;
each $R^b$ is independently $C_{1-6}$ alkyl;
B is naphthyl, 5-membered heteroaryl, 9- to 10-membered heteroaryl, naphthyl substituted with one or more $R^5$, 6-membered heteroaryl substituted with one or more $R^2$, or 9- to 10-membered heteroaryl substituted with one or more $R^3$;
each $R^2$ is independently $C_{1-6}$ alkyl;
each $R^3$ is independently $C_{1-6}$ alkyl;
U is 4- to 10-membered heterocycloalkyl, 3- to 10-membered cycloalkyl substituted with one or more $R^{2-1}$, or 4- to 10-membered heterocycloalkyl substituted with one or more $R^{2-2}$;
each $R^{2-1}$ is independently halogen;
each $R^{2-2}$ is independently halogen;
W is *-CONH-; $X^2$ is H; and
the compound represented by formula II satisfies one or two of the following conditions:

(1) $R^x$ is 6- to 9-membered bridged heterocycloalkyl; and
(2) B is 5-membered heteroaryl, 9- to 10-membered heteroaryl, or 9- to 10-membered heteroaryl substituted with one or more $R^3$.

In a certain embodiment, $R^x$ is 6- to 9-membered bridged heterocycloalkyl, 7- to 9-membered spiro heterocycloalkyl, 6- to 9-membered bridged heterocycloalkyl substituted with one or more $R^{x-2}$, or 7- to 9-membered spiro heterocycloalkyl substituted with one or more $R^{x-3}$, preferably 6- to 9-membered bridged heterocycloalkyl substituted with one or more $R^{x-2}$, 6- to 9-membered bridged heterocycloalkyl, or 7- to 9-membered spiro heterocycloalkyl, and more preferably 6- to 9-membered bridged heterocycloalkyl or 7- to 9-membered spiro heterocycloalkyl.
In a certain embodiment, each of $R^{x-1}$, $R^{x-2}$, $R^{x-3}$, and $R^{x-4}$ is independently halogen, $C_{1-6}$ alkyl, or $C_{1-6}$ alkyl substituted with one or more halogens, preferably halogen or $C_{1-6}$ alkyl, and more preferably $C_{1-3}$ alkyl, F, Cl, or Br.
In a certain embodiment, $R^x$ is 6- to 9-membered bridged heterocycloalkyl, 7- to 9-membered spiro heterocycloalkyl, 6- to 9-membered bridged heterocycloalkyl substituted with one or more $R^{x-2}$, or 7- to 9-membered spiro heterocycloalkyl substituted with one or more $R^{x-3}$;
each of $R^{x-1}$, $R^{x-2}$, $R^{x-3}$, and $R^{x-4}$ is independently halogen, $C_{1-6}$ alkyl, or $C_{1-6}$ alkyl substituted with one or more halogens, preferably halogen or $C_{1-6}$ alkyl, and more preferably $C_{1-3}$ alkyl, F, Cl, or Br.
In a certain embodiment, $R^x$ is 6- to 9-membered bridged heterocycloalkyl, 7- to 9-membered spiro heterocycloalkyl, or 6- to 9-membered bridged heterocycloalkyl substituted with one or more $R^{x-2}$;
each $R^{x-2}$; is independently $C_{1-6}$ alkyl.
In a certain embodiment, each of $R^{x-1}$, $R^{x-2}$, $R^{x-3}$, and $R^{x-4}$ is independently $C_{1-6}$ alkyl.
In a certain embodiment, L is *-$NHSO_2$-.
In a certain embodiment, L is *-$NHSO_2$- or -NH-.
In a certain embodiment, $R^{1-1}$ is $C_{1-6}$ alkyl, 3- to 6-membered monocyclic cycloalkyl, 3- to 6-membered monocyclic cycloalkyl substituted with one or more $R^b$, or $C_{1-6}$ alkyl substituted with one or more $R^a$, preferably $C_{1-3}$ alkyl, $C_{1-3}$ alkyl substituted with one or more $R^a$, or 3- to 6-membered monocyclic cycloalkyl substituted with one or more $R^b$. In a certain embodiment, $R^a$ is hydroxyl.
In a certain embodiment, each $R^b$ is independently $C_{1-6}$ alkyl.
In a certain embodiment, $R^{1-1}$ is $C_{1-6}$ alkyl, 3- to 6-membered monocyclic cycloalkyl substituted with one or more $R^b$, or $C_{1-6}$ alkyl substituted with one or more $R^a$;

$R^a$ is hydroxyl;
each $R^b$ is independently $C_{1-6}$ alkyl.

In a certain embodiment, B is phenyl, naphthyl, 5-membered heteroaryl, 6-membered heteroaryl, 9- to 10-membered heteroaryl, 6-membered heterocycloalkenyl, naphthyl substituted with one or more $R^5$, 6-membered heteroaryl substituted with one or more $R^2$, 9- to 10-membered heteroaryl substituted with one or more $R^3$, or 6-membered heterocycloalkenyl substituted with one or more $R^4$, preferably 5-membered heteroaryl, 9- to 10-membered heteroaryl, 6-membered heteroaryl substituted with one or more $R^2$, or 9- to 10-membered heteroaryl substituted with one or more $R^3$, and more preferably 9- to 10-membered heteroaryl or 9- to 10-membered heteroaryl substituted with one or more $R^3$.
In a certain embodiment, B is 5-membered heteroaryl, 9- to 10-membered heteroaryl, 6-membered heteroaryl substituted with one or more $R^2$, or 9- to 10-membered heteroaryl substituted with one or more $R^3$.
In a certain embodiment, in B, the 9- to 10-membered heteroaryl is 9-membered heteroaryl with 2 heteroatoms independently selected from 1 or 2 of N and O.

In a certain embodiment, in B, the 9- to 10-membered heteroaryl is 9-membered heteroaryl with 3 heteroatoms of N.

In a certain embodiment, in B, the 9- to 10-membered heteroaryl is 10-membered heteroaryl with 2 heteroatoms of N, and the heteroatoms are located on different rings.

In a certain embodiment, when B is 6-membered heteroaryl substituted with one or more $R^2$, $R^x$ is 8- to 9-membered bridged heterocycloalkyl.

In a certain embodiment, each of $R^2$, $R^3$, $R^4$, $R^5$, and $R^6$ is independently halogen, oxo, or $C_{1-6}$ alkyl, preferably $C_{1-3}$ alkyl.

In a certain embodiment, each of $R^2$, $R^3$, $R^4$, $R^5$, and $R^6$ is independently halogen, $C_{1-6}$ alkyl, or $C_{1-6}$ alkyl substituted with one or more halogens.

In a certain embodiment, each $R^2$ is independently $C_{1-6}$ alkyl.

In a certain embodiment, each $R^3$ is independently halogen, $C_{1-6}$ alkyl, or $C_{1-6}$ alkyl substituted with one or more halogens.

In a certain embodiment, each $R^3$ is independently halogen or $C_{1-6}$ alkyl.

In a certain embodiment, B is 9- to 10-membered heteroaryl, 6-membered heteroaryl substituted with one or more $R^2$, or 9- to 10-membered heteroaryl substituted with one or more $R^3$;

each $R^2$ is independently $C_{1-6}$ alkyl;
each $R^3$ is independently halogen or $C_{1-6}$ alkyl.

In a certain embodiment, B is 5-membered heteroaryl, 9- to 10-membered heteroaryl, 6-membered heteroaryl substituted with one or more $R^2$, or 9- to 10-membered heteroaryl substituted with one or more $R^3$;
each of $R^2$ and $R^3$ is independently halogen or $C_{1-6}$ alkyl.

In a certain embodiment, U is 3- to 10-membered cycloalkyl, 4- to 10-membered heterocycloalkyl, 3- to 10-membered cycloalkyl substituted with one or more $R^{2-1}$, or 4- to 10-membered heterocycloalkyl substituted with one or more $R^{2-2}$, preferably 4- to 9-membered heterocycloalkyl, 3- to 8-membered cycloalkyl substituted with one or more $R^{2-1}$, or 4- to 9-membered heterocycloalkyl substituted with one or more $R^{2-2}$, and more preferably 4- to 9-membered heterocycloalkyl or 4- to 9-membered heterocycloalkyl substituted with one or more $R^{2-2}$.

In a certain embodiment, in U, the 4- to 10-membered heterocycloalkyl is 4- to 6-membered monocyclic heterocycloalkyl, 6- to 9-membered bridged heterocycloalkyl, or 7- to 9-membered spiro heterocycloalkyl.

In a certain embodiment, each of $R^{2-1}$, $R^{2-2}$, and $R^{2-3}$ is independently halogen, $C_{1-6}$ alkyl, hydroxyl, $C_{1-6}$ alkoxy, or $C_{1-6}$ alkyl substituted with one or more halogens, preferably halogen, and more preferably F, Cl, or Br.

In a certain embodiment, U is 4- to 10-membered heterocycloalkyl or 4- to 10-membered heterocycloalkyl substituted with one or more $R^{2-2}$;
each $R^{2-2}$ is independently halogen.

In a certain embodiment, U is 3- to 10-membered cycloalkyl, 4- to 10-membered heterocycloalkyl, 3- to 10-membered cycloalkyl substituted with one or more $R^{2-1}$, or 4- to 10-membered heterocycloalkyl substituted with one or more $R^{2-2}$;
each of $R^{2-1}$ and $R^{2-2}$ is independently halogen, $C_{1-6}$ alkyl, hydroxyl, $C_{1-6}$ alkoxy, or $C_{1-6}$ alkyl substituted with one or more halogens, preferably F, Cl, or Br.

In a certain embodiment, G is $C(R^{10})$.

In a certain embodiment, G is $C(R^{10})$ or N; $R^{10}$ is H.

In a certain embodiment, $R^{10}$ is H, halogen, $C_{1-6}$ alkyl, or $C_{1-6}$ alkyl substituted with one or more halogens, preferably H.

In a certain embodiment, G is $C(R^{10})$; $R^{10}$ is H.

In a certain embodiment, $R^W$ is H.

In a certain embodiment, $R^{W-1}$ is H.

In a certain embodiment, W is *-CONR$^W$-; $X^2$ is $R^{W-1}$;

$R^{W-1}$ is H or halogen, preferably H;
$R^W$ is H.

In a certain embodiment, the compound represented by formula II satisfies 1 or 2 of the following conditions:

(a) $R^x$ is 6- to 9-membered bridged heterocycloalkyl; and
(b) B is 5-membered heteroaryl, 9- to 10-membered heteroaryl, 6-membered heterocycloalkenyl, 6-membered heterocycloalkenyl substituted with one or more $R^4$, or 9- to 10-membered heteroaryl substituted with one or more $R^3$, preferably 9- to 10-membered heteroaryl or 9- to 10-membered heteroaryl substituted with one or more $R^3$.

In a certain embodiment, in the compound represented by formula II, $R^x$ is 6- to 9-membered bridged heterocycloalkyl or 6- to 9-membered bridged heterocycloalkyl substituted with one or more $R^{x-2}$.

In a certain embodiment, in the compound represented by formula II, B is 9- to 10-membered heteroaryl or 9- to 10-membered heteroaryl substituted with one or more $R^3$.

In a certain embodiment, the nitrogen-containing compound represented by formula II is the following compound:

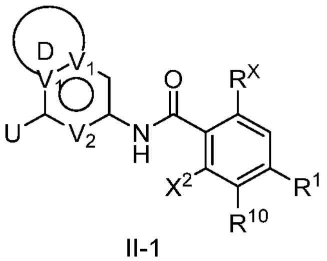

II-1 ;

each $V_1$ is independently C or N;

$V_2$ is N or C-$R^{11}$, preferably N;

D is partially saturated or unsaturated 5- to 6-membered carbocycle, or partially saturated or unsaturated 5- to 6-membered heterocycle, the 5- to 6-membered carbocycle or 5- to 6-membered heterocycle being optionally substituted with 0, 1, 2, or 3 $R^{11}$; in the "5- to 6-membered heterocycle", the heteroatom is N, O, or S, and the number of the heteroatom is independently 1, 2, 3, or 4;

$R^{11}$ is hydrogen, halogen, cyano, $C_{1-6}$ alkyl, $C_{1-6}$ alkoxy, or $C_{1-6}$ alkyl substituted with one or more halogens.

In a certain embodiment, each "5- to 6-membered monocyclic heterocycloalkyl" is independently 5- to 6-membered heterocycloalkyl comprising 1 or 2 heteroatoms, preferably

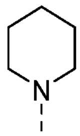 or 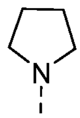 .

In a certain embodiment, each "6- to 9-membered bridged heterocycloalkyl" is independently 6-to 9-membered bridged heterocycloalkyl comprising 1 or 2 heteroatoms, preferably selected from any one of the following fragments:

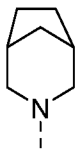 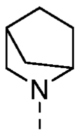 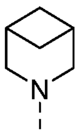 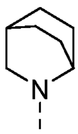 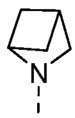 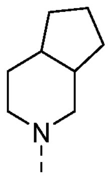 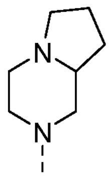 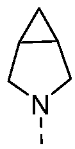 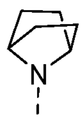 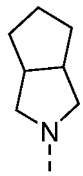 ,

and more preferably

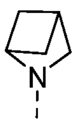 , 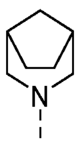 , 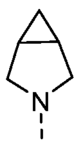 , 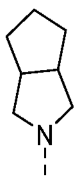 , or 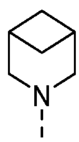 .

In a certain embodiment, each "6- to 9-membered bridged heterocycloalkyl" is independently 6-to 9-membered bridged heterocycloalkyl comprising 1 or 2 heteroatoms, preferably any one of the following fragments:

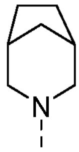 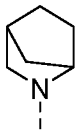 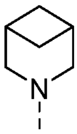 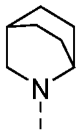 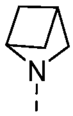 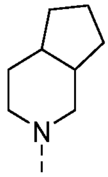 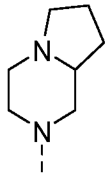 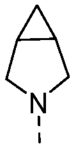 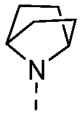 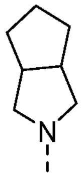

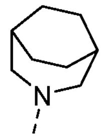 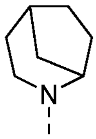 ,

and more preferably 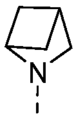 , 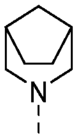 , 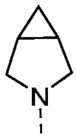 , 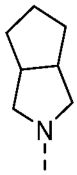 , 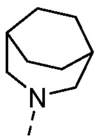 , 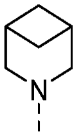 , or 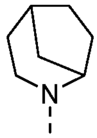 .

In a certain embodiment, each "7- to 9-membered spiro heterocycloalkyl" is independently 7- to 9-membered spiro heterocycloalkyl comprising 1 or 2 heteroatoms, preferably any one of the following fragments:

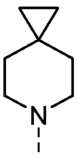 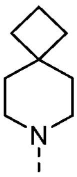 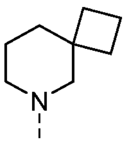 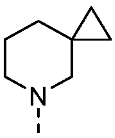 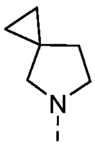 ,

and more preferably

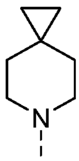 .

In a certain embodiment, each "halogen" is independently fluorine, chlorine, bromine, or iodine, preferably fluorine.

In a certain embodiment, each "$C_{1-6}$ alkyl" is independently methyl, ethyl, n-propyl, isopropyl, n-butyl, isobutyl, or *tert*-butyl, preferably methyl or ethyl.

In a certain embodiment, in $R^{1-1}$, the $C_{1-6}$ alkyl is ethyl or isopropyl.

In a certain embodiment, each "3- to 6-membered monocyclic cycloalkyl" is independently cyclopropyl, cyclobutyl, cyclopentyl, or cyclohexyl, preferably cyclopropyl.

In a certain embodiment, each "$C_{1-6}$ alkoxy" is independently methoxy, ethoxy, n-propoxy, isopropoxy, n-butoxy, isobutoxy, or tert-butoxy, preferably methoxy or ethoxy.

In a certain embodiment, the "4- to 8-membered monocyclic heterocycloalkyl" is "4- to 8-membered monocyclic heterocycloalkyl" comprising one O atom, preferably

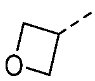 .

In a certain embodiment, each "5-membered heteroaryl" is independently 5-membered heteroaryl with "heteroatoms selected from 1 or 2 of N and S", preferably any one of the following fragments:

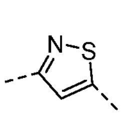 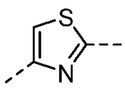 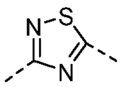 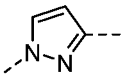 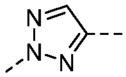 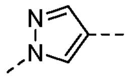 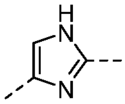 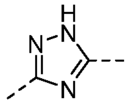 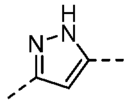

, and more preferably

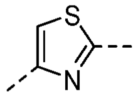

(e.g.,

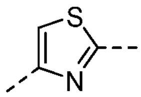

connected to U through the right dashed line).

In a certain embodiment, each "6-membered heteroaryl" is independently any one of the following fragments:

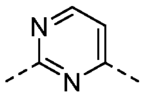 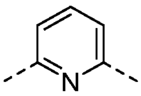 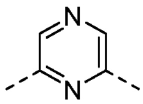 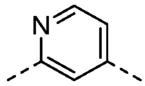 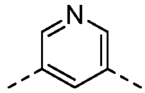 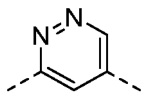 ,

preferably

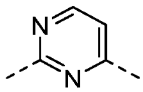 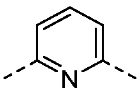 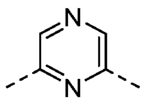 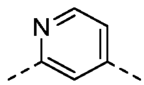 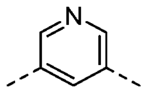 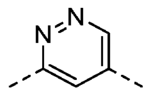

connected to U through the left dashed line, and more preferably

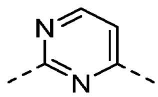

connected to U through the left dashed line.

In a certain embodiment, each "9- to 10-membered heteroaryl" is independently any one of the following fragments:

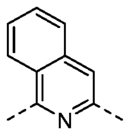 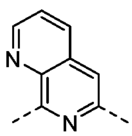 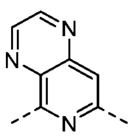 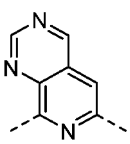 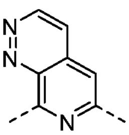 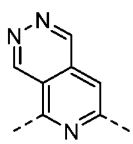 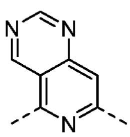 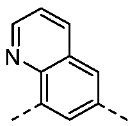

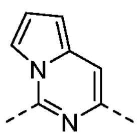 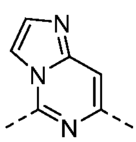 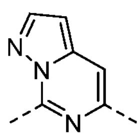 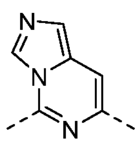 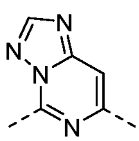 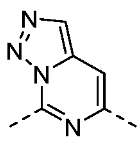 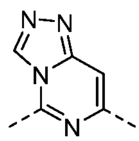

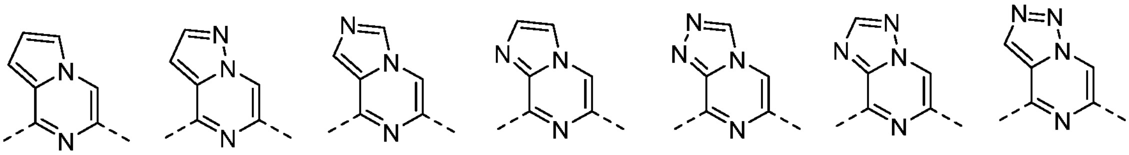

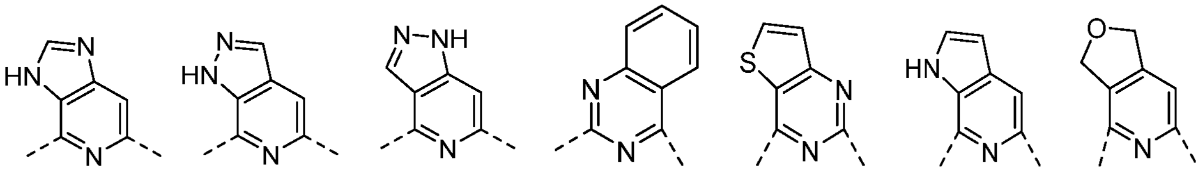

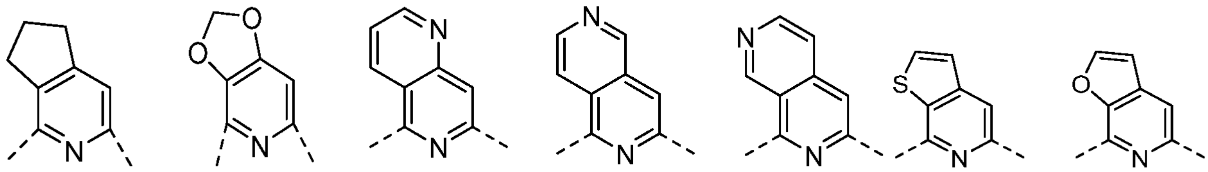

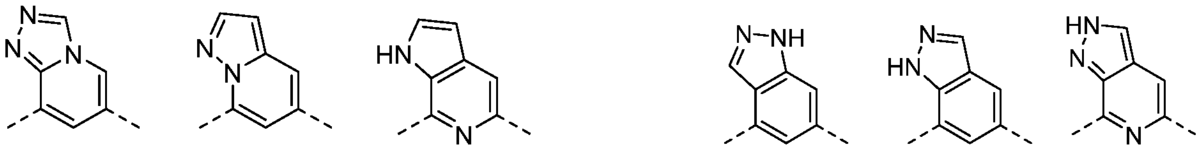

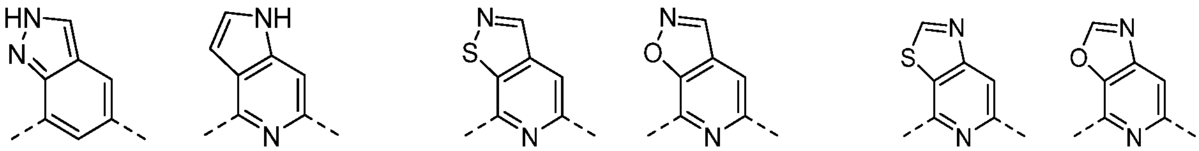

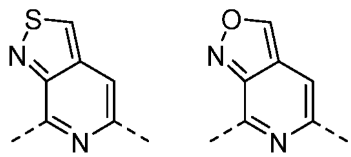 ,

preferably

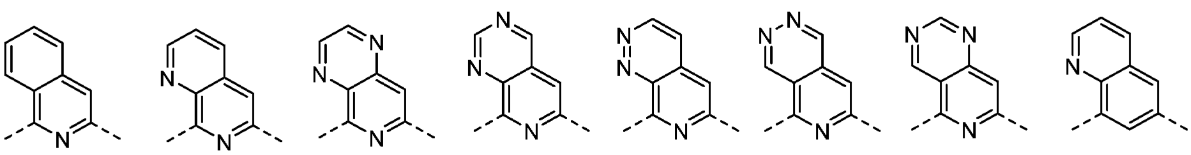

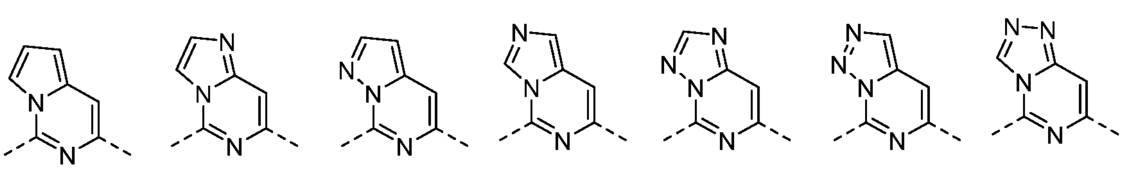

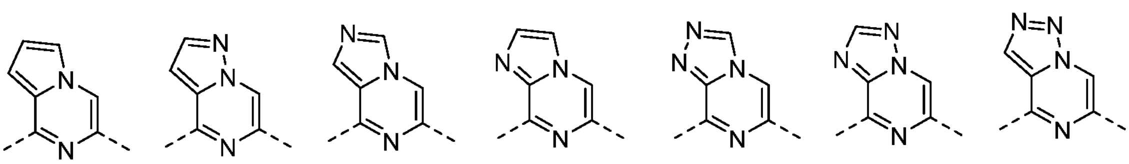

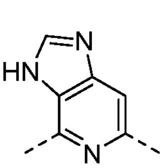 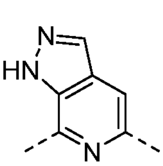 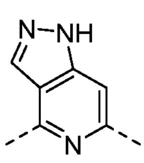 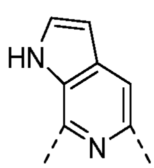 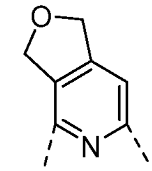 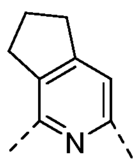 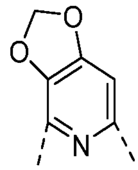

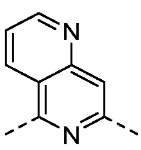 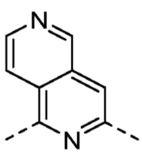 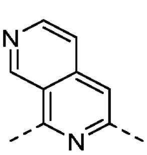

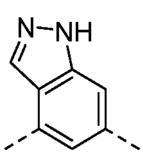 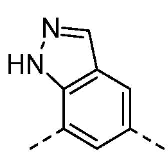 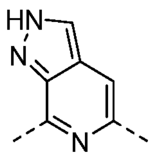 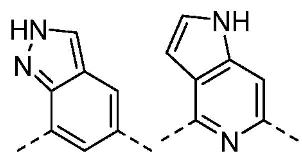 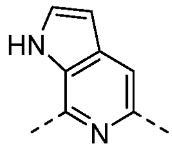

connected to U through the left dashed line, and more preferably

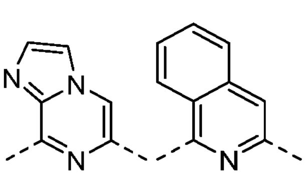 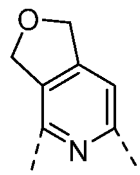

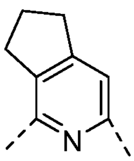 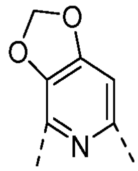 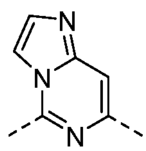 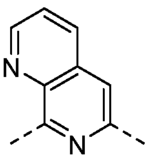 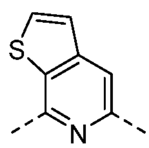

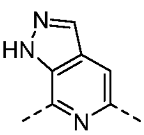 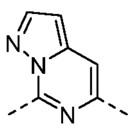 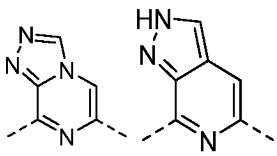 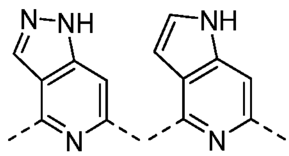 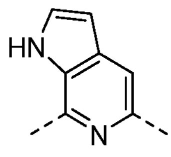

connected to U through the left dashed line.

In a certain embodiment, each "9- to 10-membered heteroaryl" is independently any one of the following fragments:

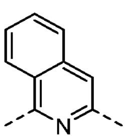 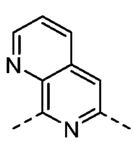 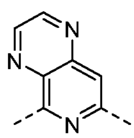 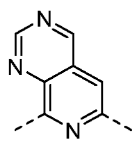 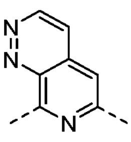 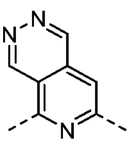 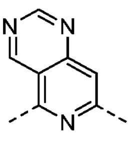 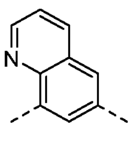

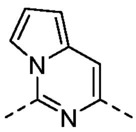 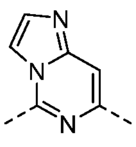 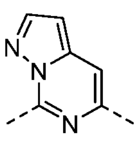 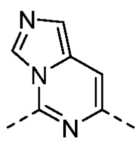 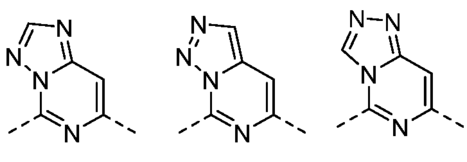

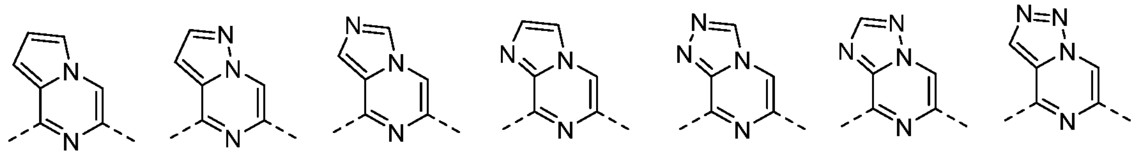

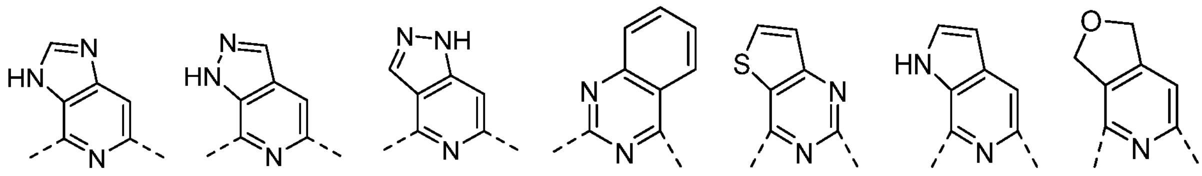

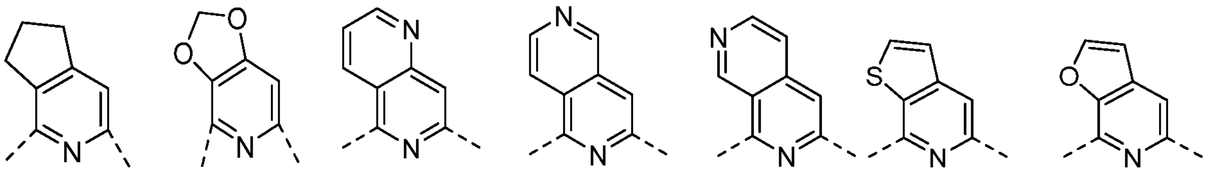

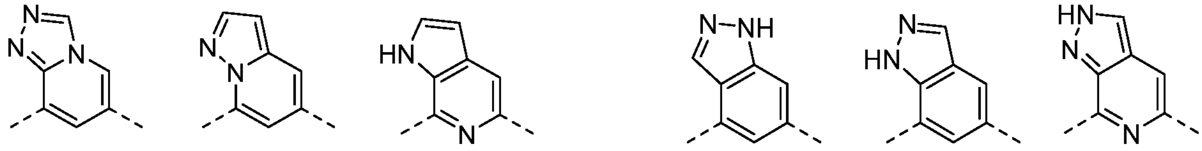

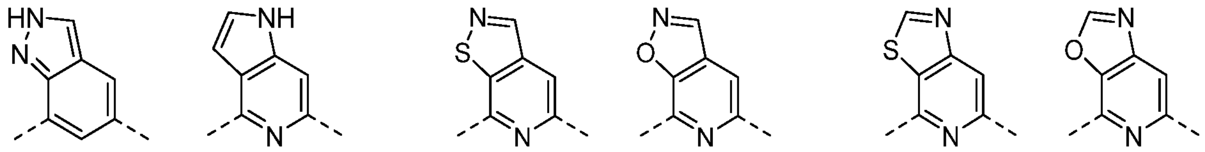

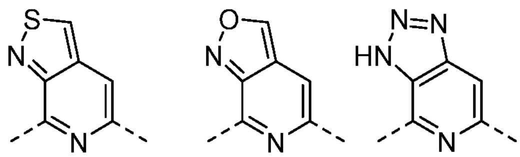,

preferably

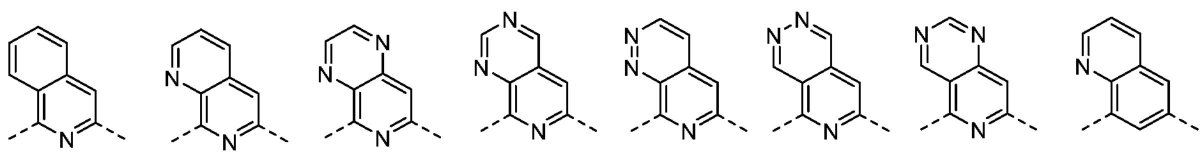

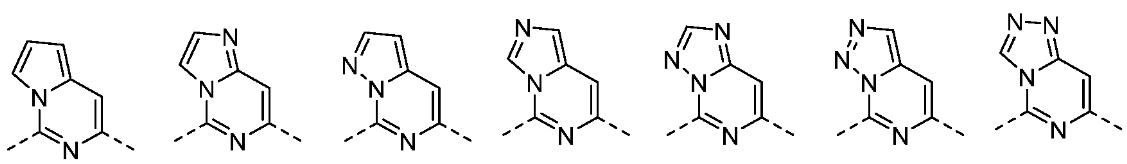

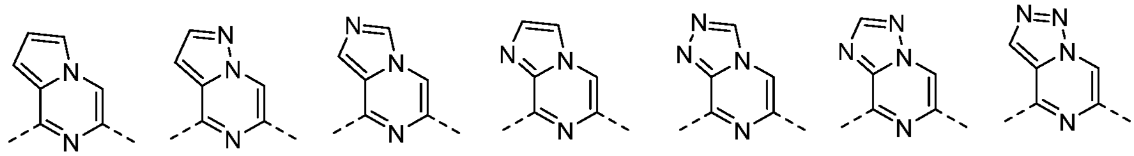

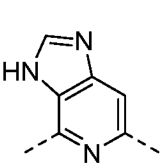 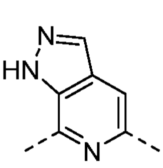 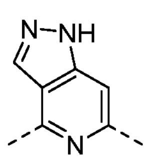 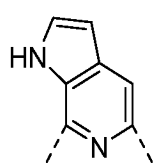 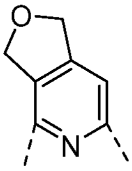 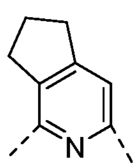 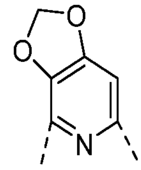

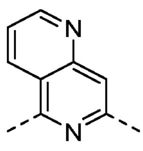 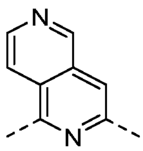 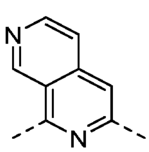

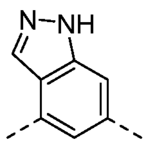 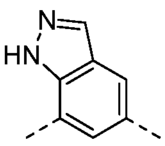 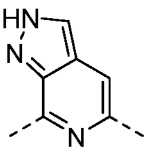 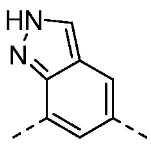 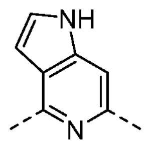 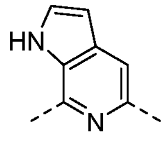

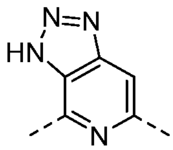

connected to U through the left dashed line, and more preferably

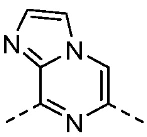

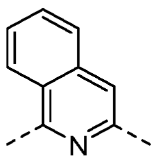 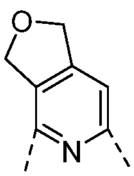 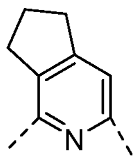 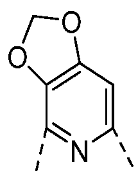 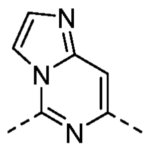 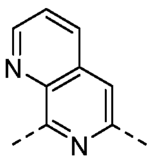 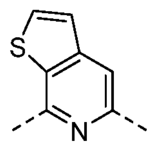

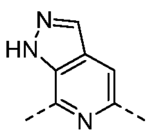 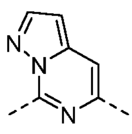 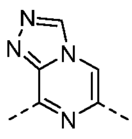 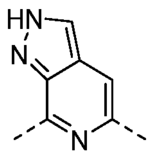 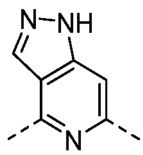 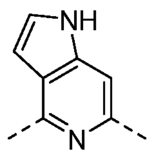 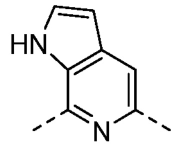

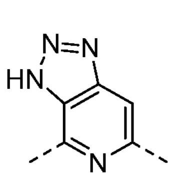 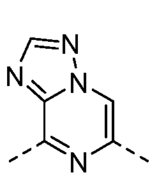

connected to U through the left dashed line.

In a certain embodiment, each "6-membered heterocycloalkenyl" is independently 6-membered heterocycloalkenyl with 1 or 2 heteroatoms of N, preferably any one of the following fragments:

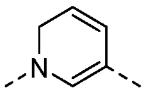 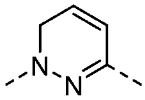 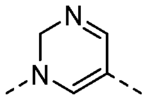 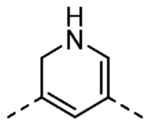 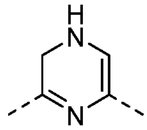 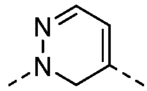 ,

and more preferably

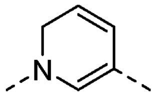 or 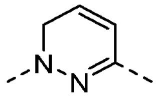

connected to U through the left dashed line.

In a certain embodiment, each "3- to 10-membered cycloalkyl" is independently 3- to 6-membered monocyclic cycloalkyl or 6- to 8-membered spiro cycloalkyl, preferably cyclopropyl, cyclobutyl, cyclopentyl, cyclohexyl,

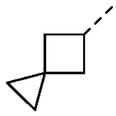 , or 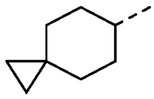 ,

and more preferably

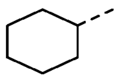 .

In a certain embodiment, each "4- to 10-membered heterocycloalkyl" is independently 4- to 6-membered monocyclic heterocycloalkyl, 6- to 8-membered spiro heterocycloalkyl, or 7- to 9-membered bridged heterocycloalkyl, preferably 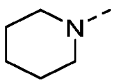 , 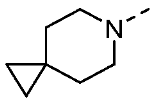 , 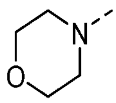 , 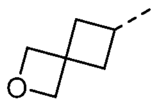 , or 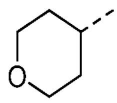 ,

and more preferably

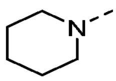 .

In a certain embodiment, each "4- to 10-membered heterocycloalkyl" is independently 4- to 6-membered monocyclic heterocycloalkyl, 6- to 8-membered spiro heterocycloalkyl, or 7- to 9-membered bridged heterocycloalkyl, preferably 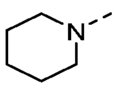 , 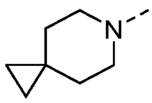 , 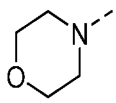 , 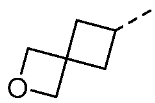 , 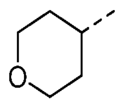 ,

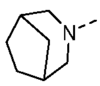 , or 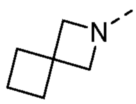 ,

and more preferably

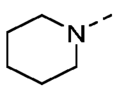 , 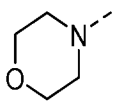 , 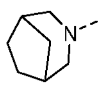 , or 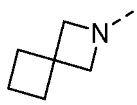 .

In a certain embodiment, the "6- to 10-membered aryl" is phenyl.

In a certain embodiment, the "5- to 10-membered heteroaryl" is 5- to 6-membered heteroaryl with 1 or 2 heteroatoms of N, preferably

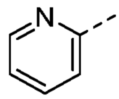
.

In a certain embodiment, when W is *-C($X^1$)$NR^W$-, and $X^1$, $X^2$, and the C atoms therebetween together form 6-membered heteroaryl, in the "6-membered heteroaryl", the heteroatom is N, and the number of the heteroatom is 1 or 2.

In a certain embodiment, $R^x$ is any one of the following fragments:

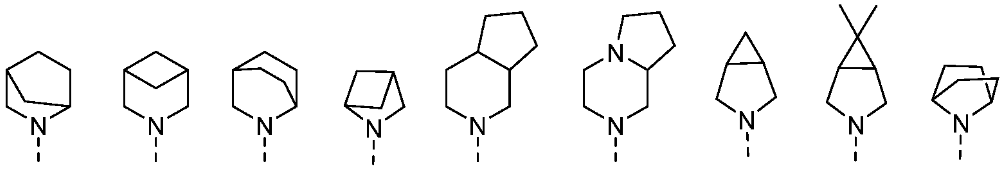

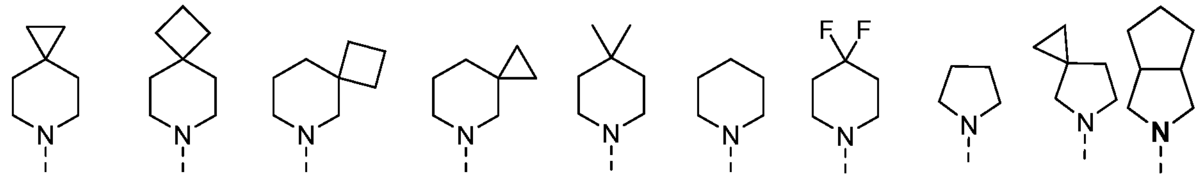

or

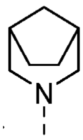
,

preferably

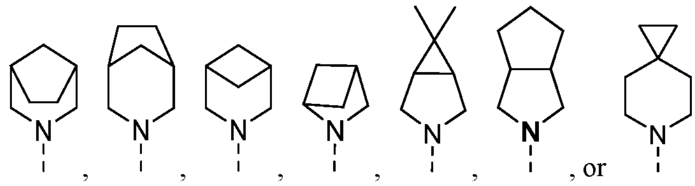
, , , , , , or .

In a certain embodiment, $R^x$ is any one of the following fragments:

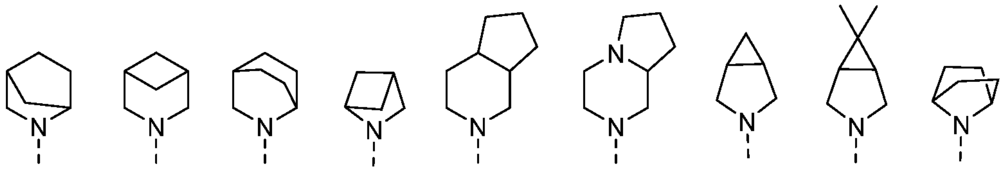

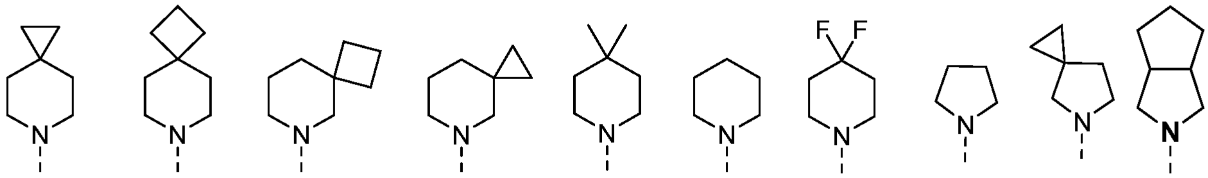

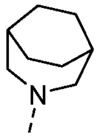 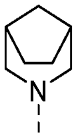 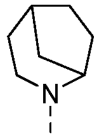 ,

preferably

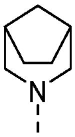 , 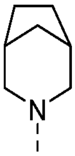 , 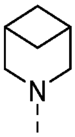 , 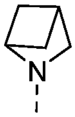 , 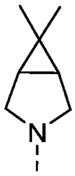 , 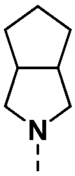 , 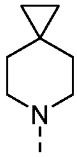 , 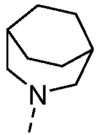 ,

or

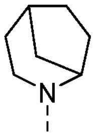 .

In a certain embodiment, $R_1$ is any one of the following fragments:

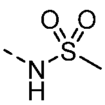 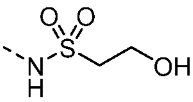 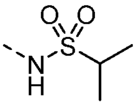 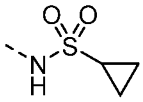 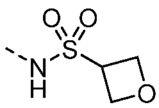 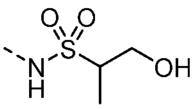

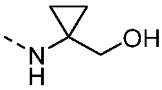 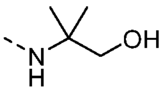 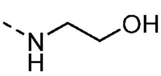 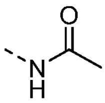 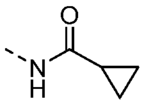 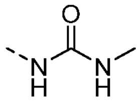

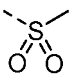 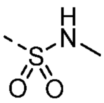 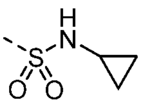 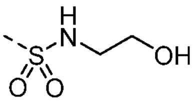 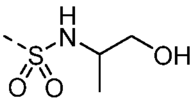 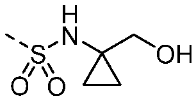

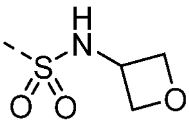 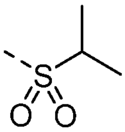 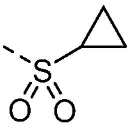 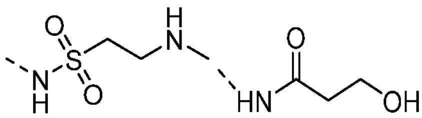 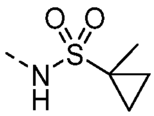

or

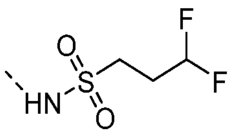 ,

preferably

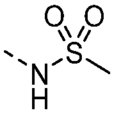, 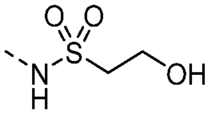, 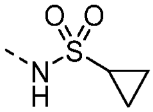, 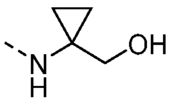,

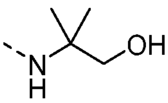, 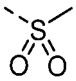, 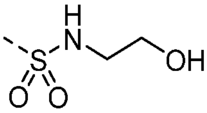, 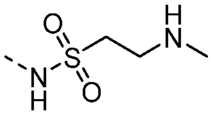, 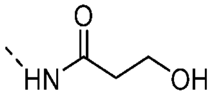, 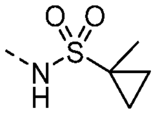,

or

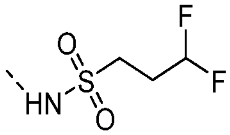.

In a certain embodiment, B is any one of the following fragments (the left dashed line in the following fragments represents the bond connected to U):

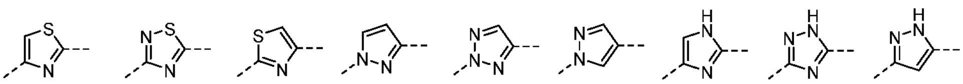

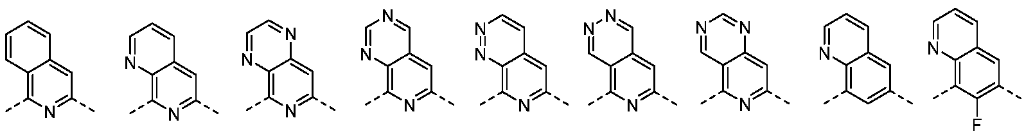

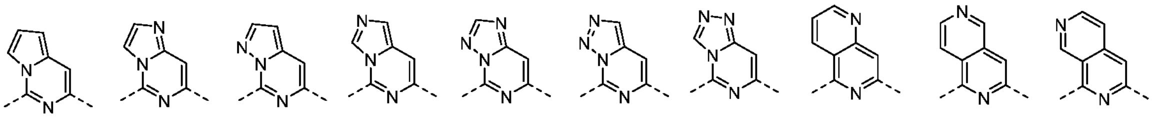

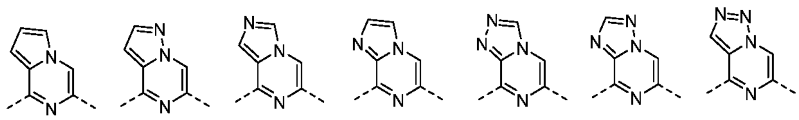

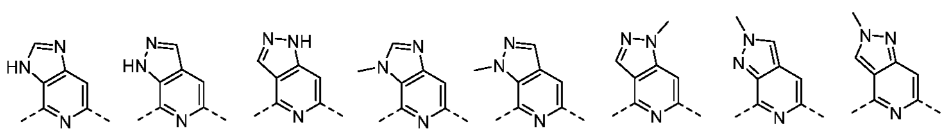

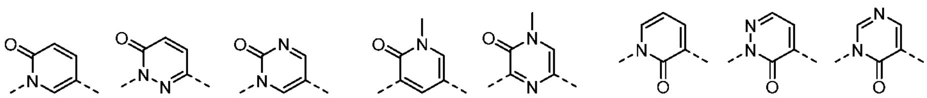

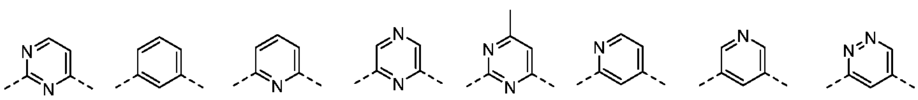

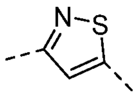 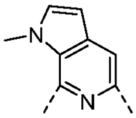 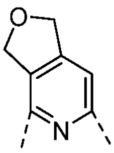 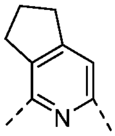 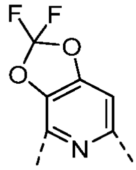 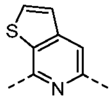 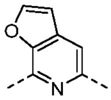

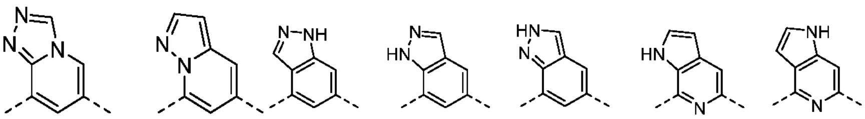,

preferably

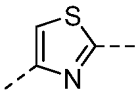,

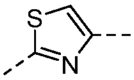, 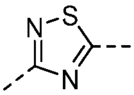, 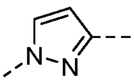, 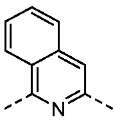, 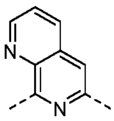, 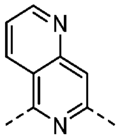, 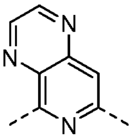, 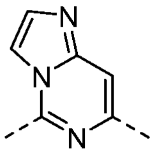,

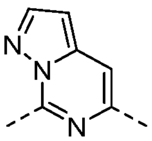, 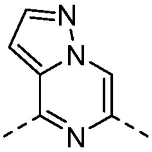, 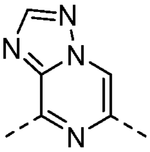, 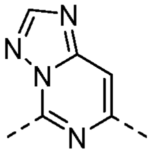, 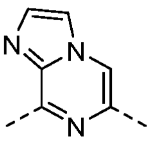, 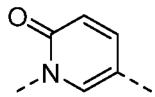,

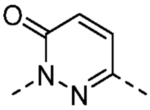, 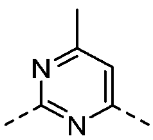, 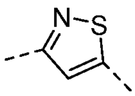, 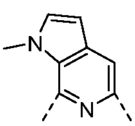, 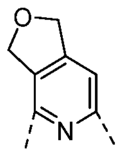, 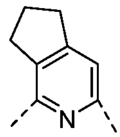, or 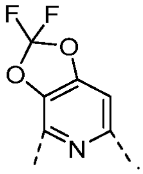.

In a certain embodiment, B is any one of the following fragments (the left dashed line in the following fragments represents the bond connected to U):

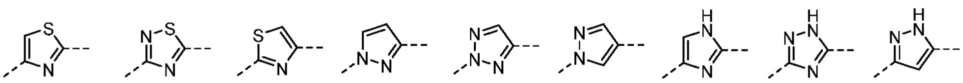

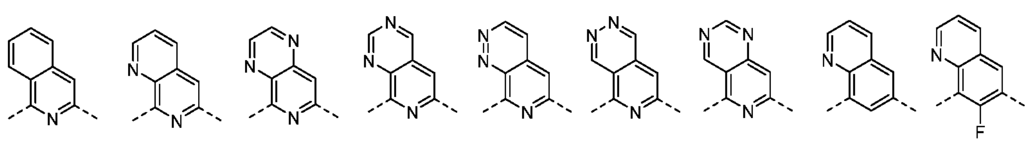

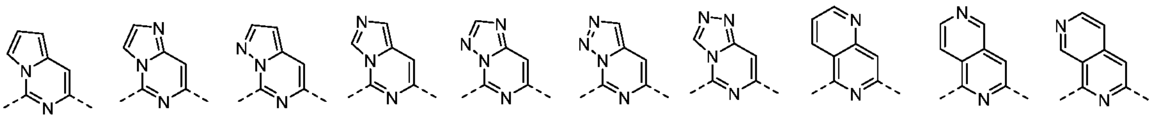

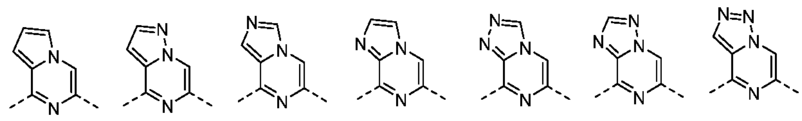

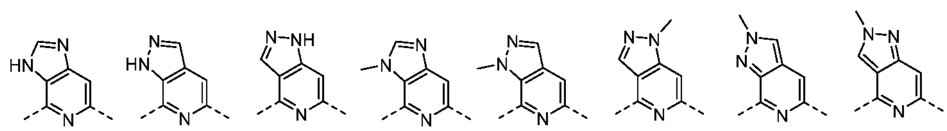

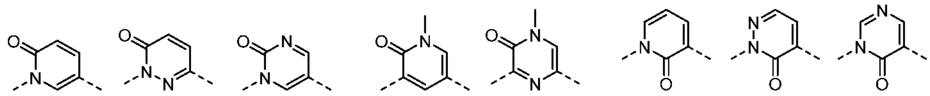

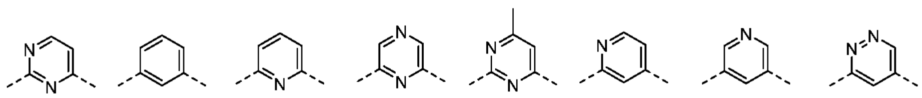

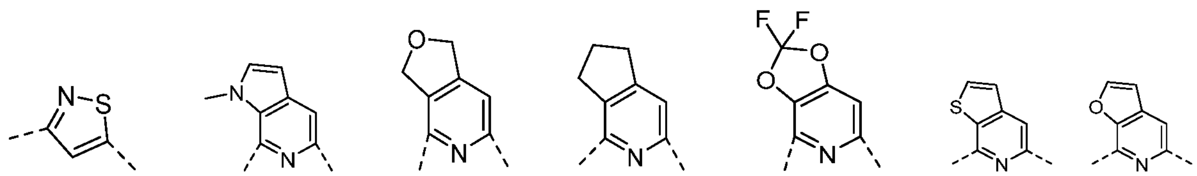

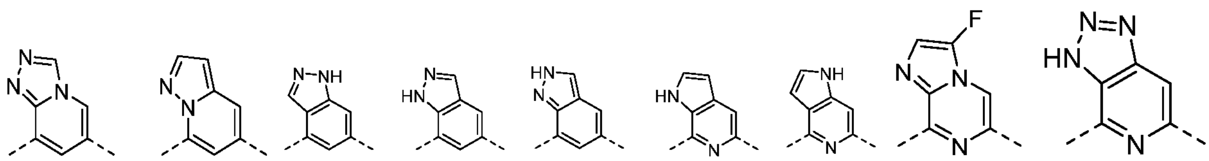

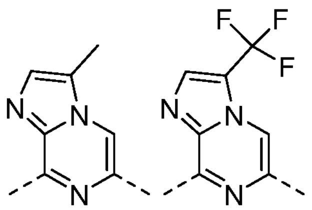 or 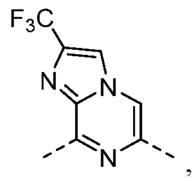,

preferably

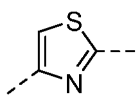, 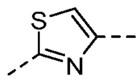, 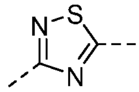,

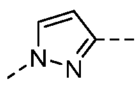, 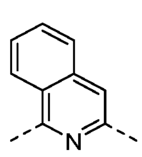, 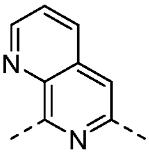, 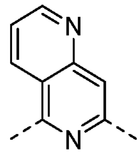, 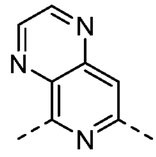, 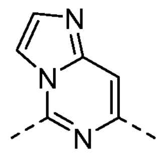, 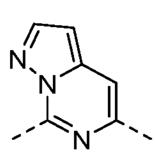,

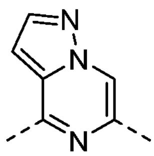, 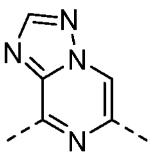, 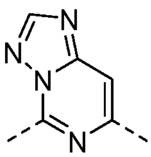, 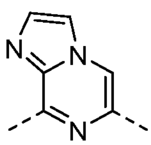, 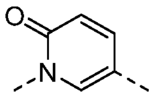, 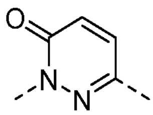,

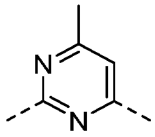, 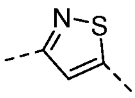, 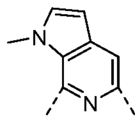, 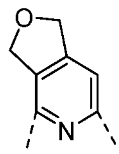, 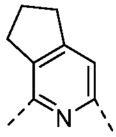, 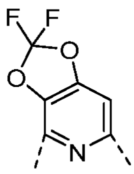, 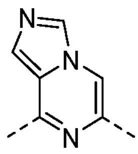,

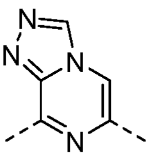, 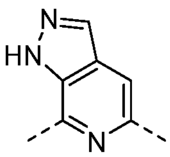, 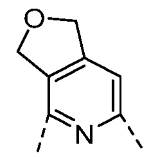, 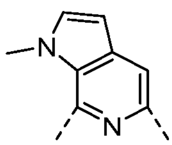, 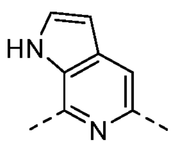, 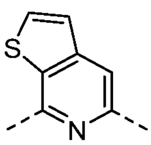,

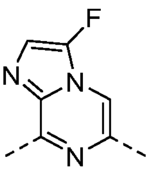, 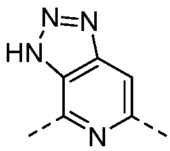, 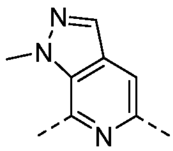, 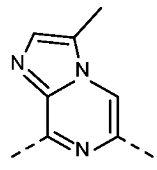, 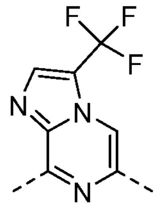, or 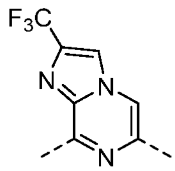.

In a certain embodiment, U is selected from any one of the following fragments:

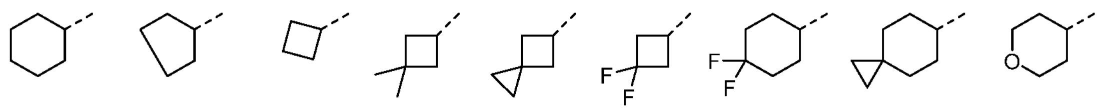

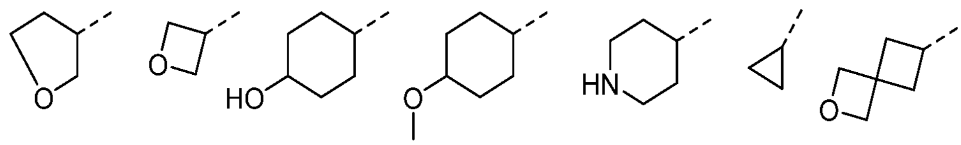

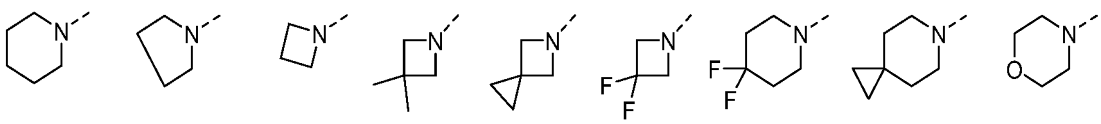

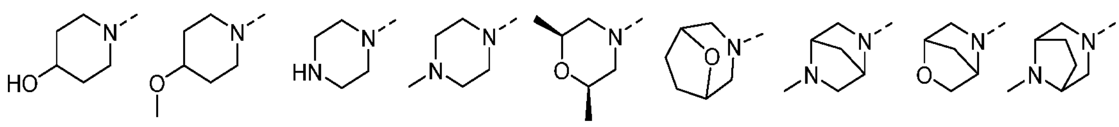

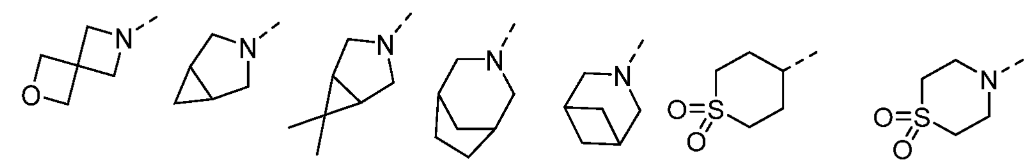

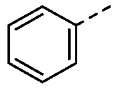 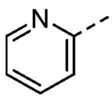 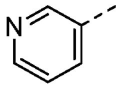 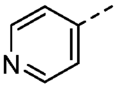 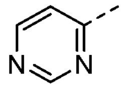 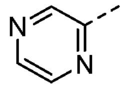 and 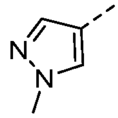,

preferably

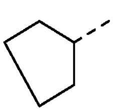, 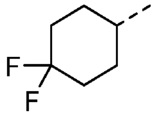, 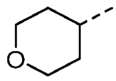, 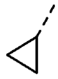, 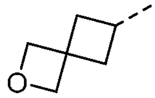, 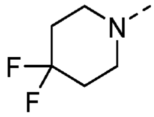, 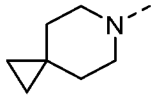, or 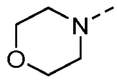.

In a certain embodiment, U is any one of the following fragments:

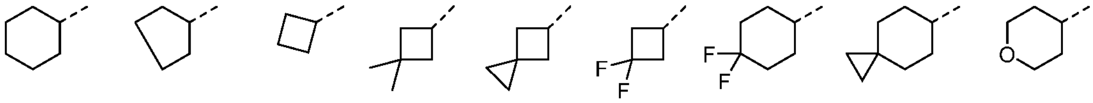

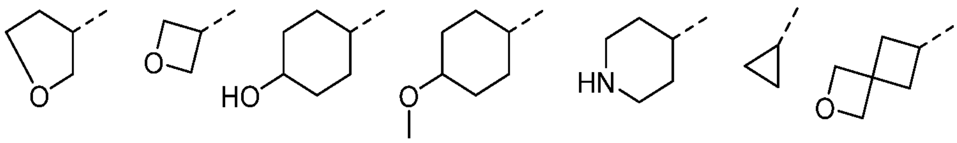

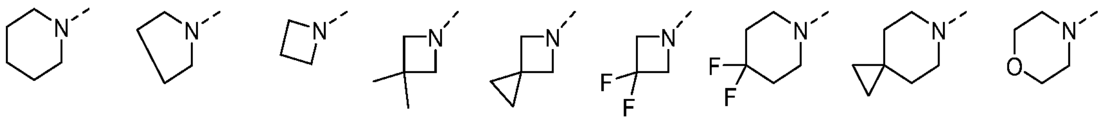

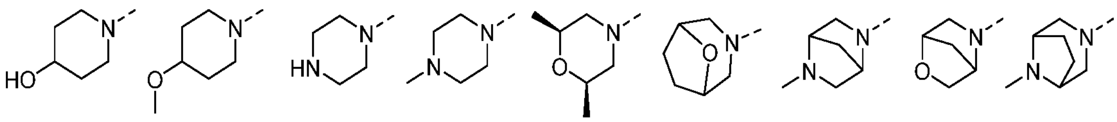

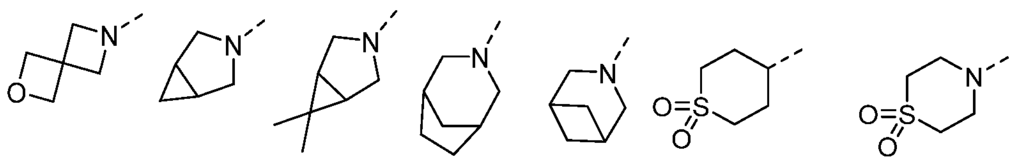

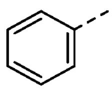 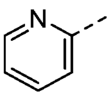 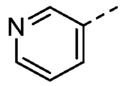 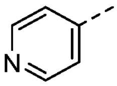 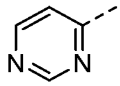 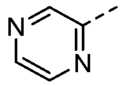 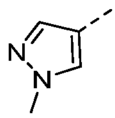 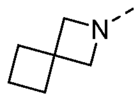 or

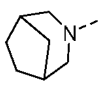,

preferably

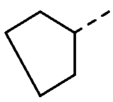, 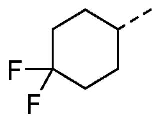, 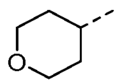, 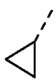, 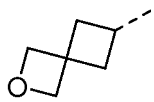, 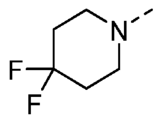,

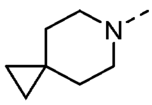 , 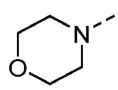 , 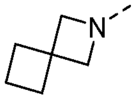 , or 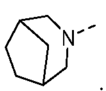 .

The present disclosure further provides any one of the following nitrogen-containing compounds:

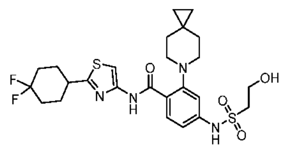 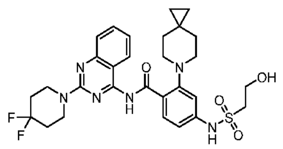 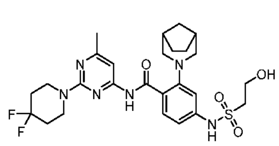 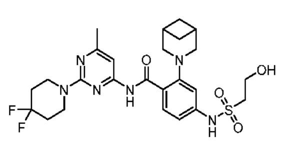

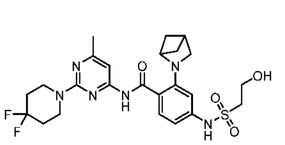 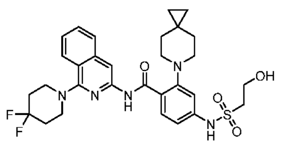 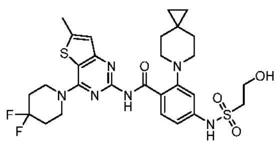 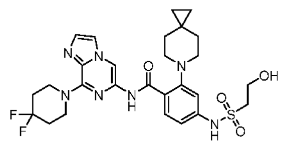

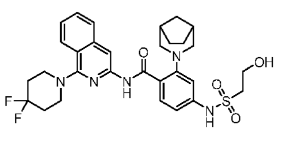 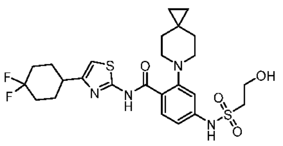 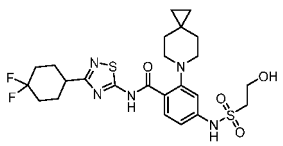 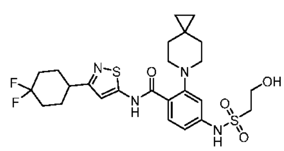

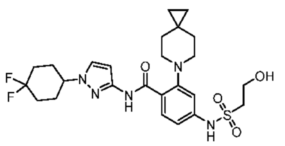 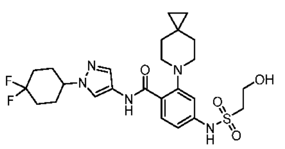 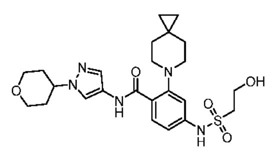 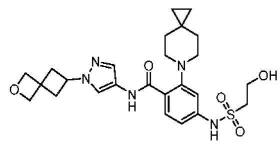

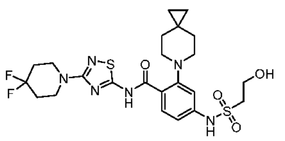 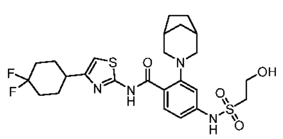 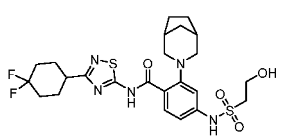 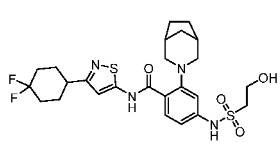

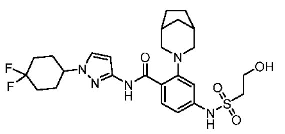 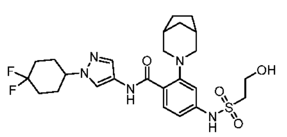 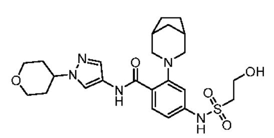 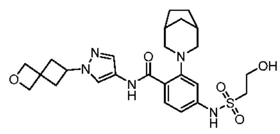

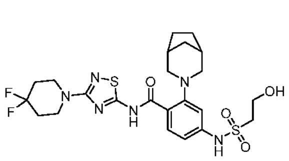 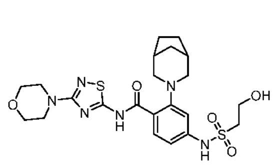 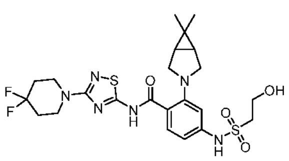 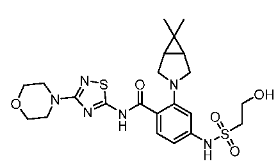

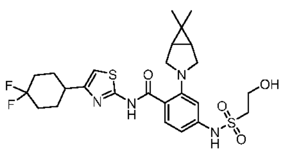 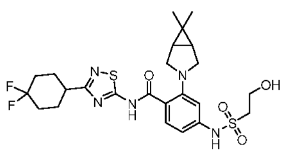 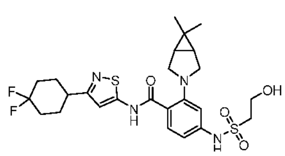

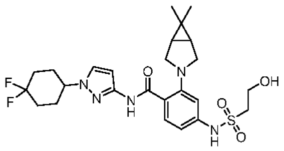
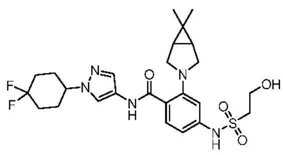
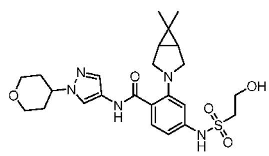
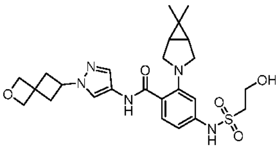

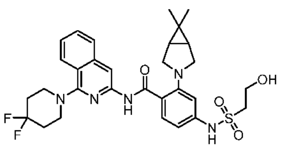
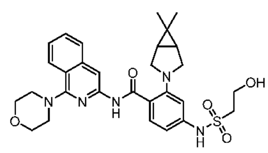
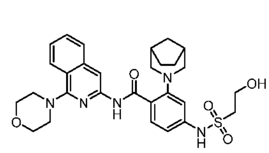

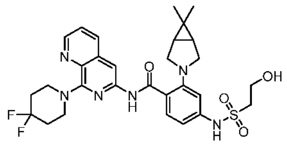
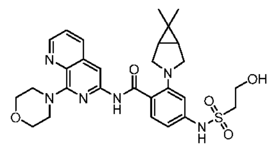
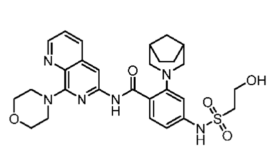
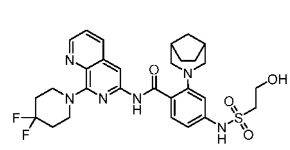

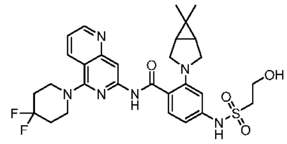
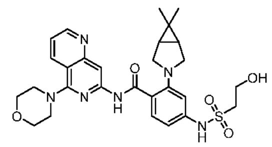
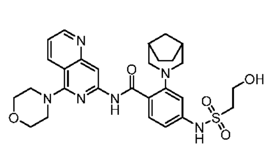
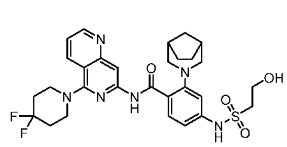

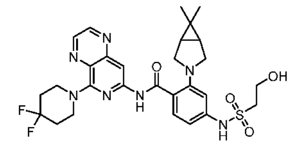
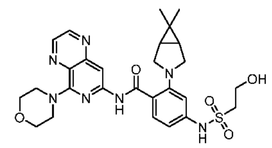
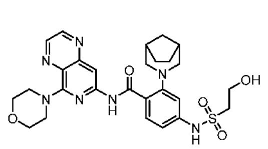
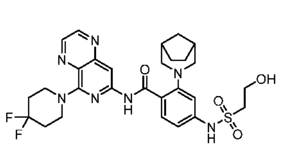

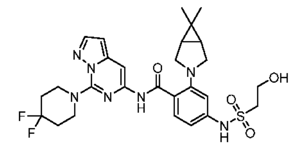
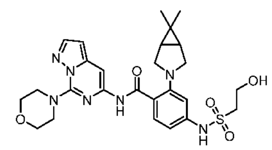
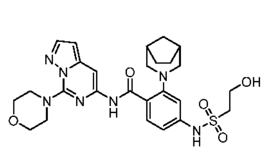
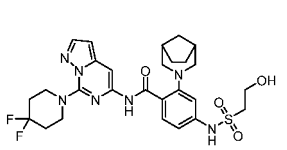

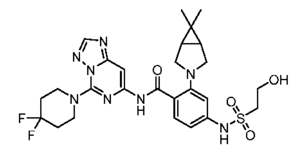
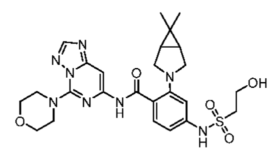
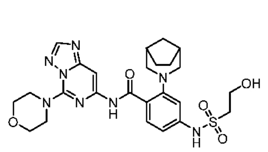
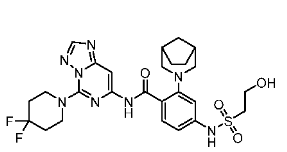

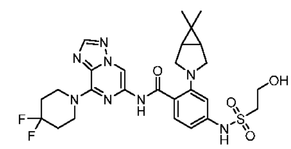
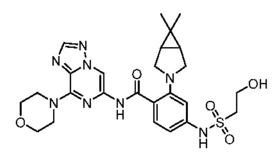
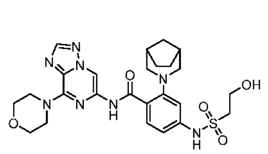
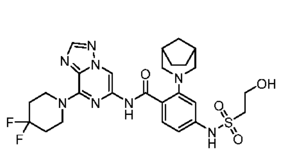

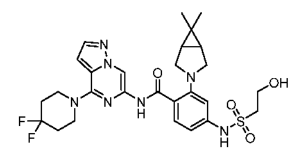
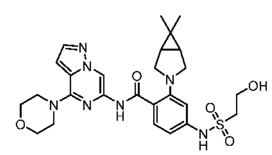
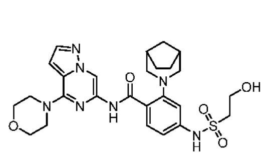
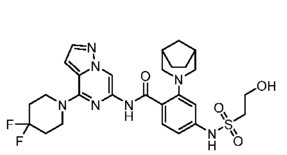

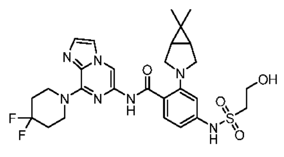 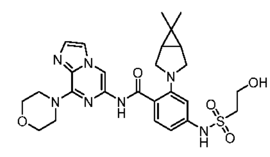 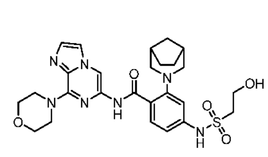 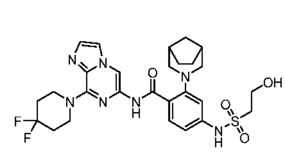

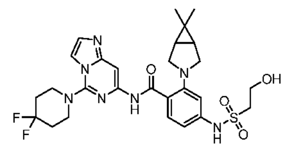 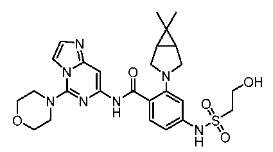 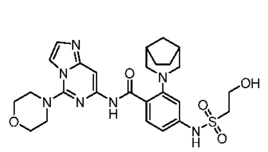 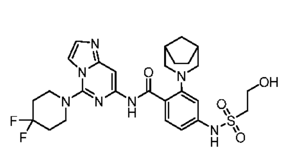

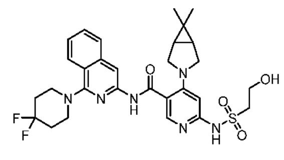 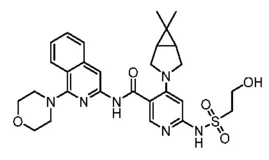 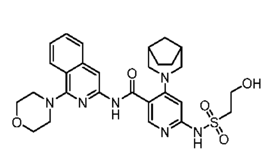 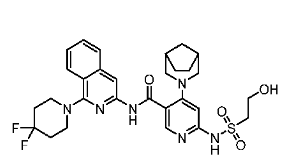

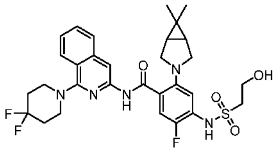 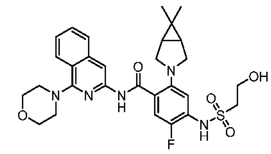 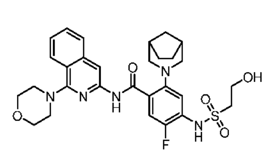 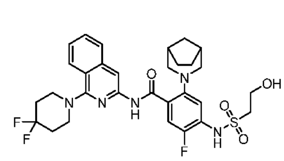

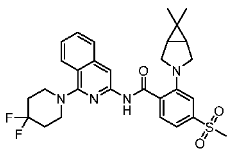 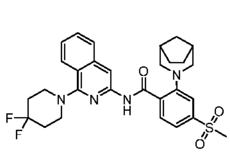 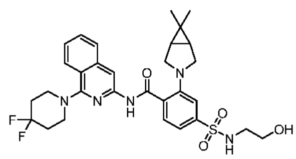 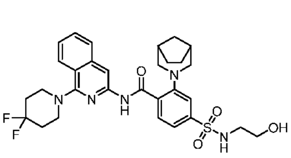

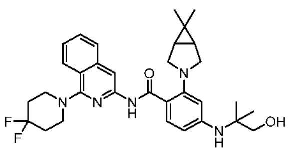 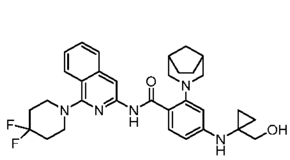 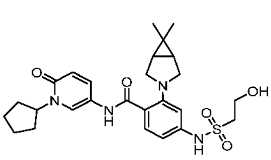 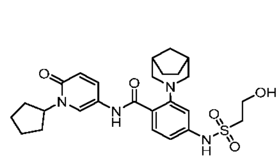

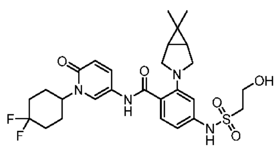 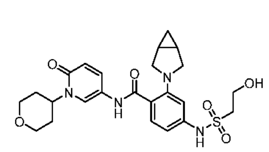 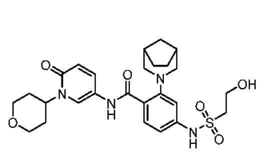 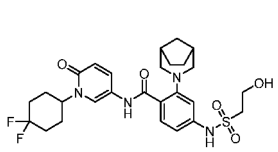

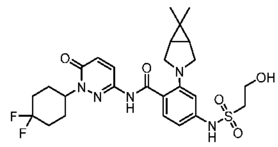 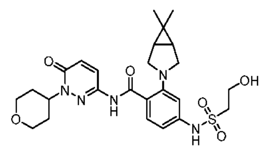 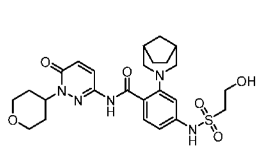 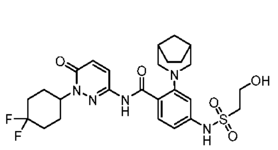

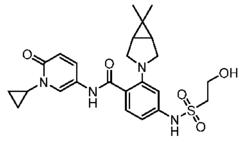 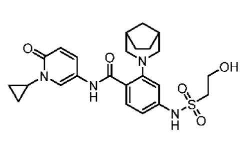

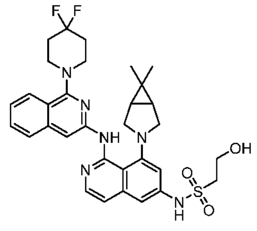
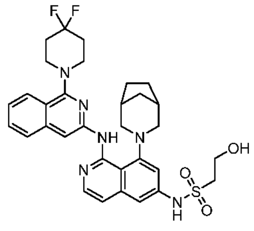
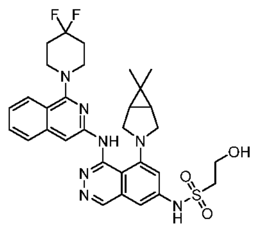
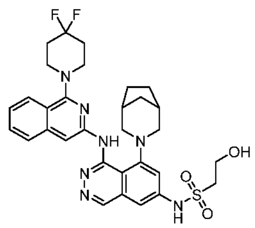

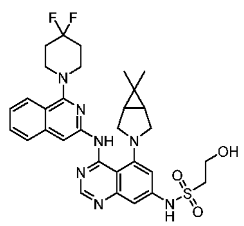
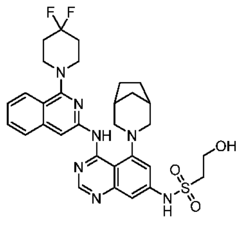

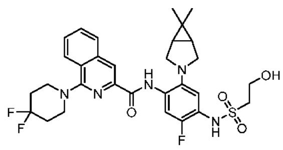
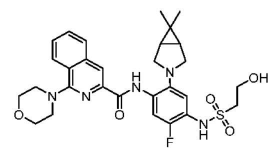
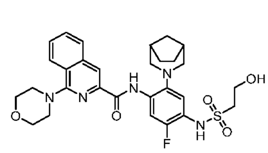
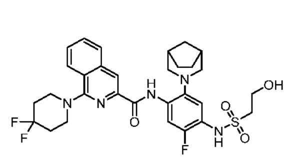

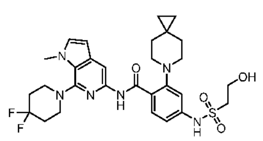
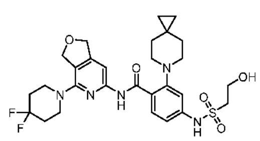
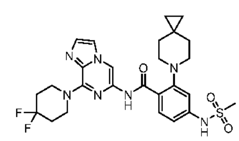
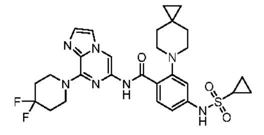

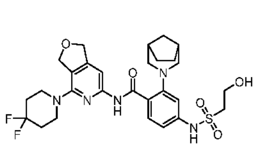
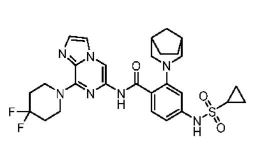
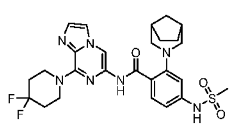
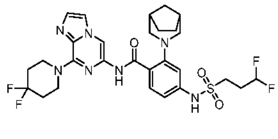

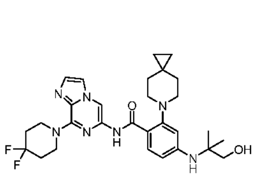
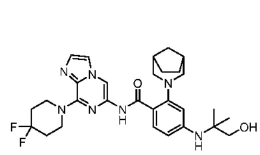
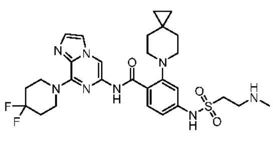
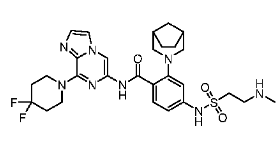

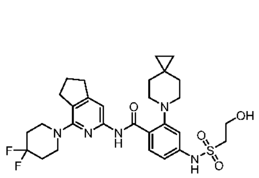
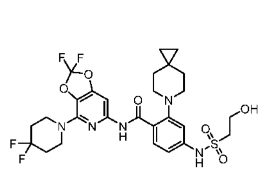
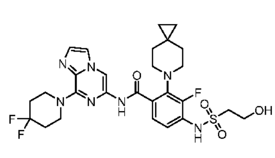
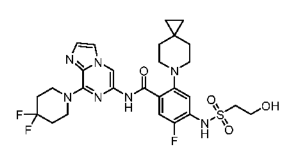

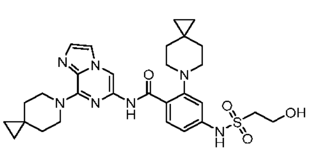
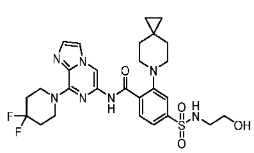
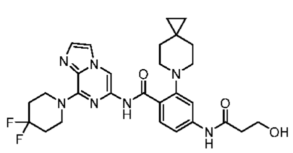
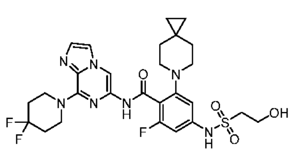

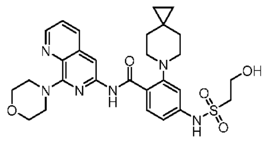 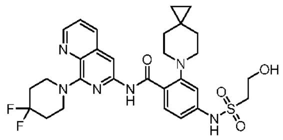 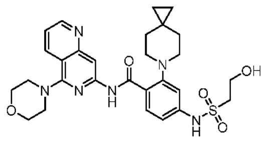 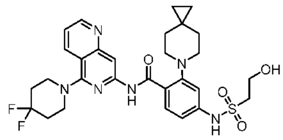

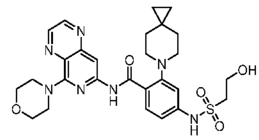 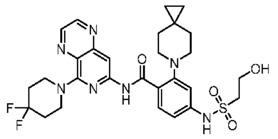 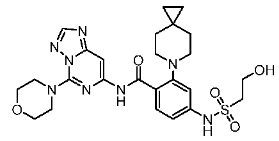 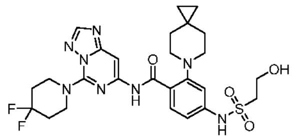

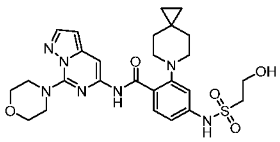 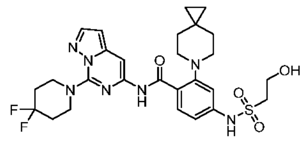 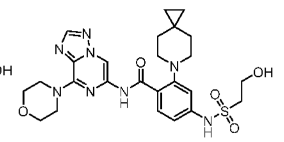 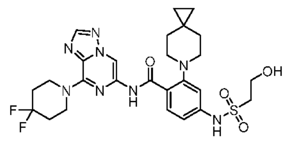

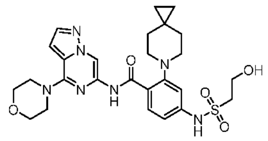 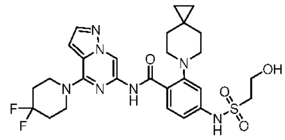 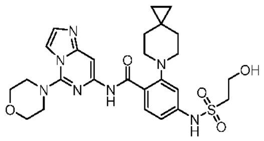 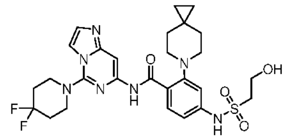

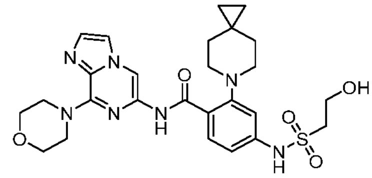 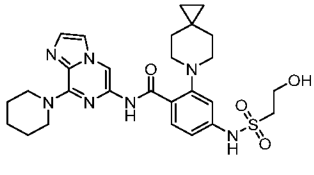 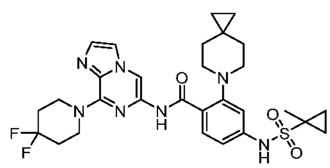

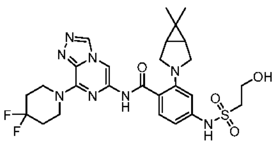 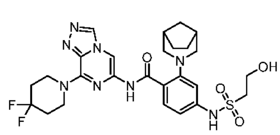 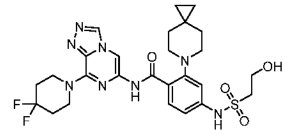

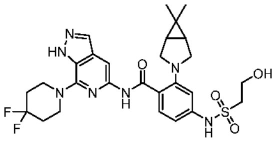 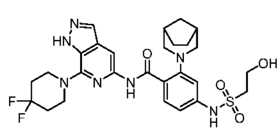 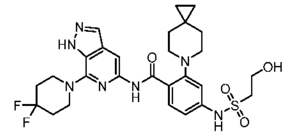

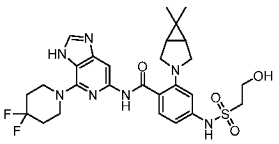 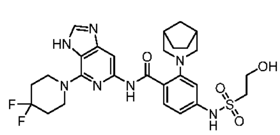 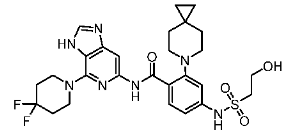

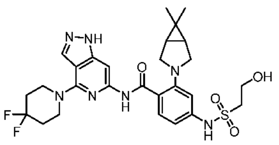 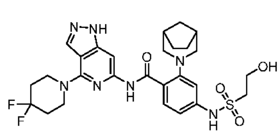 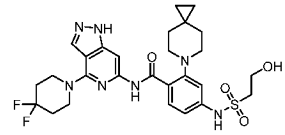

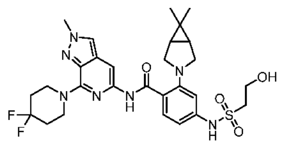
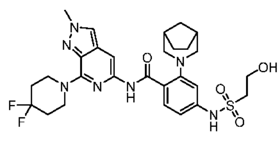
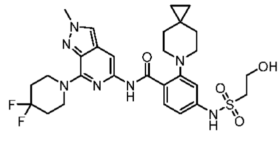

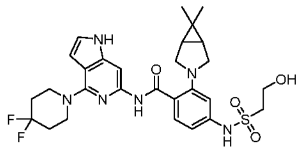
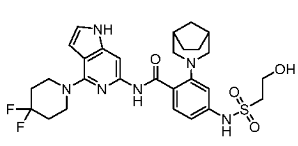
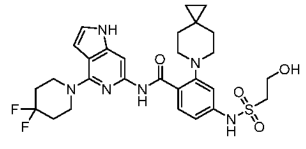

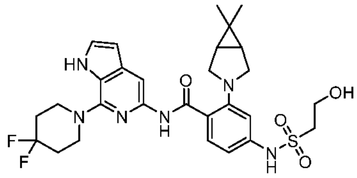
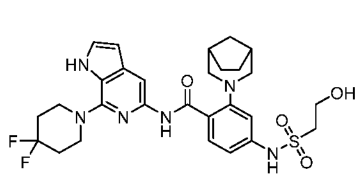
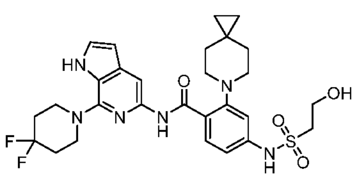

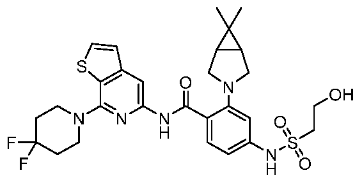
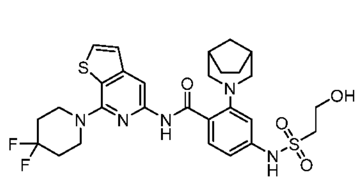
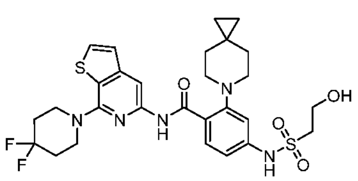

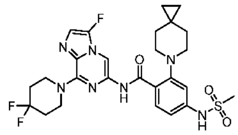
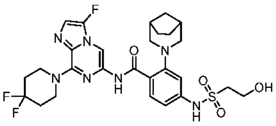
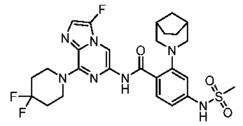
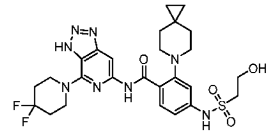

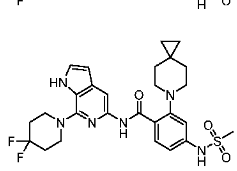
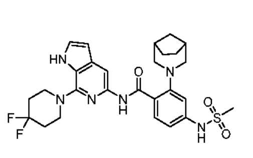
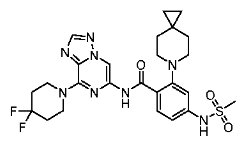
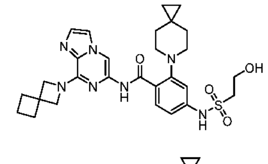

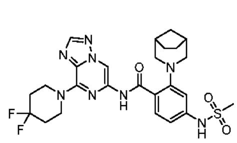
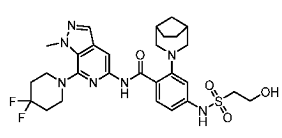
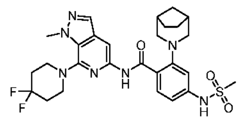
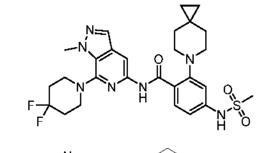

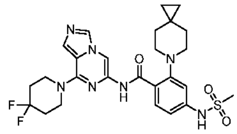
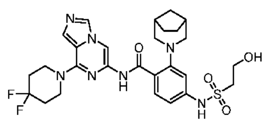
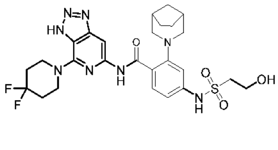

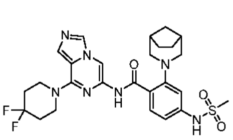
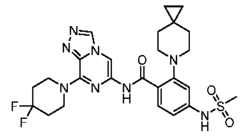
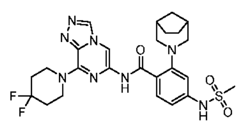
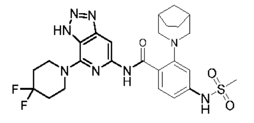
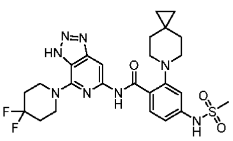
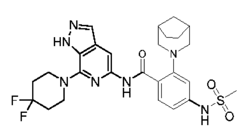
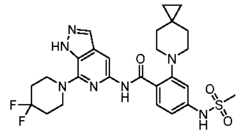
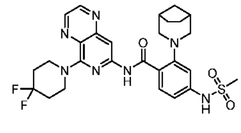
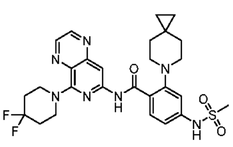
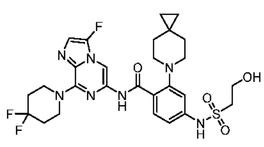
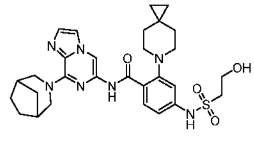
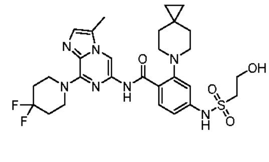
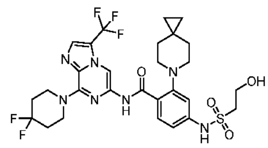
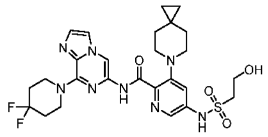
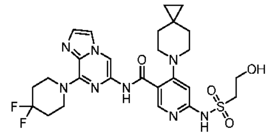
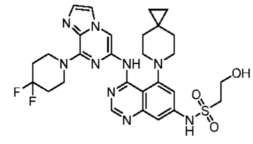
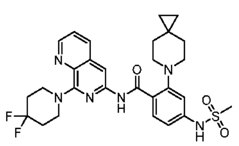
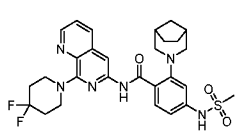
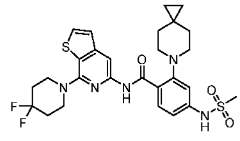
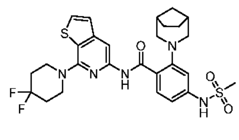
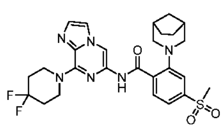
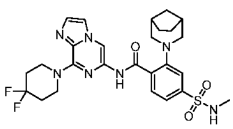
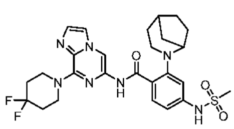
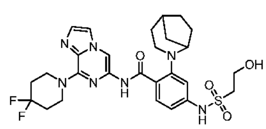
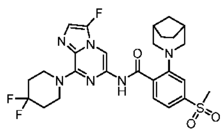
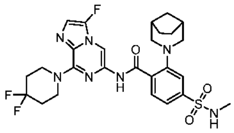
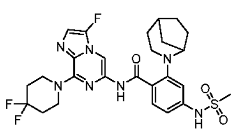
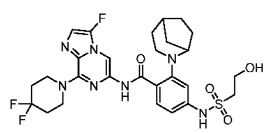
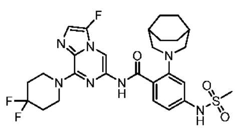
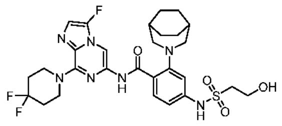
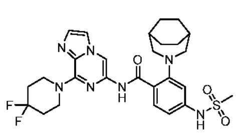
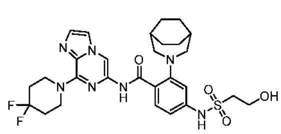
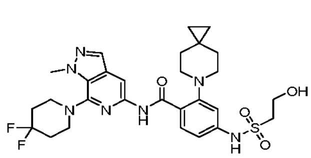
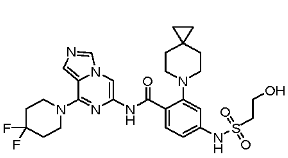
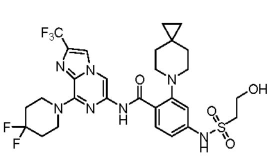
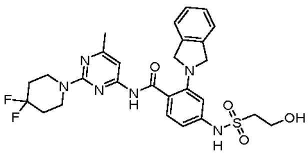
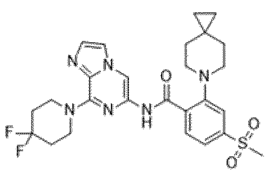
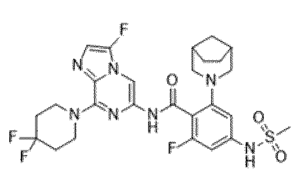
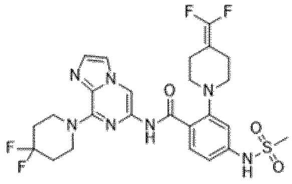

The present disclosure further provides a pharmaceutical composition, comprising:

(1) the nitrogen-containing compound represented by formula I described above, the nitrogen-containing compound represented by formula II described above, the nitrogen-containing compound described above, the pharmaceutically acceptable salt thereof (referring to the pharmaceutically acceptable salt of the nitrogen-containing compound represented by formula I described above, the nitrogen-containing compound represented by formula II described above, or the nitrogen-containing compound described above), the stereoisomer thereof (referring to the stereoisomer of the nitrogen-containing compound represented by formula I described above, the nitrogen-containing compound represented by formula II described above, or the nitrogen-containing compound described above), the tautomer thereof (referring to the tautomer of the nitrogen-containing compound represented by formula I described above, the nitrogen-containing compound represented by formula II described above, or the nitrogen-containing compound described above), the metabolite thereof (referring to the metabolite of the nitrogen-containing compound represented by formula I described above, the nitrogen-containing compound represented by formula II described above, or the nitrogen-containing compound described above), the prodrug thereof (referring to the prodrug of the nitrogen-containing compound represented by formula I described above, the nitrogen-containing compound represented by formula II described above, or the nitrogen-containing compound described above), the isotopically labeled derivative thereof (referring to the isotopically labeled derivative of the nitrogen-containing compound represented by formula I described above, the nitrogen-containing compound represented by formula II described above, or the nitrogen-containing compound described above), the solvate thereof (referring to the solvate of the nitrogen-containing compound represented by formula I described above, the nitrogen-containing compound represented by formula II described above, or the nitrogen-containing compound described above), or the solvate of the pharmaceutically acceptable salt thereof (referring to the solvate of the pharmaceutically acceptable salt of the nitrogen-containing compound represented by formula I described above, the nitrogen-containing compound represented by formula II described above, or the nitrogen-containing compound described above); and
(2) a pharmaceutically acceptable auxiliary material.

The present disclosure further provides use of the nitrogen-containing compound represented by formula I described above, the nitrogen-containing compound represented by formula II described above, the nitrogen-containing compound described above, the pharmaceutically acceptable salt thereof (referring to the pharmaceutically acceptable salt of the nitrogen-containing compound represented by formula I described above, the nitrogen-containing compound represented by formula II described above, or the nitrogen-containing compound described above), the stereoisomer thereof (referring to the stereoisomer of the nitrogen-containing compound represented by formula I described above, the nitrogen-containing compound represented by formula II described above, or the nitrogen-containing compound described above), the tautomer thereof (referring to the tautomer of the nitrogen-containing compound represented by formula I described above, the nitrogen-containing compound represented by formula II described above, or the nitrogen-containing compound described above), the metabolite thereof (referring to the metabolite of the nitrogen-containing compound represented by formula I described above, the nitrogen-containing compound represented by formula II described above, or the nitrogen-containing compound described above), the prodrug thereof (referring to the prodrug of the nitrogen-containing compound represented by formula I described above, the nitrogen-containing compound represented by formula II described above, or the nitrogen-containing compound described above), the isotopically labeled derivative thereof (referring to the isotopically labeled derivative of the nitrogen-containing compound represented by formula I described above, the nitrogen-containing compound represented by formula II described above, or the nitrogen-containing compound described above), the solvate thereof (referring to the solvate of the nitrogen-containing compound represented by formula I described above, the nitrogen-containing compound represented by formula II described above, or the nitrogen-containing compound described above), or the solvate of the pharmaceutically acceptable salt thereof (referring to the solvate of the pharmaceutically acceptable salt of the nitrogen-containing compound represented by formula I described above, the nitrogen-containing compound represented by formula II described above, or the nitrogen-containing compound described above) in the preparation of a KIF18A inhibitor.

The present disclosure further provides use of the nitrogen-containing compound represented by formula I described above, the nitrogen-containing compound represented by formula II described above, the nitrogen-containing compound described above, the pharmaceutically acceptable salt thereof (referring to the pharmaceutically acceptable salt of the nitrogen-containing compound represented by formula I described above, the nitrogen-containing compound represented by formula II described above, or the nitrogen-containing compound described above), the stereoisomer thereof (referring to the stereoisomer of the nitrogen-containing compound represented by formula I described above, the nitrogen-containing compound represented by formula II described above, or the nitrogen-containing compound described above), the tautomer thereof (referring to the tautomer of the nitrogen-containing compound represented by formula I described above, the nitrogen-containing compound represented by formula II described above, or the nitrogen-containing compound described above), the metabolite thereof (referring to the metabolite of the nitrogen-containing compound represented by formula I described above, the nitrogen-containing compound represented by formula II described above, or the nitrogen-containing compound described above), the prodrug thereof (referring to the prodrug of

the nitrogen-containing compound represented by formula I described above, the nitrogen-containing compound represented by formula II described above, or the nitrogen-containing compound described above), the isotopically labeled derivative thereof (referring to the isotopically labeled derivative of the nitrogen-containing compound represented by formula I described above, the nitrogen-containing compound represented by formula II described above, or the nitrogen-containing compound described above), the solvate thereof (referring to the solvate of the nitrogen-containing compound represented by formula I described above, the nitrogen-containing compound represented by formula II described above, or the nitrogen-containing compound described above), or the solvate of the pharmaceutically acceptable salt thereof (referring to the solvate of the pharmaceutically acceptable salt of the nitrogen-containing compound represented by formula I described above, the nitrogen-containing compound represented by formula II described above, or the nitrogen-containing compound described above) in the preparation of a medicament, wherein the medicament is used for treating a disease related to the mechanism of action of a KIF18A protein, for example, a cancer, and for another example, a cancer related to the mechanism of action of the KIF18A protein.

In a certain embodiment, the cancer is ovarian cancer, colon cancer, or ductal breast cancer.

The present disclosure further provides use of the nitrogen-containing compound represented by formula I described above, the nitrogen-containing compound represented by formula II described above, the nitrogen-containing compound described above, the pharmaceutically acceptable salt thereof (referring to the pharmaceutically acceptable salt of the nitrogen-containing compound represented by formula I described above, the nitrogen-containing compound represented by formula II described above, or the nitrogen-containing compound described above), the stereoisomer thereof (referring to the stereoisomer of the nitrogen-containing compound represented by formula I described above, the nitrogen-containing compound represented by formula II described above, or the nitrogen-containing compound described above), the tautomer thereof (referring to the tautomer of the nitrogen-containing compound represented by formula I described above, the nitrogen-containing compound represented by formula II described above, or the nitrogen-containing compound described above), the metabolite thereof (referring to the metabolite of the nitrogen-containing compound represented by formula I described above, the nitrogen-containing compound represented by formula II described above, or the nitrogen-containing compound described above), the prodrug thereof (referring to the prodrug of the nitrogen-containing compound represented by formula I described above, the nitrogen-containing compound represented by formula II described above, or the nitrogen-containing compound described above), the isotopically labeled derivative thereof (referring to the isotopically labeled derivative of the nitrogen-containing compound represented by formula I described above, the nitrogen-containing compound represented by formula II described above, or the nitrogen-containing compound described above), the solvate thereof (referring to the solvate of the nitrogen-containing compound represented by formula I described above, the nitrogen-containing compound represented by formula II described above, or the nitrogen-containing compound described above), or the solvate of the pharmaceutically acceptable salt thereof (referring to the solvate of the pharmaceutically acceptable salt of the nitrogen-containing compound represented by formula I described above, the nitrogen-containing compound represented by formula II described above, or the nitrogen-containing compound described above) in the preparation of a medicament, wherein the medicament is used for treating a cancer.

In a certain embodiment, the cancer is ovarian cancer, colon cancer, or ductal breast cancer.

Terminology

In the present disclosure, the term "pharmaceutically acceptable salt" refers to a salt obtained from the reaction of the compound with a pharmaceutically acceptable acid or base. When the compound contains a relatively acidic functional group, a base addition salt can be obtained by contacting the compound with a sufficient amount of a pharmaceutically acceptable base in a suitable inert solvent. When the compound contains a relatively basic functional group, an acid addition salt can be obtained by contacting the compound with a sufficient amount of a pharmaceutically acceptable acid in a suitable inert solvent. For more details, see Handbook of Pharmaceutical Salts: Properties, Selection, and Use (P. Heinrich Stahl, Camille G. Wermuth, 2011, 2nd Revised Edition).

In the present disclosure,

“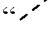”

in a structural fragment means that the structural fragment is connected to the rest of the molecule by this bond. For example,

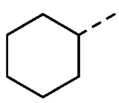

refers to cyclohexyl.

In the present disclosure, the term "one or more" refers to 1, 2, 3, 4, 5, 6, 7, 8, 9, 10, 11, 12, 13, 14, 15, 16, 17, or 18.

In the present disclosure, the term "saturated" refers to a group that is fully saturated with hydrogen.

In the present disclosure, the term "partially saturated" refers to a group that is partially saturated with hydrogen.

In the present disclosure, the term "unsaturated" refers to an aromatic group.

In the present disclosure, the term "halogen" refers to fluorine, chlorine, bromine, or iodine.

In the present disclosure, the term "alkyl" refers to a linear or branched, saturated, monovalent hydrocarbon group with a specified number of carbon atoms (e.g., $C_1$-$C_6$). The alkyl includes, but is not limited to, methyl, ethyl, *n*-propyl, isopropyl, *n*-butyl, isobutyl, *sec*-butyl, *tert*-butyl, *n*-pentyl, *n*-hexyl, and the like.

In the present disclosure, the term "alkoxy" refers to the group $R^Y$-O-, where $R^Y$ is as defined for the term "alkyl". The alkoxy includes, but is not limited to: methoxy, ethoxy, n-propoxy, isopropoxy, and the like.

In the present disclosure, the term "cycloalkyl" is monocyclic cycloalkyl, bridged cycloalkyl, or spiro cycloalkyl.

In the present disclosure, the term "monocyclic cycloalkyl" refers to a cyclic, saturated, monovalent hydrocarbon group with a specified number of carbon atoms (e.g., $C_3$-$C_6$), which is monocyclic. The monocyclic cycloalkyl includes, but is not limited to:

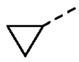 , 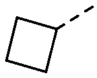 , 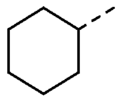 ,

and the like.

In the present disclosure, the term "carbocycle" refers to a cyclic hydrocarbon group with a specified number of carbon atoms (e.g., $C_5$-$C_6$) that shares two atoms and one bond with the rest of the molecule, such as

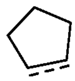

(where "- - -" represents the bond connected to the rest of the molecule).

In the present disclosure, the term "heterocycle" refers to a cyclic group with a specified number of ring atoms (e.g., 5-6 members), a specified number of heteroatoms (e.g., 1, 2, or 3) and a specified heteroatom type (one or more of N, O, and S) that shares two atoms and one bond with the rest of the molecule, such as

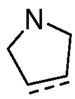

(where "- - -" represents the bond connected to the rest of the molecule).

In the present disclosure, the term "bridged cycloalkyl" refers to a cyclic, saturated, monovalent hydrocarbon group with a specified number of carbon atoms (e.g., $C_4$-$C_{10}$), which is polycyclic (e.g., bicyclic or tricyclic) with individual rings sharing two or more carbon atoms. The bridged cycloalkyl includes, but is not limited to:

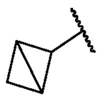 , 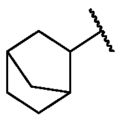 ,

and the like.

In the present disclosure, the term "spiro cycloalkyl" refers to a cyclic, saturated, monovalent hydrocarbon group with a specified number of carbon atoms (e.g., $C_5$-$C_9$), which is polycyclic (e.g., bicyclic or tricyclic) with individual rings sharing one carbon atom. The spiro cycloalkyl includes, but is not limited to:

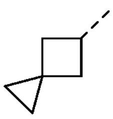 , 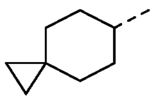 ,

and the like.

In the present disclosure, the term "heterocycloalkenyl" refers to a cyclic, partially unsaturated hydrocarbon group with a specified number of ring atoms (e.g., 5-10 members), a specified number of heteroatoms (e.g., 1, 2, or 3) and a specified heteroatom type (one or more of N, O, and S), which has one or more (e.g., 1, 2, or 3) carbon-carbon $sp^2$ double bonds and is monocyclic and non-aromatic. The heterocycloalkenyl is connected to the rest of the molecule by a carbon atom or a heteroatom. The heterocycloalkenyl includes, but is not limited to:

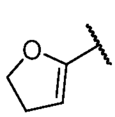,
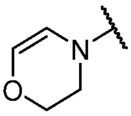,

and the like.

In the present disclosure, the term "cycloalkenyl" refers to a cyclic, partially unsaturated hydrocarbon group with a specified number of carbon atoms (e.g., 5-6 members), which has one or more (e.g., 1, 2, or 3) carbon-carbon $sp^2$ double bonds and is monocyclic and non-aromatic. The cycloalkenyl is connected to the rest of the molecule by a carbon atom.

In the present disclosure, the term "heterocycloalkyl" is monocyclic heterocycloalkyl, bridged heterocycloalkyl, or spiro heterocycloalkyl.

In the present disclosure, the term "monocyclic heterocycloalkyl" refers to a cyclic, saturated, monovalent group with a specified number of ring atoms (e.g., 3-10 members), a specified number of heteroatoms (e.g., 1, 2, or 3) and a specified heteroatom type (one or more of N, O, and S), which is monocyclic. The monocyclic heterocycloalkyl is connected to the rest of the molecule by a carbon atom or a heteroatom. The monocyclic heterocycloalkyl includes, but is not limited to:

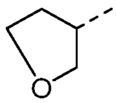,
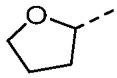,

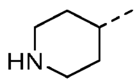,
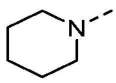,

and the like.

In the present disclosure, the term "bridged heterocycloalkyl" refers to a cyclic, saturated, monovalent group with a specified number of ring atoms (e.g., 4-10 members), a specified number of heteroatoms (e.g., 1, 2, or 3) and a specified heteroatom type (one or more of N, O, and S), which is polycyclic (e.g., bicyclic or tricyclic) with individual rings sharing two or more carbon atoms and/or heteroatoms. The bridged heterocycloalkyl is connected to the rest of the molecule through a ring with or without a heteroatom; the bridged heterocycloalkyl group is connected to the rest of the molecule by a carbon atom or a heteroatom. The bridged heterocycloalkyl includes, but is not limited to:

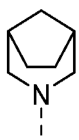,
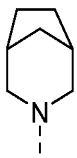,
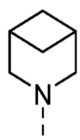,

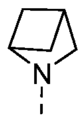,

and the like.

In the present disclosure, the term "spiro heterocycloalkyl" refers to a cyclic, saturated, monovalent group with a specified number of ring atoms (e.g., 7-10 members), a specified number of heteroatoms (e.g., 1, 2, or 3) and a specified heteroatom type (one or more of N, O, and S), which is polycyclic (e.g., bicyclic or tricyclic) with individual rings sharing one carbon atom. The spiro heterocycloalkyl is connected to the rest of the molecule through a ring with or without a

heteroatom; the spiro heterocycloalkyl group is connected to the rest of the molecule through a carbon atom or a heteroatom. The spiro heterocycloalkyl includes, but is not limited to:

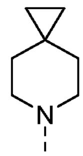 , 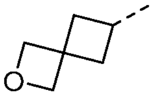 ,

and the like.

In the present disclosure, the term "heteroaryl" refers to a cyclic, unsaturated group with a specified number of ring atoms (e.g., 5-10 members), a specified number of heteroatoms (e.g., 1, 2, or 3) and a specified heteroatom type (one or more of N, O, and S), which is either monocyclic or polycyclic; when it is polycyclic, the individual rings share two atoms and one bond, and at least one of the rings is aromatic. The heteroaryl is connected to the rest of the molecule through a carbon atom or a heteroatom; the heteroaryl is connected to the rest of the molecule through a ring with or without a heteroatom; the heteroaryl is connected to the rest of the molecule through an aromatic ring or a non-aromatic ring. The heteroaryl includes, but is not limited to,

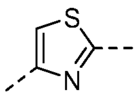 , 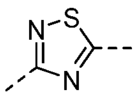 , 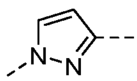 , 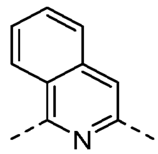 , 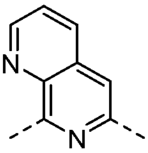 ,

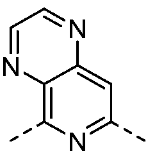 , 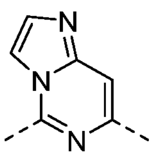 , 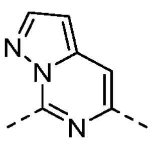 , 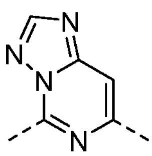 , 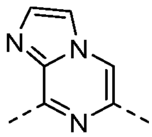 , 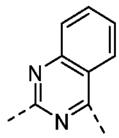 , 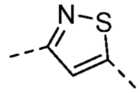 ,

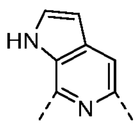 , 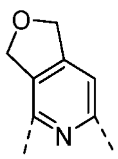 , 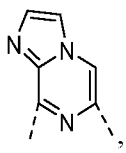 , 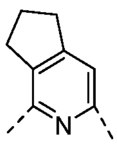 , 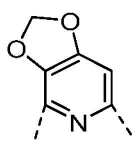 ,

and the like.

In the present disclosure, the term "aryl" refers to a cyclic, unsaturated hydrocarbon group with a specified number of carbon atoms (e.g., $C_6$-$C_{10}$), which is either monocyclic or polycyclic (e.g., bicyclic or tricyclic); when it is polycyclic, the individual rings share two atoms and one bond, and at least one of the rings is aromatic. The aryl is connected to the rest of the molecule through an aromatic ring or a non-aromatic ring. The aryl includes, but is not limited to: phenyl, naphthyl, and the like.

In the present disclosure, the term "pharmaceutically acceptable auxiliary material" refers to all substances, other than the active pharmaceutical ingredient, contained in a pharmaceutical formulation, which are generally divided into two main categories: excipients and additives. For more details, see Pharmacopoeia of the People's Republic of China (2020 Edition) and Handbook of Pharmaceutical Excipients (Paul J Sheskey, Bruno C Hancock, Gary P Moss, David J Goldfarb, 2020, 9th Edition).

In the present disclosure, the term "solvate" refers to a substance formed by association of a compound with a solvent (including, but not limited to: water, methanol, ethanol, and the like). Solvates are divided into stoichiometric solvates and non-stoichiometric solvates. Solvates include, but are not limited to, monohydrates.

In the present disclosure, the term "stereoisomer" refers to a cis-trans isomer or an optical isomer; the cis-trans isomer is an isomer caused by the inability of a double bond or a single bond of a ring-forming carbon atom to rotate freely, and the optical isomer is a stereoisomer having different optical rotation properties due to the absence of axial symmetry in the molecule.

In the present disclosure, the term "isotopically labeled derivative" refers to a compound in which one or more atoms have an isotopic abundance that differs from their natural abundance. For example, one or more atoms in the

compound are replaced by an atom with a lower natural abundance of mass number; for instance, one hydrogen atom in the compound is replaced by deuterium. For another example, compounds with the structure of the present disclosure having "deuterium" or "tritium" in place of hydrogen, or $^{18}$F-fluorine labeling (18$^{F}$ isotope) in place of fluorine, or $^{11}$C-, $^{13}$C- or $^{14}$C-enriched carbon ($^{11}$C-, $^{13}$C- or $^{14}$C-carbon labeling; $^{11}$C-, $^{13}$C- or $^{14}$C-isotope) in place of a carbon atom are within the scope of the present disclosure.

In the present disclosure, the term "prodrug" refers to any compound that can be converted *in vivo* to provide a bioactive substance (i.e., the compound represented by formula I). For example, a compound containing a carboxyl group can form a physiologically hydrolyzable ester, which acts as a prodrug by being hydrolyzed *in vivo* to yield the compound represented by formula I itself. The prodrug is preferably administered orally, as hydrolysis in many cases occurs primarily under the influence of digestive enzymes. When the ester itself is active or hydrolysis occurs in the blood, parenteral administration can be used. In addition, the prodrug can be converted to the compound of the present disclosure *in vivo* by chemical or biochemical methods.

In the present disclosure, the term "tautomer" refers to a functional isomer resulting from the rapid movement of an atom between two positions within a molecule.

In the present disclosure, the term "metabolite" refers to a substance produced or consumed in a metabolic process.

The preferred conditions described above may be combined arbitrarily to give preferred embodiments of the present disclosure without departing from the general knowledge in the art.

The reagents and starting materials used in the present disclosure are commercially available.

The positive and progressive effect of the present disclosure is that the nitrogen-containing compound of the present disclosure can effectively inhibit the overexpression of the KIF18A protein in some human tumor cells.

## DETAILED DESCRIPTION OF THE PREFERRED EMBODIMENT

The present disclosure is further illustrated by the following examples; however, these examples are not intended to limit the present disclosure. Experimental procedures without specified conditions in the following examples were conducted in accordance with conventional procedures and conditions, or in accordance with product instructions.

### Example 1: Preparation of Compound 1

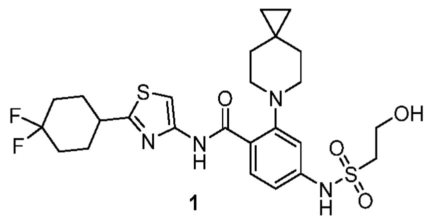

1

The synthetic route of compound 1 is as follows:

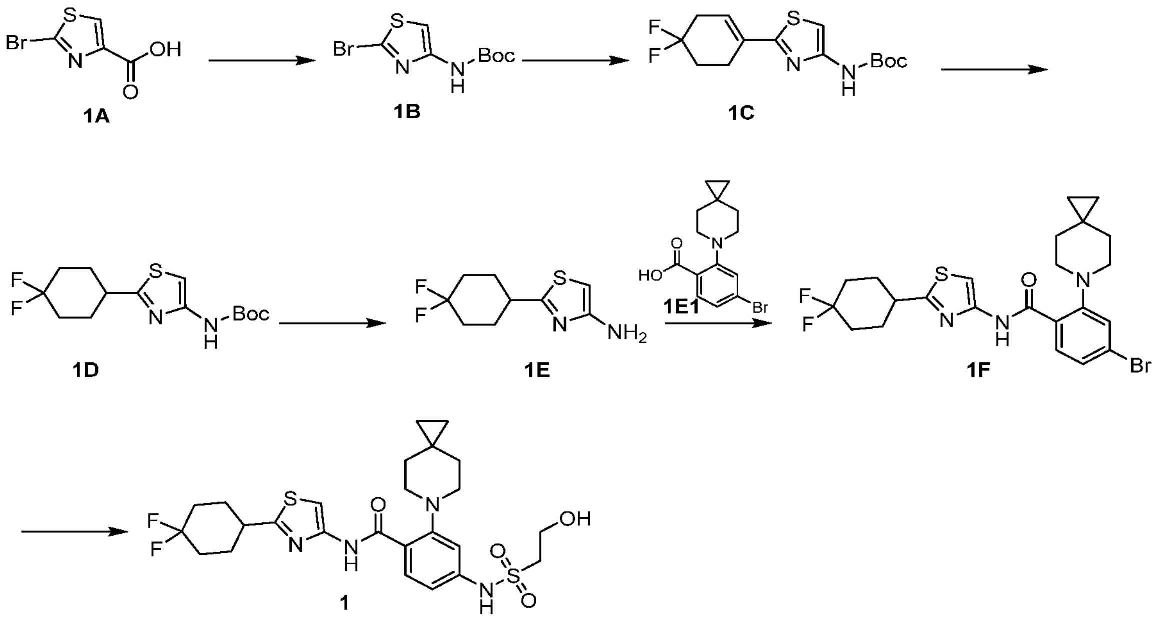

**1A** **1B** **1C** **1D** **1E** **1E1** **1F** **1**

The specific preparation method for compound 1 includes the following steps:

Step 1: synthesis of compound 1B

Compound **1A** (2.0 g, 9.7 mmol) was dissolved in tert-butanol (20 mL), and then triethylamine (1.95 g, 19.4 mmol) and diphenylphosphoryl azide (4.0 g, 14.55 mmol) were sequentially added. After the addition was completed, the mixture was purged 3 times with nitrogen, and then heated to 80 °C and allowed to react for 16 h under a nitrogen atmosphere. After the reaction was completed as detected by TLC, the mixture was diluted with water (50 mL) and extracted with ethyl acetate (30 mL × 3). The organic phases were combined, dried, and concentrated to dryness by rotary evaporation, and the residue was purified by silica gel column chromatography to give compound **1B** (500 mg, white solid, yield: 18.5%). MS (ESI): *m/z* 278.9 $[M+1]^+$

Step 2: synthesis of compound **1C**

Compound **1B** (200 mg, 0.72 mmol) was dissolved in a mixed solvent of 1,4-dioxane (4 mL) and water (0.5 mL), and then 2-(4,4-difluorocyclohex-1-en-1-yl)-4,4,5,5-tetramethyl-1,3,2-dioxaborolane (264 mg, 1.1 mmol), cesium carbonate (469 mg, 1.44 mmol), and [1,1'-bis(diphenylphosphino)ferrocenepalladium dichloride dichloromethane complex (20 mg, 0.033 mmol) were sequentially added at room temperature. After the addition was completed, the reaction liquid was purged 3 times with nitrogen, and then heated to 90 °C and allowed to react for 16 h under a nitrogen atmosphere. After the reaction was completed as detected by TLC, the reaction liquid was cooled to room temperature, diluted with water (20 mL), and extracted with ethyl acetate (20 mL × 3). The organic phases were combined, dried, and concentrated to dryness by rotary evaporation, and the residue was purified by silica gel column chromatography to give compound **1C** (200 mg, off-white solid, yield: 55.5%). MS (ESI): *m/z* 317.1 $[M+1]^+$

Step 3: synthesis of compound **1D**

Compound **1C** (200 mg, 0.63 mmol) was dissolved in methanol (5 mL), and then 10% wet palladium on carbon (50 mg) was added. After the addition was completed, the reaction liquid was purged 3 times with hydrogen and then allowed to react at 15 Psi and 50 °C for 4 h. After the reaction was completed as detected by TLC, the reaction liquid was cooled to room temperature and filtered. The filter cake was washed with methanol (5 mL), and the filtrate was concentrated to dryness by rotary evaporation to give compound **1D** (200 mg, pale yellow solid, yield: 99.4%). MS (ESI): *m/z* 263.1 $[M-55]^+$

Step 4: synthesis of compound **1E**

Compound **1D** (190 mg, 0.63 mmol) was dissolved in an 8% solution of hydrochloric acid in ethanol (2 mL), and the reaction liquid was purged 3 times with nitrogen and then allowed to react at 40 °C for 2 h. After the reaction was

completed as detected by TLC, the reaction liquid was concentrated to remove the solvent to give compound **1E** (100 mg, yellow solid, yield: 76.7%).

Step 5: synthesis of compound **1F**

Compound **1E1** (141 mg, 0.46 mmol) was dissolved in dichloromethane (2 mL), and then oxalyl chloride (233 mg, 1.82 mmol) and N,N-dimethylformamide (0.5 drop) were sequentially added dropwise. After the addition was completed, the mixture was allowed to react at room temperature for 30 min. After the reaction was completed as detected by TLC, the mixture was concentrated, and toluene (2 mL), 4-dimethylaminopyridine (223 mg, 1.82 mmol), and compound **1E** (100 mg, 0.46 mmol) were added to the resulting residue. After the addition was completed, the reaction liquid was purged 3 times with nitrogen and allowed to react at 100 °C for 1 h. After the reaction was completed as detected by TLC, the reaction liquid was cooled to room temperature, diluted with water (15 mL), and extracted with ethyl acetate (10 mL × 3). The organic phases were combined, dried, and concentrated to dryness by rotary evaporation, and the residue was purified by a thin layer chromatography plate to give compound **1F** (40 mg, yellow oil, yield: 17.1%). MS (ESI): *m*/*z* 510.1 $[M+1]^+$

Step 6: synthesis of compound **1**

Compound **1F** (40 mg, 0.08 mmol) and 2-hydroxyethane-1-sulfonamide (20 mg, 0.16 mmol) were dissolved in N,N-dimethylformamide (0.5 mL), and then cuprous iodide (30 mg, 0.16 mmol), potassium phosphate (50 mg, 0.24 mmol), and (1*R*,2*R*)-(-)-*N,N'*-dimethyl-1,2-cyclohexanediamine (11 mg, 0.08 mmol) were sequentially added. After the addition was completed, the reaction liquid was heated to 120 °C and allowed to react for 4 h. After the reaction was completed as detected by TLC, the reaction liquid was cooled to room temperature, diluted with water (20 mL), then extracted with ethyl acetate (20 mL × 3), dried, and concentrated to dryness by rotary evaporation, and the residue was purified by a TLC plate to give a crude product of the compound, which was then purified by preparative HPLC to give compound **1** (15 mg, pale yellow solid, yield: 18.5%). MS (ESI): *m*/*z* 555.2 $[M+1]^+$.

$^1$H NMR (300 MHz, dmso) δ 13.49 (s, 1H), 10.15 (br.s, 1H), 8.03 (d, *J* = 8.6 Hz, 1H), 7.52 (s, 1H), 7.26 (d, *J* = 1.9 Hz, 1H), 7.11 (dd, *J* = 8.6, 2.0 Hz, 1H), 4.93 (br.s, 1H), 3.75 (t, *J* = 6.5 Hz, 2H), 3.33 (t, *J* = 6.6 Hz, 2H), 3.22 (ddd, *J* = 8.9, 4.2, 1.7 Hz, 1H), 2.95 (t, *J* = 5.0 Hz, 4H), 2.19 - 1.78 (m, 8H), 1.67 (s, 4H), 0.37 (s, 4H).

**Example 2: Preparation of Compound 2**

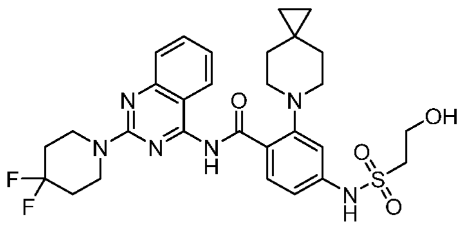

**2**

The synthetic route of compound 2 is as follows:

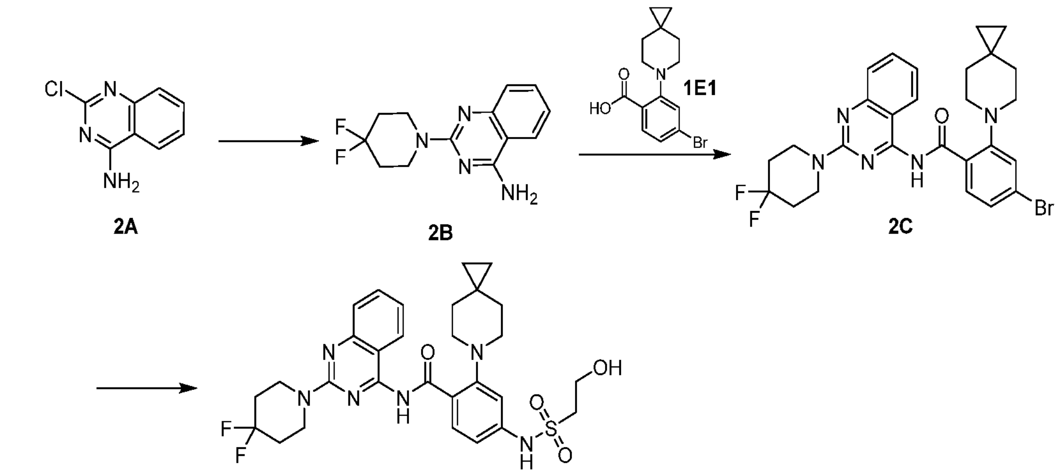

2A 2B 1E1 2C

2

The specific preparation method for compound 2 includes the following steps:

Step 1: synthesis of compound **2B**

Compound **2A** (500 mg, 2.78 mmol) was dissolved in N-methylpyrrolidone (5 mL), 4,4-difluoropiperidine hydrochloride (530 mg, 3.34 mmol), and N,N-diisopropylethylamine (720 mg, 5.56 mmol) were added. After the addition was completed, the reaction liquid was purged 3 times with nitrogen and then allowed to react at 120 °C for 16 h. After the reaction was completed as detected by TLC, the reaction liquid was diluted with water (50 mL) and then extracted with ethyl acetate (50 mL × 3). The organic phases were combined, dried, and concentrated to dryness by rotary evaporation, and the residue was purified by silica gel column chromatography to give compound **2B** (700 mg, yellow oil, yield: 95.2%). MS (ESI): *m/z* 265.1 $[M+1]^+$

Step 2: synthesis of compound **2C**

Compound **1E1** (117 mg, 0.38 mmol) was dissolved in dichloromethane (2 mL), and then oxalyl chloride (192 mg, 1.52 mmol) and N,N-dimethylformamide (1 drop) were sequentially added dropwise. After the addition was completed, the mixture was allowed to react at room temperature for 30 min. Then, the solvent was removed, and toluene (2 mL), 4-dimethylaminopyridine (184 mg, 1.52 mmol), and compound **2B** (100 mg, 0.38 mmol) were added. After the addition was completed, the reaction liquid was purged 3 times with nitrogen and allowed to react at 100 °C for 1 h. After the reaction was completed as detected by TLC, the reaction liquid was cooled to room temperature, diluted with water (15 mL), and then extracted with ethyl acetate (10 mL × 3). The organic phases were combined, dried, and concentrated to dryness by rotary evaporation, and the residue was purified by a TLC plate to give compound **2C** (50 mg, yellow solid, yield: 23.8%). MS (ESI): *m/z*556.1 $[M+1]^+$

Step 3: synthesis of compound **2**

Compound **2C** (50 mg, 0.09 mmol) and 2-hydroxyethane-1-sulfonamide (23 mg, 0.18 mmol) were dissolved in *N*,*N*-dimethylformamide (0.5 mL), and then cuprous iodide (34 mg, 0.18 mmol), potassium phosphate (58 mg, 0.27 mmol), and (1R,2R)-(-)-*N*,*N'*-dimethyl-1,2-cyclohexanediamine (13 mg, 0.09 mmol) were sequentially added. After the addition was completed, the reaction liquid was heated to 120 °C and allowed to react for 4 h. After the reaction was completed as detected by TLC, the reaction liquid was cooled to room temperature, diluted with water (10 mL), then extracted with ethyl acetate (30 mL × 3), dried, and concentrated to dryness by rotary evaporation, and the residue was purified by a TLC plate to give a crude product of the compound, which was then purified by preparative HPLC to give compound **2** (10 mg, pale yellow solid, yield: 18.5%). MS (ESI): *m/z* 601.2 $[M+1]^+$.

$^1$H NMR (300 MHz, dmso) δ 12.04 (s, 1H), 10.10 (br.s, 1H), 8.12 (d, *J* = 8.2 Hz, 1H), 7.79 - 7.64 (m, 2H), 7.47 (d, *J* = 8.3 Hz, 1H), 7.24 (t, *J* = 7.6 Hz, 1H), 7.11 - 6.96 (m, 2H), 4.97 (br.s, 1H), 3.76 (t, *J* = 6.4 Hz, 6H), 3.34 (d, *J* = 6.5 Hz, 2H), 2.85 (s, 4H), 2.04 - 1.79 (m, 4H), 1.28 (s, 4H), 0.21 (s, 4H).

**Example 3: Preparation of Compound 3**

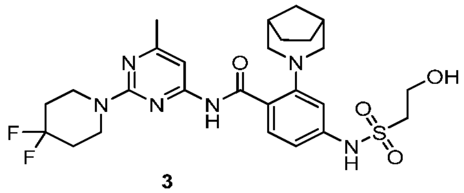

**3**

The synthetic route of compound 3 is as follows:

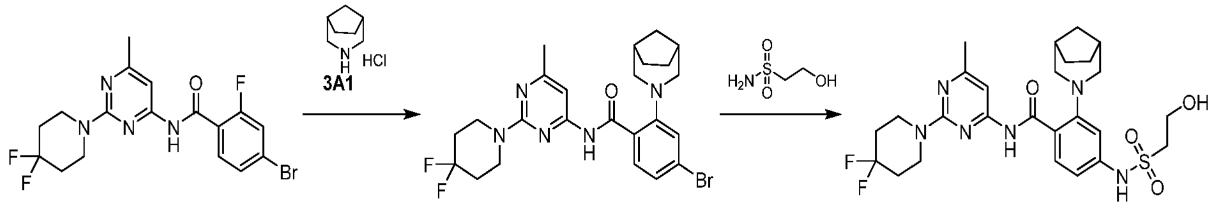


**3A** **3B** **3**

The specific preparation method for compound 3 includes the following steps:

Step 1: synthesis of compound **3B**

Compound **3A** (500 mg, 1.17 mmol, synthesized according to the preparation method in WO2020132648A1) was dissolved in N,N-dimethylformamide (5 mL), and then compound **3A1** (344 mg, 2.34 mmol) and potassium carbonate (323 mg, 2.34 mmol) were added. After the addition was completed, the mixture was allowed to react at 120 °C for 16 h. After the reaction was completed as detected by TLC, the mixture was diluted with water (30 mL) and then extracted with ethyl acetate (20 mL × 3). The organic phases were combined, washed with saturated brine (10 mL × 2), dried, and concentrated to dryness by rotary evaporation, and the residue was purified by thin layer chromatography to give compound **3B** (350 mg, white solid, yield: 57.6%). MS (ESI): *m*/*z* 260.5 $[M/2+1]^+$

Step 2: synthesis of compound **3**

**3B** (143.0 mg, 0.3 mmol) was dissolved in *N*,*N*-dimethylformamide (3.0 mL), and then potassium phosphate (116.9 mg, 0.6 mmol), 2-hydroxyethane-1-sulfonamide (69.0 mg, 0.6 mmol), (1*R*,2*R*)-(-)-*N,N'*-dimethyl-1,2-cyclohexanediamine (19.6 mg, 0.1 mmol), and cuprous iodide (52.5 mg, 0.3 mmol) were sequentially added. Then, the mixture was allowed to react at 120 °C for 3 h under a nitrogen atmosphere. After the reaction was completed as detected by TLC, the reaction liquid was diluted with saturated brine (20.0 mL) and extracted with ethyl acetate (20.0 mL × 3). The organic phases were combined, washed with saturated brine (4.0 mL × 2), dried over anhydrous sodium sulfate, and concentrated, and the resulting residue was purified by thin layer chromatography to give **compound** 3 (21.3 mg, white solid, yield: 13.7%). MS (ESI): *m*/*z* 565.2 $[M+1]^+$

$^1$H NMR (300 MHz, dmso) δ 11.02 (s, 1H), 10.08 (br.s, 1H), 7.73 (d, *J* = 8.5 Hz, 1H), 7.45 (s, 1H), 7.19 (s, 1H), 7.00 (dd, *J* = 8.5, 1.9 Hz, 1H), 4.91 (br.s, 1H), 3.92 - 3.78 (m, 4H), 3.73 (t, *J* = 6.5 Hz, 2H), 3.31 (t, *J* = 6.6 Hz, 2H), 2.93 (dd, *J* = 10.6, 3.1 Hz, 2H), 2.80 (d, *J* = 10.4 Hz, 2H), 2.29 (s, 3H), 2.24 (s, 2H), 2.03 - 1.81 (m, 6H), 1.69 - 1.42 (m, 4H).

## Example 4: Preparation of Compound 4

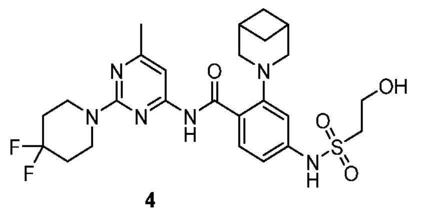

**4**

The synthetic route of compound 4 is as follows:

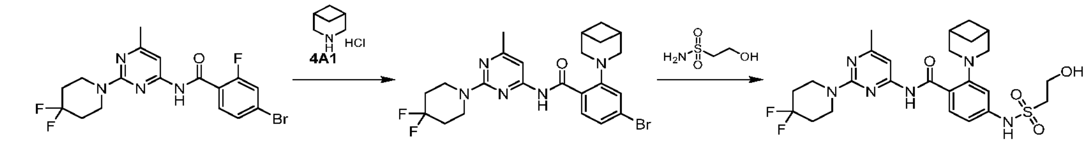


3A 4B 4

The specific preparation method for compound 4 includes the following steps:

Step 1: synthesis of compound **4B**

Compound **3A** (500 mg, 1.17 mmol) was dissolved in N,N-dimethylformamide (5 mL), and then compound **4A1** (344 mg, 2.34 mmol) and potassium carbonate (323 mg, 2.34 mmol) were added. After the addition was completed, the mixture was allowed to react at 120 °C for 16 h. After the reaction was completed as detected by TLC, the mixture was diluted with water (30 mL) and then extracted with ethyl acetate (20 mL × 3). The organic phases were combined, washed with saturated brine (10 mL × 2), dried, and concentrated to dryness by rotary evaporation, and the residue was purified by thin layer chromatography to give compound **4B** (75 mg, white solid, yield: 12.7%). MS (ESI): *m/z* 253.5 $[M/2+1]^+$

Step 2: synthesis of compound **4**

**4B** (75.0 mg, 0.15 mmol) was dissolved in *N,N*-dimethylformamide (2.0 mL), and then potassium phosphate (58.4 mg, 0.3 mmol), 2-hydroxyethane-1-sulfonamide (34.5 mg, 0.3 mmol), (1*R*,2*R*)-(-)-*N,N'*-dimethyl-1,2-cyclohexanediamine (10.0 mg, 0.05 mmol), and cuprous iodide (27.0 mg, 0.15 mmol) were sequentially added. The mixture was allowed to react at 120 °C for 3 h under a nitrogen atmosphere. After the reaction was completed as detected by TLC, the reaction liquid was diluted with saturated brine (20.0 mL) and extracted with ethyl acetate (20.0 mL × 3). The organic phases were combined, washed with saturated brine (10.0 mL × 2), dried over anhydrous sodium sulfate, and concentrated, and the resulting residue was purified by a thin layer chromatography column to give a product, which was then purified by preparative HPLC to give **compound 4** (28.0 mg, white solid, yield: 30%). MS (ESI): *m/z* 551.2 $[M+1]^+$

$^1$H NMR (300 MHz, dmso) δ 11.58 (s, 1H), 10.03 (br.s, 1H), 7.65 (d, *J* = 7.5 Hz, 1H), 7.37 (s, 1H), 7.15 (s, 1H), 6.91 (d, *J* = 8.8 Hz, 1H), 4.97 (br.s, 1H), 3.82 (s, 4H), 3.74 (t, *J* = 6.6 Hz, 2H), 3.39 (s, 4H), 3.31 (t, *J* = 6.6 Hz, 2H), 2.46-2.42 (m, 2H), 2.28 (s, 3H), 2.18 - 2.04 (m, 2H), 1.94 (ddd, *J* = 20.2, 13.6, 6.3 Hz, 4H), 1.71 (d, *J* = 5.6 Hz, 2H).

**Example 5: Preparation of Compound 5**

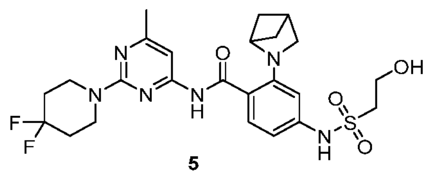

5

The synthetic route of compound 5 is as follows:

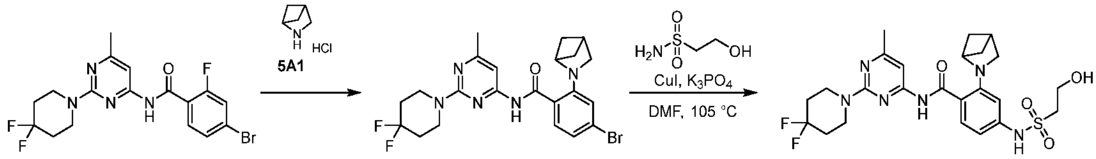


3A 5B 5

The specific preparation method for compound 5 includes the following steps:

Step 1: synthesis of compound **5B**

Compound **3A** (500 mg, 1.17 mmol) was dissolved in N,N-dimethylformamide (5 mL), and then compound **5A1** (302 mg, 2.34 mmol) and potassium carbonate (323 mg, 2.34 mmol) were added. The mixture was allowed to react at 120 °C for 16 h. After the reaction was completed as detected by TLC, the mixture was diluted with water (30 mL) and then

extracted with ethyl acetate (20 mL × 3). The organic phases were combined, washed with saturated brine (10 mL × 2), dried, and concentrated to dryness by rotary evaporation, and the residue was purified by thin layer chromatography to give compound **5B** (130 mg, white solid, yield: 22.6%). MS (ESI): *m*/*z* 246.5 $[M/2+1]^+$

Step 2: synthesis of compound **5**

Compound **5B** (100 mg, 0.203 mmol) and 2-hydroxyethane-1-sulfonamide (38.12 mg, 0.305 mmol) were dissolved in *N*,*N*-dimethylformamide (2 mL), and then cuprous iodide (38.8 mg, 0.203 mmol), potassium phosphate (86.5 mg, 0.406 mmol), and (1*R*,2*R*)-(-)-*N,N'*-dimethyl-1,2-cyclohexanediamine (14.45 mg, 0.101 mmol) were sequentially added. After the addition was completed, the mixture was allowed to react at 120 °C for 4 h under a nitrogen atmosphere. After the reaction was completed as detected by TLC, the reaction liquid was cooled to room temperature, diluted with water (20 mL), then extracted with ethyl acetate (20 mL × 3), dried, and concentrated to dryness by rotary evaporation, and the residue was purified by thin layer chromatography to give compound **5** (5 mg, pale yellow solid, yield: 4.59%). MS (ESI): *m*/*z* 537.2 $[M+1]^+$.

$^1$H NMR (300 MHz, dmso) δ 10.91 (s, 1H), 10.03 (br.s, 1H), 7.67 (d, *J* = 8.5 Hz, 1H), 7.35 (s, 1H), 6.92 (s, 1H), 6.85 (d, *J* = 8.3 Hz, 1H), 4.91 (br.s, 1H), 4.11 (d, *J* = 6.7 Hz, 1H), 3.91 - 3.77 (m, 4H), 3.73 (t, *J* = 6.5 Hz, 2H), 3.28-3.26 (m, 1H), 3.09 (s, 2H), 2.90 - 2.80 (m, 1H), 2.29 (s, 3H), 2.08 - 1.82 (m, 7H), 1.48 (d, *J* = 4.2 Hz, 2H).

**Example 6: Preparation of Compound 6**

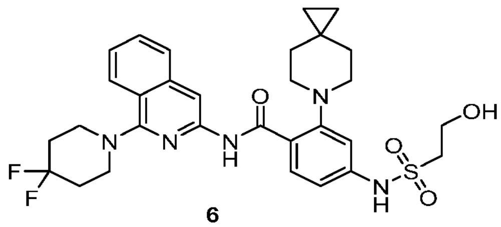

6

The synthetic route of compound 6 is as follows:

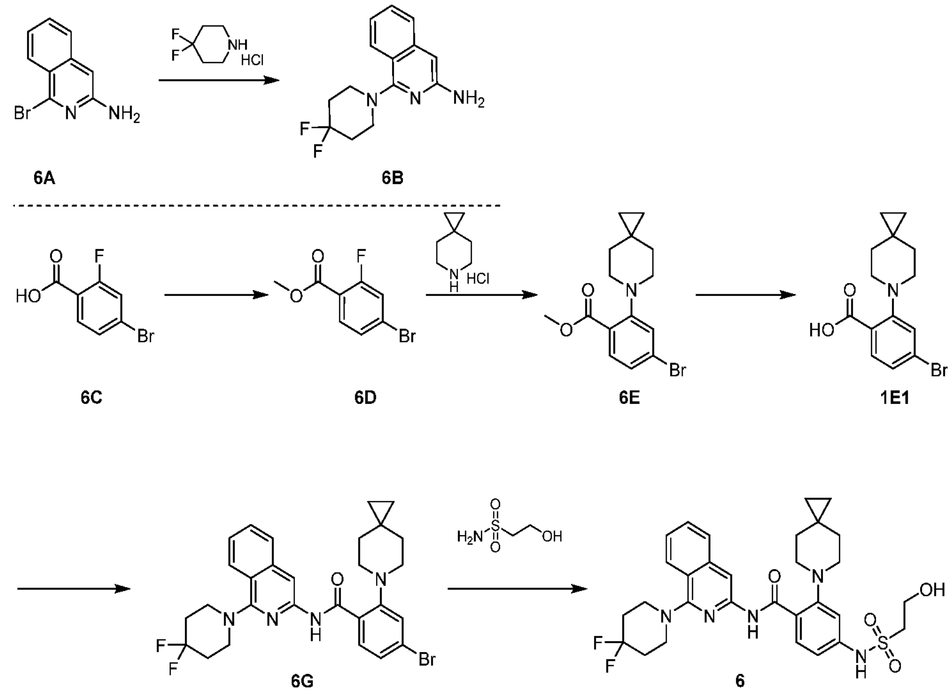

6A 6B 6C 6D 6E 1E1 6G 6

The specific preparation method for compound 6 includes the following steps:

Step 1: synthesis of compound **6B**

**6A** (500.0 mg, 2.3 mmol) was dissolved in N,N-dimethylpyrrolidone (5.0 mL), and then *N,N*-diisopropylethylamine (1.4 g, 9.0 mmol) and 4,4-difluoropiperidine hydrochloride (722.2 mg, 4.6 mmol) were sequentially added. The mixture was allowed to react in a sealed container at 150 °C for 5 h. After the reaction liquid was cooled down, it was diluted with saturated brine (20.0 mL) and extracted with ethyl acetate (40.0 mL × 2). The organic phases were combined, washed with saturated brine (10.0 mL × 2), and dried over anhydrous sodium sulfate, and then the organic solution was concentrated to dryness by rotary evaporation. The resulting residue was purified by silica gel column chromatography and concentrated to dryness by rotary evaporation under reduced pressure to give **6B** (452 mg, yellow solid, yield: 76.3%). MS (ESI): *m*/*z* 264.1 [M+1]+

Step 2: synthesis of compound **6D**

Compound **6C** (5.0 g, 22.8 mmol) was dissolved in methanol (50 mL), and then thionyl chloride (3.5 g, 29.6 mmol) was added dropwise to the reaction liquid at 0 °C. After the addition was completed, the reaction liquid was stirred at 60 °C for 5 h. After the reaction was completed as detected by TLC, the reaction liquid was concentrated under reduced pressure to remove the solvent, and the residue was purified by silica gel column chromatography to give compound **6D** (5.0 g, white solid, yield: 94.0%). MS (ESI): *m*/*z* 232.0 [M+1]+

Step 3: synthesis of compound **6E**

Compound **6D** (1.0 g, 4.29 mmol) was weighed out and added into a sealed container, and then *N*-methylpyrrolidone (10 mL), 6-aza-spiro[2.5]octane hydrochloride (760 mg, 5.15 mmol), and *N,N*-diisopropylethylamine (1.7 g, 12.9 mmol) were added. After the addition was completed, the reaction liquid was heated to 120 °C and allowed to react for 16 h. After the reaction was completed as detected by TLC, the reaction liquid was cooled to room temperature, diluted with water (100 mL), then extracted with ethyl acetate (80 mL × 3), dried, and concentrated to dryness by rotary evaporation, and the residue was purified by silica gel column chromatography to give compound **6E** (1.2 g, pale yellow oil, yield: 86.3%). MS (ESI): *m*/*z* 324.1 [M+1]+, 326.1 [M+3]+

Step 4: synthesis of compound **1E1**

Compound **6E** (1.2 g, 3.70 mmol) was dissolved in methanol (10 mL), water (10 mL), and tetrahydrofuran (5 mL), and then lithium hydroxide monohydrate (156 mg, 3.70 mmol) was added under an ice bath. After the addition was completed, the reaction liquid was allowed to react at room temperature for 16 h. After the reaction was completed as detected by TLC, methanol and tetrahydrofuran were removed, and 1 N hydrochloric acid was added to adjust the pH to 5-6. The mixture was then extracted with dichloromethane/methanol (10:1, 100 mL × 3), dried, and concentrated to dryness by rotary evaporation, and the residue was purified by silica gel column chromatography to give compound **1E1** (1.0 g, white solid, yield: 87.1%). MS (ESI): *m*/*z* 310.0 [M+1]+

Step 5: synthesis of compound **6G**

Compound **1E1** (50.0 mg, 0.2 mmol) was dissolved in anhydrous dichloromethane (2.0 mL), and then oxalyl chloride (50.0 mg, 0.4 mmol) was added at room temperature, followed by addition of half a drop of *N*,*N*-dimethylformamide. The mixture was allowed to react at 25 °C for 10 min. After the reaction was completed as detected by TLC, the mixture was concentrated to dryness by rotary evaporation, and the resulting residue was dissolved in anhydrous tetrahydrofuran (2.0 mL). Then, a solution of *N*,*N*-dimethyl-4-aminopyridine (79.1 mg, 0.6 mmol) and compound **6B** (42.5 mg, 0.2 mmol) in toluene (1.0 mL) was added. The reaction liquid was stirred at 100 °C for 1 h. After the reaction was completed as detected by TLC, the reaction liquid was diluted with saturated brine (20.0 mL) and extracted with ethyl acetate (20.0 mL × 2). The organic phases were combined, washed with saturated brine (10.0 mL × 2), dried over anhydrous sodium sulfate, and concentrated, and the resulting residue was purified by silica gel column chromatography and concentrated to dryness by rotary evaporation under reduced pressure to give compound **6G** (63 mg, yellow solid, yield: 70.3%). MS (ESI): *m*/*z* 555.2[M+1]+

Step 6: synthesis of compound **6**

Compound **6G** (63.0 mg, 0.1 mmol) was dissolved in *N*,*N*-dimethylformamide (1.5 mL), and then anhydrous potassium phosphate (48.3 mg, 0.2 mmol), 2-hydroxyethane-1-sulfonamide (21.3 mg, 0.2 mmol), (1*R*,2*R*)-(-)-*N*,*N*'-dimethyl-1,2-cyclohexanediamine (8.1 mg, 0.06 mmol), and cuprous iodide (21.7 mg, 0.1 mmol) were sequentially added.

The mixture was allowed to react at 120 °C for 3 h under a nitrogen atmosphere. After the reaction was completed as detected by TLC, the reaction liquid was cooled to room temperature, diluted with water (20 mL), then extracted with ethyl acetate (20 mL × 3), dried, and concentrated to dryness by rotary evaporation, and the residue was purified by a TLC plate to give a crude product of the compound, which was then purified by preparative HPLC to give compound 6 (19.4 mg, white solid, yield: 28.5%). MS (ESI): *m/z* 600.2 [M+1]$^+$

$^1$H NMR (300 MHz, dmso) δ 13.13 (s, 1H), 10.22 (br.s, 1H), 8.26 (s, 1H), 8.09 (d, *J* = 8.5 Hz, 1H), 8.05 (d, *J* = 8.5 Hz, 1H), 7.84 (d, *J* = 8.0 Hz, 1H), 7.65 (t, *J* = 7.5 Hz, 1H), 7.45 (t, *J* = 7.6 Hz, 1H), 7.27 (d, *J* = 1.8 Hz, 1H), 7.13 (dd, *J* = 8.6, 1.9 Hz, 1H), 4.94 (br.s, 1H), 3.75 (t, *J* = 6.5 Hz, 2H), 3.57 - 3.44 (m, 4H), 3.36 (d, *J* = 6.4 Hz, 2H), 3.00 (d, *J* = 3.6 Hz, 4H), 2.27 (ddd, *J* = 19.7, 14.4, 5.5 Hz, 4H), 2.14 - 1.34 (m, 4H), 0.39 (s, 4H).

**Example 7: Preparation of Compound 7**

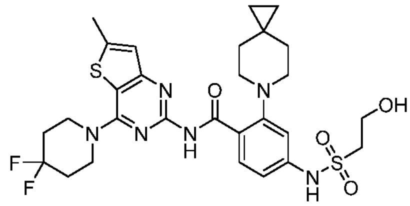

**7**

The synthetic route of compound 7 is as follows:

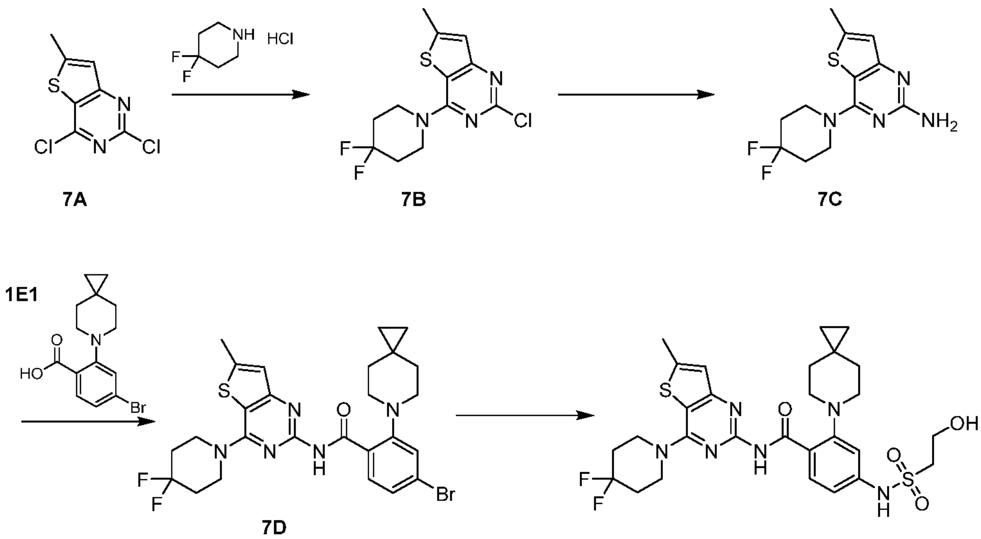


**7A** **7B** **7C**

**7D** **7**

The specific preparation method for compound 7 includes the following steps:

Step 1: synthesis of compound **7B**

Compound **7A** (500 mg, 2.29 mmol) was dissolved in acetonitrile (10 mL), and then *N,N*-diisopropylethylamine (739.7 mg, 5.73 mmol) and compound 4,4-difluoropiperidine hydrochloride (400 mg, 2.52 mmol) were sequentially added at room temperature. After the addition was completed, the reaction liquid was allowed to react at room temperature for 16 h. After the reaction was completed as detected by TLC, the reaction liquid was diluted with water (40 mL) and extracted with ethyl acetate (20 mL × 3), and the organic phases were combined and dried over anhydrous sodium sulfate to give compound **7B** (757 mg, white solid, yield: 108.9%). MS (ESI): *m/z* 304.1[M+1]$^+$

Step 2: synthesis of compound **7C**

Compound **7B** (300 mg, 0.99 mmol) was added to a solution of aqueous ammonia (6 mL), and the mixture was

allowed to react in a sealed container at 160 °C for 4 h. After the reaction was completed as detected by TLC, the reaction liquid was diluted with water (40 mL) and extracted with ethyl acetate (20 mL × 3). The organic phases were combined, dried over anhydrous sodium sulfate, and concentrated to dryness by rotary evaporation, and the residue was purified by silica gel column chromatography to give compound 7C (94 mg, white solid, yield: 33.4%). MS (ESI): *m/z* 285.1[M+1]+

Step 3: synthesis of compound **7D**

Compound **1E1** (80.5 mg, 0.26 mmol) was dissolved in dichloromethane (2 mL), and then oxalyl chloride (58 mg, 0.65 mmol) and N,N-dimethylformamide (0.5 drop) were sequentially added dropwise under an ice bath. After the addition was completed, the mixture was allowed to react at room temperature for 30 min. After the reaction was completed as detected by TLC, the mixture was concentrated to dryness by rotary evaporation, and then toluene (2 mL), 4-dimethylaminopyridine (127.3 mg, 1.04 mmol), and a solution of compound **7C** (74 mg, 0.26 mmol) in toluene (2 mL) were sequentially added. After the addition was completed, the reaction liquid was allowed to react at 100 °C for 1 h under a nitrogen atmosphere. After the reaction was completed as detected by TLC, the reaction liquid was diluted with water (30 mL) and extracted with ethyl acetate (10 mL × 3). The organic phases were combined, dried, and concentrated to dryness by rotary evaporation, and the residue was purified by a thin layer chromatography plate to give compound **7D** (55 mg, yellow solid, yield: 36.7%). MS (ESI): *m/z* 576.1 [M+1]+

Step 6: synthesis of compound **7**

Compound **7D** (45 mg, 0.078 mmol) and 2-hydroxyethane-1-sulfonamide (14.7 mg, 0.117 mmol) were dissolved in N,N-dimethylformamide (1 mL), and then cuprous iodide (15.0 mg, 0.078 mmol), potassium phosphate (33.2 mg, 0.156 mmol), and (1*R*,2*R*)-(-)-*N*,*N*'-dimethyl-1,2-cyclohexanediamine (5.6 mg, 0.039 mmol) were sequentially added. After the addition was completed, the reaction liquid was allowed to react at 120 °C for 2 h under a nitrogen atmosphere. After the reaction was completed as detected by TLC, the reaction liquid was diluted with water (20 mL) and extracted with ethyl acetate (10 mL × 3). The organic phases were combined, dried, and concentrated to dryness by rotary evaporation, and then the residue was purified by preparative HPLC to give compound 7 (2 mg, yellow solid, yield: 4.1%). MS (ESI): *m/z* 621.2 [M+1]+

**Example 8: Preparation of Compound 8**

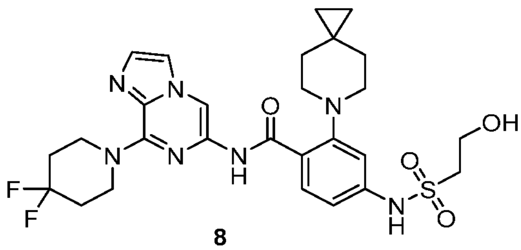

**8**

The synthetic route of compound 8 is as follows:

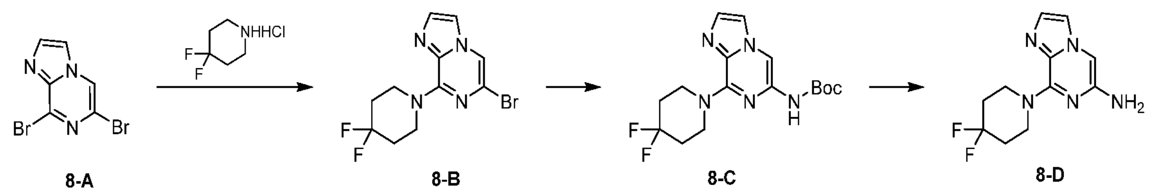

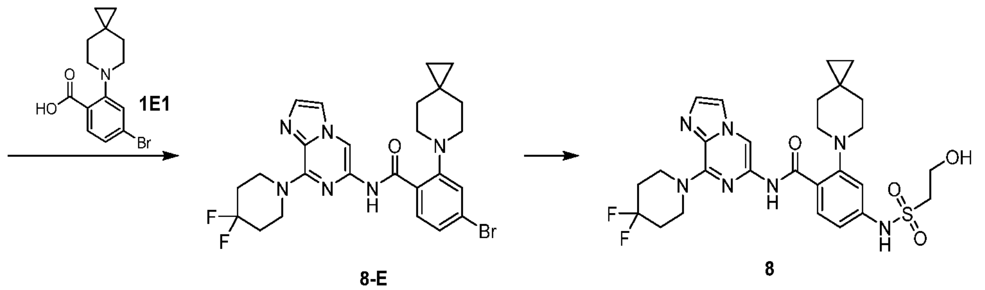

The specific preparation method for compound 8 includes the following steps:

**Step 1: synthesis of compound 8-B**

**Compound 8-A** (2.0 g, 7.27 mmol) was dissolved in N-methylpyrrolidone (50 mL), and then *N,N*-diisopropylethylamine (2.8 g, 21.9 mmol) and 4,4-difluoropiperidine hydrochloride (1.5 g, 9.4 mmol) were added. Then, the mixture was stirred at 100 °C for 3 h. After the reaction was completed as detected by TLC, the reaction liquid was cooled to room temperature, diluted with water (100 mL), and extracted with ethyl acetate (40 mL × 3). The organic phases were combined, washed with saturated brine (30 mL × 3), dried over anhydrous sodium sulfate, filtered, and concentrated under reduced pressure to remove the solvent, and the residue was purified by silica gel column chromatography to give **compound 8-B** (2.0 g, yellow solid, yield: 87.1%). MS (ESI): *m/z* 317.0 $[M+1]^+$

**Step 2: synthesis of compound 8-C**

**Compound 8-B** (860.0 mg, 2.72 mmol) was dissolved in dioxane (10.0 mL), and then *tert*-butyl carbamate (640.0 mg, 5.44 mmol), cesium carbonate (1.78 g, 5.44 mmol), 2-dicyclohexylphosphino-2',4',6'-triisopropylbiphenyl (235.0 mg, 0.41 mmol), and palladium acetate (50.0 mg, 0.21 mmol) were added. Then, the mixture was allowed to react at 100 °C for 4 h under a nitrogen atmosphere. After the reaction was completed as detected by TLC, the reaction liquid was cooled to room temperature, diluted with water (50.0 mL), and extracted with ethyl acetate (30.0 mL × 2). The organic phases were combined, washed with saturated brine (20.0 mL × 2), dried over anhydrous sodium sulfate, filtered, and concentrated under reduced pressure to remove the solvent, and the residue was purified by silica gel column chromatography and concentrated to dryness by rotary evaporation under reduced pressure to give **compound 8-C** (800 mg, brown solid, yield: 83.3%). MS (ESI): *m/z* 354.2 $[M+1]^+$

**Step 3: synthesis of compound 8-D**

**Compound 8-C** (400.0 mg, 1.13 mmol) was dissolved in anhydrous dichloromethane (10.0 mL), and then 2,6-lutidine (364 mg, 3.4 mmol) and trimethylsilyl trifluoromethanesulfonate (755 mg, 3.4 mmol) were sequentially added at room temperature. Then, the mixture was stirred at room temperature for 1 h. After the reaction was completed as detected by TLC, the reaction liquid was diluted with ice water (40 mL) and extracted with dichloromethane (30 mL × 3). The organic phases were combined, washed with saturated brine (20.0 mL × 2), dried over anhydrous sodium sulfate, filtered, and concentrated under reduced pressure to remove the solvent to give a crude product of **compound 8-D** (300 mg). MS (ESI): *m/z* 254.1 $[M+1]^+$.

$^1$H NMR (400 MHz, DMSO-$d_6$) δ(ppm) 8.20 (s, 1H), 7.91 (d, *J* = 1.2 Hz, 1H), 7.58 (d, *J* = 1.2 Hz, 1H), 4.35 (t, *J* = 8.0 Hz, 4H), 2.14 - 2.04 (m, 4H).

**Step 4: synthesis of compound 8-E**

Compound 1E1 (330.0 mg, 1.1 mmol) was dissolved in dichloromethane (10 mL), and then oxalyl chloride (415

mg, 3.3 mmol) and *N,N*-dimethylformamide (0.5 drop) were sequentially added dropwise under an ice bath. Then, the mixture was stirred at room temperature for 30 min. After the reaction was completed as detected by TLC, the reaction liquid was concentrated to dryness by rotary evaporation, and then the residue was dissolved in anhydrous tetrahydrofuran (6 mL). A solution of **compound 8-D** (300 mg, 1.1 mmol) and *N,N*-diisopropylethylamine (426 mg, 3.3 mmol) in tetrahydrofuran (8 mL) was added at room temperature. Then, the mixture was stirred at 50 °C for 1 h under a nitrogen atmosphere. After the reaction was completed as detected by TLC, the reaction liquid was diluted with water (40 mL) and extracted with ethyl acetate (30 mL × 3). The organic phases were combined, washed with saturated brine (20.0 mL), dried over anhydrous sodium sulfate, filtered, and concentrated under reduced pressure to remove the solvent, and the residue was purified by a silica gel column to give **compound 8-E** (300 mg, bright yellow solid, yield: 50.1%). MS (ESI): *m/z* 273.1 $[M/2+1]^+$

**Step 5: synthesis of compound 8**

Compound **8-E** (100 mg, 0.18 mmol) and 2-hydroxyethane-1-sulfonic acid (46.3 mg, 0.37 mmol) were dissolved in *N,N*-dimethylformamide (3 mL), and then cuprous iodide (35.0 mg, 0.18 mmol), potassium phosphate (78 mg, 0.37 mmol), and (1*R*,2*R*)-(-)-*N,N'*-dimethyl-1,2-cyclohexanediamine (26 mg, 0.18 mmol) were sequentially added. Then, the mixture was stirred at 120 °C for 3 h under a nitrogen atmosphere. After the reaction was completed as detected by TLC, the reaction liquid was diluted with water (40 mL) and extracted with ethyl acetate (30 mL × 3). The organic phases were combined, washed with saturated brine (20.0 mL), dried over anhydrous sodium sulfate, filtered, and concentrated under reduced pressure to remove the solvent, and the residue was purified by preparative HPLC to give compound 8 (60 mg, off-white solid). MS (ESI): *m/z* 590.2 $[M+1]^+$.

$^1$H NMR (300 MHz, DMSO-$d_6$) δ(ppm) 12.88 (s, 1H), 10.20 (br.s, 1H), 8.82 (s, 1H), 8.10 - 8.02 (m, 2H), 7.54 (d, *J* = 1.0 Hz, 1H), 7.26 (d, *J* = 2.1 Hz, 1H), 7.12 (dd, *J* = 8.7, 2.1 Hz, 1H), 4.99 (br.s, 1H), 4.51 - 4.35 (m, 4H), 3.74 (t, *J* = 6.5 Hz, 2H), 3.35 (t, *J* = 6.5 Hz, 2H), 3.03 - 2.89 (m, 4H), 2.26 - 2.00 (m, 5H), 1.91 - 1.50 (m, 3H), 0.39 (s, 4H).

**Example 9: Preparation of Compound 9**

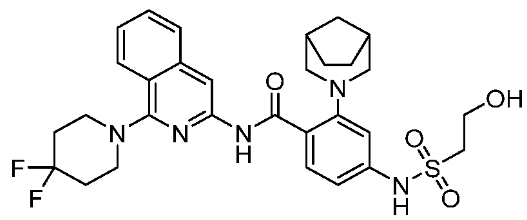

**9**

The synthetic route of compound 9 is as follows:

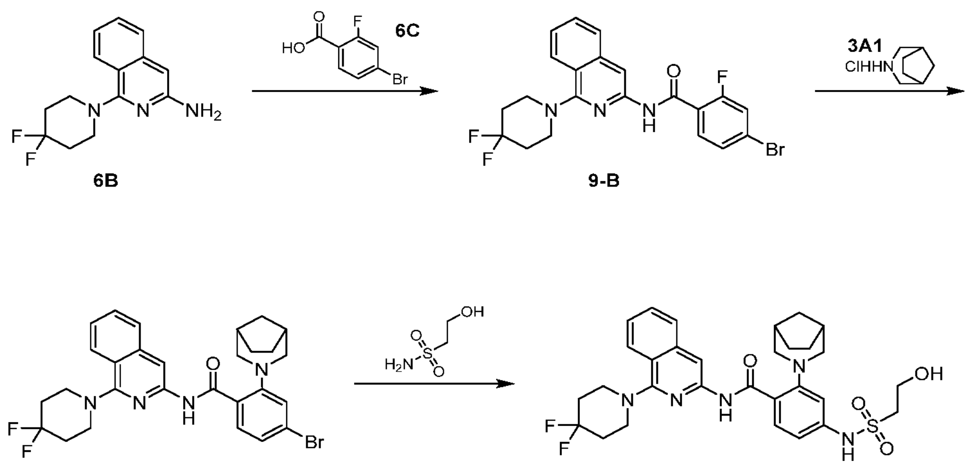


6B 9-B

9-C 9

**The preparation method for compound 9 specifically includes the following steps:**

**Step 1: synthesis of compound 9-B**

**Compound 6C** (50 mg, 0.23 mmol) was dissolved in thionyl chloride (1 mL), and then the mixture was stirred at 80 °C for 2 h. After the reaction was completed as detected by TLC, the reaction liquid was concentrated under reduced pressure to remove the solvent to give a crude product of the intermediate. **Compound 6B** (60 mg, 0.23 mmol) and *N,N*-diisopropylethylamine (0.16 mL, 0.91 mmol) were dissolved in tetrahydrofuran (1 mL), and then the solution of the intermediate in tetrahydrofuran (0.5 mL) was added dropwise to the above reaction liquid. Then, the mixture was stirred at 50 °C for 2 h. After the reaction was completed as detected by TLC, the reaction liquid was cooled to room temperature, diluted with water (10 mL), and then extracted with ethyl acetate (10 mL × 3). The organic phases were combined, washed with saturated brine (10 mL), dried over anhydrous sodium sulfate, filtered, and concentrated under reduced pressure to remove the solvent, and the residue was purified by column chromatography to give **compound 9-B** (110 mg, yellow solid). MS (ESI): *m*/*z* 464.1 $[M+1]^+$

**Step 2: synthesis of compound 9-C**

**Compound 9-B** (110 mg, 0.24 mmol) was dissolved in *N,N*-dimethylformamide (1 mL), and then **compound 3A1** (70 mg, 0.47 mmol) and potassium carbonate (66 mg, 0.47 mmol) were added. Then, the mixture was stirred at 120 °C for 12 h. After the reaction was completed as detected by TLC, the reaction liquid was cooled to room temperature, diluted with water (10 mL), and then extracted with ethyl acetate (10 mL × 3). The organic phases were combined, washed with saturated brine (10 mL × 3), dried over anhydrous sodium sulfate, filtered, and concentrated under reduced pressure to remove the solvent, and the residue was purified by column chromatography to give **compound 9-C** (80 mg, yellow solid, yield: 60%). MS (ESI): *m*/*z* 555.2 $[M+1]^+$

**Step 3: synthesis of compound 9**

**Compound 9-C** (80 mg, 0.14 mmol) and 2-hydroxyethane-1-sulfonamide (37 mg, 0.28 mmol) were dissolved in anhydrous *N,N*-dimethylformamide (2 mL), and then cuprous iodide (3 mg, 0.01 mmol), potassium phosphate (61 mg, 0.28 mmol) and (1*R*,2*R*)-(-)-*N,N'*-dimethyl-1,2-cyclohexanediamine (11 mg, 0.07 mmol) were added. The reaction liquid was purged three times with nitrogen and then stirred at 120 °C for 16 h. After the reaction was completed as detected by TLC, the reaction liquid was cooled to room temperature, diluted with water (10 mL), and then extracted with ethyl acetate (10 mL × 3). The organic phases were combined, washed with saturated brine (10 mL × 3), dried over anhydrous sodium sulfate, filtered, and concentrated under reduced pressure to remove the solvent, and the residue was purified by column chromatography to give a crude product of the compound, which was then purified by prep-HPLC to give **compound 9** (23 mg, pale yellow solid). MS (ESI): *m*/*z* 600.2 $[M+1]^+$.

$^1$H NMR (300 MHz, DMSO-$d_6$) δ(ppm) 10.73 (s, 1H), 10.05 (br.s, 1H), 8.32 (s, 1H), 8.11 - 7.99 (m, 1H), 7.85 (d, *J* = 8.3 Hz, 1H), 7.72 (d, *J* = 8.5 Hz, 1H), 7.65 (ddd, *J* = 8.1, 6.8, 1.2 Hz, 1H), 7.46 (ddd, *J* = 8.3, 6.8, 1.2 Hz, 1H), 7.16 (d, *J* = 2.1 Hz, 1H), 7.00 (dd, *J* = 8.5, 2.0 Hz, 1H), 4.95 (br.s, 1H), 3.74 (t, *J* = 6.6 Hz, 2H), 3.51 - 3.40 (m, 4H), 3.31 - 3.26 (m, 2H), 3.01 (dd, *J* = 10.7, 3.5 Hz, 2H), 2.80 (d, *J* = 10.6 Hz, 2H), 2.28 - 2.16 (m, 6H), 2.03 - 1.92 (m, 2H), 1.63 - 1.44 (m, 4H).

**Example 10: Preparation of Compound 10**

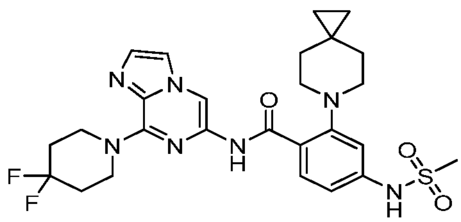

**10**

**Synthetic route:**

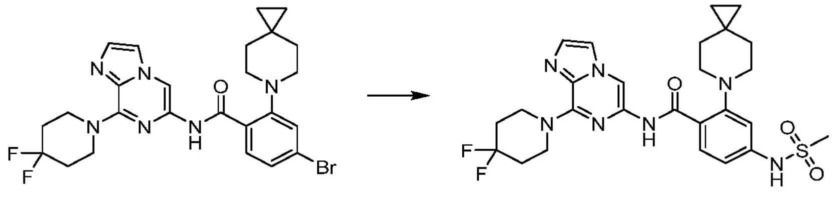

8-E 10

**Preparation method:**

**Step 1: synthesis of compound 10**

**Compound 8-E** (70 mg, 0.128 mmol) and **methanesulfonamide** (16 mg, 0.384 mmol) were dissolved in *N,N*-dimethylformamide (1 mL), and then cuprous iodide (15 mg, 0.077 mmol), potassium phosphate (77 mg, 0.371 mmol), and (1*R*,2*R*)-(-)-*N,N'*-dimethyl-1,2-cyclohexanediamine (15 mg, 0.110 mmol) were sequentially added. Then, the reaction liquid was purged three times with nitrogen and stirred at 100 °C for 16 h. After the reaction was completed as detected by LCMS, the reaction liquid was cooled to room temperature, diluted with water (10 mL), and then extracted with ethyl acetate (10 mL × 3). The organic phases were combined, washed with saturated brine (10 mL × 3), dried over anhydrous sodium sulfate, filtered, and concentrated under reduced pressure to remove the solvent, and the residue was purified by preparative HPLC to give **compound 10** (10 mg, off-white solid). MS (ESI): *m/z* 560.2 $[M+1]^+$.

$^1H$ NMR (300 MHz, DMSO-$d_6$) δ(ppm) 12.88 (s, 1H), 10.24 (br.s, 1H), 8.83 (s, 1H), 8.08 (dd, *J* = 4.9, 3.8 Hz, 2H), 7.54 (d, *J* = 1.1 Hz, 1H), 7.25 (d, *J = 2.1* Hz, 1H), 7.13 (dd, *J* = 8.7, 2.1 Hz, 1H), 4.48 - 4.37 (m, 4H), 3.11 (s, 3H), 3.03 - 2.95 (m, 4H), 2.23 - 1.99 (m, 5H), 1.88 - 1.59 (m, 3H), 0.39 (s, 4H).

**Example 11: Preparation of Compound 11**

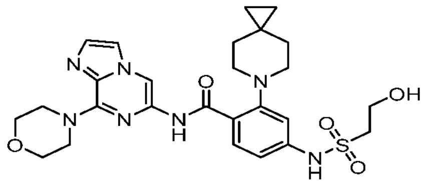

11

**Synthetic route:**

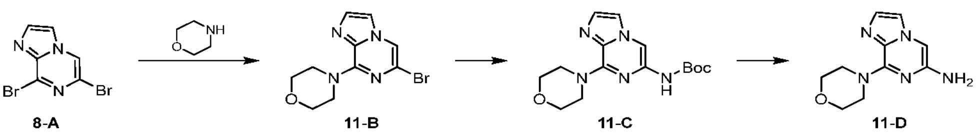

8-A 11-B 11-C 11-D

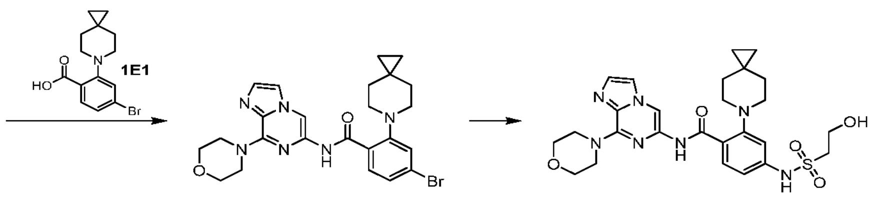


11-E 11

**Preparation method:**

**Step 1: synthesis of compound 11-B**

**Compound 8-A** (1.0 g, 3.61 mmol) was dissolved in *N*-methylpyrrolidone (10 mL), and then *N,N*-diisopropylethylenediamine (1.4 g, 10.8 mmol) and **morpholine** (410 mg, 4.69 mmol) were added. Then, the mixture was stirred at 100 °C for 4 h under a nitrogen atmosphere. After the reaction was completed as detected by TLC, the reaction liquid was cooled to room temperature, diluted with water (100 mL), and extracted with ethyl acetate (50 mL × 3). The organic phases were combined, washed with saturated brine (30 mL × 2), dried over anhydrous sodium sulfate, filtered, and concentrated under reduced pressure to remove the solvent, and the residue was purified by silica gel column chromatography to give **compound 11-B** (1 g, yellow oil, yield: 97.8%). MS (ESI): *m*/*z* 283.0 $[M+1]^+$

**Step 2: synthesis of compound 11-C**

**Compound 11-B** (1.0 g, 3.53 mmol) was dissolved in dioxane (10 mL), and then *tert*-butyl carbamate (833 mg, 7.06 mmol), 2-dicyclohexylphosphino-2',4',6'-triisopropylbiphenyl (340 mg, 0.71 mmol), cesium carbonate (2.3 g, 7.06 mmol), palladium acetate (80 mg, 0.35 mmol) were added, and the mixture was stirred at 100 °C for 16 h under a nitrogen atmosphere. After the reaction was completed as detected by TLC, the reaction liquid was cooled to room temperature, diluted with water (100 mL), and extracted with ethyl acetate (50 mL × 2). The organic phases were combined, washed with saturated brine (30 mL × 2), dried over anhydrous sodium sulfate, filtered, and concentrated under reduced pressure to remove the solvent, and the residue was purified by silica gel column chromatography to give **compound 11-C** (1.0 g, yellow solid, yield: 88.7%). MS (ESI): *m*/*z* 320.2 $[M+1]^+$

**Step 3: synthesis of compound 11-D**

**Compound 11-C** (500 mg, 1.57 mmol) was dissolved in anhydrous dichloromethane (5.0 mL), and then 2,6-lutidine (504 mg, 4.71 mmol) and trimethylsilyl trifluoromethanesulfonate (1.1 g, 4.71 mmol) were sequentially added at room temperature. Then, the mixture was stirred at room temperature for 1 h. After the reaction was completed as detected by TLC, the reaction liquid was diluted with ice water (50 mL), adjusted to pH = 7-8 with a saturated ammonium bicarbonate solution, and then extracted with dichloromethane (50 mL × 3). The organic phases were combined, washed with saturated brine (10 mL × 2), dried over anhydrous sodium sulfate, filtered, and concentrated under reduced pressure to remove the solvent to give a crude product of **compound 11-D** (300 mg, yellow oil). MS (ESI): *m*/*z* 220.1 $[M+1]^+$

**Step 4: synthesis of compound 11-E**

**Compound 1E1** (214 mg, 0.68 mmol) was dissolved in dichloromethane (5.0 mL), and then oxalyl chloride (263 mg, 2.04 mmol) was added dropwise. Then, the mixture was stirred at room temperature for 30 min. After the reaction was completed as detected by TLC, the reaction liquid was concentrated to dryness by rotary evaporation, and then the residue was dissolved in anhydrous tetrahydrofuran (10 mL). **Compound 11-D** (150 mg, 0.68 mmol) and *N,N*-diisopropylethylenediamine (268 mg, 2.04 mmol) were sequentially added at room temperature, and then the reaction liquid was stirred at 50 °C for 1 h. After the reaction was completed as detected by TLC, the reaction liquid was diluted with water (40 mL) and extracted with ethyl acetate (15 mL × 3). The organic phases were combined, washed with saturated brine (10 mL), dried over anhydrous sodium sulfate, filtered, and concentrated under reduced pressure to remove the solvent, and the residue was purified by a silica gel column to give **compound 11-E** (110 mg, yellow solid, yield: 34.3%). MS (ESI): *m*/*z* 511.1 $[M+1]^+$

**Step 5: synthesis of compound 11**

**Compound 11-E** (100 mg, 0.20 mmol) and **2-hydroxyethane-1-sulfonamide** (30 mg, 0.24 mmol) were dissolved in *N,N*-dimethylformamide (3 mL), and then cuprous iodide (4 mg, 0.02 mmol), potassium phosphate (84 mg, 0.40 mmol), and (1R,2R)-(-)-N,N'-dimethyl-1,2-cyclohexanediamine (6 mg, 0.04 mmol) were sequentially added. Then, the mixture was stirred at 120 °C for 3 h under a nitrogen atmosphere. After the reaction was completed as detected by TLC, the reaction liquid was diluted with water (20 mL) and extracted with ethyl acetate (15 mL × 3). The organic phases were combined, washed with saturated brine (8 mL × 3), dried over anhydrous sodium sulfate, filtered, and concentrated under reduced pressure to remove the solvent, and the residue was purified by preparative HPLC to give **compound 11** (15 mg, yellow solid). MS (ESI): *m*/*z* 556.2 $[M+1]^+$.

$^1H$ NMR (300 MHz, DMSO-$d_6$) δ(ppm) 12.95 (s, 1H), 10.19 (s, 1H), 8.79 (s, 1H), 8.12 - 8.03 (m, 2H), 7.52 (d, *J* = 1.1 Hz, 1H), 7.27 (d, *J* = 2.1 Hz, 1H), 7.13 (dd, *J* = 8.6, 2.2 Hz, 1H), 4.92 (s, 1H), 4.29 - 4.23 (m, 4H), 3.79 - 3.76 (m, 4H), 3.34 (t, *J* = 6.4 Hz, 2H), 3.16 (t, *J* = 9.3, 4.4 Hz, 2H), 3.00 - 2.94 (m, 4H), 1.83 - 1.61 (m, 3H), 1.44 - 1.39 (m, 1H), 0.38 (s, 4H).

**Example 12: Preparation of Compound 12**

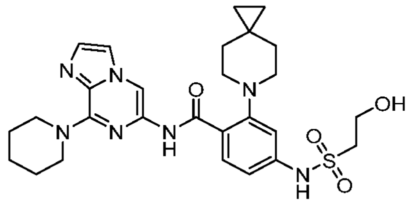

**12**

**Synthetic route:**

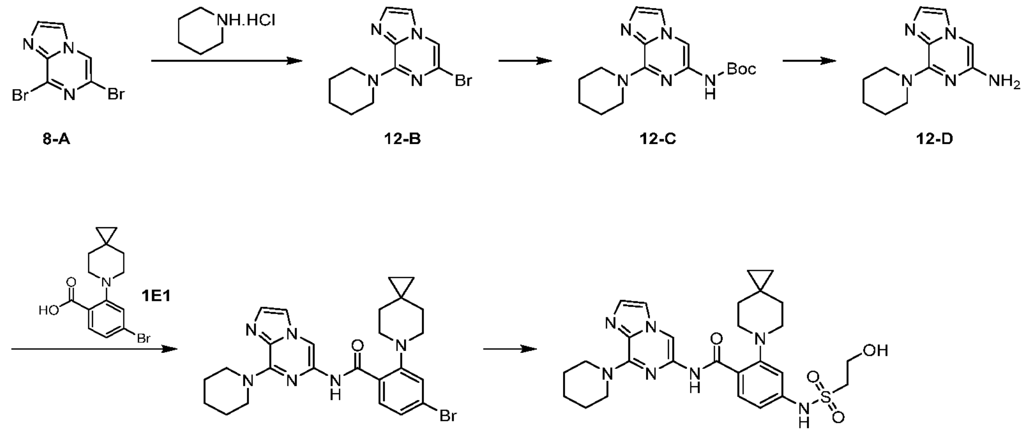


8-A 12-B 12-C 12-D

12-E 12

**Preparation method:**

**Step 1: synthesis of compound 12-B**

**Compound 8-A** (500 mg, 1.81 mmol) was dissolved in N-methylpyrrolidone (30 mL), and then N,N-diisopropylethylenediamine (938 mg, 13.0 mmol) and **piperidine hydrochloride** (308 mg, 3.62 mmol) were added. Then, the mixture was stirred at 100 °C for 4 h under a nitrogen atmosphere. After the reaction was completed as detected by TLC, the reaction liquid was cooled to room temperature, diluted with water (50 mL), and extracted with ethyl acetate (30 mL × 3). The organic phases were combined, washed with saturated brine (20 mL × 3), dried over anhydrous sodium sulfate, filtered, and concentrated under reduced pressure to remove the solvent, and the residue was purified by silica gel column chromatography to give **compound 12-B** (400 mg, yellow oil, yield: 78.8%). MS (ESI): *m/z* 281.0 $[M+1]^+$

**Step 2: synthesis of compound 12-C**

**Compound 12-B** (400 mg, 1.42 mmol) was dissolved in dioxane (5.0 mL), and then *tert-butyl* carbamate (336 mg, 2.84 mmol), 2-dicyclohexylphosphino-2',4',6'-triisopropylbiphenyl (145 mg, 0.28 mmol), cesium carbonate (932 mg, 2.84 mmol), and palladium acetate (32 mg, 0.14 mmol) were added. Then, the mixture was stirred at 100 °C for 16 h under a nitrogen atmosphere. After the reaction was completed as detected by TLC, the reaction liquid was cooled to room temperature, diluted with water (100 mL), and extracted with ethyl acetate (50.0 mL × 2). The organic phases were combined, washed with saturated brine (30 mL × 2), dried over anhydrous sodium sulfate, filtered, and concentrated under reduced pressure to remove the solvent, and the residue was purified by silica gel column chromatography and concentrated to dryness by rotary evaporation under reduced pressure to give **compound 12-C** (200 mg, yellow solid, yield: 44.3%). MS (ESI): *m/z* 318.2 $[M+1]^+$

**Step 3: synthesis of compound 12-D**

**Compound 12-C** (200.0 mg, 0.63 mmol) was dissolved in anhydrous dichloromethane (5.0 mL), and then 2,6-lutidine (203 mg, 1.89 mmol) and trimethylsilyl trifluoromethanesulfonate (420 mg, 1.89 mmol) were sequentially added at room temperature. Then, the mixture was stirred at room temperature for 1 h. After the reaction was completed as detected by TLC, the reaction liquid was diluted with ice water (20 mL), adjusted to pH = 7-8 with a saturated sodium bicarbonate solution, and then extracted with dichloromethane (20 mL × 3). The organic phases were combined, washed with saturated brine (10 mL × 2), dried over anhydrous sodium sulfate, filtered, and concentrated under reduced pressure to remove the solvent to give a crude product of **compound 12-D** (150 mg, yellow oil). MS (ESI): *m/z* 218.1 $[M+1]^+$

**Step 4: synthesis of compound 12-E**

**Compound 1E1** (214 mg, 0.69 mmol) was dissolved in dichloromethane (5 mL), and then oxalyl chloride (263 mg, 2.07 mmol) was added dropwise under an ice bath. Then, the mixture was stirred at room temperature for 30 min. After the reaction was completed as detected by TLC, the reaction liquid was concentrated to dryness by rotary evaporation, and then the residue was dissolved in anhydrous tetrahydrofuran (10 mL). **Compound 12-D** (150 mg, 0.69 mmol) and N,N-diisopropylethylenediamine (268 mg, 2.07 mmol) were sequentially added at room temperature, and then the reaction liquid was stirred at 50 °C for 1 h. After the reaction was completed as detected by TLC, the reaction liquid was diluted with water (40 mL) and extracted with ethyl acetate (15 mL × 3). The organic phases were combined, washed with saturated brine (10 mL), dried over anhydrous sodium sulfate, filtered, and concentrated under reduced pressure to remove the solvent, and the residue was purified by a silica gel column to give **compound 12-E** (110 mg, yellow solid, yield: 31.3%). MS (ESI): *m/z* 509.2 $[M+1]^+$

**Step 5: synthesis of compound 12**

**Compound 12-E** (100 mg, 0.20 mmol) and **2-hydroxyethane-1-sulfonamide** (30 mg, 0.24 mmol) were dissolved in *N,N*-dimethylformamide (3 mL), and then cuprous iodide (4 mg, 0.02 mmol), potassium phosphate (84 mg, 0.40 mmol), and (1*R*,2*R*)-(-)-*N,N'*-dimethyl-1,2-cyclohexanediamine (6 mg, 0.04 mmol) were sequentially added. Then, the mixture was stirred at 120 °C for 3 h under a nitrogen atmosphere. After the reaction was completed as detected by TLC, the reaction liquid was diluted with water (20 mL) and extracted with ethyl acetate (15 mL × 3). The organic phases were combined, washed with saturated brine (8 mL), dried over anhydrous sodium sulfate, filtered, and concentrated under reduced pressure to remove the solvent, and the residue was purified by preparative HPLC to give **compound 12** (28 mg, off-white solid). MS (ESI): *m/z* 554.3 $[M+1]^+$.

$^1$H NMR (300 MHz, DMSO-$d_6$) δ(ppm) 12.80 (s, 1H), 10.15 (br.s, 1H), 8.72 (s, 1H), 8.07 (d, *J* = 8.6 Hz, 1H), 8.00 (d, *J* = 1.1 Hz, 1H), 7.49 (d, *J* = 1.0 Hz, 1H), 7.27 (d, *J* = 2.1 Hz, 1H), 7.12 (dd, *J* = 8.6, 2.1 Hz, 1H), 4.94 (br.s, 1H), 4.34 - 4.21 (m, 4H), 3.75 (t, *J* = 6.5 Hz, 2H), 3.34 (t, *J* = 6.5 Hz, 2H), 3.02 - 2.93 (m, 4H), 1.83 - 1.58 (m, 10H), 0.38 (s, 4H).

**Example 13: Preparation of Compound 13**

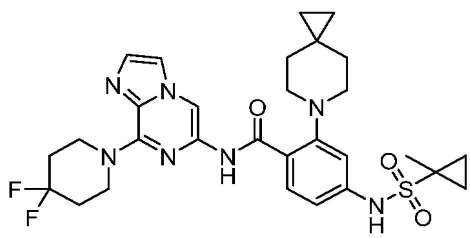

**13**

**Synthetic route:**

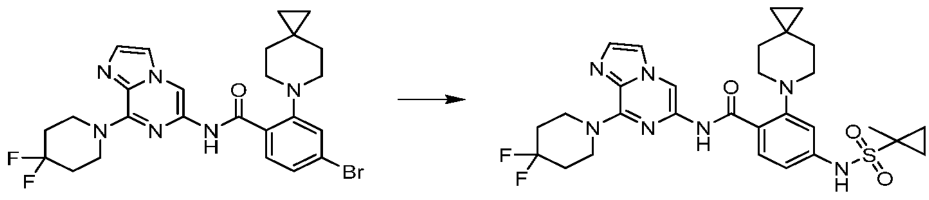

8-E 13

**Preparation method:**

**Step 1: synthesis of compound 13**

**Compound 8-E** (70 mg, 0.128 mmol) and **1-methylcyclopropane-1-sulfonamide** (23 mg, 0.384 mmol) were dissolved in *N,N*-dimethylformamide (1 mL), and then cuprous iodide (15 mg, 0.077 mmol), potassium phosphate (77 mg, 0.371 mmol), and (1*R*,2*R*)-(-)-*N,N'*-dimethyl-1,2-cyclohexanediamine (15 mg, 0.110 mmol) were sequentially added. Then, the reaction liquid was purged three times with nitrogen and stirred at 100 °C for 16 h. After the reaction was completed as detected by LCMS, the reaction liquid was cooled to room temperature, diluted with water (10 mL), and then extracted with ethyl acetate (10 mL × 3). The organic phases were combined, washed with saturated brine (20 mL × 3), dried over anhydrous sodium sulfate, filtered, and concentrated under reduced pressure to remove the solvent, and the residue was purified by preparative HPLC to give compound **13** (25 mg, off-white solid, 32.59%). MS (ESI): *m/z* 600.3 $[M+1]^+$.

$^1$H NMR (300 MHz, DMSO-$d_6$) δ(ppm) 12.92 (s, 1H), 10.25 (br.s, 1H), 8.84 (s, 1H), 8.12 - 8.00 (m, 2H), 7.55 (d, *J* = 1.1 Hz, 1H), 7.36 (d, *J* = 2.1 Hz, 1H), 7.17 (dd, *J* = 8.6, 2.1 Hz, 1H), 4.48 - 4.39 (m, 4H), 3.01 - 2.92 (m, 4H), 2.22 - 2.01 (m, 5H), 1.90 - 1.61 (m, 3H), 1.40 (s, 3H), 1.25 - 1.17 (m, 2H), 0.88 - 0.79 (m, 2H), 0.41 (s, 4H).

**Example 14: Preparation of Compound 14**

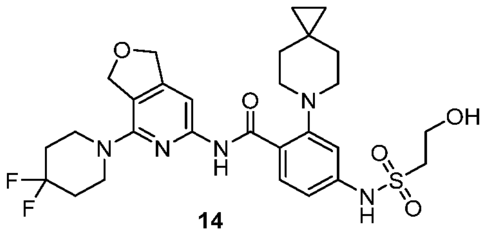

14

**Synthetic route:**

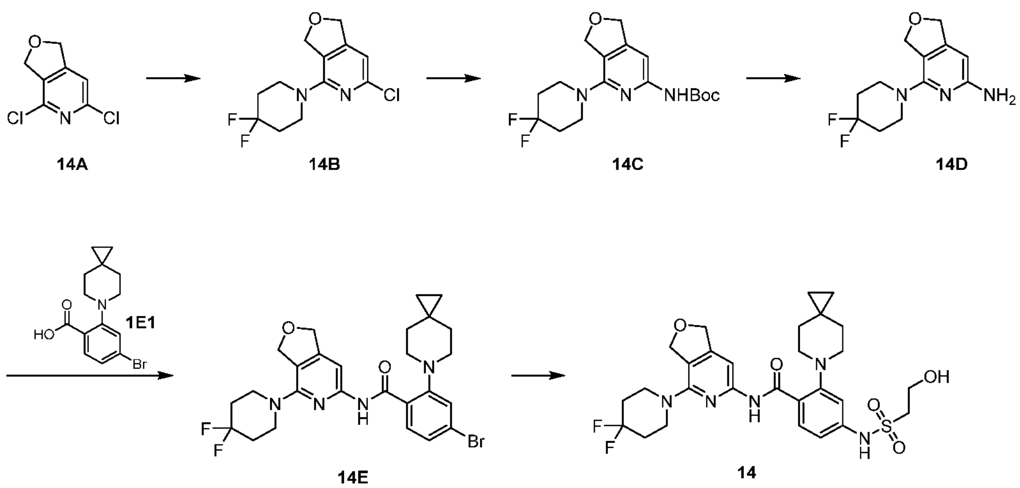

14A 14B 14C 14D

1E1

14E 14

**Preparation method:**

**Step 1: synthesis of compound 14B**

**Compound 14A** (900 mg, 4.74 mmol) was dissolved in N-methylpyrrolidone (10 mL), and then N,N-diisopropylethylenediamine (1.2 g, 9.48 mmol) and **4,4-difluoropiperidine hydrochloride** (900 mg, 5.69 mmol) were added. Then, the mixture was stirred at 120 °C for 16 h under a nitrogen atmosphere. After the reaction was completed as detected by TLC, the reaction liquid was cooled to room temperature, diluted with water (100 mL), and extracted with ethyl acetate (80 mL × 3). The organic phases were combined, washed with saturated brine (50 mL × 3), dried over anhydrous sodium sulfate, filtered, and concentrated under reduced pressure to remove the solvent, and the residue was purified by silica gel column chromatography to give **compound 14B** (1.0 g, yellow solid, yield: 76.9%). MS (ESI): *m/z* 275.1 $[M+1]^+$

**Step 2: synthesis of compound 14C**

**Compound 14B** (1.0 g, 3.64 mmol) was dissolved in dioxane (10.0 mL), and then ***tert-butyl* carbamate** (854 mg, 7.28 mmol), 2-dicyclohexylphosphino-2',4',6'-triisopropylbiphenyl (348 mg, 0.73 mmol), cesium carbonate (2.37 mg, 7.28 mmol), and palladium acetate (81 mg, 0.36 mmol) were added. Then, the mixture was allowed to react at 100 °C for 16 h under a nitrogen atmosphere. After the reaction was completed as detected by TLC, the reaction liquid was cooled to room temperature, diluted with water (50 mL), and extracted with ethyl acetate (50 mL × 3). The organic phases were combined, washed with saturated brine (30 mL × 2), dried over anhydrous sodium sulfate, filtered, and concentrated under reduced pressure to remove the solvent, and the residue was purified by silica gel column chromatography to give **compound 14C** (1.0 g, yellow solid, yield: 77.3%). MS (ESI): *m/z* 356.2 $[M+1]^+$

**Step 3: synthesis of compound 14D**

**Compound 14C** (100 mg, 0.28 mmol) was dissolved in a solution of hydrochloric acid in ethyl acetate (2 mL, 8%), and then the mixture was stirred at room temperature for 16 h. After the reaction was completed as detected by TLC, the reaction liquid was concentrated under reduced pressure to remove the solvent to give a crude product of **compound 14D** (100 mg). MS (ESI): *m/z* 256.1 $[M+1]^+$

**Step 4: synthesis of compound 14E**

**Compound 1E1** (87 mg, 0.28 mmol) was dissolved in dichloromethane (2 mL), and then oxalyl chloride (90 mg, 0.70 mmol) was added dropwise under an ice bath. Then, the mixture was stirred at room temperature for 30 min. After the reaction was completed as detected by TLC, the reaction liquid was concentrated to dryness by rotary evaporation. The residue was dissolved in anhydrous tetrahydrofuran (10 mL), and then **compound 14D** (72 mg, 0.28 mmol) and N,N-diisopropylethylenediamine (146 mg, 1.12 mmol) were sequentially added at room temperature. Then, the mixture was stirred at 50 °C for 1 h. After the reaction was completed as detected by TLC, the reaction liquid was diluted with water (20 mL) and extracted with ethyl acetate (15 mL × 3). The organic phases were combined, washed with saturated brine (10 mL), dried over anhydrous sodium sulfate, filtered, and concentrated under reduced pressure to remove the solvent, and the residue was purified by a silica gel column to give **compound 14E** (80 mg, yellow solid, yield: 51.8%). MS (ESI): *m/z* 547.1 $[M+1]^+$

**Step 5: synthesis of compound 14**

**Compound 14E** (70 mg, 0.13 mmol) and **2-hydroxyethane-1-sulfonamide** (20 mg, 0.16 mmol) were dissolved in *N,N*-dimethylformamide (1.5 mL), and then cuprous iodide (3 mg, 0.01 mmol), potassium phosphate (56 mg, 0.26 mmol), and (1*R*,2*R*)-(-)-*N,N'*-dimethyl-1,2-cyclohexanediamine (4 mg, 0.03 mmol) were sequentially added. Then, the mixture was stirred at 120 °C for 3 h under a nitrogen atmosphere. After the reaction was completed as detected by TLC, the reaction liquid was diluted with water (10 mL) and extracted with ethyl acetate (8 mL × 3). The organic phases were combined, washed with saturated brine (5 mL × 2), dried over anhydrous sodium sulfate, filtered, and concentrated under reduced pressure to remove the solvent to give compound 14 (14 mg, off-white solid). MS (ESI): *m/z* 592.2 $[M+1]^+$.

$^1H$ NMR (300 MHz, DMSO-$d_6$) δ 12.86 (s, 1H), 10.21 (br.s, 1H), 8.05 (d, *J* = 8.6 Hz, 1H), 7.69 (s, 1H), 7.25 (d, *J* = 2.2 Hz, 1H), 7.11 (dd, *J* = 8.6, 2.1 Hz, 1H), 5.08 - 5.05 (m, 2H), 4.98 - 4.89 (m, 3H), 3.75 (t, *J* = 6.5 Hz, 2H), 3.60 - 3.54 (m, 4H), 3.35 (t, *J* = 6.5 Hz, 2H), 3.00 - 2.94 (m, 4H), 2.10 - 1.96 (m, 5H), 1.81 - 1.56 (m, 3H), 0.38 (s, 4H).

**Example 15: Preparation of Compound 15**

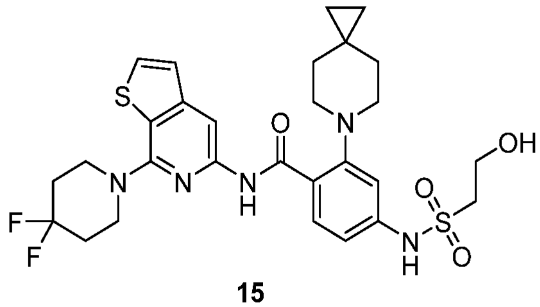

**15**

**Synthetic route:**

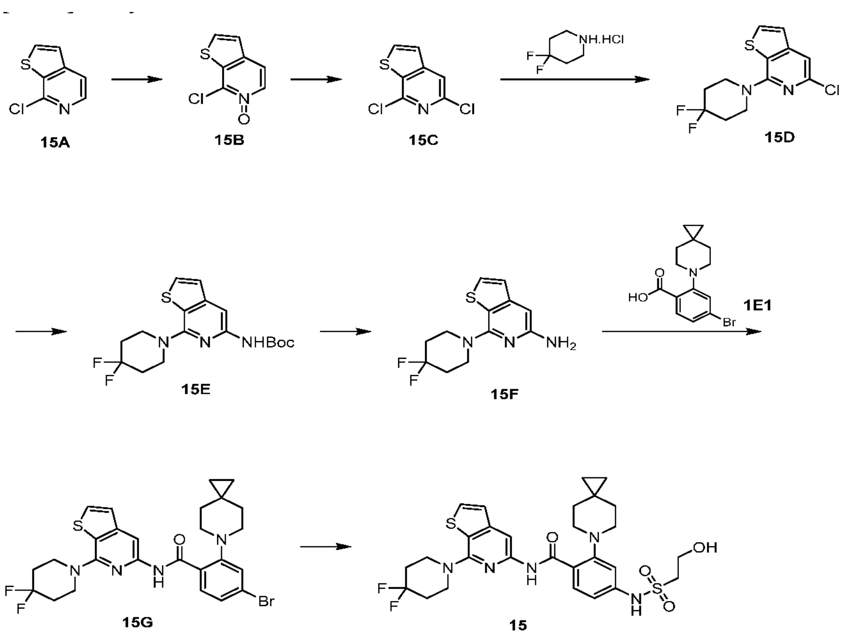

15A 15B 15C 15D 15E 15F 1E1 15G 15

**Preparation method:**

**Step 1: synthesis of compound 15B**

**Compound 15A** (900 mg, 5.31 mmol) was dissolved in dichloromethane (10 mL), and then m-chloroperoxybenzoic acid (1.8 g, 10.6 mmol) was added. Then, the mixture was stirred at room temperature for 12 h. After the reaction was completed as detected by TLC, the reaction liquid was concentrated under reduced pressure to remove the solvent, and the residue was purified by silica gel column chromatography to give a crude product of **compound 15B** (1.15 g, yellow solid). MS (ESI): *m/z* 186.0 [M+1]$^+$

**Step 2: synthesis of compound 15C**

The crude product of **compound 15B** (1.15 g) was dissolved in phosphorus oxychloride (7 mL), and then the mixture was stirred at 115 °C for 12 h. After the reaction was completed as detected by TLC, the reaction liquid was concentrated under reduced pressure to remove the solvent, and the residue was dissolved in dichloromethane (50 mL), then adjusted to pH = 8 with a saturated sodium bicarbonate solution, and extracted with dichloromethane (30 mL × 3). The organic phases were combined, washed with saturated brine (20 mL), dried over anhydrous sodium sulfate, filtered, and concentrated under reduced pressure to remove the solvent, and the residue was purified by silica gel column

chromatography to give **compound 15C** (350 mg, yellow solid). MS (ESI): *m/z* 203.9 $[M+1]^+$

**Step 3: synthesis of compound 15D**

**Compound 15C** (350 mg, 1.72 mmol) was dissolved in N-methylpyrrolidone (5 mL), and then potassium carbonate (711 mg, 5.15 mmol) and **4,4-difluoropiperidine hydrochloride** (406 mg, 2.57 mmol) were added. Then, the mixture was stirred at 120 °C for 12 h. After the reaction was completed as detected by TLC, the reaction liquid was cooled to room temperature, then diluted with water (20 mL), and extracted with ethyl acetate (20 mL × 3). The organic phases were combined, washed with saturated brine (20 mL × 3), dried over anhydrous sodium sulfate, filtered, and concentrated under reduced pressure to remove the solvent, and the residue was purified by silica gel column chromatography to give **compound 15D** (380 mg, yellow liquid, yield: 76.5%). MS (ESI): *m/z* 289.0 $[M+1]^+$

**Step 4: synthesis of compound 15E**

**Compound 15D** (350 mg, 1.21 mmol) was dissolved in dioxane (5.0 mL), and then ***tert-butyl*** **carbamate** (284 mg, 2.42 mmol), 2-dicyclohexylphosphino-2',4',6'-triisopropylbiphenyl (116 mg, 0.24 mmol), cesium carbonate (792 mg, 2.42 mmol), and palladium acetate (27 mg, 0.12 mmol) were added. Then, the mixture was stirred at 110 °C for 16 h under a nitrogen atmosphere. After the reaction was completed as detected by TLC, the reaction liquid was cooled to room temperature, diluted with water (20 mL), and extracted with ethyl acetate (30 mL × 2). The organic phases were combined, washed with saturated brine (20 mL × 2), dried over anhydrous sodium sulfate, filtered, and concentrated under reduced pressure to remove the solvent, and the residue was purified by silica gel column chromatography to give **compound 15E** (400 mg, yellow solid, yield: 89.3%). MS (ESI): *m/z* 370.1 $[M+1]^+$

**Step 5: synthesis of compound 15F**

**Compound 15E** (100 mg, 0.27 mmol) was dissolved in a solution of hydrochloric acid in ethyl acetate (2.0 mL, 8%), and the mixture was stirred at room temperature for 2 h. After the reaction was completed as detected by TLC, the mixture was concentrated under reduced pressure to remove the solvent to give a crude product of **compound 15F** (100 mg, yellow oil). MS (ESI): *m/z* 270.1 $[M+1]^+$

**Step 6: synthesis of compound 15G**

**Compound 1E1** (84 mg, 0.27 mmol) was dissolved in dichloromethane (2.0 mL), and then oxalyl chloride (86 mg, 0.675 mmol) was added dropwise. Then, the mixture was stirred at room temperature for 30 min. After the reaction was completed as detected by TLC, the reaction liquid was concentrated to dryness by rotary evaporation, and the residue was dissolved in anhydrous tetrahydrofuran (5 mL). **Compound 15F** (73.0 mg, 0.27 mmol) and N,N-diisopropylethylenediamine (105 mg, 0.81 mmol) were sequentially added at room temperature, and then the mixture was stirred at 50 °C for 1 h. After the reaction was completed as detected by TLC, the reaction liquid was diluted with water (20 mL) and extracted with ethyl acetate (15 mL × 3). The organic phases were combined, washed with saturated brine (10 mL), dried over anhydrous sodium sulfate, filtered, and concentrated under reduced pressure to remove the solvent, and the residue was purified by a silica gel column to give **compound 15G** (110 mg, yellow solid, yield: 72.3%). MS (ESI): *m/z* 561.1 $[M+1]^+$

**Step 7: synthesis of compound 15**

**Compound 15G** (50.0 mg, 0.09 mmol) and **2-hydroxyethane-1-sulfonamide** (14 mg, 0.18 mmol) were dissolved in *N,N*-dimethylformamide (3 mL), and then cuprous iodide (2 mg, 0.01 mmol), potassium phosphate (38 mg, 0.18 mmol), and (1*R*,2*R*)-(-)-*N,N'*-dimethyl-1,2-cyclohexanediamine (2.5 mg, 0.02 mmol) were sequentially added. Then, the mixture was stirred at 120 °C for 3 h under a nitrogen atmosphere. After the reaction was completed as detected by TLC, the reaction liquid was diluted with water (20 mL) and extracted with ethyl acetate (15 mL × 3). The organic phases were combined, washed with saturated brine (8 mL), dried over anhydrous sodium sulfate, filtered, and concentrated under reduced pressure to remove the solvent, and the residue was purified by HPLC to give **compound 15** (38 mg, yellow solid). MS (ESI): *m/z* 606.2 $[M+1]^+$.

$^1$H NMR (300 MHz, DMSO-$d_6$) δ(ppm) 12.91 (s, 1H), 10.20 (br.s, 1H), 8.29 (s, 1H), 8.08 (d, *J* = 8.6 Hz, 1H), 8.00 (d, *J* = 5.4 Hz, 1H), 7.49 (d, *J* = 5.5 Hz, 1H), 7.26 (d, *J* = 2.1 Hz, 1H), 7.12 (dd, *J* = 8.6, 2.1 Hz, 1H), 4.95 (br.s, 1H), 3.83 - 3.74 (m, 6H), 3.35 (t, *J* = 6.4 Hz, 2H), 3.02 - 2.94 (m, 4H), 2.24 - 2.05 (m, 5H), 1.89 - 1.60 (m, 3H), 0.38 (s, 4H).

**Example 16: Preparation of Compound 16**

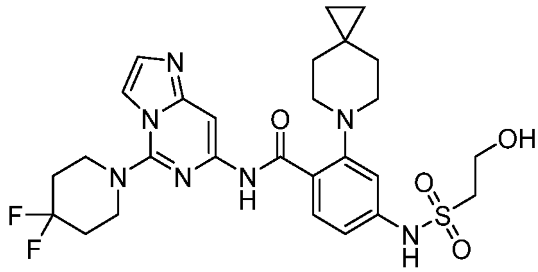

**16**

**Synthetic route:**

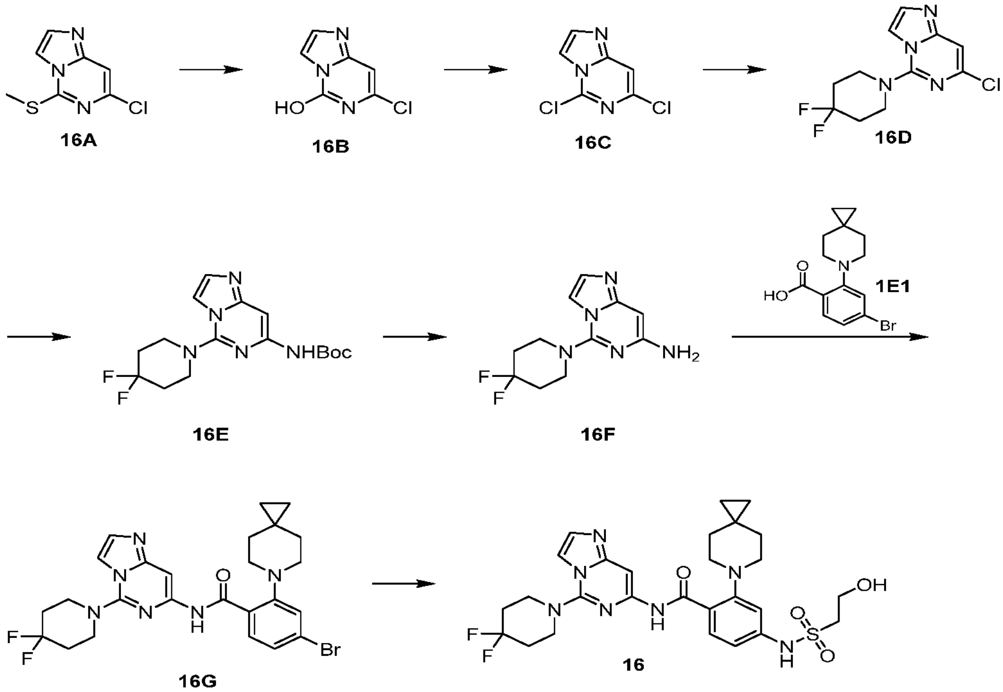

**Preparation method:**

**Step 1: synthesis of compound 16B**

**Compound 16A** (5.0 g, 25.1 mmol) was dissolved in methanol (25 mL), and then a potassium hydroxide solution (50 mL, 2 mol/L) was added. Then, the mixture was stirred at 70 °C for 2 h. After the reaction was completed as detected by TLC, the mixture was cooled to room temperature and concentrated under reduced pressure to remove the solvent, and the residue was purified by a silica gel column to give **compound 16B** (3.0 g, yellow solid, yield: 70.5%). MS (ESI): *m/z* 170.0 $[M+1]^+$

**Step 2: synthesis of compound 16C**

**Compound 16B** (3.0 g, 17.7 mmol) was dissolved in phosphorus oxychloride (30 mL), and then the mixture was stirred at 120 °C for 16 h. After the reaction was completed as detected by TLC, the mixture was cooled to room temperature and concentrated under reduced pressure to remove the solvent, and the residue was adjusted to pH = 8 with

a saturated sodium bicarbonate solution and then extracted with ethyl acetate (50 mL × 3). The organic phases were combined, washed with saturated brine (30 mL), dried over anhydrous sodium sulfate, filtered, and concentrated under reduced pressure to remove the solvent, and the residue was purified by a silica gel column to give **compound 16C** (3.0 g, yellow solid, yield: 90.4%). MS (ESI): *m*/*z* 188.0 $[M+1]^+$

**Step 3: synthesis of compound 16D**

**Compound 16C** (3.0 g, 16.0 mmol) was dissolved in N-methylpyrrolidone (30 mL), and then *N,N*-diisopropylethylamine (6.2 g, 48.0 mmol) and **4,4-difluoropiperidine hydrochloride** (3.3 g, 20.8 mmol) were added. Then, the mixture was stirred at 100 °C for 3 h. After the reaction was completed as detected by TLC, the reaction liquid was cooled to room temperature, poured into ice water (50 mL), and then extracted with ethyl acetate (50 mL × 3). The organic phases were combined, washed with saturated brine (30 mL × 3), dried over anhydrous sodium sulfate, filtered, and concentrated under reduced pressure to remove the solvent, and the residue was purified by a silica gel column to give **compound 16D** (4.0 g, yellow oil, yield: 91.8%). MS (ESI): *m*/*z* 273.1 $[M+1]^+$

**Step 4: synthesis of compound 16E**

**Compound 16D** (200 mg, 0.74 mmol) was dissolved in dioxane (3 mL), and then ***tert*-butyl carbamate** (130 mg, 1.11 mmol), cesium carbonate (479 mg, 1.48 mmol), 2-dicyclohexylphosphino-2',4',6'-triisopropylbiphenyl (35 mg, 0.07 mmol), and tris(dibenzylideneacetone)dipalladium (34 mg, 0.04 mmol) were added. Then, the mixture was stirred at 100 °C for 4 h under a nitrogen atmosphere. After the reaction was completed as detected by TLC, the mixture was cooled to room temperature, poured into ice water (10 mL), and extracted with ethyl acetate (10 mL × 3). The organic phases were combined, washed with saturated brine (15 mL), dried over anhydrous sodium sulfate, filtered, and concentrated under reduced pressure to remove the solvent, and the residue was purified by a silica gel column to give **compound 16E** (150 mg, yellow oil, yield: 56.8%). MS (ESI): *m*/*z* 354.2 $[M+1]^+$

**Step 5: synthesis of compound 16F**

**Compound 16E** (700 mg, 1.98 mmol) was dissolved in a solution of hydrochloric acid in ethyl acetate (10 mL, 8%), and then the mixture was stirred at room temperature for 2 h. After the reaction was completed as detected by TLC, the reaction liquid was diluted with water (50 mL), adjusted to pH = 8 by adding aqueous ammonia, and then extracted with ethyl acetate (30 mL × 3). The organic phases were combined, washed with saturated brine (15 mL), dried over anhydrous sodium sulfate, filtered, and concentrated under reduced pressure to remove the solvent to give a crude product of **compound 16F** (400 mg, yellow solid). MS (ESI): *m*/*z* 254.1$[M+1]^+$

**Step 6: synthesis of compound 16G**

**Compound 1E1** (487 mg, 1.58 mmol) was dissolved in dichloromethane (10 mL), and then oxalyl chloride (500 mg, 3.95 mmol) was added dropwise under an ice bath. Then, the mixture was stirred at room temperature for 30 min. After the reaction was completed as detected by TLC, the reaction liquid was concentrated under reduced pressure to remove the solvent. The residue was dissolved in anhydrous tetrahydrofuran (10 mL), and then **compound 16F** (400 mg, 1.58 mmol) and *N,N*-diisopropylethylenediamine (609 mg, 4.74 mmol) were sequentially added at room temperature. Then, the mixture was stirred at 50 °C for 1 h. After the reaction was completed as detected by TLC, the reaction liquid was diluted with water (40 mL) and extracted with ethyl acetate (20 mL × 3). The organic phases were combined, washed with saturated brine (15 mL), dried over anhydrous sodium sulfate, filtered, and concentrated under reduced pressure to remove the solvent, and the residue was purified by a silica gel column to give **compound 16G** (70 mg, yellow solid, yield: 8.1%). MS (ESI): *m*/*z* 545.1 547.1 $[M+1]^+$

**Step 7: synthesis of compound 16**

**Compound 16G** (70 mg, 0.13 mmol) and **2-hydroxyethane-1-sulfonamide** (20 mg, 0.16 mmol) were dissolved in *N,N*-dimethylformamide (2 mL), and then cuprous iodide (3 mg, 0.01 mmol), potassium phosphate (56 mg, 0.26 mmol), and (1*R*,2*R*)-(-)-*N,N'*-dimethyl-1,2-cyclohexanediamine (4 mg, 0.03 mmol) were sequentially added. Then, the mixture was stirred at 120 °C for 2 h under a nitrogen atmosphere. After the reaction was completed as detected by TLC, the reaction liquid was diluted with water (10 mL) and extracted with ethyl acetate (8 mL × 3). The organic phases were combined, washed with saturated brine (8 mL), dried over anhydrous sodium sulfate, filtered, and concentrated under reduced pressure to remove the solvent, and the residue was purified by HPLC to give compound **16** (7 mg, yellow solid). MS (ESI): *m*/*z* 590.2 $[M+1]^+$.

$^1$H NMR (300 MHz, DMSO-$d_6$) δ(ppm) 13.30 (s, 1H), 10.24 (br.s, 1H), 8.16 - 7.74 (m, 3H), 7.67 - 7.48 (m, 1H), 7.26 (s, 1H), 7.12 (d, $J$ = 8.6 Hz, 1H), 5.05 (br.s, 1H), 3.75 (t, $J$ = 6.5 Hz, 2H), 3.61 - 3.54 (m, 4H), 3.34 (t, $J$ = 6.5 Hz, 2H), 3.00 - 2.93 (m, 4H), 2.33 - 2.16 (m, 5H), 1.85 - 1.60 (m, 3H), 0.38 (s, 4H).

**Example 17: Preparation of Compound 17**

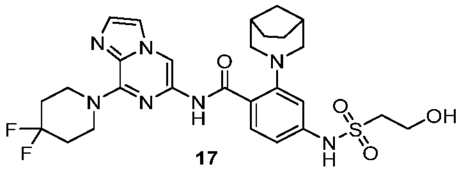

17

**Synthetic route:**

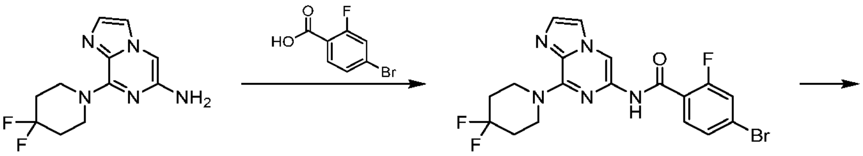

8-D

17B

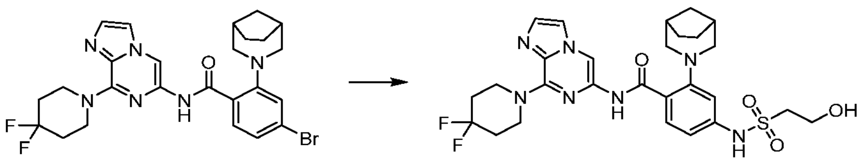

17C

17

**Preparation method:**

**Step 1: synthesis of compound 17B**

**2-Fluoro-4-bromobenzoic acid** (173 mg, 0.79 mmol) was dissolved in thionyl chloride (5 mL), and the mixture was stirred at 80 °C for 2 h. After the reaction was completed as detected by TLC, the reaction liquid was concentrated under reduced pressure to remove the solvent. The residue was dissolved in anhydrous tetrahydrofuran (2 mL), and then **compound 8-D** (200 mg, 0.79 mmol) and *N,N*-diisopropylethylenediamine (408 mg, 3.16 mmol) were sequentially added at room temperature. Then, the mixture was stirred at 50 °C for 2 h. After the reaction was completed as detected by TLC, the reaction liquid was diluted with water (40 mL) and extracted with ethyl acetate (15 mL × 3). The organic phases were combined, washed with saturated brine (10 mL), dried over anhydrous sodium sulfate, filtered, and concentrated under reduced pressure to remove the solvent, and the residue was purified by a silica gel column (petroleum ether/ethyl acetate = 1/0 to 3/1 (volume ratio)) to give **compound 17B** (110 mg, yellow solid, yield: 30.7%). MS (ESI): *m/z* 454.0 [M+1]$^+$

**Step 2: synthesis of compound 17C**

**Compound 17B** (100 mg, 0.22 mmol) was dissolved in N,N-dimethylformamide (2 mL), and then **3-azabicyclo [3.2.1]octane hydrochloride** (38 mg, 0.26 mmol) and potassium carbonate (60 mg, 0.44 mmol) were added. Then, the mixture was stirred at 120 °C for 16 h under a nitrogen atmosphere. After the reaction was completed as detected by TLC, the reaction liquid was diluted with water (20 mL) and extracted with ethyl acetate (15 mL × 3). The organic phases were combined, washed with saturated brine (15 mL × 3), dried over anhydrous sodium sulfate, filtered, and concentrated under reduced pressure to remove the solvent, and the residue was purified by a silica gel column to give **compound 17C**

(70 mg, yellow solid, yield: 66.2%). MS (ESI): *m/z* 545.1 $[M+1]^+$

**Step 3: synthesis of compound 17**

**Compound 17C** (70 mg, 0.13 mmol) and **2-hydroxyethane-1-sulfonamide** (20 mg, 0.16 mmol) were dissolved in *N,N*-dimethylformamide (2 mL), and then cuprous iodide (3 mg, 0.01 mmol), potassium phosphate (56 mg, 0.26 mmol), and (1*R*,2*R*)-(-)-*N,N'*-dimethyl-1,2-cyclohexanediamine (4 mg, 0.03 mmol) were sequentially added. Then, the mixture was stirred at 120 °C for 2 h under a nitrogen atmosphere. After the reaction was completed as detected by TLC, the reaction liquid was diluted with water (10 mL) and extracted with ethyl acetate (8 mL × 3). The organic phases were combined, washed with saturated brine (8 mL), dried over anhydrous sodium sulfate, filtered, and concentrated under reduced pressure to remove the solvent, and the residue was purified by HPLC to give compound **17** (16 mg, yellow solid). MS (ESI): *m/z* 590.2 $[M+1]^+$.

$^1H$ NMR (300 MHz, DMSO-$d_6$) δ(ppm) 10.54 (s, 1H), 10.04 (br.s, 1H), 8.89 (s, 1H), 8.09 (d, *J* = 1.1 Hz, 1H), 7.70 (d, *J* = 8.4 Hz, 1H), 7.55 (d, *J* = 1.1 Hz, 1H), 7.16 (d, *J* = *2.1* Hz, 1H), 7.00 (dd, *J* = 8.5, 2.0 Hz, 1H), 4.95 (br.s, 1H), 4.44 - 4.36 (m, 4H), 3.75 (t, *J* = 6.6 Hz, 2H), 3.31 (t, *J* = 6.6 Hz, 2H), 3.06 - 2.95 (m, 2H), 2.80 (d, *J* = 10.5 Hz, 2H), 2.29 - 2.21 (m, 2H), 2.14 - 1.95 (m, 6H), 1.63 - 1.47 (m, 4H).

**Example 18: Preparation of Compound 18**

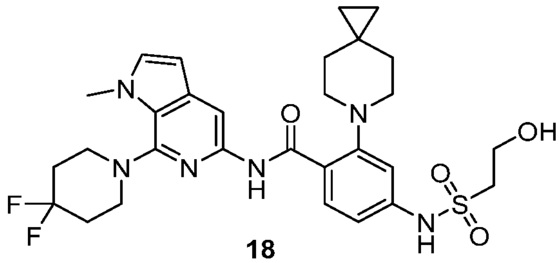

18

**Synthetic route:**

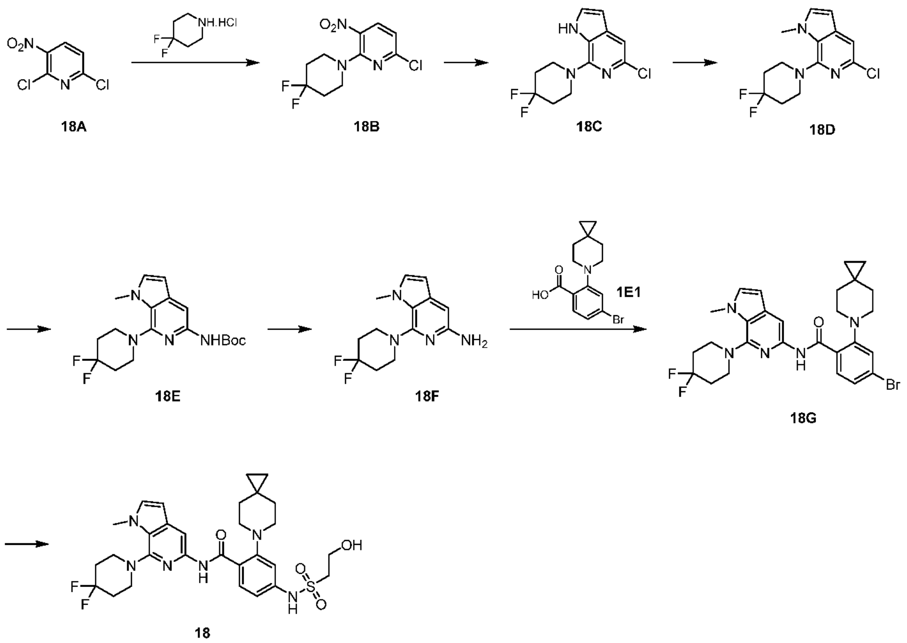


18A 18B 18C 18D

18E 18F 18G

18

**Preparation method:**

**Step 1: synthesis** of **compound 18B**

**Compound 18A** (5 g, 25.9 mmol) was dissolved in isopropanol (50 mL), and then sodium carbonate (6.84 g, 64.8 mmol) and **4,4-difluoropiperidine hydrochloride** (4.09 g, 25.9 mmol) were sequentially added under an ice bath. Then, the mixture was stirred at room temperature for 12 h. After the reaction was completed as detected by TLC, the reaction liquid was diluted with water (50 mL) and then extracted with ethyl acetate (100 mL × 3). The organic phases were combined, washed with saturated brine (50 mL), dried over anhydrous sodium sulfate, filtered, and concentrated under reduced pressure to remove the solvent, and the residue was purified by a silica gel column (petroleum ether/ethyl acetate = 1/0 to 5/1 (volume ratio)) to give **compound 18B** (4.4 g, yellow solid, yield: 61.2%). MS (ESI): *m/z* 278.0 $[M+1]^+$

**Step 2: synthesis of compound 18C**

**Compound 18B** (4.4 g, 15.8 mmol) was dissolved in anhydrous tetrahydrofuran (50 mL), and then a **vinylmagnesium bromide solution** (55.3 mL, 55.3 mmol) was added dropwise to the reaction liquid at - 70 °C under a nitrogen atmosphere. Then, the mixture was naturally warmed to room temperature over one hour. After the reaction was completed as detected by TLC, the reaction liquid was slowly poured into saturated ammonium chloride (100 mL) under an ice bath and then extracted with ethyl acetate (100 mL × 3). The organic phases were combined, washed with saturated brine (50 mL), dried over anhydrous sodium sulfate, filtered, and concentrated under reduced pressure to remove the solvent, and the residue was purified by a silica gel column to give **compound 18C** (1.9 g, yellow oil, yield: 44.3%). MS (ESI): *m/z* 272.1 $[M+1]^+$

**Step 3: synthesis of compound 18D**

**Compound 18C** (1.9 g, 7.0 mmol) was dissolved in anhydrous tetrahydrofuran (50 mL), and then sodium hydride (420 mg, 10.5 mmol) was added under an ice bath. The mixture was stirred for 10 min under the ice bath. Subsequently, **iodomethane** (1.19 g, 8.4 mmol) was added dropwise to the reaction liquid under the ice bath, and then the mixture was stirred at room temperature for 1 h. After the reaction was completed as detected by TLC, the reaction liquid was slowly poured into saturated ammonium chloride (100 mL) under an ice bath and then extracted with ethyl acetate (50 mL × 3). The organic phases were combined, washed with saturated brine (30 mL × 3), dried over anhydrous sodium sulfate, filtered, and concentrated under reduced pressure to remove the solvent, and the residue was purified by a silica gel column to give **compound 18D** (1.2 g, yellow oil, yield: 60%). MS (ESI): *m/z* 286.1 $[M+1]^+$

**Step 4: synthesis of compound 18E**

**Compound 18D** (1.15 g, 4.0 mmol) was dissolved in anhydrous dioxane (50 mL), and then *tert*-**butyl carbamate** (707 mg, 6.0 mmol), cesium carbonate (2.62 g, 8.0 mmol), 2-dicyclohexylphosphino-2',4',6'-triisopropylbiphenyl (192 mg, 0.4 mmol), and tris(dibenzylideneacetone)dipalladium (184 mg, 0.2 mmol) were added. Then, the mixture was stirred at 100 °C for 4 h under a nitrogen atmosphere. After the reaction was completed as detected by TLC, the mixture was cooled to room temperature, diluted with water (30 mL), and extracted with ethyl acetate (50 mL × 3). The organic phases were combined, washed with saturated brine (40 mL), dried over anhydrous sodium sulfate, filtered, and concentrated under reduced pressure to remove the solvent, and the residue was purified by a silica gel column to give **compound 18E** (1.2 g, yellow oil, yield: 81.9%). MS (ESI): *m/z* 367.2 $[M+1]^+$

**Step 5: synthesis of compound 18F**

**Compound 18E** (500 mg, 1.36 mmol) was dissolved in a solution of hydrochloric acid in ethyl acetate (10 mL, 8%), and then the mixture was stirred at room temperature for 2 h. After the reaction was completed as detected by TLC, the reaction liquid was diluted with water (50 mL), and then aqueous ammonia was added until pH = 8. Then, the mixture was extracted with ethyl acetate (30 mL × 3). The organic phases were combined, washed with saturated brine (20 mL), dried over anhydrous sodium sulfate, filtered, and concentrated under reduced pressure to remove the solvent to give a crude product of **compound 18F** (400 mg, yellow solid). MS (ESI): *m/z* 267.1 $[M+1]^+$

**Step 6: synthesis of compound 18G**

**Compound 1E1** (468 mg, 1.50 mmol) was dissolved in dichloromethane (10 mL), and then oxalyl chloride (477 mg, 3.75 mmol) was added dropwise under an ice bath. The mixture was stirred at room temperature for 30 min. After the reaction was completed as detected by TLC, the reaction liquid was concentrated under reduced pressure to remove the solvent. The residue was dissolved in anhydrous tetrahydrofuran (10 mL), and then **compound 18F** (400 mg, 1.50 mmol)

and *N,N*-diisopropylethylenediamine (582 mg, 4.50 mmol) were sequentially added at room temperature. Then, the mixture was stirred at 50 °C for 1 h. After the reaction was completed as detected by TLC, the reaction liquid was diluted with water (40 mL) and extracted with ethyl acetate (20 mL × 3). The organic phases were combined, washed with saturated brine (20 mL), dried over anhydrous sodium sulfate, filtered, and concentrated under reduced pressure to remove the solvent, and the residue was purified by a silica gel column to give **compound 18G** (500 mg, yellow solid, yield: 59.6%). MS (ESI): *m/z* 558.2 $[M+1]^+$

**Step 7: synthesis of compound 18**

**Compound 18G** (100 mg, 0.18 mmol) and **2-hydroxyethane-1-sulfonamide** (27 mg, 0.22 mmol) were dissolved in *N,N*-dimethylformamide (2 mL), and then cuprous iodide (3 mg, 0.02 mmol), potassium phosphate (76 mg, 0.39 mmol), and (1R,2R)-(-)-N,N-dimethyl-1,2-cyclohexanediamine (5 mg, 0.04 mmol) were sequentially added. Then, the mixture was stirred at 120 °C for 2 h under a nitrogen atmosphere. After the reaction was completed as detected by TLC, the reaction liquid was diluted with water (10 mL) and extracted with ethyl acetate (10 mL × 3). The organic phases were combined, washed with saturated brine (8 mL), dried over anhydrous sodium sulfate, filtered, and concentrated under reduced pressure to remove the solvent, and the residue was purified by HPLC to give **compound 18** (7 mg, pale yellow solid). MS (ESI): *m/z* 603.3 $[M+1]^+$.

$^1H$ NMR (300 MHz, DMSO-$d_6$) δ(ppm) 12.87 (s, 1H), 10.14 (br.s, 1H), 8.14 (s, 1H), 8.07 (d, *J* = 8.6 Hz, 1H), 7.44 (d, *J* = 3.0 Hz, 1H), 7.24 (d, *J* = 2.1 Hz, 1H), 7.10 (dd, *J* = 8.6, 2.1 Hz, 1H), 6.45 (d, *J* = 3.0 Hz, 1H), 4.94 (br.s, 1H), 4.03 (s, 3H), 3.74 (t, *J* = 6.5 Hz, 2H), 3.34 (t, *J* = 6.5 Hz, 2H), 3.29 - 3.24 (m, 4H), 3.00 - 2.93 (m, 4H), 2.31 - 2.13 (m, 5H), 1.94 - 1.51 (m, 3H), 0.37 (s, 4H).

**Example** 19: **Preparation of Compound 19**

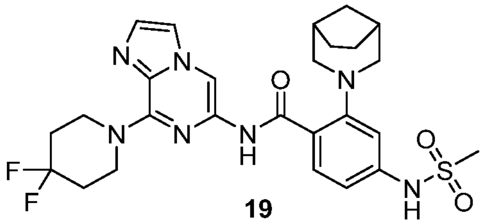

**19**

Synthetic route:

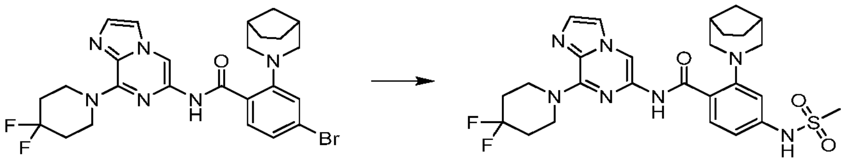

**17C** **19**

**Preparation** method:

**Step 1: synthesis of compound 19**

**Compound 17C** (70 mg, 0.13 mmol) and **methanesulfonamide** (15 mg, 0.38 mmol) were dissolved in *N,N*-dimethylformamide (2 mL), and then cuprous iodide (3 mg, 0.01 mmol), potassium phosphate (55 mg, 0.26 mmol), and (1*R*,2*R*)-(-)-*N,N'*-dimethyl-1,2-cyclohexanediamine (4 mg, 0.03 mmol) were sequentially added. Then, the mixture was stirred at 120 °C for 16 h under a nitrogen atmosphere. After the reaction was completed as detected by TLC, the reaction liquid was diluted with water (20 mL) and extracted with ethyl acetate (10 mL × 3). The organic phases were combined, washed with saturated brine (8 mL), dried over anhydrous sodium sulfate, filtered, and concentrated under reduced pressure to remove the solvent, and the residue was purified by HPLC to give **compound 19** (16 mg, pale yellow solid). MS (ESI): *m/z* 280.7 $[1/2M+1]^+$.

$^1$H NMR (300 MHz, DMSO-$d_6$) δ(ppm) 10.53 (s, 1H), 10.10 (br.s, 1H), 8.89 (s, 1H), 8.09 (s, 1H), 7.71 (d, $J$ = 8.4 Hz, 1H), 7.55 (s, 1H), 7.15 (d, $J$ = 2.1 Hz, 1H), 7.01 (dd, $J$ = 8.5, 2.0 Hz, 1H), 4.44 - 4.34 (m, 4H), 3.07 (s, 3H), 3.05 - 2.95 (m, 2H), 2.86 - 2.77 (m, 2H), 2.28 - 2.21 (m, 2H), 2.17 - 1.95 (m, 6H), 1.66 - 1.44 (m, 4H).

**Example 20: Preparation of Compound 20**

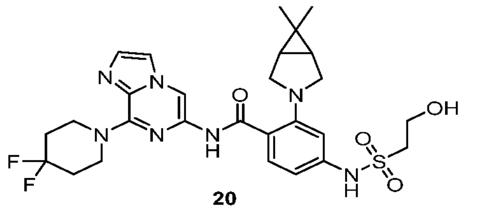

**20**

**Synthetic route:**

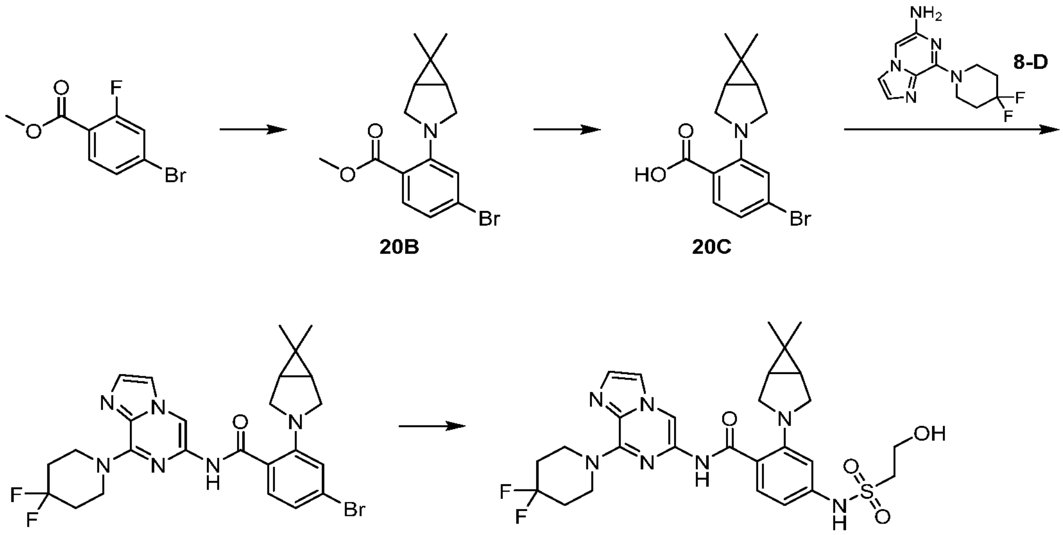

**20B** **20C** **8-D**

**20D** **20**

**Preparation method:**

**Step 1: synthesis of compound 20B**

**Methyl 2-fluoro-4-bromobenzoate** (1.6 g, 6.87 mmol) was dissolved in N,N-dimethylformamide (10 mL), and then 6,6-dimethyl-3-azabicyclo[3.1.0]hexane (940 mg, 8.24 mmol) and potassium carbonate (2.0 g, 13.7 mmol) were added. Then, the mixture was stirred at 120 °C for 16 h under a nitrogen atmosphere. After the reaction was completed as detected by TLC, the reaction liquid was cooled to room temperature, diluted with water (80 mL), and then extracted with ethyl acetate (50 mL × 3). The organic phases were combined, washed with saturated brine (20 mL × 3), dried over anhydrous sodium sulfate, filtered, and concentrated under reduced pressure to remove the solvent, and the residue was purified by silica gel column chromatography to give **compound 20B** (2.1 g, yellow oil, yield: 94.3%). MS (ESI): $m/z$ 324.0 $[M+1]^+$

**Step 2: synthesis of compound 20C**

**Compound 20B** (2.0 g, 6.17 mmol) was dissolved in methanol (20 mL), water (20 mL), and tetrahydrofuran (10 mL), and then lithium hydroxide monohydrate (260 mg, 6.17 mmol) was added under an ice bath. Then, the mixture was allowed to react at 70 °C for 16 h. After the reaction was completed as detected by TLC, the reaction liquid was

concentrated under reduced pressure to remove the methanol and tetrahydrofuran, then adjusted to pH = 5-6 with 1 N hydrochloric acid under an ice bath, and filtered. The filter cake was washed with water (50 mL) and dried to give **compound 20C** (1.5 g, pale yellow solid, yield: 78.4%). MS (ESI): *m*/*z* 310.0 $[M+1]^+$

**Step 3: synthesis of compound 20D**

**Compound 20C** (122 mg, 0.39 mmol) was dissolved in dichloromethane (5 mL), and then oxalyl chloride (125 mg, 0.98 mmol) was added dropwise under an ice bath. Then, the mixture was stirred at room temperature for 30 min. After the reaction was completed as detected by TLC, the reaction liquid was concentrated to dryness by rotary evaporation, and then the residue was dissolved in anhydrous tetrahydrofuran (10 mL). **Compound 8-D** (100 mg, 0.39 mmol) and N,N-diisopropylethylamine (191 mg, 1.56 mmol) were sequentially added at room temperature, and then the reaction liquid was stirred at 50 °C for 1 h. After the reaction was completed as detected by TLC, the reaction liquid was diluted with water (20 mL) and extracted with ethyl acetate (15 mL × 3). The organic phases were combined, washed with saturated brine (10 mL), dried over anhydrous sodium sulfate, filtered, and concentrated under reduced pressure to remove the solvent, and the residue was purified by a silica gel column to give **compound 20D** (120 mg, yellow solid, yield: 55.9%).

**Step 3: synthesis of compound 20**

**Compound 20D** (120 mg, 0.22 mmol) and **2-hydroxyethane-1-sulfonamide** (33 mg, 0.29 mmol) were dissolved in *N,N*-dimethylformamide (4 mL), and then cuprous iodide (4 mg, 0.02 mmol), potassium phosphate (94 mg, 0.44 mmol), and (1*R*,2*R*)-(-)-*N*,*N'*-dimethyl-1,2-cyclohexanediamine (6 mg, 0.04 mmol) were sequentially added. Then, the mixture was stirred at 120 °C for 16 h under a nitrogen atmosphere. After the reaction was completed as detected by TLC, the reaction liquid was diluted with water (20 mL) and extracted with ethyl acetate (15 mL × 3). The organic phases were combined, washed with saturated brine (8 mL), dried over anhydrous sodium sulfate, filtered, and concentrated under reduced pressure to remove the solvent, and the residue was purified by HPLC to give **compound 20** (30 mg, yellow solid). MS (ESI): *m*/*z* 295.6 $[1/2M+1]^+$.

$^1H$ NMR (300 MHz, DMSO-$d_6$) δ(ppm) 10.34 (s, 1H), 9.67 (br.s, 1H), 8.69 (s, 1H), 8.05 (d, *J* = 1.1 Hz, 1H), 7.50 (d, *J* = 1.1 Hz, 1H), 7.13 (d, *J* = 8.7 Hz, 1H), 6.55 - 6.47 (m, 2H), 4.92 (br.s, 1H), 4.38 - 4.29 (m, 4H), 3.71 (t, *J* = 6.8 Hz, 2H), 3.43 - 3.35 (m, 2H), 3.25 - 3.15 (m, 4H), 2.12 - 1.96 (m, 4H), 1.47 - 1.37 (m, 2H), 0.95 (s, 3H), 0.80 (s, 3H).

**Example 21: Preparation of Compound 21**

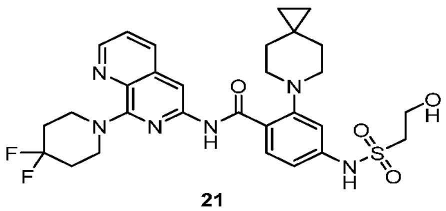

**21**

**Synthetic route:**

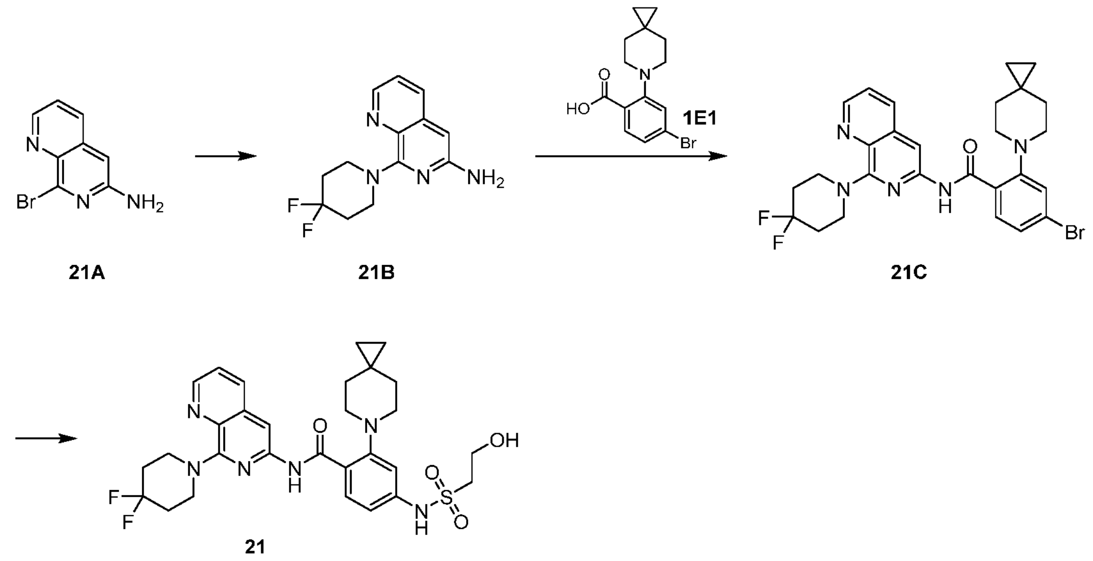

21A 21B 1E1 21C

21

**Preparation method:**

**Step 1: synthesis of compound 21B**

**Compound 21A** (100 mg, 0.45 mmol) was dissolved in N-methylpyrrolidone (3 mL), and then N,N-diisopropylethylamine (230 mg, 1.80 mmol) and **4,4-difluoropiperidine hydrochloride** (114 mg, 0.90 mmol) were added. Then, the mixture was stirred at 150 °C for 5 h under a nitrogen atmosphere. After the reaction was completed as detected by TLC, the reaction liquid was cooled to room temperature, diluted with water (20 mL), and extracted with ethyl acetate (20 mL × 3). The organic phases were combined, washed with saturated brine (10 mL × 3), dried over anhydrous sodium sulfate, filtered, and concentrated under reduced pressure to remove the solvent, and the residue was purified by silica gel column chromatography to give **compound 21B** (110 mg, yellow solid, yield: 93.3%). MS (ESI): *m/z* 265.1 $[M+1]^+$

**Step 2: synthesis of compound 21C**

**Compound 1E1** (152 mg, 0.49 mmol) was dissolved in dichloromethane (5 mL), and then oxalyl chloride (156 mg, 1.23 mmol) was added dropwise under an ice bath. Then, the mixture was stirred at room temperature for 30 min. After the reaction was completed as detected by TLC, the reaction liquid was concentrated to dryness by rotary evaporation, and then the residue was dissolved in anhydrous tetrahydrofuran (10 mL). **Compound 21B** (130 mg, 0.49 mmol) and N,N-diisopropylethylamine (191 mg, 1.47 mmol) were sequentially added at room temperature, and then the reaction liquid was stirred at 50 °C for 1 h. After the reaction was completed as detected by TLC, the reaction liquid was diluted with water (20 mL) and extracted with ethyl acetate (15 mL × 3). The organic phases were combined, washed with saturated brine (10 mL), dried over anhydrous sodium sulfate, filtered, and concentrated under reduced pressure to remove the solvent, and the residue was purified by a silica gel column to give **compound 21C** (200 mg, yellow solid, yield: 73.1%). MS (ESI): *m/z* 279.4 $[1/2M+1]^+$

**Step 3: synthesis of compound 21**

**Compound 21C** (100 mg, 0.18 mmol) and **2-hydroxyethane-1-sulfonamide** (20 mg, 0.16 mmol) were dissolved in *N,N*-dimethylformamide (2 mL), and then cuprous iodide (4 mg, 0.02 mmol), potassium phosphate (76 mg, 0.36 mmol), and (1R,2R)-(-)-N,N'-dimethyl-1,2-cyclohexanediamine (5 mg, 0.04 mmol) were sequentially added. Then, the mixture was stirred at 120 °C for 16 h under a nitrogen atmosphere. After the reaction was completed as detected by TLC, the reaction liquid was diluted with water (10 mL) and extracted with ethyl acetate (15 mL × 3). The organic phases were combined, washed with saturated brine (8 mL × 3), dried over anhydrous sodium sulfate, filtered, and concentrated under reduced pressure to remove the solvent, and the residue was purified by HPLC to give **compound 21** (20 mg, white solid). MS (ESI): *m/z* 301.3 $[1/2M+1]^+$.

$^1$H NMR (300 MHz, DMSO-$d_6$) δ(ppm) 12.90 (s, 1H), 10.21 (br.s, 1H), 8.72 (dd, *J* = 4.2, 1.7 Hz, 1H), 8.24 (dd, *J* = 8.5, 1.7 Hz, 1H), 8.13 - 8.07 (m, 2H), 7.61 (dd, *J* = 8.4, 4.1 Hz, 1H), 7.28 (d, *J* = 2.1 Hz, 1H), 7.14 (dd, *J* = 8.6, 2.1 Hz, 1H), 4.96 (br.s, 1H), 4.23 - 4.16 (m, 4H), 3.76 (t, *J* = 6.5 Hz, 2H), 3.36 (t, *J* = 6.5 Hz, 2H), 3.04 - 2.97 (m, 4H), 2.28 - 2.06 (m, 5H), 1.93 - 1.58 (m, 3H), 0.41 (s, 4H).

**Example 22: Preparation of Compound 22**

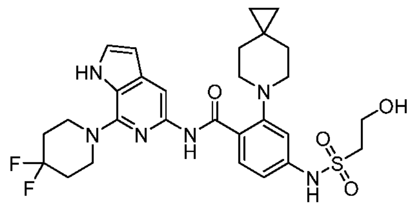

22

**Synthetic route:**

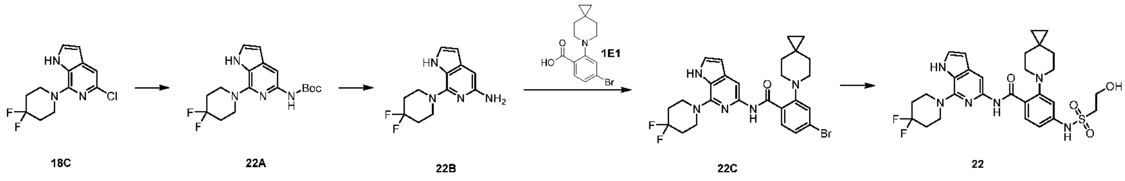


**Preparation method:**

**Step 3: synthesis of compound 22A**

**Compound 18C** (550 mg, 2.0 mmol) was dissolved in dioxane (7 mL), and then *tert-butyl* carbamate (352 mg, 3.0 mmol), cesium carbonate (1.32 g, 4.0 mmol), 2-dicyclohexylphosphino-2',4',6'-triisopropylbiphenyl (99 mg, 0.2 mmol), and tris(dibenzylideneacetone)dipalladium (88 mg, 0.1 mmol) were added. Then, the mixture was stirred at 100 °C for 16 h under a nitrogen atmosphere. After the reaction was completed as detected by TLC, the reaction liquid was cooled to room temperature, diluted with water (20 mL), and then extracted with ethyl acetate (15 mL × 3). The organic phases were combined, washed with saturated brine (15 mL), dried over anhydrous sodium sulfate, filtered, and concentrated under reduced pressure to remove the solvent, and the residue was purified by a silica gel column to give **compound 22A** (550 mg, yellow oil, yield: 80%). MS (ESI): *m*/*z* 353.1 $[M+1]^+$

**Step 4: synthesis of compound 22B**

**Compound 22A** (322 mg, 0.9 mmol) was dissolved in dichloromethane (5 mL), and then trifluoroacetic acid (1 mL) was added. Then, the mixture was stirred at 25 °C for 16 h. After the reaction was completed as detected by TLC, the reaction liquid was concentrated under reduced pressure to remove the solvent, and the residue was dissolved in methanol (2 mL) and water (2 mL). A sodium hydroxide solid (182 mg, 4.5 mmol) was slowly added under an ice bath. Then, the mixture was stirred at 25 °C for 16 h. After the reaction was completed as detected by TLC, the reaction liquid was diluted with water (20 mL) and extracted with ethyl acetate (15 mL × 3). The organic phases were combined, washed with saturated brine (20 mL), dried over anhydrous sodium sulfate, then filtered to remove the drying agent, and concentrated under reduced pressure to remove the solvent, and the residue was purified by silica gel column chromatography to give **compound 22B** (130 mg, brown oil, yield: 55.6%). MS (ESI): *m*/*z* 253.1 $[M+1]^+$

**Step 5: synthesis of compound 22C**

**Compound 1E1** (120 mg, 0.4 mmol) was dissolved in dichloromethane (10 mL), and then oxalyl chloride (130 mg, 1.0 mmol) was added dropwise under an ice bath. Then, the mixture was stirred at room temperature for 30 min. After the reaction was completed as detected by TLC, the reaction liquid was concentrated to dryness by rotary evaporation, and the residue was dissolved in anhydrous tetrahydrofuran (10 mL). **Compound 22B** (100 mg, 0.4 mmol) and N,N'-diisopropylethylamine (200 mg, 1.6 mmol) were added at room temperature, and then the mixture was stirred at 50 °C for 1 h. After the reaction was completed as detected by TLC, the reaction liquid was diluted with water (20 mL) and extracted with ethyl acetate (15 mL × 3). The organic phases were combined, washed with saturated brine (20 mL), dried

over anhydrous sodium sulfate, then filtered to remove the drying agent, and concentrated under reduced pressure to remove the solvent, and the residue was purified by a silica gel column to give **compound 22C** (20 mg, yellow solid, yield: 10%). MS (ESI): *m/z* 544.1 [M+1]$^+$

**Step 6: synthesis of compound 22**

**Compound 22C** (20 mg, 0.04 mmol) and **2-hydroxyethane-1-sulfonamide** (6 mg, 0.52 mmol) were dissolved in *N,N*-dimethylformamide (2 mL), and then cuprous iodide (4 mg, 0.02 mmol), potassium phosphate (23 mg, 0.12 mmol), and (1*R*,2*R*)-(-)-*N*,*N'*-dimethyl-1,2-cyclohexanediamine (5 mg, 0.03 mmol) were sequentially added. Then, the mixture was stirred at 120 °C for 2 h under a nitrogen atmosphere. After the reaction was completed as detected by TLC, the reaction liquid was diluted with water (10 mL) and extracted with ethyl acetate (15 mL × 3). The organic phases were combined, washed with saturated brine (8 mL), dried over anhydrous sodium sulfate, filtered, and concentrated under reduced pressure to remove the solvent, and the residue was purified by preparative HPLC to give compound **22** (0.62 mg, pale yellow solid). MS (ESI): *m/z* 589.2 [M+1]$^+$

**Example 23: Preparation of Compound 23**

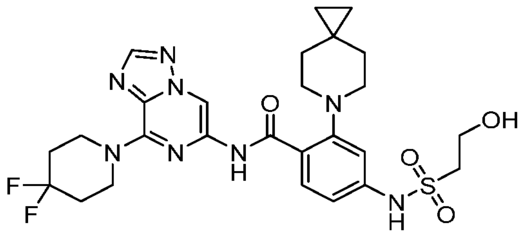

**23**

**Synthetic route:**

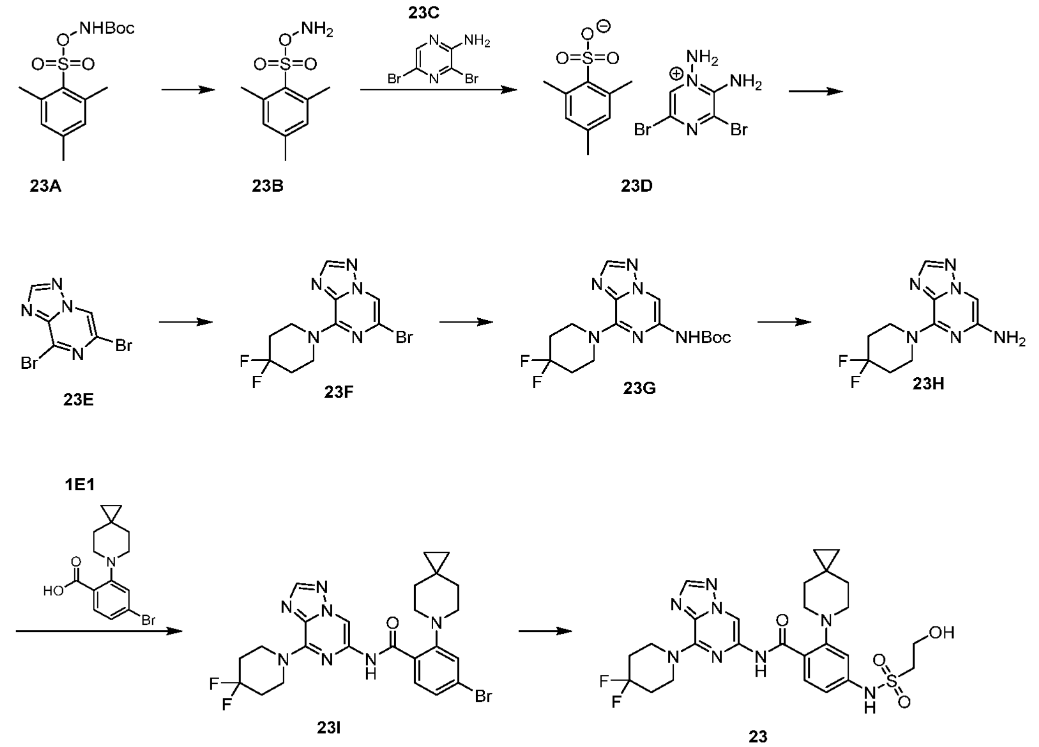


23A 23B 23D

23E 23F 23G 23H

23I 23

**Preparation method:**

**Step 1: synthesis of compound 23B**

**Compound 23A** (500 mg, 1.59 mmol) was added to trifluoroacetic acid (1.5 mL) under an ice bath, and the mixture was stirred for 1.5 h under the ice bath. After the reaction was completed as detected by TLC, an ice-water mixture (20 mL) was added to the reaction liquid, and then the mixture was stirred at room temperature for 5 min. The suspension was filtered, and the filter cake was washed with water until the pH of the filtrate was 6. The filter cake was collected and dissolved in dichloromethane (30 mL), and the mixture was dried over anhydrous sodium sulfate, then filtered to remove the drying agent, and concentrated under reduced pressure to remove the solvent to give **compound 23B** (290 mg, white solid, yield: 84.8%).

**Step 2: synthesis of compound 23D**

**Compound 23B** (290 mg, 1.35 mmol) was dissolved in dichloromethane (10 mL), and then **compound 23C** (341 mg, 1.35 mmol) was added at room temperature. The mixture was stirred at room temperature for 16 h. After the reaction was completed as detected by LCMS, the reaction liquid was filtered, and the filter cake was washed with dichloromethane (1 mL × 3) and dried to give a crude product of **compound 23D** (500 mg, white solid). MS (ESI): *m*/*z* 268.9 $[M]^+$

**Step 3: synthesis of compound 23E**

The crude product of **compound 23D** (500 mg) was dissolved in triethyl orthoformate (5 mL), and then the mixture was stirred at 100 °C for 1 h. After the reaction was completed as detected by LCMS, the reaction liquid was concentrated under reduced pressure to remove the solvent, and the residue was purified by silica gel column chromatography to give **compound 23E** (300 mg, yellow solid). MS (ESI): *m*/*z* 278.8 $[M+1]^+$

**Step 4: synthesis of compound 23F**

**Compound 23E** (300 mg, 1.08 mmol) was dissolved in N-methylpyrrolidone (5 mL), and then *N,N*-diisopropylethylamine (230 mg, 4.32 mmol) and **4,4-difluoropiperidine hydrochloride** (170 mg, 2.16 mmol) were added. Then, the mixture was stirred at 100 °C for 16 h under a nitrogen atmosphere. After the reaction was completed as detected by TLC, the reaction liquid was cooled to room temperature, diluted with water (60 mL), and extracted with ethyl acetate (50 mL × 3). The organic phases were combined, washed with saturated brine (30 mL × 3), dried over anhydrous sodium sulfate, then filtered to remove the drying agent, and concentrated under reduced pressure to remove the solvent, and the residue was purified by silica gel column chromatography to give **compound 23F** (220 mg, brownish-yellow solid, yield: 64.1%). MS (ESI): *m*/*z* 318.0 $[M+1]^+$

**Step 5: synthesis of compound 23G**

Compound 23F (200 mg, 0.63 mmol) was dissolved in dioxane (5 mL), and then *tert-butyl* carbamate (148 mg, 1.26 mmol), 2-dicyclohexylphosphino-2',4',6'-triisopropylbiphenyl (30 mg, 0.06 mmol), cesium carbonate (411 mg, 1.26 mmol), tris(dibenzylideneacetone)dipalladium (29 mg, 0.03 mmol) were added. Then, the mixture was stirred at 100 °C for 16 h under a nitrogen atmosphere. After the reaction was completed as detected by TLC, the reaction liquid was cooled to room temperature, diluted with water (20 mL), and extracted with ethyl acetate (30 mL × 3). The organic phases were combined, washed with saturated brine (30 mL × 2), dried over anhydrous sodium sulfate, then filtered to remove the drying agent, and concentrated under reduced pressure to remove the solvent, and the residue was purified by silica gel column chromatography and concentrated to dryness by rotary evaporation under reduced pressure to give compound 23G (190 mg, yellow solid, yield: 75.3%). MS (ESI): *m*/*z* 355.2 $[M+1]^+$

**Step 6: synthesis of compound 23H**

**Compound 23G** (200 mg, 0.56 mmol) was dissolved in dichloromethane (5 mL), and then trifluoroacetic acid (1 mL) was added. The mixture was stirred at room temperature for 2 h. After the reaction was completed as detected by TLC, the reaction liquid was concentrated under reduced pressure to give a **crude product of compound 23H** (200 mg, yellow oil). MS (ESI): *m*/*z* 255.1 $[M+1]^+$

**Step 7: synthesis of compound 23I**

**Compound 1E1** (182 mg, 0.59 mmol) was dissolved in dichloromethane (5 mL), and then oxalyl chloride (188 mg, 1.48 mmol) was added dropwise under an ice bath. Then, the mixture was stirred at room temperature for 30 min. After the reaction was completed as detected by TLC, the reaction liquid was concentrated to dryness by rotary evaporation, and the residue was dissolved in anhydrous tetrahydrofuran (10 mL). **Compound 23H** (150 mg, 0.59 mmol) and N,N-diisopropylethylamine (305 mg, 2.36 mmol) were sequentially added at room temperature, and then the mixture was stirred at 50 °C for 1 h. After the reaction was completed as detected by TLC, the reaction liquid was diluted with water (40 mL) and extracted with ethyl acetate (20 mL × 3). The organic phases were combined, washed with saturated brine (10 mL), dried over anhydrous sodium sulfate, filtered, and concentrated under reduced pressure to remove the solvent, and the residue was purified by a silica gel column to give **compound 23I** (100 mg, yellow solid, yield: 31.0%). MS (ESI): *m/z* 273.6 274.4 [1/2M+1]$^+$

**Step 8: synthesis of compound 23**

**Compound 23I** (90 mg, 0.16 mmol) and **2-hydroxyethane-1-sulfonamide** (21 mg, 0.19 mmol) were dissolved in *N,N*-dimethylformamide (2 mL), and then cuprous iodide (3 mg, 0.02 mmol), potassium phosphate (70 mg, 0.32 mmol), and (1*R*,2*R*)-(-)-*N,N'*-dimethyl-1,2-cyclohexanediamine (5 mg, 0.04 mmol) were sequentially added. Then, the mixture was stirred at 120 °C for 16 h under a nitrogen atmosphere. After the reaction was completed as detected by TLC, the reaction liquid was diluted with water (10 mL) and extracted with ethyl acetate (20 mL × 3). The organic phases were combined, washed with saturated brine (8 mL × 3), dried over anhydrous sodium sulfate, then filtered to remove the drying agent, and concentrated under reduced pressure to remove the solvent, and the residue was purified by HPLC to give **compound 23** (30 mg, white solid). MS (ESI): *m/z* 591.4 [M+1]$^{+}_{o}$ $^1$H NMR (300 MHz, DMSO-$d_6$) δ(ppm) 13.30 (s, 1H), 10.17 (br.s, 1H), 8.97 (s, 1H), 8.50 (s, 1H), 8.10 (d, *J* = 8.6 Hz, 1H), 7.30 (d, *J* = 2.1 Hz, 1H), 7.14 (dd, *J* = 8.7, 2.1 Hz, 1H), 4.92 (br.s, 1H), 4.42 - 4.36 (m, 4H), 3.76 (t, *J* = 6.5 Hz, 2H), 3.35 (t, *J* = 6.2 Hz, 2H), 3.02 - 2.96 (m, 4H), 2.23 - 2.09 (m, 5H), 1.81 - 1.68 (m, 3H), 0.42 (s, 4H).

**Example 24: Preparation of Compound 24**

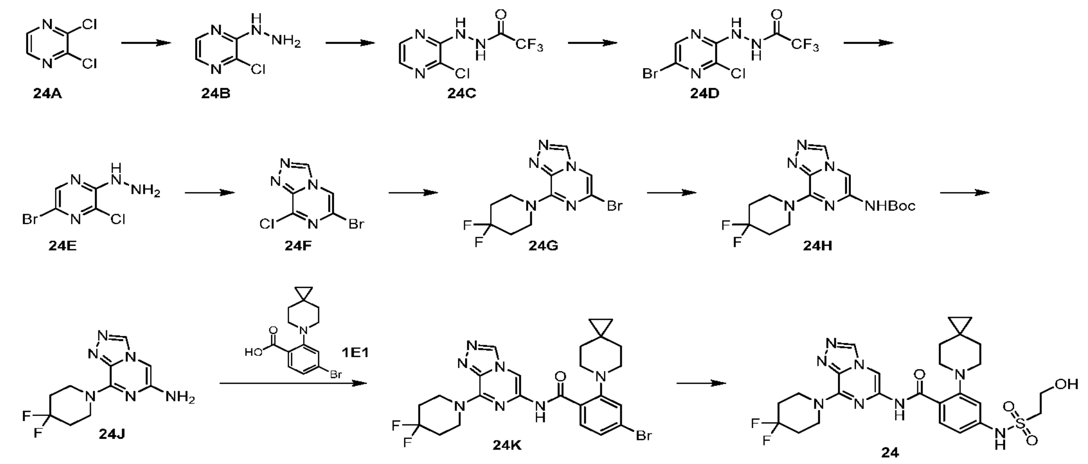
24A
24B
24C
24D
24E
24F
24G
24H
24J
1E1
24K
24

**Step 1: synthesis of compound 24B**

**Compound 24A** (50 g, 335.6 mmol) was dissolved in ethanol, and then hydrazine hydrate (39.5 g, 671.3 mmol, 85% pure) was added. Then, the mixture was stirred at 90 °C for 12 h. After the reaction was completed as detected by LCMS, the reaction liquid was cooled to room temperature and filtered, and the filter cake was washed with ice ethanol and dried to give a crude product of **compound 24B** (54 g, yellow solid). MS (ESI): *m/z* 145.0 [M+1]$^+$

**Step 2: synthesis of compound 24C**

The crude product of **compound 24B** (54 g, 373.5 mmol) was dissolved in tetrahydrofuran, and then trifluoroacetic anhydride (86.3 g, 410.9 mmol) was added dropwise under an ice bath. Then, the mixture was stirred for 3 h under

the ice bath. After the reaction was completed as detected by LCMS, the reaction liquid was concentrated under reduced pressure to remove the solvent, then diluted with water (200 mL), and extracted with dichloromethane (200 mL × 3). The organic phases were combined, washed with saturated brine (100 mL), dried over anhydrous sodium sulfate, filtered, and concentrated under reduced pressure to remove the solvent to give a crude product of **compound 24C** (40 g, orange liquid). MS (ESI): *m/z* 241.0 $[M+1]^+$

**Step 3: synthesis of compound 24D**

The crude product of **compound 24C** (40 g, 166.3 mmol) was dissolved in dichloromethane (400 mL), and then NBS (86.3 g, 182.9 mmol) was added in portions at -40 °C. Then, the mixture was naturally warmed to 0 °C over 1 h. After the reaction was completed as detected by LCMS, the reaction was quenched with water (200 mL) under an ice bath, and the mixture was extracted with dichloromethane (200 mL × 3). The organic phases were combined, washed with saturated brine (100 mL), dried over anhydrous sodium sulfate, filtered, and concentrated under reduced pressure to remove the solvent, and the residue was purified by silica gel column chromatography to give **compound 24D** (15 g, yellow liquid). MS (ESI): *m/z* 318.8 320.8 $[M+1]^+$

**Step 4: synthesis of compound 24E**

**Compound 24D** (15 g, 47.0 mmol) was dissolved in ethanol (160 mL), and then hydrochloric acid (12 mL, 12 mol/L) was added. Then, the mixture was stirred at 100 °C for 8 h. After the reaction was completed as detected by LCMS, the reaction liquid was concentrated under reduced pressure to remove the ethanol, and then the reaction was quenched with a sodium bicarbonate solid until pH = 7. Then, the reaction liquid was extracted with dichloromethane/methanol (80 mL × 3, 10:1). The organic phases were combined, washed with saturated brine (60 mL), dried over anhydrous sodium sulfate, filtered, and concentrated under reduced pressure to remove the solvent to give a crude product of **compound 24E** (7 g, grey solid). MS (ESI): *m/z* 222.9 224.9 $[M+1]^+$

**Step 5: synthesis of compound 24F**

**Compound 24E** (7 g, 31.3 mmol) was dissolved in triethyl orthoformate (70 mL), and the mixture was stirred at 130 °C for 2 h. After the reaction was completed as detected by LCMS, the reaction liquid was concentrated under reduced pressure to remove the solvent, and the residue was purified by silica gel column chromatography to give **compound 24F** (5.2 g, yellow solid). MS (ESI): *m/z* 232.8 234.8 $[M+1]^+$

**Step 6: synthesis of compound 24G**

**Compound 24F** (800 mg, 3.43 mmol) was dissolved in N-methylpyrrolidone (10 mL), and then *N,N*-diisopropylethylamine (1.8 g, 13.7 mmol) and **4,4-difluoropiperidine hydrochloride** (1.1 g, 6.86 mmol) were added. Then, the mixture was stirred at 60 °C for 4 h under a nitrogen atmosphere. After the reaction was completed as detected by TLC, the reaction liquid was cooled to room temperature, diluted with water (80 mL), and extracted with ethyl acetate (50 mL × 3). The organic phases were combined, washed with saturated brine (40 mL × 2), dried over anhydrous sodium sulfate, filtered, and concentrated under reduced pressure to remove the solvent, and the residue was purified by silica gel column chromatography to give **compound 24G** (220 mg, brownish-red solid, yield: 20.2%). MS (ESI): *m/z 318.0* 320.0 $[M+1]^+$

**Step 7: synthesis of compound 24H**

Compound 24G (400 mg, 1.26 mmol) was dissolved in dioxane (10 mL), and then *tert-butyl* carbamate (298 mg, 2.52 mmol), 2-dicyclohexylphosphino-2',4',6'-triisopropylbiphenyl (60 mg, 0.13 mmol), cesium carbonate (823 mg, 2.52 mmol), tris(dibenzylideneacetone)dipalladium (21 mg, 0.06 mmol) were added. Then, the mixture was stirred at 110 °C for 16 h under a nitrogen atmosphere. After the reaction was completed as detected by TLC, the reaction liquid was cooled to room temperature, diluted with water (50 mL), and extracted with ethyl acetate (40 mL × 3). The organic phases were combined, dried over anhydrous sodium sulfate, filtered, and concentrated under reduced pressure to remove the solvent, and the residue was purified by silica gel column chromatography to give compound 24H (150 mg, brown solid, yield: 24.6%). MS (ESI): *m/z* 355.1 $[M+1]^+$

**Step 8: synthesis of compound 24J**

**Compound 24H** (100 mg, 0.56 mmol) was dissolved in dichloromethane (5 mL), and then trifluoroacetic acid (1 mL) was added. The mixture was stirred at room temperature for 2 h. After the reaction was completed as detected by TLC,

the mixture was concentrated under reduced pressure to remove the solvent, diluted with water (20 mL), then adjusted to pH = 7-8 with a sodium bicarbonate solid, and extracted with ethyl acetate (20 mL × 4). The organic phases were combined, dried over anhydrous sodium sulfate, filtered, and concentrated under reduced pressure to remove the solvent to give a **crude product of compound 24J** (100 mg, blackish-green solid). MS (ESI): *m/z* 255.1 [M+1]$^+$

**Step 9: synthesis of compound 24K**

**Compound 1E1** (122 mg, 0.39 mmol) was dissolved in dichloromethane (5 mL), and then oxalyl chloride (125 mg, 0.98 mmol) was added dropwise under an ice bath. Then, the mixture was stirred at room temperature for 30 min. After the reaction was completed as detected by TLC, the reaction liquid was concentrated to dryness by rotary evaporation, and then the residue was dissolved in anhydrous tetrahydrofuran (5 mL). **Compound 24J** (100 mg, 0.39 mmol) and *N,N*-diisopropylethylamine (152 mg, 1.17 mmol) were sequentially added at room temperature, and then the reaction liquid was stirred at 50 °C for 2 h. After the reaction was completed as detected by TLC, the reaction liquid was diluted with water (20 mL) and extracted with ethyl acetate (20 mL × 3). The organic phases were combined, dried over anhydrous sodium sulfate, filtered, and concentrated under reduced pressure to remove the solvent, and the residue was purified by a silica gel column to give **compound 24K** (100 mg, yellow solid, purity: 53.8%, yield: 25.0%). MS (ESI): *m/z* 273.6 274.4 [M/2+1]$^+$

**Step 10: synthesis of compound 24**

**Compound 24K** (100 mg, 0.18 mmol) and **2-hydroxyethane-1-sulfonamide** (28 mg, 0.22 mmol) were dissolved in *N,N*-dimethylformamide (2 mL), and then cuprous iodide (4 mg, 0.02 mmol), potassium phosphate (78 mg, 0.36 mmol), and (1*R*,2*R*)-(-)-*N,N'*-dimethyl-1,2-cyclohexanediamine (5 mg, 0.04 mmol) were sequentially added. Then, the mixture was stirred at 120 °C for 2 h under a nitrogen atmosphere. After the reaction was completed as detected by TLC, the reaction liquid was diluted with water (10 mL) and extracted with ethyl acetate (10 mL × 3). The organic phases were combined, washed with saturated brine (8 mL × 3), dried over anhydrous sodium sulfate, filtered, and concentrated under reduced pressure to remove the solvent, and the residue was purified by preparative HPLC to give **compound 24** (40 mg, off-white solid). MS (ESI): *m/z* 591.1 [M+1]$^+$。 $^1$H NMR (300 MHz, DMSO-$d_6$) δ(ppm) 13.05 (s, 1H), 10.20 (br.s, 1H), 9.34 (s, 1H), 8.76 (s, 1H), 8.05 (d, *J* = 8.6 Hz, 1H), 7.26 (d, *J* = 2.1 Hz, 1H), 7.11 (dd, *J* = 8.7, 2.1 Hz, 1H), 4.91 (br.s, 1H), 4.48 - 4.35 (m, 4H), 3.73 (t, *J* = 6.5 Hz, 2H), 3.33 (t, *J* = 6.4 Hz, 2H), 3.00 - 2.93 (m, 4H), 2.22 - 2.07 (m, 5H), 1.81 - 1.56 (m, 3H), 0.38 (s, 4H).

**Example 25: Preparation of Compound 25**

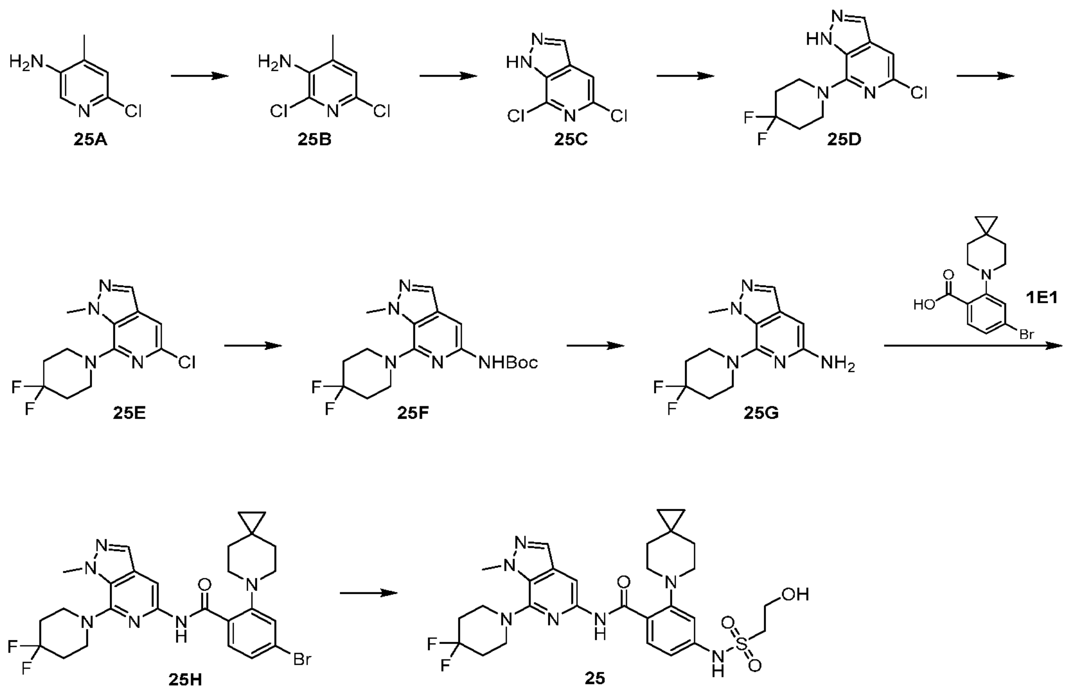
25A
25B
25C
25D
25E
25F
25G
1E1
25H
25

**Step 1: synthesis of compound 25B**

**Compound 25A** (5.0 g, 35.1 mmol) was dissolved in glacial acetic acid (50 mL), and then *N*-chlorosuccinimide (5.6 g, 42.1 mmol) was added in portions under an ice bath. Then, the mixture was stirred at room temperature for 16 h. After the reaction was completed as detected by TLC, the reaction liquid was diluted with water (300 mL), then adjusted to pH = 7-8 with sodium bicarbonate, and extracted with ethyl acetate (150 mL × 3). The organic phases were combined, washed with saturated brine (100 mL × 2), dried over anhydrous sodium sulfate, filtered, and concentrated under reduced pressure to remove the solvent, and the residue was purified by silica gel column chromatography to give **compound 25B** (3.7 g, pale red solid, yield: 56.9%). MS (ESI): *m/z* 176.9 $[M+1]^+$

**Step 2: synthesis of compound 25C**

**Compound 25B** (3.5 g, 19.8 mmol) was dissolved in glacial acetic acid (35 mL), and then a solution of sodium nitrite (1.5 g, 21.8 mmol) in water (5 mL) was slowly added dropwise under an ice bath. After the addition was completed, the mixture was stirred at room temperature for 16 h. After the reaction was completed as detected by TLC, the reaction liquid was diluted with water (150 mL), then adjusted to pH = 7-8 with sodium bicarbonate, and extracted with ethyl acetate (100 mL × 3). The organic phases were combined, washed with saturated brine (80 mL × 2), dried over anhydrous sodium sulfate, filtered, and concentrated under reduced pressure to remove the solvent, and the residue was purified by silica gel column chromatography to give **compound 25C** (500 m g, pale red solid, yield: 64.1%). MS (ESI): *m/z* 187.9 $[M+1]^+$

**Step 3: synthesis of compound 25D**

**Compound 25C** (800 mg, 4.26 mmol) was dissolved in N-methylpyrrolidone (10 mL), and then N,N-diisopropylethylamine (2.2 g, 17.0 mmol) and **4,4-difluoropiperidine hydrochloride** (1.3 g, 8.52 mmol) were added. Then, the mixture was stirred at 80 °C for 16 h under a nitrogen atmosphere. After the reaction was completed as detected by TLC, the reaction liquid was cooled to room temperature, diluted with water (30 mL), and extracted with ethyl acetate (30 mL × 3). The organic phases were combined, washed with saturated brine (20 mL × 3), dried over anhydrous sodium sulfate, filtered, and concentrated under reduced pressure to remove the solvent, and the residue was purified by silica gel column chromatography to give **compound 25D** (800 mg, brownish-yellow solid, yield: 62.5%). MS (ESI): *m/z* 273.1 $[M+1]^+$

**Step 4: synthesis of compound 25E**

**Compound 25D** (1.0 g, 3.49 mmol) was dissolved in anhydrous N,N-dimethylformamide (10 mL), and then sodium hydride (300 mg, 6.98 mmol) was added in portions under an ice bath. The mixture was stirred for 30 min under the ice bath, and then iodomethane (785 mg, 5.24 mmol) was added dropwise to the reaction liquid. After the addition was completed, the mixture was slowly warmed to room temperature and stirred for 2 h. After the reaction was completed as detected by TLC, the reaction was slowly quenched with a saturated ammonium chloride solution (30 mL) under an ice bath, and the mixture was extracted with ethyl acetate (50 mL × 3). The organic phases were combined, washed with saturated brine (50 mL × 2), dried over anhydrous sodium sulfate, filtered, and concentrated under reduced pressure to remove the solvent, and the residue was purified by silica gel column chromatography to give **compound 25E** (600 mg, brown oil, purity: 70.5%, yield: 40.2%). MS (ESI): *m/z* 287.0 $[M+1]^+$

**Step 5: synthesis of compound 25F**

Compound 25E (600 mg, 2.09 mmol) was dissolved in dioxane (10 mL), and then *tert-butyl* carbamate (492 mg, 4.18 mmol), 2-dicyclohexylphosphino-2',4',6'-triisopropylbiphenyl (198 mg, 0.42 mmol), cesium carbonate (1.37 g, 4.18 mmol), and palladium acetate (192 mg, 0.21 mmol) were added. Then, the mixture was stirred at 110 °C for 16 h under a nitrogen atmosphere. After the reaction was completed as detected by TLC, the reaction liquid was cooled to room temperature, diluted with water (80 mL), and extracted with ethyl acetate (50 mL × 3). The organic phases were combined, washed with saturated brine (30 mL × 2), dried over anhydrous sodium sulfate, filtered, and concentrated under reduced pressure to remove the solvent, and the residue was purified by silica gel column chromatography to give compound 25F (600 mg, yellow solid, yield: 70.2%). MS (ESI): *m/z* 368.2 $[M+1]^+$

**Step 6: synthesis of compound 25G**

**Compound 25F** (800 mg, 2.18 mmol) was dissolved in dichloromethane (10 mL), and then trifluoroacetic acid (2 mL) was added. The mixture was stirred at room temperature for 2 h. After the reaction was completed as detected by TLC, the reaction liquid was concentrated under reduced pressure to remove the solvent to give a **crude product of compound**

**25G** (800 mg, yellow oil). MS (ESI): *m/z* 268.1 [M+1]$^+$

**Step 7: synthesis of compound 25H**

**Compound 1E1** (578 mg, 1.87 mmol) was dissolved in dichloromethane (5 mL), and then oxalyl chloride (594 mg, 4.68 mmol) was added dropwise under an ice bath. Then, the mixture was stirred at room temperature for 30 min. After the reaction was completed as detected by TLC, the reaction liquid was concentrated to dryness by rotary evaporation, and then the residue was dissolved in anhydrous tetrahydrofuran (10 mL). **Compound 25G** (500 mg, 1.87 mmol) and N,N-diisopropylethylamine (966 mg, 7.48 mmol) were added at room temperature, and then the reaction liquid was stirred at 50 °C for 1 h. After the reaction was completed as detected by TLC, the reaction liquid was diluted with water (20 mL) and extracted with ethyl acetate (40 mL × 3). The organic phases were combined, washed with saturated brine (30 mL), dried over anhydrous sodium sulfate, filtered, and concentrated under reduced pressure to remove the solvent, and the residue was purified by a silica gel column to give **compound 25H** (400 mg, yellowish-brown solid, yield: 36.7%). MS (ESI): *m/z* 280.1 281.1 [M/2+1]$^+$

**Step 8: synthesis of compound 25**

**Compound 25H** (400 mg, 0.71 mmol) and **2-hydroxyethane-1-sulfonamide** (108 mg, 0.85 mmol) were dissolved in *N,N*-dimethylformamide (6 mL), and then cuprous iodide (14 mg, 0.07 mmol), potassium phosphate (304 mg, 1.42 mmol), and (1*R*,2*R*)-(-)-*N,N'*-dimethyl-1,2-cyclohexanediamine (20 mg, 0.14 mmol) were sequentially added. Then, the mixture was stirred at 120 °C for 2 h under a nitrogen atmosphere. After the reaction was completed as detected by TLC, the reaction liquid was diluted with water (30 mL) and extracted with ethyl acetate (30 mL × 3). The organic phases were combined, washed with saturated brine (20 mL × 2), dried over anhydrous sodium sulfate, filtered, and concentrated under reduced pressure to remove the solvent, and the residue was purified by preparative HPLC to give **compound 25** (60 mg, pale yellow solid). MS (ESI): *m/z* 302.7 [M/2+1]$^+$。$^1$H NMR (300 MHz, DMSO-$d_6$) δ(ppm) 12.51 (s, 1H), 10.26 (br.s, 1H), 8.24 (s, 1H), 8.07 (d, *J* = 8.6 Hz, 1H), 7.82 (s, 1H), 7.25 (d, *J* = 2.1 Hz, 1H), 7.11 (dd, *J* = 8.6, 2.1 Hz, 1H), 4.95 (s, 1H), 4.33 - 4.24 (m, 4H), 4.14 (s, 3H), 3.76 (t, *J* = 6.5 Hz, 2H), 3.35 (t, *J* = 6.5 Hz, 2H), 3.04 - 2.93 (m, 4H), 2.23 - 1.99 (m, 5H), 1.90 - 1.61 (m, 3H), 0.40 (s, 4H).

**Example 26: Preparation of Compound 26**

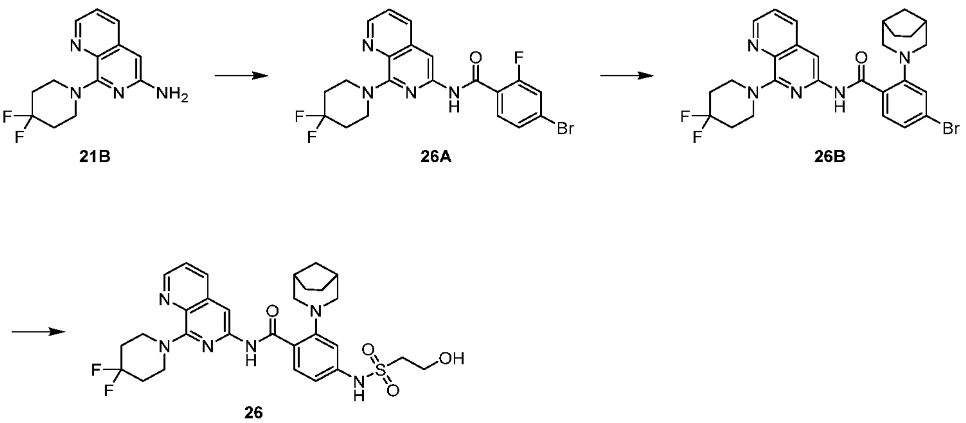

21B 26A 26B 26

**Preparation method:**

**Step 1: synthesis of compound 26A**

**2-Fluoro-4-bromobenzoic acid** (165 mg, 0.76 mmol) was dissolved in thionyl chloride (4 mL), and the mixture was stirred at 80 °C for 2 h. After the reaction was completed as detected by TLC, the reaction liquid was concentrated to dryness by rotary evaporation, and then the residue was dissolved in anhydrous tetrahydrofuran (2 mL). **Compound 21B** (100 mg, 0.76 mmol) and N,N-diisopropylethylamine (391 mg, 3.04 mmol) were sequentially added at room temperature, and then the mixture was stirred at 50 °C for 2 h. After the reaction was completed as detected by TLC, the reaction liquid was diluted with water (40 mL) and extracted with ethyl acetate (15 mL × 3). The organic phases were combined, washed

with saturated brine (10 mL), dried over anhydrous sodium sulfate, filtered, and concentrated under reduced pressure to remove the solvent, and the residue was purified by a silica gel column to give **compound 26A** (200 mg, yellow solid, purity: 85.8%, yield: 48.7%). MS (ESI): *m/z* 465.0 466.9 $[M+1]^+$

**Step 2: synthesis of compound 26B**

**Compound 26A** (100 mg, 0.21 mmol) was dissolved in N,N-dimethylformamide (2 mL), and then **3-azabicyclo [3.2.1]octane hydrochloride** (32 mg, 0.25 mmol) and potassium carbonate (60 mg, 0.42 mmol) were added. Then, the mixture was stirred at 120 °C for 16 h under a nitrogen atmosphere. After the reaction was completed as detected by TLC, the reaction liquid was diluted with water (20 mL) and extracted with ethyl acetate (15 mL × 3). The organic phases were combined, washed with saturated brine (10 mL × 3), dried over anhydrous sodium sulfate, filtered, and concentrated to dryness by rotary evaporation, and the residue was purified by a silica gel column to give **compound 26B** (120 mg, yellow solid, purity: 63.1%, yield: 63.3%). MS (ESI): *m/z* 278.6 279.7 $[M/2+1]^+$

**Step 3: synthesis of compound 26**

**Compound 26B** (100 mg, 0.18 mmol) and **2-hydroxyethane-1-sulfonamide** (27 mg, 0.22 mmol) were dissolved in *N,N*-dimethylformamide (4 mL), and then cuprous iodide (3 mg, 0.02 mmol), potassium phosphate (76 mg, 0.36 mmol), and (1*R*,2*R*)-(-)-*N,N'*-dimethyl-1,2-cyclohexanediamine (5 mg, 0.04 mmol) were sequentially added. Then, the mixture was stirred at 120 °C for 2 h under a nitrogen atmosphere. After the reaction was completed as detected by TLC, the reaction liquid was diluted with water (10 mL) and extracted with ethyl acetate (10 mL × 3). The organic phases were combined, washed with saturated brine (8 mL × 2), dried over anhydrous sodium sulfate, filtered, and concentrated under reduced pressure to remove the solvent, and the residue was purified by preparative HPLC to give compound 26 (27 mg, off-white solid). MS (ESI): *m/z* 301.1 $[M/2+1]^+$。 $^1H$ NMR (300 MHz, DMSO-$d_6$) δ(ppm) 10.50 (s, 1H), 10.02 (br.s, 1H), 8.71 (dd, *J* = 4.1, 1.7 Hz, 1H), 8.24 (dd, *J* = 8.5, 1.7 Hz, 1H), 8.12 (s, 1H), 7.69 - 7.55 (m, 2H), 7.12 (d, *J* = 2.0 Hz, 1H), 6.98 (dd, *J* = 8.4, 2.0 Hz, 1H), 4.95 (br.s, 1H), 4.20 - 4.10 (m, 4H), 3.76 (t, *J* = 6.6 Hz, 2H), 3.31 (t, *J* = 6.6 Hz, 2H), 3.09 - 2.98 (m, 2H), 2.79 (d, *J* = 10.5 Hz, 2H), 2.27 - 2.02 (m, 6H), 1.98 - 1.88 (m, 2H), 1.58 - 1.44 (m, 4H).

**Example 27: Preparation of Compound 27**

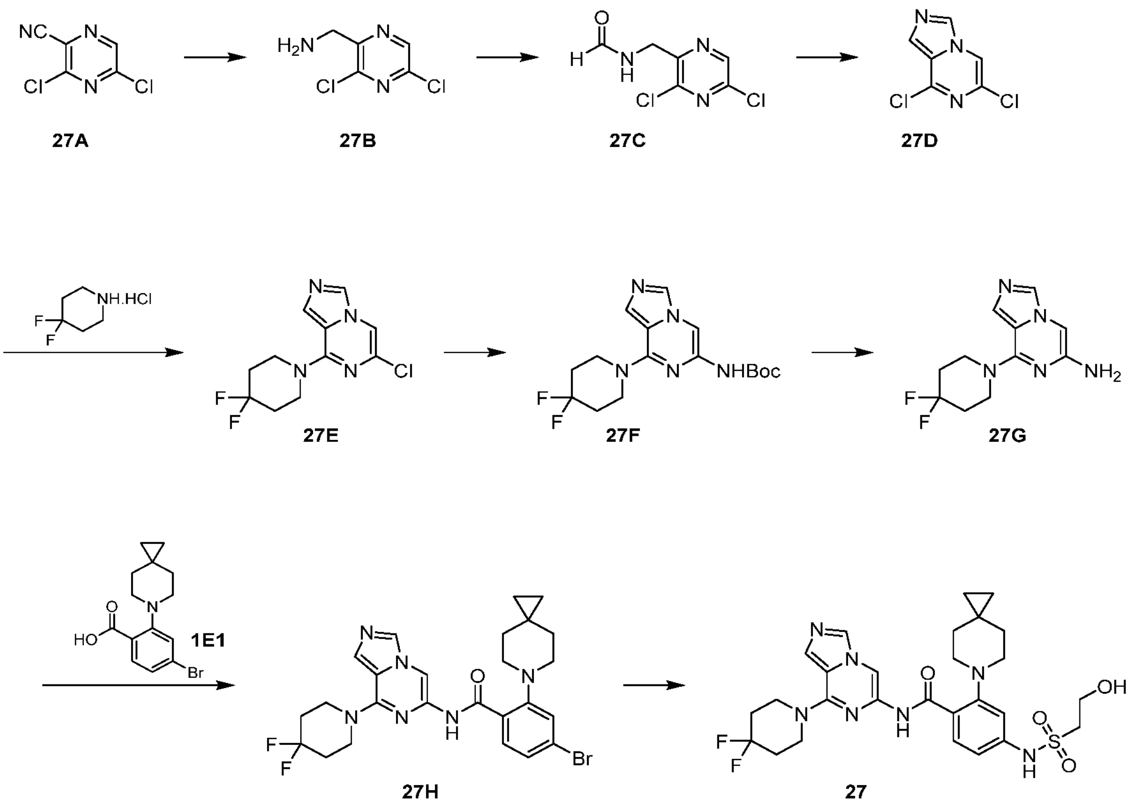

**27A** **27B** **27C** **27D**

**27E** **27F** **27G**

**1E1**

**27H** **27**

**Step 1: synthesis of compound 27B**

**Compound 27A** (5 g, 28.7 mmol) was dissolved in acetic acid (50 mL), and then Raney nickel (2 g) was added. Then, the mixture was stirred at 55 °C for 48 h under a hydrogen atmosphere (40 psi). After the reaction was completed as detected by LCMS, the reaction liquid was filtered through celite, and the filtrate was concentrated under reduced pressure to remove the solvent to give a crude product of compound **27B** (6 g, brown oil). MS (ESI): *m/z* 178.0 $[M+1]^+$

**Step 2: synthesis of compound 27C**

Acetic anhydride (25 mL) was added to formic acid (25 mL), and then the mixture was stirred at 50 °C for 2 h. Subsequently, the solution was cooled to room temperature, and a solution of **compound 27B** (6 g) in dichloromethane (50 mL) was added dropwise to the reaction liquid at room temperature. Then, the mixture was stirred at room temperature for 3 h. After the reaction was completed as detected by LCMS, the reaction liquid was concentrated under reduced pressure to remove the solvent, and the residue was quenched with saturated sodium bicarbonate until pH = 7 and then extracted with ethyl acetate (50 mL × 3). The organic phases were combined, washed with saturated brine (50 mL), dried over anhydrous sodium sulfate, filtered, and concentrated under reduced pressure to remove the solvent to give a crude product of **compound 27C** (5 g, brown solid). MS (ESI): *m/z* 205.8 $[M+1]^+$

**Step 3: synthesis of compound 27D**

The crude product of **compound 27C** (5 g, 24.3 mmol) was dissolved in acetonitrile (50 mL), and then thionyl chloride (4.33 g, 36.4 mmol) was added at room temperature. Then, the mixture was stirred at 80 °C for 0.5 h. After the reaction was completed as detected by LCMS, the reaction liquid was concentrated under reduced pressure to remove the solvent, and the residue was quenched with saturated sodium bicarbonate until pH = 7 and then extracted with ethyl acetate (50 mL × 3). The organic phases were combined, washed with saturated brine (50 mL), dried over anhydrous sodium sulfate, filtered, and concentrated under reduced pressure to remove the solvent, and the residue was purified by silica gel column chromatography to give **compound 27D** (3.2 g, brown solid). MS (ESI): *m/z* 187.9 $[M+1]^+$

**Step 4: synthesis of compound 27E**

**Compound 27D** (270 mg, 1.44 mmol) was dissolved in N-methyl-2-pyrrolidone (6 mL), and then **4,4-difluoropiperidine hydrochloride** (296 mg, 1.87 mmol) and N,N-diisopropylethylamine (558 mg, 4.32 mmol) were added. After the addition was completed, the mixture was stirred at 80 °C for 4 h under a nitrogen atmosphere. After the reaction was completed as detected by TLC, the reaction liquid was diluted with water (20 mL) at 0 °C and then extracted with ethyl acetate (20 mL × 2). The organic phases were combined, washed with saturated brine (15 mL × 3), dried over anhydrous sodium sulfate, then filtered to remove the drying agent, and concentrated under reduced pressure to remove the solvent, and the residue was purified by silica gel column chromatography to give **compound 27E** (383 mg, 1.40 mmol, yellow oil, yield: 97.2%). MS (ESI): *m/z* 273.0 $[M+1]^+$

**Step 5: synthesis of compound 27F**

**Compound 27E** (383 mg, 1.40 mmol) was dissolved in dioxane (7 mL), and then *tert-butyl* **carbamate** (247 mg, 2.1 mmol), cesium carbonate (1.4 g, 4.2 mmol), 2-dicyclohexylphosphino-2',4',6'-triisopropylbiphenyl (67 mg, 0.14 mmol), and tris(dibenzylideneacetone)dipalladium (65 mg, 0.07 mmol) were added. Then, the mixture was stirred at 100 °C for 5 h under a nitrogen atmosphere. After the reaction was completed as detected by TLC, the reaction liquid was cooled to room temperature, diluted with water (20 mL), and extracted with ethyl acetate (15 mL × 2). The organic phases were combined, washed with saturated brine (15 mL), dried over anhydrous sodium sulfate, filtered, and concentrated under reduced pressure to remove the solvent, and the residue was purified by a silica gel column to give **compound 27F** (220 mg, yellow oil, yield: 44%). MS (ESI): *m/z* 354.2 $[M+1]^+$

**Step 6: synthesis of compound 27G**

**Compound 27F** (220 mg, 0.62 mmol) was dissolved in dichloromethane (6 mL), and then trifluoroacetic acid (1.5 mL) was added. Then, the mixture was stirred at room temperature for 5 h. After the reaction was completed as detected by TLC, the mixture was concentrated under reduced pressure to remove the solvent to give a crude product of **compound 27G** (151 mg, yellow oil). MS (ESI): *m/z* 254.1 $[M+1]^+$

**Step 7: synthesis of compound 27H**

**Compound 1E1** (184 mg, 0.6 mmol) was dissolved in dichloromethane (2 mL), and then oxalyl chloride (189 mg, 1.5 mmol) was added dropwise under an ice bath. Then, the mixture was stirred at room temperature for 30 min. After the reaction was completed as detected by TLC, the reaction liquid was concentrated to dryness by rotary evaporation, and the residue was dissolved in anhydrous tetrahydrofuran (2 mL). **Compound 27G** (151 mg, 0.6 mmol) and N,N-diisopropylethylamine (309 mg, 2.4 mmol) were added to the reaction liquid under an ice bath, and then the mixture was stirred at room temperature for 1 h. After the reaction was completed as detected by TLC, the reaction liquid was diluted with water (20 mL) and extracted with ethyl acetate (15 mL × 3). The organic phases were combined, washed with saturated brine (10 mL), dried over anhydrous sodium sulfate, filtered, and concentrated under reduced pressure to remove the solvent, and the residue was purified by a silica gel column to give **compound 27H** (98 mg, yellow solid, yield: 30%). MS (ESI): *m/z* 545.2 547.1 $[M+1]^+$

**Step 8: synthesis of compound 27**

**Compound 27H** (98 mg, 0.18 mmol) and **2-hydroxyethane-1-sulfonamide** (27 mg, 0.24 mmol) were dissolved in *N,N*-dimethylformamide (5 mL), and then cuprous iodide (18 mg, 0.11 mmol), potassium phosphate (99 mg, 0.52 mmol), and (1*R*,2*R*)-(-)-*N,N'*-dimethyl-1,2-cyclohexanediamine (20 mg, 0.15 mmol) were sequentially added. Then, the mixture was stirred at 120 °C for 2 h under a nitrogen atmosphere. After the reaction was completed as detected by TLC, the reaction liquid was diluted with water (20 mL) and extracted with ethyl acetate (15 mL × 3). The organic phases were combined, washed with saturated brine (10 mL × 3), dried over anhydrous sodium sulfate, filtered, and concentrated under reduced pressure to remove the solvent, and the residue was purified by preparative HPLC to give compound 27 (6 mg, yellow solid). MS (ESI): *m/z* 590.2 $[M+1]^+$。 $^1H$ NMR (300 MHz, DMSO-$d_6$) δ(ppm) 12.81 (s, 1H), 10.20 (br.s, 1H), 8.71 (s, 1H), 8.53 (s, 1H), 8.07 (d, *J* = 8.6 Hz, 1H), 7.87 (s, 1H), 7.28 (d, *J* = 2.2 Hz, 1H), 7.14 (dd, *J* = 8.7, 2.1 Hz, 1H), 4.94 (br.s, 1H), 4.01 - 3.93 (m, 4H), 3.76 (t, *J* = 6.5 Hz, 2H), 3.35 (t, *J*= 6.5 Hz, 2H), 3.01-2.94 (m, 4H), 2.23 - 2.04 (m, 5H), 1.82 - 1.59 (m, 3H), 0.40 (s, 4H).

**Example 28: Preparation of Compound 28**

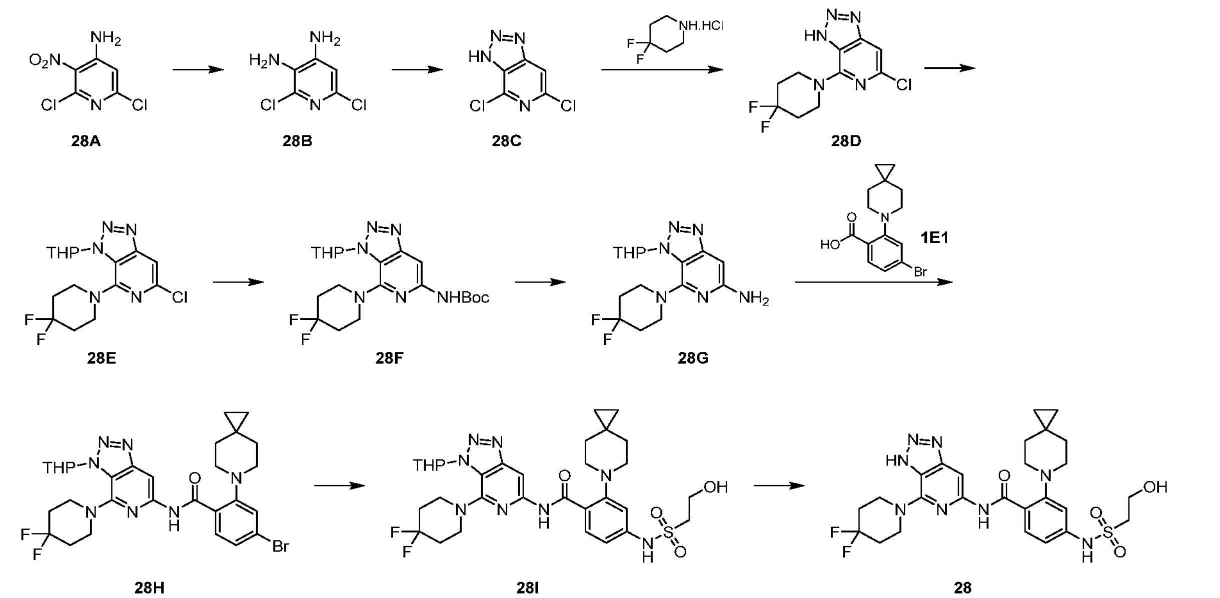

28A 28B 28C 28D

28E 28F 28G 1E1

28H 28I 28

**Step 1: synthesis of compound 28B**

**Compound 28A** (8.0 g, 38.5 mmol) was dissolved in ethanol (160 mL) and water (30 mL), and then iron powder (10.7 g, 192.5 mmol) and concentrated hydrochloric acid (20 mL) were added. The mixture was stirred at 95 °C for 16 h. After the reaction was completed as detected by TLC, the reaction liquid was cooled to room temperature and filtered, and the filtrate was diluted with water (150 mL), adjusted to pH = 7 with a sodium bicarbonate solid, and then extracted with ethyl acetate (50 mL × 3). The organic phases were combined, washed with saturated brine (100 mL), dried over anhydrous

sodium sulfate, then filtered to remove the drying agent, and concentrated under reduced pressure to remove the solvent to give a crude product of **compound 28B** (10.0 g, yellow solid). MS (ESI): *m/z* 178.0 $[M+1]^+$

**Step 2: synthesis of compound 28C**

The crude product of **compound 28B** (10.0 g, 56.1 mmol) was dissolved in hydrochloric acid (25 mL, 2 mol/L) and water (20 mL), and then a sodium nitrite solid (3.26 g, 56.1 mmol) was added in portions at 0 °C. Then, the mixture was stirred at 0 °C for 4 h. After the reaction was completed as detected by TLC, ice water (150 mL) was added to the reaction liquid, and a solid was precipitated. The mixture was filtered, and the filter cake was washed twice with water, collected, and dried to give a crude product of **compound 28C** (6.5 g, yellow solid). MS (ESI): *m/z* 188.9 $[M+1]^+$

**Step 3: synthesis of compound 28D**

**Compound 28C** (2.0 g, 10.6 mmol) was dissolved in N-methyl-2-pyrrolidone (20 mL), and then *N,N*-diisopropylethylamine (4.1 g, 31.8 mmol) and **4,4-difluoropiperidine hydrochloride** (2.2 g, 13.8 mmol) were added. Then, the mixture was stirred at 100 °C for 16 h. After the reaction was completed as detected by TLC, the reaction liquid was cooled to room temperature, diluted with water (50 mL), and then extracted with ethyl acetate (20 mL × 3). The organic phases were combined, washed with saturated brine (30 mL × 3), dried over anhydrous sodium sulfate, then filtered to remove the drying agent, and concentrated under reduced pressure to remove the solvent, and the residue was purified by silica gel column chromatography to give **compound 28D** (2.6 g, yellow oil, yield: 89.6%). MS (ESI): *m/z* 274.1 $[M+1]^+$

**Step 4: synthesis of compound 28E**

**Compound 28D** (1.0 g, 3.7 mmol) was dissolved in tetrahydrofuran (10 mL), and then 3,4-dihydro-2H-pyran (615 mg, 7.4 mmol) and p-toluenesulfonic acid monohydrate (69 mg, 0.37 mmol) were added. Then, the mixture was stirred at room temperature for 5 h. After the reaction was completed as detected by TLC, the reaction liquid was diluted with water (20 mL) and extracted with ethyl acetate (30 mL × 3). The organic phases were combined, washed with saturated brine (30 mL), dried over anhydrous sodium sulfate, then filtered to remove the drying agent, and concentrated under reduced pressure to remove the solvent, and the residue was purified by silica gel column chromatography to give a crude product of **compound 28E** (1.6 g, yellow solid). MS (ESI): *m/z* 358.1 $[M+1]^+$

**Step 5: synthesis of compound 28F**

**Compound 28E** (1.6 g, 4.5 mmol) was dissolved in dioxane (16 mL), and then ***tert*-butyl carbamate** (787 mg, 6.8 mmol), cesium carbonate (4.38 g, 13.5 mmol), 2-dicyclohexylphosphino-2',4',6'-triisopropylbiphenyl (213 mg, 0.45 mmol), and tris(dibenzylideneacetone)dipalladium (205 mg, 0.23 mmol) were added. Then, the mixture was stirred at 100 °C for 16 h under a nitrogen atmosphere. After the reaction was completed as detected by TLC, the reaction liquid was cooled to room temperature, diluted with water (50 mL), and extracted with ethyl acetate (30 mL × 2). The organic phases were combined, washed with saturated brine (30 mL), dried over anhydrous sodium sulfate, filtered, and concentrated under reduced pressure to remove the solvent, and the residue was purified by a silica gel column to give compound 28F (1.7 g, yellow oil, yield: 88%). MS (ESI): *m/z* 439.3 $[M+1]^+$

**Step 6: synthesis of compound 28G**

**Compound 28F** (1.7 g, 4.0 mmol) was dissolved in toluene (20 mL), and then 300-400 mesh silica gel (1.7 g) was added. Then, the mixture was stirred at 120 °C for 16 h. After the reaction was completed as detected by TLC, the reaction liquid was filtered. The filter cake was washed with dichloromethane/methanol (5/1, 30 mL), and the filtrate was concentrated under reduced pressure to remove the solvent to give a crude product of **compound 28G** (860 mg, brown oil). MS (ESI): *m/z* 339.2 $[M+1]^+$

**Step 7: synthesis of compound 28H**

**Compound 28G** (860 mg, 2.5 mmol) and **compound 1E1** (732 mg, 2.4 mmol) were dissolved in pyridine (8 mL), and then phosphorus oxychloride (1.92 g, 12.5 mmol) was added dropwise under an ice bath. Then, the mixture was stirred at room temperature for 3 h. After the reaction was completed as detected by TLC, the reaction liquid was concentrated to dryness by rotary evaporation, diluted with ice water (20 mL) and adjusted to pH = 5 with 4 N hydrochloric acid, and then extracted with ethyl acetate (30 mL × 3). The organic phases were combined, washed with saturated brine (20 mL), dried over anhydrous sodium sulfate, filtered, and concentrated under reduced pressure to remove the solvent, and the residue

was purified by a silica gel column to give **compound 28H** (162 mg, yellow solid, yield: 12%). MS (ESI): *m/z* 273.7 274.8 [(M-84)/2+1]$^+$

**Step 8: synthesis of compound 28I**

**Compound 28H** (162 mg, 0.3 mmol) and **2-hydroxyethane-1-sulfonamide** (53 mg, 0.4 mmol) were dissolved in *N,N*-dimethylformamide (5 mL), and then cuprous iodide (37 mg, 0.18 mmol), potassium phosphate (200 mg, 0.87 mmol), and (1R,2R)-(-)-N,N'-dimethyl-1,2-cyclohexanediamine (40 mg, 0.26 mmol) were sequentially added. Then, the mixture was stirred at 120 °C for 2 h under a nitrogen atmosphere. After the reaction was completed as detected by TLC, the reaction liquid was diluted with water (10 mL) and extracted with ethyl acetate (8 mL × 3). The organic phases were combined, washed with saturated brine (8 mL), dried over anhydrous sodium sulfate, filtered, and concentrated under reduced pressure to remove the solvent, and the residue was purified by a silica gel column to give **compound 28I** (25 mg, white solid). MS (ESI): *m/z* 675.2 [M+1]$^+$

**Step 9: synthesis of compound 28**

**Compound 28I** (25 mg) was dissolved in a solution of hydrochloric acid in ethanol (2 mL, 4 mol/L), and the mixture was stirred at 25 °C for 0.5 h. After the reaction was completed as detected by TLC, the mixture was concentrated under reduced pressure to remove the solvent, and the residue was purified by preparative HPLC to give compound 28 (8 mg, white solid). MS (ESI): *m/z* 591.3 [M+1]$^+$。 $^1$H NMR (300 MHz, DMSO-$d_6$) δ(ppm) 12.97 (s, 1H), 10.29 (br.s, 1H), 8.09 (d, *J* = 8.6 Hz, 1H), 7.85 (s, 1H), 7.28 (d, *J* = 2.2 Hz, 1H), 7.13 (dd, *J* = 8.6, 2.1 Hz, 1H), 4.99 (br.s, 1H), 4.50 - 4.28 (m, 4H), 3.76 (t, *J* = 6.4 Hz, 2H), 3.36 (t, *J* = 6.4 Hz, 2H), 3.05 - 2.94 (m, 4H), 2.45 - 2.02 (m, 5H), 1.87 - 1.61 (m, 3H), 0.41 (s, 4H).

**Example 29: Preparation of Compound 29**

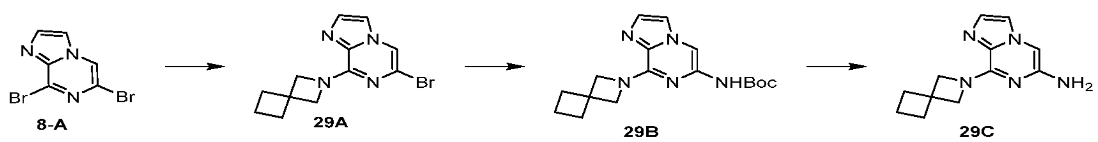
8-A
29A
29B
29C

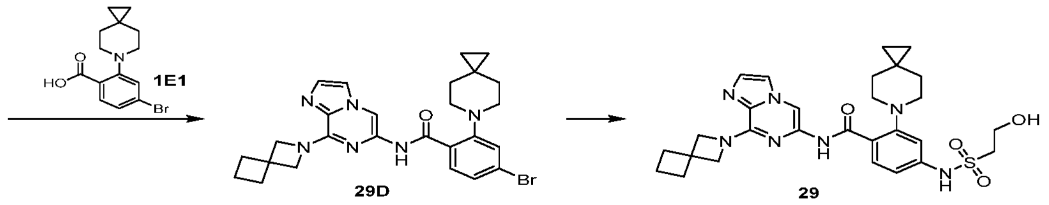
1E1
29D
29

**Step 1: synthesis of compound 29A**

**Compound 8-A** (1.1 g, 3.9 mmol) was dissolved in N-methyl-2-pyrrolidone (12 mL), and then N,N-diisopropylethylamine (2.06 g, 15.6 mmol) and 2-azaspiro[3.3]heptane hemioxalate (792 mg, 2.7 mmol) were added. Then, the mixture was stirred at 100 °C for 5 h under a nitrogen atmosphere. After the reaction was completed as detected by TLC, the reaction liquid was diluted with water (30 mL) at 0 °C and extracted with ethyl acetate (50 mL × 3). The organic phases were combined, washed with saturated brine (30 mL × 3), dried over anhydrous sodium sulfate, then filtered to remove the drying agent, and concentrated under reduced pressure to remove the solvent, and the residue was purified by silica gel column chromatography to give **compound 29A** (1.1 g, yellow oil, yield: 94.8%). MS (ESI): *m/z* 293.0 295.0 [M+1]$^+$

**Step 2: synthesis of compound 29B**

**Compound 29A** (600 mg, 2.1 mmol) was dissolved in dioxane (7 mL), and then ***tert*-butyl carbamate** (360 mg, 3.2 mmol), cesium carbonate (1.33 g, 4.2 mmol), 2-dicyclohexylphosphino-2',4',6'-triisopropylbiphenyl (92 mg, 0.2 mmol), and palladium acetate (24 mg, 0.1 mmol) were added. Then, the mixture was stirred at 100 °C for 5 h under a nitrogen atmosphere. After the reaction was completed as detected by TLC, the reaction liquid was cooled to room temperature, diluted with water (20 mL), and extracted with ethyl acetate (30 mL × 3). The organic phases were combined, washed with

saturated brine (15 mL), dried over anhydrous sodium sulfate, then filtered to remove the drying agent, and concentrated under reduced pressure to remove the solvent, and the residue was purified by a silica gel column to give **compound 29B** (550 mg, yellow oil, yield: 80.9%). MS (ESI): *m*/*z* 330.2 [M+1]$^+$

**Step 3: synthesis of compound 29C**

**Compound 29B** (550 mg, 1.7 mmol) was dissolved in dichloromethane (7 mL), and then trifluoroacetic acid (1.5 mL) was added. Then, the mixture was stirred at room temperature for 2 h. After the reaction was completed as detected by TLC, the mixture was concentrated under reduced pressure to remove the solvent to give a crude product of **compound 29C** (330 mg, brown oil). MS (ESI): *m*/*z* 230.1 [M+1]$^+$

**Step 4: synthesis of compound 29D**

**Compound 1E1** (440 mg, 1.4 mmol) was dissolved in dichloromethane (5 mL), and then oxalyl chloride (451 mg, 3.5 mmol) was added dropwise under an ice bath. Then, the mixture was stirred at room temperature for 30 min. After the reaction was completed as detected by TLC, the reaction liquid was concentrated to dryness by rotary evaporation, and the residue was dissolved in anhydrous tetrahydrofuran (5 mL). **Compound 29C** (330 mg, 1.4 mmol) and N,N-diisopropylethylamine (737 mg, 5.6 mmol) were added to the reaction liquid at room temperature, and then the mixture was stirred at room temperature for 1 h. After the reaction was completed as detected by TLC, the reaction liquid was diluted with water (20 mL) and extracted with ethyl acetate (15 mL × 3). The organic phases were combined, washed with saturated brine (15 mL), dried over anhydrous sodium sulfate, filtered, and concentrated under reduced pressure to remove the solvent, and the residue was purified by a silica gel column to give **compound 29D** (648 mg, yellow solid, yield: 85.7%). MS (ESI): *m*/*z* 261.3 262.4 [M/2+1]$^+$

**Step 5: synthesis of compound 29**

**Compound 29D** (120 mg, 0.23 mmol) and **2-hydroxyethane-1-sulfonamide** (37 mg, 0.3 mmol) were dissolved in *N,N*-dimethylformamide (5 mL), and then cuprous iodide (26 mg, 0.13 mmol), potassium phosphate (144 mg, 0.67 mmol), and (1R,2R)-(-)-N,N'-dimethyl-1,2-cyclohexanediamine (28 mg, 0.2 mmol) were sequentially added. Then, the mixture was stirred at 120 °C for 2 h under a nitrogen atmosphere. After the reaction was completed as detected by TLC, the reaction liquid was diluted with water (10 mL) and extracted with ethyl acetate (8 mL × 3). The organic phases were combined, washed with saturated brine (8 mL), dried over anhydrous sodium sulfate, filtered, and concentrated under reduced pressure to remove the solvent, and the residue was purified by preparative HPLC to give **compound 29** (5 mg, white solid). MS (ESI): *m*/*z* 566.0 [M+1]$^+$。 $^1$H NMR (300 MHz, DMSO-$d_6$) δ(ppm) 13.07 (s, 1H), 10.19 (s, 1H), 8.62 (s, 1H), 8.06 (d, *J* = 8.6 Hz, 1H), 7.99 (d, *J* = 1.0 Hz, 1H), 7.49 (d, *J* = 1.0 Hz, 1H), 7.28 (d, *J* = 2.1 Hz, 1H), 7.14 (dd, *J* = 8.6, 2.1 Hz, 1H), 4.94 (br.s, 1H), 4.43 - 4.31 (m, 4H), 3.76 (t, *J* = 6.4 Hz, 2H), 3.37 (t, *J* = 6.4 Hz, 2H), 3.03 - 2.94 (m, 4H), 2.22 (t, *J* = 7.5 Hz, 5H), 1.93 - 1.69 (m, 5H), 0.43 (s, 4H).

**Example 30: Preparation of Compound 30**

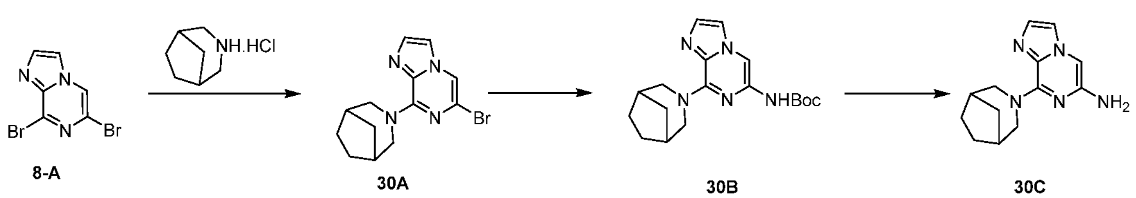

8-A 30A 30B 30C

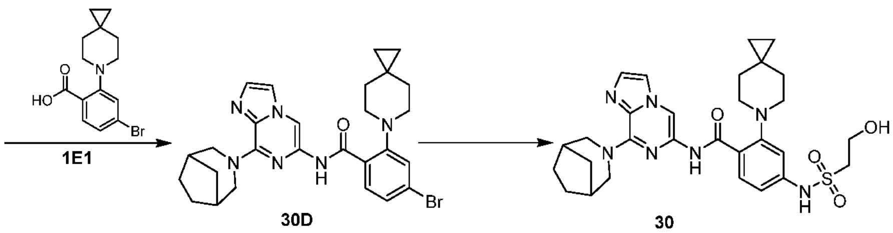


30D 30

**Step 1: synthesis of compound 30A**

**Compound 8-A** (1 g, 3.9 mmol) was dissolved in *N*-methyl-2-pyrrolidone (12 mL), and then *N,N*-diisopropylethylamine (2.06 g, 15.6 mmol) and **3-azabicyclo[3.2.1]octane hydrochloride** (1.6 g, 11.7 mmol) were added. Then, the mixture was stirred at 100 °C for 5 h under a nitrogen atmosphere. After the reaction was completed as detected by TLC, the reaction liquid was diluted with water (30 mL) at 0 °C and extracted with ethyl acetate (50 mL × 3). The organic phases were combined, washed with saturated brine (30 mL × 3), dried over anhydrous sodium sulfate, then filtered to remove the drying agent, and concentrated under reduced pressure to remove the solvent, and the residue was purified by silica gel column chromatography to give **compound 30A** (260 mg, yellow oil, yield: 24%). MS (ESI): *m*/*z* 307.0 309.0 $[M+1]^+$

**Step 2: synthesis of compound 30B**

**Compound 30A** (260 mg, 0.85 mmol) was dissolved in dioxane (7 mL), and then ***tert*-butyl** carbamate (148 mg, 1.27 mmol), cesium carbonate (559 mg, 1.7 mmol), 2-dicyclohexylphosphino-2',4',6'-triisopropylbiphenyl (39 mg, 0.09 mmol), and palladium acetate (13 mg, 0.04 mmol) were added. Then, the mixture was stirred at 100 °C for 5 h under a nitrogen atmosphere. After the reaction was completed as detected by TLC, the reaction liquid was cooled to room temperature, diluted with water (20 mL), and extracted with ethyl acetate (30 mL × 3). The organic phases were combined, washed with saturated brine (15 mL), dried over anhydrous sodium sulfate, then filtered to remove the drying agent, and concentrated under reduced pressure to remove the solvent, and the residue was purified by a silica gel column to give **compound 30B** (234 mg, yellow oil, yield: 80%). MS (ESI): *m*/*z* 344.2 $[M+1]^+$

**Step 3: synthesis of compound 30C**

**Compound 30B** (234 mg, 0.68 mmol) was dissolved in dichloromethane (7 mL), and then trifluoroacetic acid (1.5 mL) was added. Then, the mixture was stirred at room temperature for 2 h. After the reaction was completed as detected by TLC, the mixture was concentrated under reduced pressure to remove the solvent to give a crude product of **compound 30C** (164 mg, brown oil). MS (ESI): *m*/*z* 244.1 $[M+1]^+$

**Step 4: synthesis of compound 30D**

**Compound 1E1** (207 mg, 0.67 mmol) was dissolved in dichloromethane (5 mL), and then oxalyl chloride (214 mg, 1.67 mmol) was added dropwise under an ice bath. Then, the mixture was stirred at room temperature for 30 min. After the reaction was completed as detected by TLC, the reaction liquid was concentrated to dryness by rotary evaporation, and the residue was dissolved in anhydrous tetrahydrofuran (5 mL). **Compound 30C** (164 mg, 0.67 mmol) and N,N-diisopropylethylamine (348 mg, 2.68 mmol) were added to the reaction liquid at room temperature, and then the mixture was stirred at room temperature for 1 h. After the reaction was completed as detected by TLC, the reaction liquid was diluted with water (20 mL) and extracted with ethyl acetate (15 mL × 3). The organic phases were combined, washed with saturated brine (15 mL), dried over anhydrous sodium sulfate, filtered, and concentrated under reduced pressure to remove the solvent, and the residue was purified by a silica gel column to give **compound 30D** (132 mg, yellow solid, yield: 37.3%). MS (ESI): *m*/*z* 268.3 269.0 $[M/2+1]^+$

**Step 5: synthesis of compound 30**

**Compound 30D** (132 mg, 0.25 mmol) and **2-hydroxyethane-1-sulfonamide** (33 mg, 0.25 mmol) were dissolved in *N,N*-dimethylformamide (5 mL), and then cuprous iodide (26 mg, 0.15 mmol), potassium phosphate (152 mg, 0.73 mmol), and (1*R*,2*R*)-(-)-*N,N'*-dimethyl-1,2-cyclohexanediamine (20 mg, 0.22 mmol) were sequentially added. Then, the mixture was stirred at 120 °C for 2 h under a nitrogen atmosphere. After the reaction was completed as detected by TLC, the reaction liquid was diluted with water (10 mL) and extracted with ethyl acetate (8 mL × 3). The organic phases were combined, washed with saturated brine (8 mL), dried over anhydrous sodium sulfate, filtered, and concentrated under reduced pressure to remove the solvent, and the residue was purified by preparative HPLC to give **compound 30** (25 mg, white solid). MS (ESI): *m*/*z* 290.7$[M/2+1]^+$。$^1H$ NMR (300 MHz, DMSO-$d_6$) δ(ppm) 12.83 (s, 1H), 10.34 (br.s, 1H), 8.71 (s, 1H), 8.20 - 7.93 (m, 2H), 7.50 (s, 1H), 7.27 (s, 1H), 7.13 (d, *J* = 8.4 Hz, 1H), 5.27 (s, 2H), 4.98 (br.s, 1H), 3.76 (t, *J* = 6.5 Hz, 2H), 3.35 (t, *J* = 6.4 Hz, 2H), 3.09 (d, *J* = 12.6 Hz, 2H), 3.03 - 2.92 (m, 4H), 2.39 - 2.28 (m, 2H), 1.97 - 1.42 (m, 10H), 0.39 (s, 4H).

**Example 31: Preparation of Compound 31**

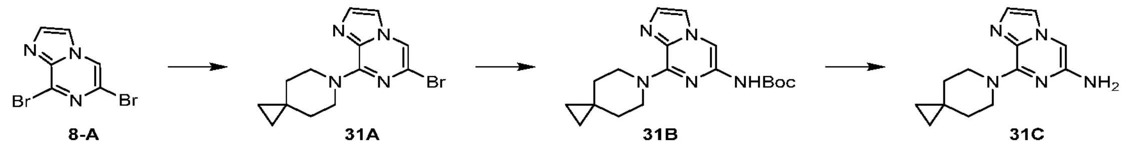

8-A 31A 31B 31C

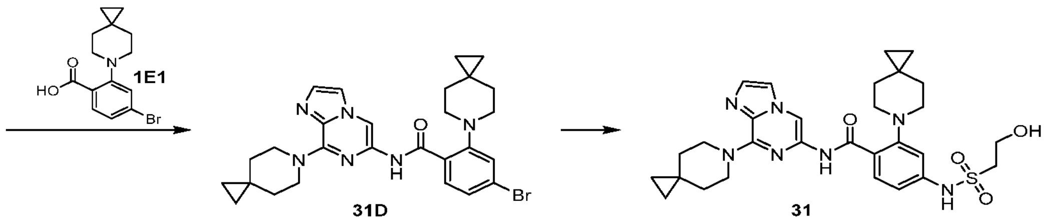

1E1 31D 31

**Step 1: synthesis of compound 31A**

Compound 8-A (1.0 g, 3.61 mmol) was dissolved in N-methylpyrrolidone (10 mL), and then *N,N*-diisopropylethylamine (930 mg, 7.22 mmol) and 6-aza-spiro[2.5]octane hydrochloride (640 mg, 4.33 mmol) were added. Then, the mixture was stirred at 100 °C for 16 h. After the reaction was completed as detected by TLC, the reaction liquid was cooled to room temperature, diluted with water (100 mL), and extracted with ethyl acetate (100 mL × 3). The organic phases were combined, washed with saturated brine (70 mL × 3), dried over anhydrous sodium sulfate, filtered, and concentrated under reduced pressure to remove the solvent, and the residue was purified by silica gel column chromatography and concentrated to dryness by rotary evaporation under reduced pressure to give compound 31A (1.0 g, pink solid, yield: 86.2%). MS (ESI): *m/z* 307.0 309.0 $[M+1]^+$

**Step 2: synthesis of compound 31B**

Compound 31A (500 mg, 1.63 mmol) was dissolved in dioxane (5 mL), and then *tert-butyl* carbamate (382 mg, 3.26 mmol), 2-dicyclohexylphosphino-2',4',6'-triisopropylbiphenyl (156 mg, 0.33 mmol), cesium carbonate (1.06 mg, 3.26 mmol), and palladium acetate (37 mg, 0.16 mmol) were added. Then, the mixture was allowed to react at 100 °C for 16 h under a nitrogen atmosphere. After the reaction was completed as detected by TLC, the reaction liquid was cooled to room temperature, diluted with water (50 mL), and extracted with ethyl acetate (60 mL × 3). The organic phases were combined, dried over anhydrous sodium sulfate, filtered, and concentrated under reduced pressure to remove the solvent, and the residue was purified by silica gel column chromatography to give compound 31B (400 mg, yellow solid, yield: 71.6%). MS (ESI): *m/z* 344.1$[M+1]^+$

**Step 3: synthesis of compound 31C**

**Compound 31B** (100 mg, 0.29 mmol) was dissolved in dichloromethane (2 mL), and then trifluoroacetic acid (0.4 mL) was added. The mixture was stirred at room temperature for 16 h. After the reaction was completed as detected by TLC, the mixture was concentrated under reduced pressure to remove the solvent to give a **crude product of compound 31C** (100 mg, yellow oil). MS (ESI): m/z 244.1$[M+1]^+$

**Step 4: synthesis of compound 31D**

**Compound 1E1** (89 mg, 0.29 mmol) was dissolved in dichloromethane (2 mL), and then oxalyl chloride (91 mg, 0.73 mmol) was added dropwise under an ice bath. Then, the mixture was stirred at room temperature for 30 min. After the reaction was completed as detected by TLC, the reaction liquid was concentrated to dryness by rotary evaporation, and then the residue was dissolved in anhydrous tetrahydrofuran (5 mL). A mixed solution of **compound 31C** (70 mg, 0.29 mmol) and *N,N*-diisopropylethylamine (148 mg, 1.16 mmol) in tetrahydrofuran was added at room temperature, and then the reaction liquid was stirred at 50 °C for 2 h. After the reaction was completed as detected by TLC, the reaction liquid was diluted with water (30 mL) and extracted with ethyl acetate (30 mL × 3). The organic phases were combined, dried over anhydrous sodium sulfate, filtered, and concentrated under reduced pressure to remove the solvent, and the residue was purified by a silica gel column to give **compound 31D** (80 mg, yellow solid, purity: 65.8%, yield: 31.7%). MS (ESI): *m/z* 268.1 269.1 $[M/2+1]^+$

**Step 5: synthesis of compound 31**

**Compound 31D** (70 mg, 0.13 mmol) and **2-hydroxyethane-1-sulfonamide** (20 mg, 0.16 mmol) were dissolved in *N,N*-dimethylformamide (2 mL), and then cuprous iodide (3 mg, 0.01 mmol), potassium phosphate (56 mg, 0.26 mmol), and (1*R*,2*R*)-(-)-*N,N'*-dimethyl-1,2-cyclohexanediamine (5 mg, 0.03 mmol) were sequentially added. Then, the mixture was stirred at 120 °C for 2 h under a nitrogen atmosphere. After the reaction was completed as detected by TLC, the reaction liquid was diluted with water (10 mL) and extracted with ethyl acetate (10 mL × 3). The organic phases were combined, washed with saturated brine (8 mL × 3), dried over anhydrous sodium sulfate, filtered, and concentrated under reduced pressure to remove the solvent, and the residue was purified by preparative HPLC to give **compound 31** (15 mg, off-white solid). MS (ESI): *m/z* 290.7[M/2+1]$^+$。 $^1$H NMR (300 MHz, DMSO-$d_6$) δ(ppm) 12.77 (s, 1H), 10.19 (br.s, 1H), 8.74 (s, 1H), 8.14 - 7.93 (m, 2H), 7.51 (d, *J* = 1.1 Hz, 1H), 7.27 (d, *J = 2.1* Hz, 1H), 7.13 (dd, *J* = 8.6, 2.1 Hz, 1H), 4.94 (br.s, 1H), 4.40 - 4.29 (m, 4H), 3.76 (t, *J* = 6.5 Hz, 2H), 3.35 (t, *J* = 6.5 Hz, 2H), 3.02 - 2.93 (m, 4H), 1.91 - 1.40 (m, 8H), 0.39 (s, 4H), 0.36 (s, 4H).

**Example 32: Preparation of Compound 32**

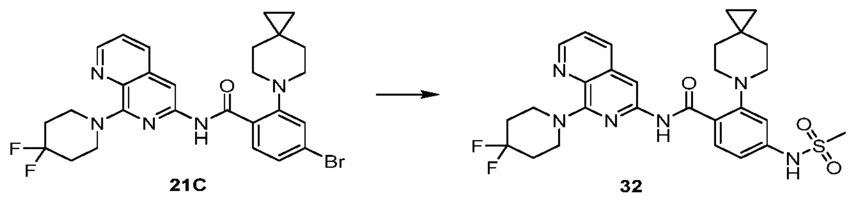

21C 32

**Step 1: synthesis of compound 32**

**Compound 21C** (100 mg, 0.18 mmol) and **methanesulfonamide** (21 mg, 0.22 mmol) were dissolved in N,N-dimethylformamide (2 mL), and then cuprous iodide (4 mg, 0.02 mmol), potassium phosphate (78 mg, 0.36 mmol), and (1R,2R)-(-)-N,N'-dimethyl-1,2-cyclohexanediamine (5 mg, 0.04 mmol) were sequentially added. Then, the mixture was stirred at 120 °C for 2 h under a nitrogen atmosphere. After the reaction was completed as detected by TLC, the reaction liquid was diluted with water (15 mL) and extracted with ethyl acetate (20 mL × 3). The organic phases were combined, washed with saturated brine (8 mL × 3), dried over anhydrous sodium sulfate, filtered, and concentrated under reduced pressure to remove the solvent, and the residue was purified by preparative HPLC to give **compound 32** (30 mg, white solid). MS (ESI): *m/z* 286.2 [M/2+1]$^+$. $^1$H NMR (300 MHz, DMSO-$d_6$) δ(ppm) 12.90 (s, 1H), 10.25 (s, 1H), 8.72 (dd, *J* = 4.1, 1.7 Hz, 1H), 8.24 (dd, *J* = 8.5, 1.7 Hz, 1H), 8.17 - 8.04 (m, 2H), 7.61 (dd, *J* = 8.4, 4.1 Hz, 1H), 7.27 (d, *J* = 2.1 Hz, 1H), 7.14 (dd, *J* = 8.6, 2.1 Hz, 1H), 4.24 - 4.16 (m, 4H), 3.13 (s, 3H), 3.05 - 2.96 (m, 4H), 2.27 - 2.07 (m, 5H), 1.86 - 1.61 (m, 3H), 0.40 (s, 4H).

**Example 33: Preparation of Compound 33**

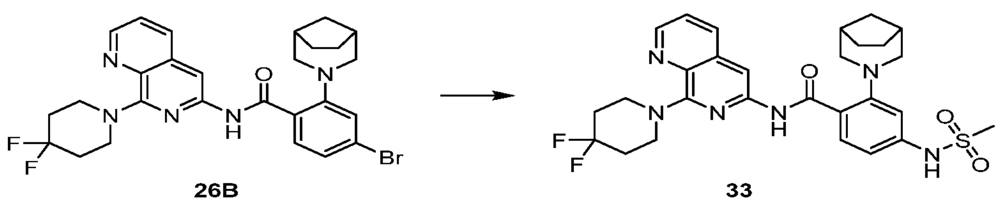

26B 33

**Step 3: synthesis of compound 33**

**Compound 26B** (800 mg, 0.14 mmol) and **methanesulfonamide** (17 mg, 0.17 mmol) were dissolved in *N,N*-dimethylformamide (2 mL), and then cuprous iodide (3 mg, 0.01 mmol), potassium phosphate (61 mg, 0.28 mmol), and (1R,2R)-(-)-N,N'-dimethyl-1,2-cyclohexanediamine (4 mg, 0.03 mmol) were sequentially added. Then, the mixture was stirred at 120 °C for 2 h under a nitrogen atmosphere. After the reaction was completed as detected by TLC, the reaction liquid was diluted with water (10 mL) and extracted with ethyl acetate (20 mL × 3). The organic phases were combined,

washed with saturated brine (8 mL × 3), dried over anhydrous sodium sulfate, filtered, and concentrated under reduced pressure to remove the solvent, and the residue was purified by preparative HPLC to give **compound 33** (15 mg, white solid).

MS (ESI): *m/z* 286.2 $[M/2+1]^+$.

$^1$H NMR (300 MHz, DMSO-$d_6$) δ(ppm) 10.49 (s, 1H), 10.04 (s, 1H), 8.71 (dd, *J* = 4.1, 1.7 Hz, 1H), 8.25 (dd, *J* = 8.5, 1.8 Hz, 1H), 8.12 (s, 1H), 7.71 - 7.54 (m, 2H), 7.11 (d, *J* = 2.1 Hz, 1H), 6.99 (dd, *J* = 8.4, 2.0 Hz, 1H), 4.21 - 4.10 (m, 4H), 3.07 (s, 3H), 3.06 - 3.01 (m, 2H), 2.80 (d, *J* = 10.5 Hz, 2H), 2.27 - 2.03 (m, 6H), 1.93 (d, *J* = 7.6 Hz, 2H), 1.58 - 1.44 (m, 4H).

**Example 34: Preparation of Compound 34**

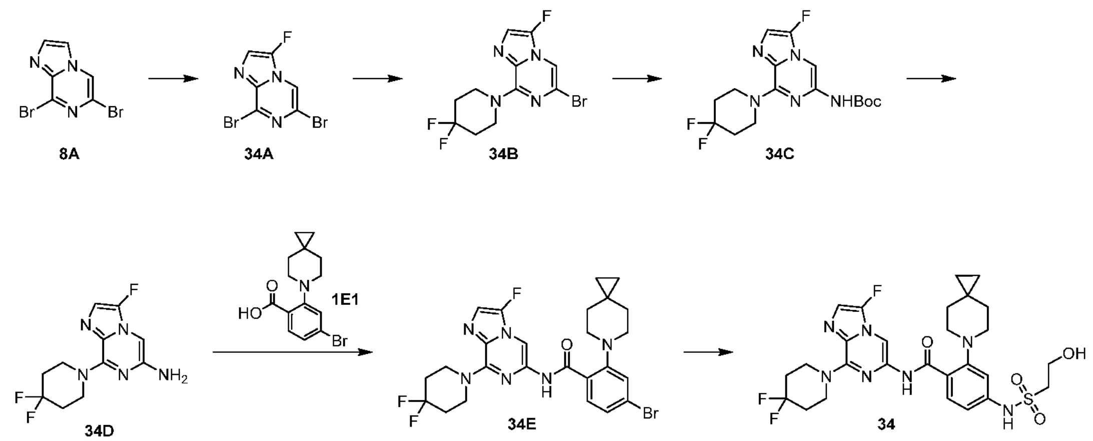

8A 34A 34B 34C 34D 1E1 34E 34

**Step 1: synthesis of compound 34A**

**Compound 8-A** (20 g, 72.2 mmol) was dissolved in acetonitrile (180 mL), and then 1-chloromethyl-4-fluoro-1,4-diazobicyclo[2.2.2]octane bis(tetrafluoroborate) (28.1 g, 79.4 mmol) was added at room temperature. Then, the mixture was stirred at 80 °C for 5 h. After the reaction was completed as detected by LCMS, the reaction liquid was concentrated under reduced pressure to remove the solvent, and the residue was purified by silica gel column chromatography to give a crude product, which was then purified by a reversed-phase column to give **34A** (3.1 g, yellow solid, yield: 14.5%). MS (ESI): *m/z* 295.8 $[M+1]^+$

**Step 2: synthesis of compound 34B**

Compound 34A (1.0 g, 3.39 mmol) was dissolved in N-methylpyrrolidone (10 mL), and then*N,N*-diisopropylethylamine (884 mg, 6.78 mmol) and 4,4-difluoropiperidine hydrochloride (650 mg, 4.07 mmol) were added. Then, the mixture was stirred at 100 °C for 16 h. After the reaction was completed as detected by TLC, the reaction liquid was cooled to room temperature, diluted with water (80 mL), and extracted with ethyl acetate (80 mL × 3). The organic phases were combined, dried over anhydrous sodium sulfate, filtered, and concentrated under reduced pressure to remove the solvent, and the residue was purified by silica gel column chromatography to give compound 34B (1.0 g, pale yellow solid, purity: 73.5%, yield: 64.7%). MS (ESI): *m/z* 335.0 337.0 $[M+1]^+$

**Step 3: synthesis of compound 34C**

Compound 34B (400 mg, 1.19 mmol) was dissolved in dioxane (10 mL), and then *tert-butyl* carbamate (280 mg, 2.38 mmol), 2-dicyclohexylphosphino-2',4',6'-triisopropylbiphenyl (57 mg, 0.12 mmol), cesium carbonate (340 mg, 2.38 mmol), and palladium acetate (13 mg, 0.06 mmol) were added. Then, the mixture was stirred at 100 °C for 16 h under a nitrogen atmosphere. After the reaction was completed as detected by TLC, the reaction liquid was cooled to room temperature, diluted with water (50 mL), and extracted with ethyl acetate (40 mL × 3). The organic phases were combined, dried over anhydrous sodium sulfate, filtered, and concentrated under reduced pressure to remove the solvent, and the residue was purified by silica gel column chromatography to give compound 34C (300 mg, off-white solid, purity: 75.2%, yield: 50.9%). MS (ESI): *m/z* 372.1 $[M+1]^+$

**Step 4: synthesis of compound 34D**

**Compound 34C** (300 mg, 0.81 mmol) was dissolved in dichloromethane (5 mL), and then trifluoroacetic acid (1 mL) was added. The mixture was stirred at room temperature for 2 h. After the reaction was completed as detected by TLC, the mixture was concentrated under reduced pressure to remove the solvent to give a **crude product of compound 34D** (300 mg, yellow oil). MS (ESI): *m/z* 272.1[M+1]$^+$

**Step 5: synthesis of compound 34E**

**Compound 1E1** (228 mg, 0.74 mmol) was dissolved in dichloromethane (5 mL), and then oxalyl chloride (234 mg, 1.85 mmol) was added dropwise under an ice bath. Then, the mixture was stirred at room temperature for 30 min. After the reaction was completed as detected by TLC, the reaction liquid was concentrated to dryness by rotary evaporation, and the residue was dissolved in anhydrous tetrahydrofuran (5 mL). A mixed solution of **compound 34D** (200 mg, 0.74 mmol) and N,N-diisopropylethylamine (381 mg, 2.96 mmol) was added at room temperature. Then, the mixture was stirred at 50 °C for 2 h. After the reaction was completed as detected by TLC, the reaction liquid was diluted with water (30 mL) and extracted with ethyl acetate (30 mL × 3). The organic phases were combined, dried over anhydrous sodium sulfate, filtered, and concentrated under reduced pressure to remove the solvent, and the residue was purified by a silica gel column to give **compound 34E** (200 mg, pale yellow solid, purity: 65.8%, yield: 31.7%). MS (ESI): *m/z* 282.8 283.9 [M/2+1]$^+$

**Step 6: synthesis of compound 34**

**Compound 34E** (100 mg, 0.18 mmol) and 2-hydroxyethane-1-sulfonamide (27 mg, 0.22 mmol) were dissolved in *N,N*-dimethylformamide (2 mL), and then cuprous iodide (3 mg, 0.02 mmol), potassium phosphate (75 mg, 0.36 mmol), and (1R,2R)-(-)-N,N'-dimethyl-1,2-cyclohexanediamine (5 mg, 0.04 mmol) were sequentially added. Then, the mixture was stirred at 120 °C for 2 h under a nitrogen atmosphere. After the reaction was completed as detected by LCMS, the reaction liquid was diluted with water (10 mL) and extracted with ethyl acetate (20 mL × 3). The organic phases were combined, washed with saturated brine (8 mL), dried over anhydrous sodium sulfate, filtered, and concentrated under reduced pressure to remove the solvent, and the residue was purified by preparative HPLC to give **compound 34** (40 mg, pale yellow solid). MS (ESI): *m/z* 304.5 [M/2+1]$^+$. $^1$H NMR (300 MHz, DMSO-$d_6$) δ (ppm) 13.03 (s, 1H), 10.30 (br.s, 1H), 8.43 (s, 1H), 8.08 (d, *J* = 8.6 Hz, 1H), 7.40 (d, *J* = 7.2 Hz, 1H), 7.29 (d, *J* = 2.2 Hz, 1H), 7.14 (dd, *J* = 8.6, 2.1 Hz, 1H), 4.95 (br.s, 1H), 4.46 - 4.36 (m, 4H), 3.76 (t, *J* = 6.5 Hz, 2H), 3.36 (t, *J* = 6.5 Hz, 2H), 3.03 - 2.94 (m, 4H), 2.21 - 2.02 (m, 5H), 1.85 - 1.61 (m, 3H), 0.41 (s, 4H).

**Example 35: Preparation of Compound 35**

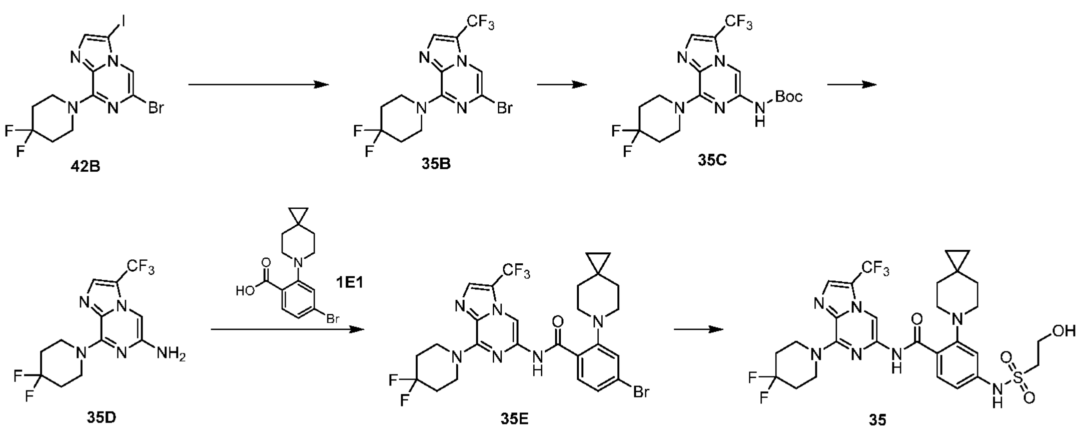

42B 35B 35C
35D 35E 35

**Preparation method:**

**Step 1: synthesis of compound 35B**

**Compound 42B** (1 g, 2.26 mmol) was dissolved in N,N-dimethylformamide (10 mL), and then cuprous iodide

(2.15 g, 11.3 mmol), cesium fluoride (1.03 g, 6.78 mmol), and (trifluoromethyl)trimethylsilane (963 mg, 6.78 mmol) were added. Then, the mixture was stirred at 95 °C for 12 h under a nitrogen atmosphere. After the reaction was completed as detected by LCMS, the reaction liquid was cooled to room temperature, diluted with water (50 mL), and extracted with ethyl acetate (50 mL × 3). The organic phases were combined, washed with saturated brine (50 mL × 3), dried over anhydrous sodium sulfate, filtered, and concentrated under reduced pressure to remove the solvent, and the residue was purified by a silica gel column to give **compound 35B** (110 mg, yellow solid, yield: 12.6%). MS (ESI): *m/z* 384.9 387.0 $[M+1]^+$

**Step 2: synthesis of compound 35C**

**Compound 35B** (110 mg, 0.29 mmol) was dissolved in dioxane (6 mL), and then *tert-butyl* carbamate (55 mg, 0.44 mmol), cesium carbonate (187 g, 0.58 mmol), 2-dicyclohexylphosphino-2',4',6'-triisopropylbiphenyl (14 mg, 0.03 mmol), and palladium acetate (4 mg, 0.02 mmol) were added. Then, the mixture was stirred at 100 °C for 5 h under a nitrogen atmosphere. After the reaction was completed as detected by TLC, the mixture was cooled to room temperature, diluted with water (20 mL), and extracted with ethyl acetate (15 mL × 2). The organic phases were combined, washed with saturated brine (15 mL), dried over anhydrous sodium sulfate, filtered, and concentrated under reduced pressure to remove the solvent, and the residue was purified by a silica gel column to give **compound 35C** (69 mg, yellow oil, yield: 55.1%). MS (ESI): *m/z*422.1 $[M+1]^+$

**Step 4: synthesis of compound 35D**

**Compound 35C** (69 mg, 0.16 mmol) was dissolved in dichloromethane (2 mL), and then trifluoroacetic acid (0.5 mL) was added. After the addition was completed, the mixture was stirred at 25 °C for 2 h. After **the reaction was completed as detected by** TLC, the mixture was concentrated under reduced pressure to remove the solvent to give a crude product of **compound 35D** (50 mg, brown oil). MS (ESI): *m/z* 322.1 $[M+1]^+$

**Step 4: synthesis of compound 35E**

**Compound 1E1** (48 mg, 0.16 mmol) was dissolved in dichloromethane (2 mL), and then oxalyl chloride (49 mg, 0.4 mmol) was added dropwise under an ice bath. Then, the mixture was stirred at room temperature for 30 min. After the reaction was completed as detected by TLC, the reaction liquid was concentrated to dryness by rotary evaporation, and then the residue was dissolved in anhydrous tetrahydrofuran (2 mL). **Compound 35D** (50 mg, 0.16 mmol) and N,N-diisopropylethylamine (80 mg, 0.64 mmol) were added at room temperature, and then the mixture was stirred at room temperature for 1 h. After the reaction was completed as detected by TLC, the reaction liquid was diluted with water (20 mL) and extracted with ethyl acetate (15 mL × 3). The organic phases were combined, dried over anhydrous sodium sulfate, filtered, and concentrated under reduced pressure to remove the solvent, and the residue was purified by a silica gel column to give **compound 35E** (50 mg, yellow solid, yield: 50%).

**Step 5: synthesis of compound 35**

**Compound 35E** (50 mg, 0.08 mmol) and 2-hydroxyethane-1-sulfonamide (13 mg, 0.10 mmol) were dissolved in *N,N*-dimethylformamide (5 mL), and then cuprous iodide (9 mg, 0.05 mmol), potassium phosphate (50 mg, 0.23 mmol), and (1R,2R)-(-)-N,N'-dimethyl-1,2-cyclohexanediamine (10 mg, 0.07 mmol) were sequentially added. Then, the mixture was stirred at 120 °C for 2 h under a nitrogen atmosphere. After the reaction was completed as detected by TLC, the reaction liquid was diluted with water (10 mL) and extracted with ethyl acetate (10 mL × 3). The organic phases were combined, washed with saturated brine (8 mL), dried over anhydrous sodium sulfate, filtered, and concentrated under reduced pressure to remove the solvent, and the residue was purified by preparative HPLC to give **compound 35** (15 mg, white solid). MS (ESI): *m/z* 329.7 $[M/2+1]^+$ $^1$H NMR (300 MHz, DMSO-$d_6$) δ(ppm) 13.22 (s, 1H), 10.27 (br.s, 1H), 8.80 (s, 1H), 8.21 - 8.01 (m, 2H), 7.29 (s, 1H), 7.14 (d, *J* = 8.3 Hz, 1H), 4.95 (br.s, 1H), 4.41 - 4.33 (m, 4H), 3.76 (t, *J* = 6.4 Hz, 2H), 3.36 (t, *J* = 6.4 Hz, 2H), 3.05 - 2.92 (m, 4H), 2.28 - 2.04 (m, 5H), 1.87 - 1.59 (m, 3H), 0.41 (s, 4H).

**Example 36: Preparation of Compound 36**

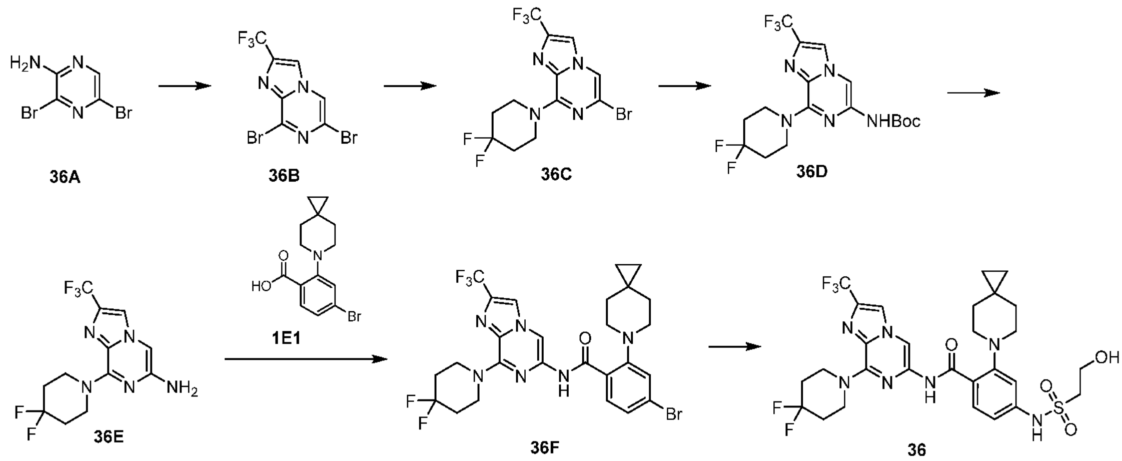

36A 36B 36C 36D 1E1 36E 36F 36

**Preparation method:**

**Step 1: synthesis of compound 36B**

Compound 36A (1.0 g, 3.95 mmol) was dissolved in N,N-dimethylacetamide (10 mL), and then 3-bromo-1,1,1-trifluoroacetone (1.9 g, 9.88 mmol) was added. Then, the mixture was stirred at 90 °C for 16 h. After the reaction was completed as detected by TLC, the reaction liquid was cooled to room temperature, diluted with water (50 mL), and extracted with ethyl acetate (50 mL × 3). The organic phases were combined, washed with saturated brine (50 mL × 2), dried over anhydrous sodium sulfate, filtered, and concentrated under reduced pressure to remove the solvent, and the residue was purified by silica gel column chromatography to give a crude product of compound 36B (600 mg, brown oil). MS (ESI): *m/z* 345.8 $[M+1]^+$

**Step 2: synthesis of compound 36C**

Compound 36B (600 mg, 1.74 mmol) was dissolved in N-methylpyrrolidone (10 mL), and then N,N-diisopropylethylamine (451 mg, 3.48 mmol) and 4,4-difluoropiperidine hydrochloride (332 mg, 2.09 mmol) were added. Then, the mixture was stirred at 100 °C for 4 h. After the reaction was completed as detected by TLC, the reaction liquid was cooled to room temperature, diluted with water (80 mL), and extracted with ethyl acetate (40 mL × 3). The organic phases were combined, dried over anhydrous sodium sulfate, filtered, and concentrated under reduced pressure to remove the solvent, and the residue was purified by silica gel column chromatography to give compound 36C (400 mg, pale yellow solid, purity: 71.8%, yield: 42.9%). MS (ESI): *m/z* 385.0 387.0 $[M+1]^+$

**Step 3: synthesis of compound 36D**

Compound 36C (200 mg, 0.52 mmol) was dissolved in dioxane (5 mL), and then *tert-butyl* carbamate (122 mg, 1.04 mmol), 2-dicyclohexylphosphino-2',4',6'-triisopropylbiphenyl (25 mg, 0.05 mmol), cesium carbonate (340 mg, 1.04 mmol), and palladium acetate (6 mg, 0.03 mmol) were added. Then, the mixture was stirred at 100 °C for 16 h under a nitrogen atmosphere. After the reaction was completed as detected by TLC, the reaction liquid was cooled to room temperature, diluted with water (50 mL), and extracted with ethyl acetate (40 mL × 3). The organic phases were combined, dried over anhydrous sodium sulfate, filtered, and concentrated under reduced pressure to remove the solvent, and the residue was purified by silica gel column chromatography to give compound 36D (160 mg, yellow solid, purity: 81.4%, yield: 59.5%). MS (ESI): *m/z* 366.1 $[M-55]^+$

**Step 4: synthesis of compound 36E**

**Compound 36D** (180 mg, 0.43 mmol) was dissolved in dichloromethane (5 mL), and then trifluoroacetic acid (1 mL) was added. The mixture was stirred at room temperature for 2 h. After the reaction was completed as detected by TLC, the mixture was concentrated under reduced pressure to remove the solvent to give a crude product of **compound 36E** (180 mg, yellow oil). MS (ESI): *m/z* 322.1$[M+1]^+$

**Step 5: synthesis of compound 36F**

**Compound 1E1** (135 mg, 0.44 mmol) was dissolved in dichloromethane (5 mL), and then oxalyl chloride (139 mg, 1.10 mmol) was added dropwise under an ice bath. Then, the mixture was stirred at room temperature for 30 min. After the reaction was completed as detected by TLC, the reaction liquid was concentrated to dryness by rotary evaporation, and then the residue was dissolved in anhydrous tetrahydrofuran (5 mL). **Compound 36E** (140 mg, 0.44 mmol) and N,N-diisopropylethylamine (225 mg, 1.76 mmol) were added at room temperature, and then the mixture was stirred at 50 °C for 2 h. After the reaction was completed as detected by TLC, the reaction liquid was diluted with water (30 mL) and extracted with ethyl acetate (30 mL × 3). The organic phases were combined, dried over anhydrous sodium sulfate, filtered, and concentrated under reduced pressure to remove the solvent, and the residue was purified by a silica gel column to give **compound 36F** (200 mg, pale yellow solid, purity: 81.3%, yield: 60.8%). MS (ESI): *m/z* 307.1 308.2 [M/2+1]$^+$

**Step 6: synthesis of compound 36**

**Compound 36F** (100 mg, 0.16 mmol) and 2-hydroxyethane-1-sulfonamide (25 mg, 0.19 mmol) were dissolved in N,N-dimethylformamide (2 mL), and then cuprous iodide (3 mg, 0.02 mmol), potassium phosphate (69 mg, 0.32 mmol), and (1R,2R)-(-)-N,N'-dimethyl-1,2-cyclohexanediamine (5 mg, 0.03 mmol) were sequentially added. Then, the mixture was stirred at 120 °C for 2 h under a nitrogen atmosphere. After the reaction was completed as detected by TLC, the reaction liquid was diluted with water (10 mL) and extracted with ethyl acetate (10 mL × 3). The organic phases were combined, washed with saturated brine (8 mL × 3), dried over anhydrous sodium sulfate, filtered, and concentrated under reduced pressure to remove the solvent, and the residue was purified by preparative HPLC to give compound 36 (38 mg, pale yellow solid). MS (ESI): *m/z* 329.7 [M/2+1]$^+$。 $^1$H NMR (300 MHz, DMSO-$d_6$) δ(ppm) 13.03 (s, 1H), 10.21 (br.s, 1H), 8.88 (s, 1H), 8.71 (d, *J* = 1.0 Hz, 1H), 8.08 (d, *J* = 8.6 Hz, 1H), 7.29 (d, *J* = 2.1 Hz, 1H), 7.15 (dd, *J* = 8.6, 2.1 Hz, 1H), 4.95 (br.s, 1H), 4.44 - 4.32 (m, 4H), 3.76 (t, *J* = 6.5 Hz, 2H), 3.36 (t, *J* = 6.4 Hz, 2H), 3.03 - 2.92 (m, 4H), 2.29 - 1.99 (m, 5H), 1.83 - 1.58 (m, 3H), 0.41 (s, 4H).

**Example 37: Preparation of Compound 37**

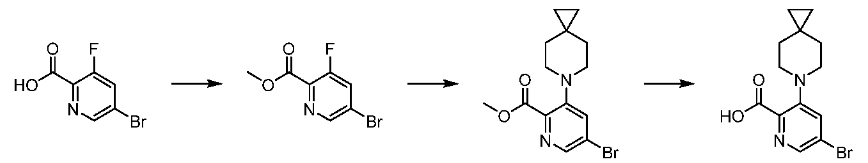

37A 37B 37C 37D

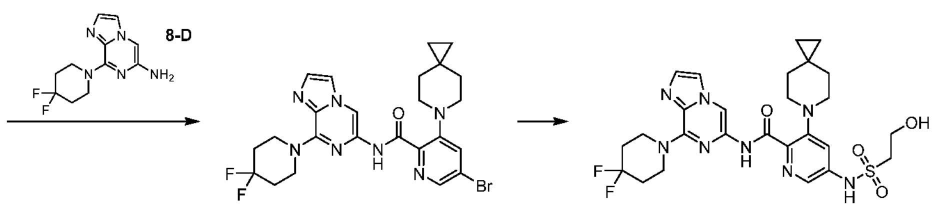


37E 37

**Preparation method:**

**Step 1: synthesis of compound 37B**

**Compound 37A** (900 mg, 4.1 mmol) was dissolved in methanol (10 mL), and then thionyl chloride (974 mg, 8.2 mmol) was added dropwise under an ice bath. Then, the mixture was stirred at room temperature for 12 h. After the reaction was completed as detected by TLC, the reaction liquid was concentrated to dryness by rotary evaporation, and the residue was adjusted to pH = 7 with saturated sodium bicarbonate and then extracted with ethyl acetate (30 mL × 3). The organic phases were combined, washed with saturated brine (20 mL), dried over anhydrous sodium sulfate, filtered, and concentrated under reduced pressure to remove the solvent to give a crude product of **compound 37B** (300 mg, off-white solid).

**Step 2: synthesis of compound 37C**

The crude product of **compound 37B** (900 mg, 3.8 mmol) was dissolved in N-methylpyrrolidone (10 mL), and then 6-aza-spiro[2.5]octane hydrochloride (681 g, 4.6 mmol) and N,N-diisopropylethylamine (2.7 mL, 15.4 mmol) were added. Then, the mixture was stirred at 120 °C for 3 h. After the reaction was completed as detected by LCMS, the reaction liquid was diluted with water (60 mL) under an ice bath and extracted with ethyl acetate (60 mL × 3). The organic phases were combined, washed with saturated brine (30 mL × 3), dried over anhydrous sodium sulfate, filtered, and concentrated under reduced pressure to remove the solvent, and the residue was purified by a silica gel column to give **compound 37C** (1.4 g, yellow solid). MS (ESI): 325.0 327.0 *m/z* $[M+1]^+$

**Step 3: synthesis of compound 37D**

**Compound 37C** (1.34 g, 4.1 mmol) was dissolved in methanol (5 mL) and water (5 mL), and then sodium hydroxide (330 mg, 8.2 mmol) was added. Then, the mixture was stirred at room temperature for 12 h. After the reaction was completed as detected by LCMS, the reaction liquid was adjusted to pH = 6 with a hydrochloric acid solution (1 mol/L) under an ice bath and extracted with ethyl acetate (60 mL × 3). The organic phases were combined, washed with saturated brine (30 mL × 2), dried over anhydrous sodium sulfate, filtered, and concentrated under reduced pressure to remove the solvent, and the residue was purified by a silica gel column to give **compound 37D** (1 g, off-white solid). MS (ESI): 311.0 313.0 *m/z* $[M+1]^+$

**Step 4: synthesis of compound 37E**

**Compound 37D** (372 mg, 1.18 mmol) was dissolved in dichloromethane (5 mL), and then oxalyl chloride (378 mg, 2.96 mmol) was added dropwise under an ice bath. Then, the mixture was stirred at room temperature for 30 min. After the reaction was completed as detected by TLC, the reaction liquid was concentrated to dryness by rotary evaporation, and then the residue was dissolved in anhydrous tetrahydrofuran (8 mL). A mixture of **compound 8-D** (300 mg, 1.18 mmol) and N,N-diisopropylethylamine (462 mg, 4.72 mmol) was added at room temperature, and then the reaction liquid was stirred at 50 °C for 1 h. After the reaction was completed as detected by TLC, the reaction liquid was diluted with water (50 mL) and extracted with ethyl acetate (40 mL × 3). The organic phases were combined, washed with saturated brine (20 mL × 2), dried over anhydrous sodium sulfate, filtered, and concentrated under reduced pressure to remove the solvent, and the residue was purified by a silica gel column to give **compound 37E** (300 mg, yellowish-brown solid, purity: 77.5%, yield: 24.0%). MS (ESI): 273.6 274.5 *m/z* $[M/2+1]^+$

**Step 5: synthesis of compound 37**

**Compound 37E** (5 mg, 0.009 mmol) and 2-hydroxyethane-1-sulfonamide (1.4 mg, 0.011 mmol) were dissolved in N,N-dimethylformamide (0.5 mL), and then cuprous iodide (0.2 mg, 0.001 mmol), cesium carbonate (3.9 mg, 0.018 mmol), and (1R,2R)-(-)-N,N'-dimethyl-1,2-cyclohexanediamine (0.3 mg, 0.002 mmol) were sequentially added. Then, the mixture was stirred at 120 °C for 2 h under a nitrogen atmosphere. After the reaction was completed as detected by TLC, the reaction liquids were combined and filtered. The filtrate was then concentrated under reduced pressure to remove the solvent, and the residue was purified by preparative HPLC to give compound 37 (8 mg, yellow solid, formate). MS (ESI): 591.1 *m/z* $[M+1]^+$。 $^1$H NMR (300 MHz, DMSO-$d_6$) δ(ppm) 11.38 (s, 1H), 10.37 (s, 1H), 8.80 (s, 1H), 8.12 (d, *J* = 1.1 Hz, 1H), 7.60 - 7.47 (m, 2H), 4.99 (br.s, 1H), 4.51 - 4.28 (m, 4H), 3.78 (t, *J* = 6.6 Hz, 2H), 3.40 (t, *J* = 6.2 Hz, 2H), 3.09 - 2.99 (m, 4H), 2.22 - 1.97 (m, 5H), 1.80 - 1.58 (m, 4H), 0.36 (s, 4H).

**Example 38: Preparation of Compound 38**

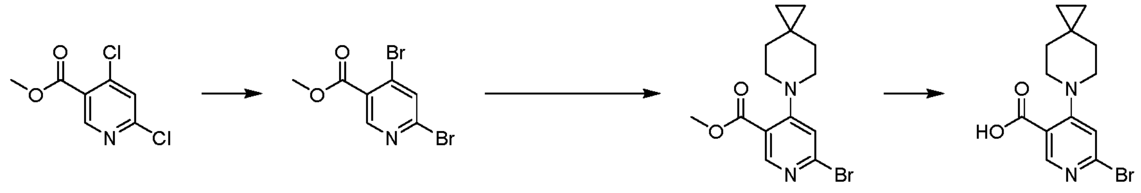

38A 38B 38C 38D

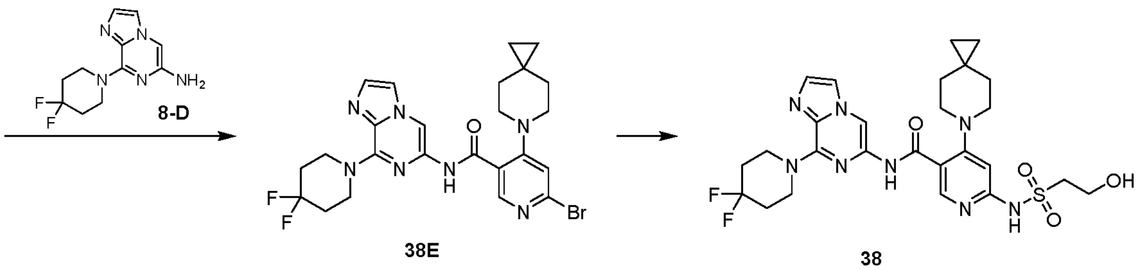

8-D

38E 38

**Step 1: synthesis of compound 38B**

**Compound 38A** (5 g, 24.3 mmol) was dissolved in a 33% solution of hydrobromic acid in acetic acid (40 mL), and then the mixture was stirred at 60 °C for 5 h. After the reaction was completed as detected by LCMS, the reaction liquid was diluted with water (60 mL) under an ice bath and then stirred for 10 min under the ice bath. The suspension was filtered, and the filter cake was washed with ice water (10 mL × 2). The filter cake was dissolved in ethyl acetate (60 mL) and water (30 mL), and then neutralized with an aqueous sodium hydroxide solution (1 mol/L) until pH = 7. The aqueous phase was extracted with ethyl acetate (60 mL × 3). The organic phases were combined, washed with saturated brine (50 mL), dried over anhydrous sodium sulfate, filtered, and concentrated under reduced pressure to remove the solvent to give a crude product of **compound 38B** (2.5 g, off-white solid). MS (ESI): *m/z* 295.9 $[M+1]^+$

**Step 2: synthesis of compound 38C**

**Compound 38B** (2 g, 6.78 mmol) was dissolved in N-methylpyrrolidone (20 mL), and then 6-aza-spiro[2.5] octane hydrochloride (1.2 g, 8.14 mmol) and N,N-diisopropylethylamine (3.6 mL, 20.3 mmol) were added. Then, the mixture was stirred at 120 °C for 12 h. After the reaction was completed as detected by LCMS, the reaction liquid was diluted with water (60 mL) under an ice bath and extracted with ethyl acetate (60 mL × 3). The organic phases were combined, washed with saturated brine (30 mL × 3), dried over anhydrous sodium sulfate, filtered, and concentrated under reduced pressure to remove the solvent, and the residue was purified by a silica gel column to give **compound 38C** (1.1 g, off-white solid). MS (ESI): *m/z* 325.0 327.0 $[M+1]^+$

**Step 3: synthesis of compound 38D**

**Compound 38C** (1.1 g, 3.4 mmol) was dissolved in methanol (5 mL) and water (5 mL), and then sodium hydroxide (271 mg, 6.8 mmol) was added. Then, the mixture was stirred at room temperature for 12 h. After the reaction was completed as detected by LCMS, the reaction liquid was adjusted to pH = 6 with a hydrochloric acid solution (1 mol/L) under an ice bath and extracted with ethyl acetate (60 mL × 3). The organic phases were combined, washed with saturated brine (30 mL × 2), dried over anhydrous sodium sulfate, filtered, and concentrated under reduced pressure to remove the solvent, and the residue was purified by a silica gel column to give **compound 38D** (800 mg, white solid). MS (ESI): *m/z* 311.0 313.0 $[M+1]^+$

**Step 4: synthesis of compound 38E**

**Compound 38D** (100 mg, 0.32 mmol) was dissolved in dichloromethane (2 mL), and then oxalyl chloride (102 mg, 0.8 mmol) and one drop of N,N-dimethylformamide were added dropwise under an ice bath. Then, the mixture was stirred at room temperature for 30 min. After the reaction was completed as detected by TLC, the reaction liquid was concentrated to dryness by rotary evaporation, and then the residue was dissolved in anhydrous tetrahydrofuran (2 mL). **Compound 8-D** (81 mg, 0.32 mmol) and *N,N*-diisopropylethylamine (165 mg, 1.28 mmol) were added at room

temperature, and then the mixture was stirred at room temperature for 1 h. After the reaction was completed as detected by TLC, the reaction liquid was diluted with water (20 mL) and extracted with ethyl acetate (15 mL × 3). The organic phases were combined, dried over anhydrous sodium sulfate, filtered, and concentrated under reduced pressure to remove the solvent, and the residue was purified by a silica gel column to give **compound 38E** (130 mg, yellow solid, yield: 75%). MS (ESI): *m/z* 273.6 274.5 [M/2+1]$^+$

**Step 5: synthesis of compound 38**

**Compound 38E** (70 mg, 0.13 mmol) and 2-hydroxyethane-1-sulfonamide (21 mg, 0.17 mmol) were dissolved in N,N-dimethylformamide (2 mL), and then cuprous iodide (14 mg, 0.08 mmol), potassium phosphate (77 mg, 0.38 mmol), and (1R,2R)-(-)-N,N'-dimethyl-1,2-cyclohexanediamine (15 mg, 0.11 mmol) were sequentially added. Then, the mixture was stirred at 120 °C for 2 h under a nitrogen atmosphere. After the reaction was completed as detected by TLC, the reaction liquid was diluted with water (10 mL) and extracted with ethyl acetate (10 mL × 3). The organic phases were combined, washed with saturated brine (8 mL × 3), dried over anhydrous sodium sulfate, filtered, and concentrated under reduced pressure to remove the solvent, and the residue was purified by preparative HPLC to give **compound 38** (5 mg, white solid, formate). MS (ESI): *m/z* 591.3 [M+1]$^+$。$^1$H NMR (300 MHz, DMSO-$d_6$) δ(ppm) 10.80 (br.s, 1H), 8.72 (s, 1H), 8.05 (s, 1H), 7.52 (d, *J* = 1.1 Hz, 1H), 6.69 (s, 1H), 4.83 (br.s, 1H), 4.42 - 4.31 (m, 4H), 3.73 (t, *J* = 6.8 Hz, 2H), 3.52 - 3.34 (m, 2H), 3.01 - 2.99 (m, 5H), 2.16 - 2.00 (m, 5H), 1.64 - 1.51 (m, 4H), 0.31 (s, 4H).

**Example 39: Preparation of Compound 39**

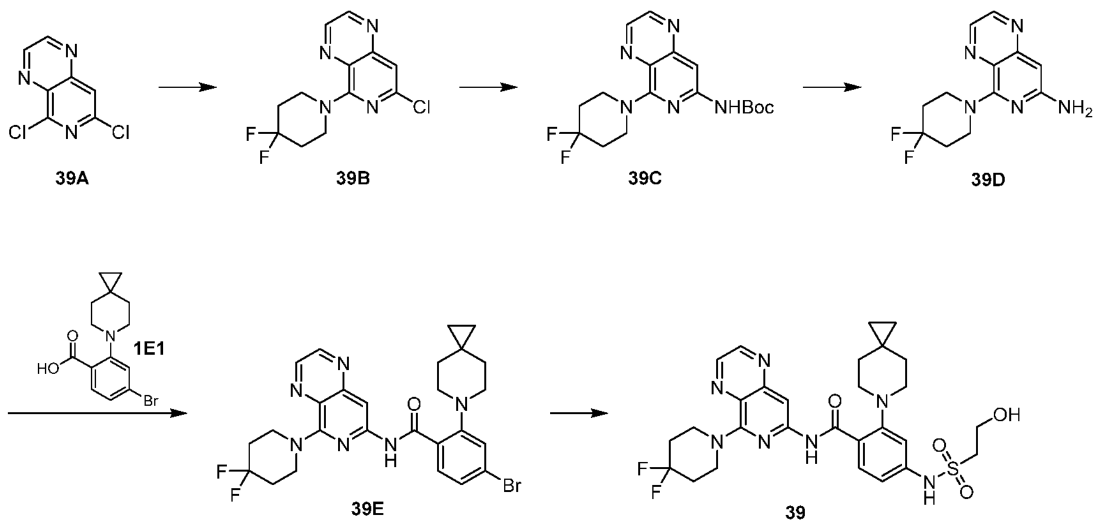
39A 39B 39C 39D
1E1
39E 39

**Step 1: synthesis of compound 39B**

**Compound 39A** (500 mg, 2.50 mmol) was dissolved in tetrahydrofuran (5 mL), and then triethylamine (508 mg, 5.00 mmol) and 4,4-difluoropiperidine hydrochloride (476 mg, 3.00 mmol) were added. Then, the mixture was stirred at room temperature for 16 h. After the reaction was completed as detected by TLC, the mixture was concentrated under reduced pressure to remove the solvent to give a crude product of **compound 39B** (1 g, yellow solid). MS (ESI): *m/z* 285.0 [M+1]$^+$

**Step 2: synthesis of compound 39C**

Compound 39B (400 mg, 1.41 mmol) was dissolved in dioxane (10 mL), and then *tert-butyl* carbamate (330 mg, 2.82 mmol), 2-dicyclohexylphosphino-2',4',6'-triisopropylbiphenyl (63 mg, 0.14 mmol), cesium carbonate (918 mg, 2.82 mmol), and palladium acetate (16 mg, 0.07 mmol) were added. Then, the mixture was stirred at 100 °C for 16 h under a nitrogen atmosphere. After the reaction was completed as detected by TLC, the reaction liquid was cooled to room temperature, diluted with water (80 mL), and extracted with ethyl acetate (40 mL × 3). The organic phases were combined, dried over anhydrous sodium sulfate, filtered, and concentrated under reduced pressure to remove the solvent, and the residue was purified by silica gel column chromatography to give compound 39C (400 mg, orange-yellow solid, yield:

75.7%). MS (ESI): *m/z* 366.1 $[M+1]^+$

**Step 3: synthesis of compound 39D**

**Compound 39C** (300 mg, 0.82 mmol) was dissolved in dichloromethane (5 mL), and then trifluoroacetic acid (1 mL) was added. The mixture was stirred at room temperature for 2 h. After the reaction was completed as detected by TLC, the mixture was concentrated under reduced pressure to remove the solvent, diluted with water (20 mL), then adjusted to pH = 7-8 with a sodium bicarbonate solid, and extracted with ethyl acetate (20 mL × 4). The organic phases were combined, dried over anhydrous sodium sulfate, filtered, and concentrated under reduced pressure to remove the solvent to give a crude product of **compound 39D** (180 mg, brownish-red solid). MS (ESI): *m/z* 266.0 $[M+1]^+$

**Step 4: synthesis of compound 39E**

**Compound 1E1** (211 mg, 0.68 mmol) was dissolved in dichloromethane (5 mL), and then oxalyl chloride (215 mg, 1.70 mmol) was added dropwise under an ice bath. Then, the mixture was stirred at room temperature for 30 min. After the reaction was completed as detected by TLC, the reaction liquid was concentrated to dryness by rotary evaporation, and then the residue was dissolved in anhydrous tetrahydrofuran (5 mL). **Compound 39D** (180 mg, 0.68 mmol) and N,N-diisopropylethylamine (350 mg, 2.04 mmol) were sequentially added at room temperature, and then the mixture was stirred at 50 °C for 2 h. After the reaction was completed as detected by TLC, the reaction liquid was diluted with water (30 mL) and extracted with ethyl acetate (30 mL × 3). The organic phases were combined, dried over anhydrous sodium sulfate, filtered, and concentrated under reduced pressure to remove the solvent, and the residue was purified by a silica gel column to give a crude product of **compound 39E** (200 mg, brownish-red solid).

**Step 5: synthesis of compound 39**

**Compound 39E** (100 mg, 0.18 mmol) and 2-hydroxyethane-1-sulfonamide (27 mg, 0.22 mmol) were dissolved in N,N-dimethylformamide (2 mL), and then cuprous iodide (3 mg, 0.02 mmol), potassium phosphate (76 mg, 0.36 mmol), and (1R,2R)-(-)-N,N'-dimethyl-1,2-cyclohexanediamine (5 mg, 0.04 mmol) were sequentially added. Then, the mixture was stirred at 120 °C for 2 h under a nitrogen atmosphere. After the reaction was completed as detected by TLC, the reaction liquid was diluted with water (20 mL) and extracted with ethyl acetate (10 mL × 3). The organic phases were combined, washed with saturated brine (10 mL), dried over anhydrous sodium sulfate, filtered, and concentrated under reduced pressure to remove the solvent, and the residue was purified by preparative HPLC to give **compound 39** (8 mg, yellow solid). MS (ESI): *m/z* 301.7 $[M/2+1]^+$。 $^1H$ NMR (300 MHz, DMSO-$d_6$) δ(ppm) 13.10 (s, 1H), 10.27 (br.s, 1H), 8.93 (d, *J* = 1.9 Hz, 1H), 8.69 (d, *J* = 1.9 Hz, 1H), 8.20 - 8.05 (m, 2H), 7.28 (d, *J* = 2.1 Hz, 1H), 7.13 (dd, *J* = 8.7, 2.1 Hz, 1H), 4.99 (br.s, 1H), 4.28 - 4.20 (m, 4H), 3.76 (t, *J* = 6.5 Hz, 2H), 3.36 (t, *J* = 6.5 Hz, 2H), 3.06 - 2.97(m, 4H), 2.31 - 2.05 (m, 5H), 1.75 (s, 3H), 0.41 (s, 4H).

**Example 40: Preparation of Compound 40**

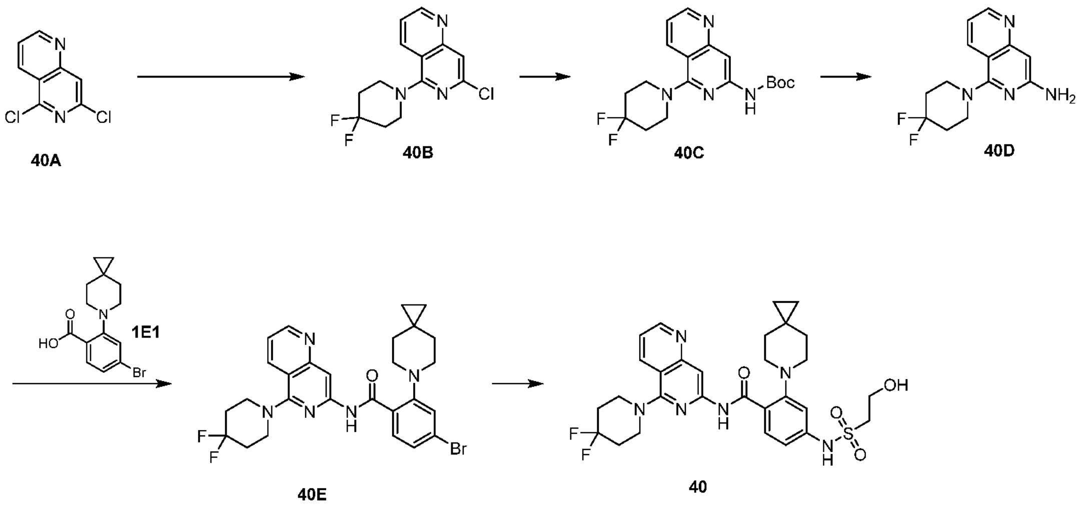

40A 40B 40C 40D 1E1 40E 40

**Step 1: synthesis of compound 40B**

**Compound 40A** (550 mg, 2.7 mmol) was dissolved in N-methyl-2-pyrrolidone (7 mL), and then N,N-diisopropylethylamine (1.06 g, 8.1 mmol) and 4,4-difluoropiperidine hydrochloride (561 mg, 3.5 mmol) were added. Then, the mixture was stirred at 80 °C for 5 h under a nitrogen atmosphere. After the reaction was completed as detected by TLC, the reaction liquid was diluted with water (30 mL) at 0 °C and extracted with ethyl acetate (20 mL × 3). The organic phases were combined, washed with saturated brine (15 mL × 3), dried over anhydrous sodium sulfate, then filtered to remove the drying agent, and concentrated under reduced pressure to remove the solvent, and the residue was purified by silica gel column chromatography to give a crude product of **compound 40B** (990 mg, yellow oil). MS (ESI):*m*/*z* 284.1 286.1 $[M+1]^+$

**Step 2: synthesis of compound 40C**

The crude product of **compound 40B** (990 mg, 3.5 mmol) was dissolved in dioxane (12 mL), and then *tert-butyl* carbamate (616 mg, 5.3 mmol), cesium carbonate (2.3 mg, 7.0 mmol), 2-dicyclohexylphosphino-2',4',6'-triisopropylbiphenyl (165 mg, 0.35 mmol), and tris(dibenzylideneacetone)dipalladium (165 mg, 0.18 mmol) were added. Then, the mixture was stirred at 100 °C for 5 h under a nitrogen atmosphere. After the reaction was completed as detected by TLC, the mixture was cooled to room temperature, diluted with water (40 mL), and extracted with ethyl acetate (20 mL × 3). The organic phases were combined, washed with saturated brine (15 mL), dried over anhydrous sodium sulfate, filtered, and concentrated under reduced pressure to remove the solvent, and the residue was purified by a silica gel column to give a crude product of **compound 40C** (1.4 g, yellow oil). MS (ESI): *m*/*z* 365.2 $[M+1]^+$

**Step 3: synthesis of compound 40D**

**Compound 40C** (500 mg, 1.4 mmol) was dissolved in dichloromethane (5 mL), and then trifluoroacetic acid (1 mL) was added. Then, the mixture was stirred at 25 °C for 2 h. After the reaction was completed as detected by TLC, the mixture was concentrated under reduced pressure to remove the solvent to give a crude product of compound **40D** (400 mg, brown oil). MS (ESI): *m*/*z* 265.1 $[M+1]^+$

**Step 4: synthesis of compound 40E**

**Compound 1E1** (468 mg, 1.5 mmol) was dissolved in dichloromethane (5 mL), and then oxalyl chloride (480 mg, 3.8 mmol) was added dropwise under an ice bath. Then, the mixture was stirred at room temperature for 30 min. After the reaction was completed as detected by TLC, the reaction liquid was concentrated to dryness by rotary evaporation, and the residue was dissolved in anhydrous tetrahydrofuran (5 mL). Subsequently, **compound 40D** (400 mg, 1.5 mmol) and N,N-diisopropylethylamine (781 mg, 6.0 mmol) were added to the reaction liquid, and then the mixture was stirred at 65 °C for 16 h. After the reaction was completed as detected by TLC, the reaction liquid was diluted with water (20 mL) and extracted with ethyl acetate (15 mL × 3). The organic phases were combined, washed with saturated brine (10 mL), dried over anhydrous sodium sulfate, filtered, and concentrated under reduced pressure to remove the solvent, and the residue was purified by a silica gel column to give **compound 40E** (280 mg, yellow solid, yield: 33.3%). MS (ESI): 278.6 279.6 $[M/2+1]^+$

**Step 5: synthesis of compound 40**

**Compound 40E** (280 mg, 0.5 mmol) and 2-hydroxyethane-1-sulfonamide (84 mg, 0.65 mmol) were dissolved in N,N-dimethylformamide (6 mL), and then cuprous iodide (57 mg, 0.3 mmol), potassium phosphate (308 mg, 1.45 mmol), and (1R,2R)-(-)-N,N'-dimethyl-1,2-cyclohexanediamine (60 mg, 0.43 mmol) were sequentially added. Then, the mixture was stirred at 120 °C for 2 h under a nitrogen atmosphere. After the reaction was completed as detected by TLC, the reaction liquid was diluted with water (10 mL) and extracted with ethyl acetate (10 mL × 3). The organic phases were combined, washed with saturated brine (8 mL), dried over anhydrous sodium sulfate, filtered, and concentrated under reduced pressure to remove the solvent, and the residue was purified by preparative HPLC to give **compound 40** (41 mg, yellow solid). MS (ESI): *m*/*z* 601.2 $[M+1]^+$ $^1$H NMR (300 MHz, DMSO-$d_6$) δ(ppm) 13.30 (s, 1H), 10.06 (br.s, 1H), 8.95 (dd, *J* = 4.2, 1.6 Hz, 1H), 8.44 (d, *J* = 8.4 Hz, 1H), 8.32 (s, 1H), 8.13 (d, *J* = *8.5* Hz, 1H), 7.43 (dd, *J* = 8.4, 4.3 Hz, 1H), 7.29 (d, *J* = 2.1 Hz, 1H), 7.15 (dd, *J* = 8.6, 2.1 Hz, 1H), 4.98 (br.s, 1H), 3.77 (t, *J* = 6.4 Hz, 2H), 3.61 - 3.51 (m, 4H), 3.37 (t, *J* = 6.4 Hz, 2H), 3.01 (d, *J* = 6.4 Hz, 4H), 2.25 (d, *J* = 16.0 Hz, 5H), 1.94 - 1.74 (m, 3H), 0.40 (s, 4H).

**Example 41: Preparation of Compound 42**

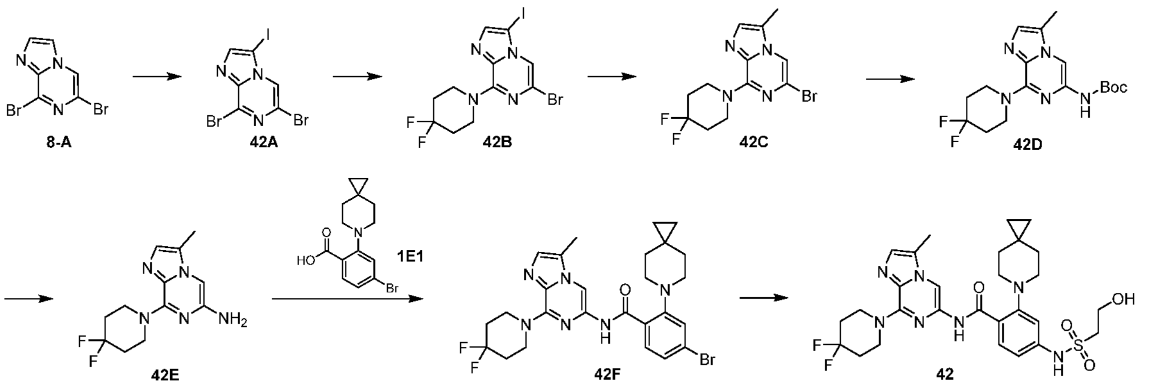


**Step 1: synthesis of compound 42A**

**Compound 8-A** (5 g, 18.1 mmol) was dissolved in DMF (40 mL), and then N-iodosuccinimide (4.47 g, 19.9 mmol) was added. Then, the mixture was stirred at 60 °C for 3 h. After the reaction was completed as detected by LCMS, the reaction liquid was cooled to room temperature, and the reaction was quenched with a saturated sodium thiosulfate solution (80 mL). Then, the reaction liquid was extracted with ethyl acetate (100 mL × 3). The organic phases were combined, washed with saturated brine (60 mL × 3), dried over anhydrous sodium sulfate, filtered, and concentrated under reduced pressure to remove the solvent, and the residue was purified by silica gel column chromatography to give **compound 42A** (6.8 g, yellow solid, yield: 93.5%). MS (ESI): *m*/*z* 403.7 $[M+1]^+$

**Step 2: synthesis of compound 42B**

**Compound 42A** (1 g, 2.5 mmol) was dissolved in N-methylpyrrolidone (40 mL), and then 4,4-difluoropiperidine hydrochloride (430 mg, 2.7 mmol) was added. Then, the mixture was stirred at 100 °C for 3 h. After the reaction was completed as detected by LCMS, the reaction liquid was cooled to room temperature, diluted with water (60 mL), and extracted with ethyl acetate (50 mL × 3). The organic phases were combined, washed with saturated brine (30 mL × 3), dried over anhydrous sodium sulfate, filtered, and concentrated under reduced pressure to remove the solvent, and the residue was purified by silica gel column chromatography and concentrated to dryness by rotary evaporation under reduced pressure to give **compound 42B** (900 mg, off-white solid, yield: 81.8%). MS (ESI): *m*/*z* 442.9 444.9 $[M+1]^+$

**Step 3: synthesis of compound 42C**

**Compound 42B** (3.5 g, 7.9 mmol) was dissolved in dioxane (40 mL) and water (4 mL), and then trimethylboroxine (6.8 mL, 23.7 mmol, a 3.5 mol/L solution in tetrahydrofuran), [1,1'-bis(diphenylphosphino)ferrocene]palladium dichloride dichloromethane complex (320 mg, 0.4 mmol), and potassium carbonate (2.18 g, 15.8 mmol) were added. Then, the mixture was stirred at 75 °C for 3 h under a nitrogen atmosphere. After the reaction was completed as detected by LCMS, the reaction liquid was cooled to room temperature, diluted with water, and extracted with ethyl acetate. The organic phases were combined, washed with saturated brine, dried over anhydrous sodium sulfate, filtered, and concentrated under reduced pressure to remove the solvent, and the residue was purified by reversed-phase preparative chromatography to give **compound 42C** (290 mg, off-white solid, yield: 11.1%). MS (ESI): *m*/*z* 331.0 333.0 $[M+1]^+$

**Step 4: synthesis of compound 42D**

Compound 42C (280 mg, 0.85 mmol) was dissolved in dioxane (5 mL), and then *tert-butyl* carbamate (200 mg, 1.70 mmol), 2-dicyclohexylphosphino-2',4',6'-triisopropylbiphenyl (40 mg, 0.09 mmol), cesium carbonate (553 mg, 1.70 mmol), tris(dibenzylideneacetone)dipalladium (40 mg, 0.04 mmol) were added. Then, the mixture was allowed to react at 110 °C for 16 h under a nitrogen atmosphere. After the reaction was completed as detected by TLC, the reaction liquid was cooled to room temperature, diluted with water (50 mL), and extracted with ethyl acetate (40 mL × 3). The organic phases were combined, dried over anhydrous sodium sulfate, filtered, and concentrated under reduced pressure to remove the solvent, and the residue was purified by silica gel column chromatography and concentrated to dryness by rotary evaporation under reduced pressure to give compound 42D (250 mg, yellow solid, purity: 94.1%, yield: 75.7%). MS (ESI): *m*/*z* 368.2 $[M+1]^+$

**Step 5: synthesis of compound 42E**

**Compound 42D** (250 mg, 0.68 mmol) was dissolved in dichloromethane (5 mL), and then trifluoroacetic acid (1 mL) was added. The mixture was stirred at room temperature for 2 h. After the reaction was completed as detected by TLC, the mixture was concentrated under reduced pressure to remove the solvent to give a crude product of **compound 42E** (250 mg, yellow oil). MS (ESI): *m/z* 268.1$[M+1]^+$

**Step 5: synthesis of compound 42F**

**Compound 1E1** (208 mg, 0.67 mmol) was dissolved in dichloromethane (5 mL), and then oxalyl chloride (218 mg, 1.68 mmol) was added dropwise under an ice bath. Then, the mixture was stirred at room temperature for 30 min. After the reaction was completed as detected by TLC, the reaction liquid was concentrated to dryness by rotary evaporation, and then the residue was dissolved in anhydrous tetrahydrofuran (10 mL). **Compound 42E** (180 mg, 0.67 mmol) and N,N-diisopropylethylamine (350 mg, 2.68 mmol) were sequentially added at room temperature, and then the reaction liquid was stirred at 50 °C for 1 h. After the reaction was completed as detected by TLC, the reaction liquid was diluted with water (40 mL) and extracted with ethyl acetate (20 mL × 3). The organic phases were combined, washed with saturated brine (10 mL), dried over anhydrous sodium sulfate, filtered, and concentrated under reduced pressure to remove the solvent, and the residue was purified by a silica gel column to give **compound 42F** (110 mg, yellow solid, purity: 63.2%, yield: 18.5%). MS (ESI): *m/z* 280.1 281.1 $[M/2+1]^+$

**Step 3: synthesis of compound 42**

**Compound 42F** (100 mg, 0.18 mmol) and 2-hydroxyethane-1-sulfonamide (27 mg, 0.22 mmol) were dissolved in N,N-dimethylformamide (2 mL), and then cuprous iodide (3 mg, 0.02 mmol), potassium phosphate (76 mg, 0.44 mmol), and (1R,2R)-(-)-N,N'-dimethyl-1,2-cyclohexanediamine (5 mg, 0.04 mmol) were sequentially added. Then, the mixture was stirred at 120 °C for 2 h under a nitrogen atmosphere. After the reaction was completed as detected by TLC, the reaction liquid was diluted with water (10 mL) and extracted with ethyl acetate (10 mL × 3). The organic phases were combined, washed with saturated brine (8 mL × 2), dried over anhydrous sodium sulfate, filtered, and concentrated under reduced pressure to remove the solvent, and the residue was purified by preparative HPLC to give **compound 42** (20 mg, white solid). MS (ESI): *m/z* 604.3 $[M+1]^+$。$^1$H NMR (300 MHz, DMSO-$d_6$) δ 12.90 (s, 1H), 10.21 (br.s, 1H), 8.50 (s, 1H), 8.08 (d, *J* = 8.6 Hz, 1H), 7.38 (s, 1H), 7.28 (d, *J* = 2.2 Hz, 1H), 7.14 (dd, *J* = 8.7, 2.1 Hz, 1H), 4.97 (br.s, 1H), 4.49 - 4.33 (m, 4H), 3.76 (t, *J* = 6.5 Hz, 2H), 3.36 (t, *J* = 6.4 Hz, 2H), 3.05 - 2.94 (m, 4H), 2.42 (s, 3H), 2.21 - 1.98 (m, 5H), 1.87 - 1.61 (m, 3H), 0.40 (s, 4H).

**Example 42: Preparation of Compound 43**

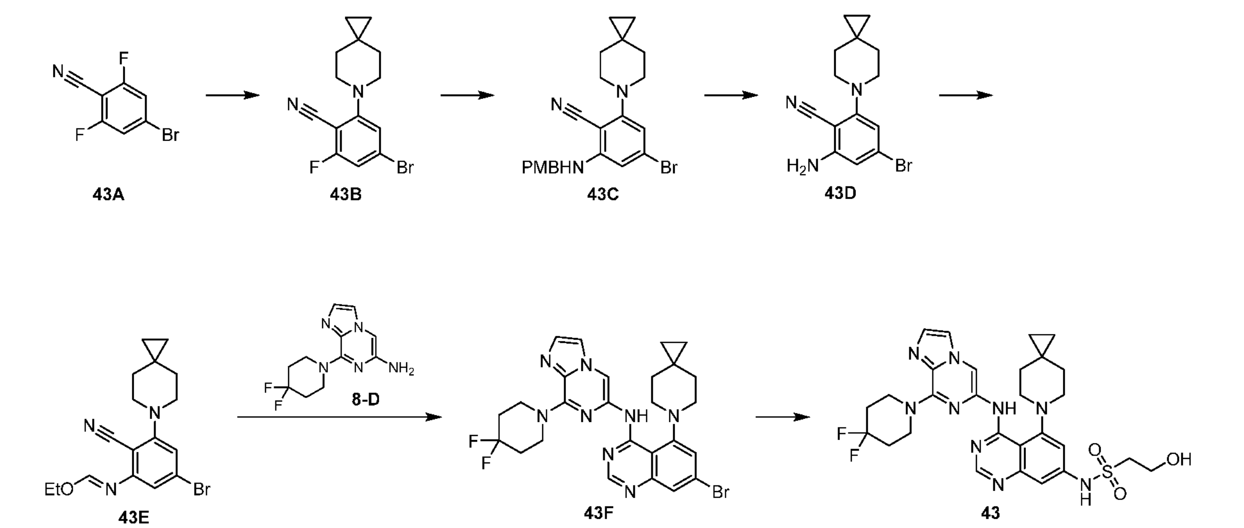


43A 43B 43C 43D 43E 43F 43

**Step 1: synthesis of compound 43B**

**Compound 43A** (2.0 g, 9.17 mmol) was dissolved in dimethyl sulfoxide (20 mL), and then 6-aza-spiro[2.5]octane hydrochloride (1.6 g, 11.0 mmol) and N,N-diisopropylethylamine (3.4 g, 22.9 mmol) were added. Then, the mixture was stirred at room temperature for 16 h under a nitrogen atmosphere. After the reaction was completed as detected by TLC, the reaction liquid was diluted with water (100 mL) and extracted with ethyl acetate (80 mL × 3). The organic phases were combined, washed with saturated brine (50 mL × 3), dried over anhydrous sodium sulfate, filtered, and concentrated under reduced pressure to remove the solvent, and the residue was purified by silica gel column chromatography to give **compound 43B** (2.8 g, white solid, yield: 97.7%). MS (ESI): *m/z* 308.9 311.0 $[M+1]^+$

**Step 2: synthesis of compound 43C**

**Compound 43B** (2.9 g, 9.38 mmol) was dissolved in N-methylpyrrolidone (30 mL), and then 4-methoxybenzylamine (1.6 g, 11.3 mmol) and N,N-diisopropylethylamine (2.4 g, 18.8 mmol) were added. Then, the mixture was stirred at 120 °C for 16 h under a nitrogen atmosphere. After the reaction was completed as detected by TLC, the reaction liquid was diluted with water (100 mL) and extracted with ethyl acetate (80 mL × 3). The organic phases were combined, washed with saturated brine (80 mL × 2), dried over anhydrous sodium sulfate, filtered, and concentrated under reduced pressure to remove the solvent, and the residue was purified by silica gel column chromatography to give **compound 43C** (3.7 g, yellow oily solid, yield: 83.0%).

**Step 3: synthesis of compound 43D**

**Compound 43C** (3.7 g, 8.68 mmol) was dissolved in trifluoroacetic acid (20 mL), and then trifluoromethanesulfonic acid (2 mL) was added. Then, the mixture was stirred at room temperature for 2 h. After the reaction was completed as detected by TLC, the reaction liquid was diluted with water (100 mL), adjusted to pH = 7-8 with a sodium bicarbonate solid, and then extracted with ethyl acetate (80 mL × 3). The organic phases were combined, then washed with saturated brine (50 mL), dried over anhydrous sodium sulfate, filtered, and concentrated under reduced pressure to remove the solvent, and the residue was purified by silica gel column chromatography to give **compound 43D** (2.0 g, yellow oil, yield: 73.0%). MS (ESI): *m/z* 306.1 308.0 $[M+1]^+$

**Step 4: synthesis of compound 43E**

**Compound 43D** (500 mg, 1.63 mmol) was dissolved in triethyl orthoformate (5 mL), and then acetic anhydride (167 mg, 1.39 mmol) was added. Then, the mixture was stirred at 145 °C for 3 h. After the reaction was completed as detected by TLC, the mixture was concentrated under reduced pressure to remove the solvent to give a crude product of **compound 43E** (500 mg, brown solid). MS (ESI): *m/z* 362.1 364.0 $[M+1]^+$

**Step 5: synthesis of compound 43F**

**Compound 43E** (500 mg, 1.38 mmol) and **compound 8-D** (350 mg, 1.38 mmol) were dissolved in glacial acetic acid (5 mL), and then the mixture was stirred at 125 °C for 1 h. After the reaction was completed as detected by TLC, the reaction liquid was cooled to room temperature, diluted with water (50 mL), adjusted to pH = 7-8 with a sodium bicarbonate solid, and then extracted with ethyl acetate (50 mL × 3). The organic phases were combined, dried over anhydrous sodium sulfate, filtered, and concentrated under reduced pressure to remove the solvent, and the residue was purified by a silica gel column to give **compound 43F** (200 mg, yellow solid, purity: 83.2%, yield: 21.2%). MS (ESI): *m/z* 285.1 286.2 $[M/2+1]^+$

**Step 6: synthesis of compound 43**

**Compound 43F** (200 mg, 0.35 mmol) and 2-hydroxyethane-1-sulfonamide (53 mg, 0.42 mmol) were dissolved in N,N-dimethylformamide (2 mL), and then cuprous iodide (7 mg, 0.04 mmol), potassium phosphate (149 mg, 0.70 mmol), and (1R,2R)-(-)-N,N'-dimethyl-1,2-cyclohexanediamine (10 mg, 0.07 mmol) were sequentially added. Then, the mixture was stirred at 120 °C for 16 h under a nitrogen atmosphere. After the reaction was completed as detected by TLC, the reaction liquid was diluted with water (20 mL) and extracted with ethyl acetate (30 mL × 3). The organic phases were combined, washed with saturated brine (10 mL), dried over anhydrous sodium sulfate, filtered, and concentrated under reduced pressure to remove the solvent, and the residue was purified by preparative HPLC to give **compound 43** (30 mg, yellow solid). MS (ESI): *m/z* 614.2. $[M+1]^+$。 $^1H$ NMR (300 MHz, DMSO-$d_6$) δ(ppm) 12.88 (s, 1H), 10.30 (br.s, 1H), 9.21 (s, 1H), 8.60 (s, 1H), 8.13 (d, *J* = 1.1 Hz, 1H), 7.56 (d, *J* = 1.1 Hz, 1H), 7.36 (d, *J* = 2.1 Hz, 2H), 5.03 (br.s, 1H), 4.51 - 4.39 (m, 4H), 3.78 (t, *J* = 6.4 Hz, 2H), 3.40 (t, *J* = 6.4 Hz, 2H), 3.20 (d, *J* = *10.9* Hz, 2H), 2.86 (t, *J* = 11.4 Hz, 2H), 2.64 - 2.53 (m, 2H), 2.22 -

2.06 (m, 4H), 1.04 (d, $J$ = 12.9 Hz, 2H), 0.52 - 0.37 (m, 4H).

**Example 43: Preparation of Compound 44**

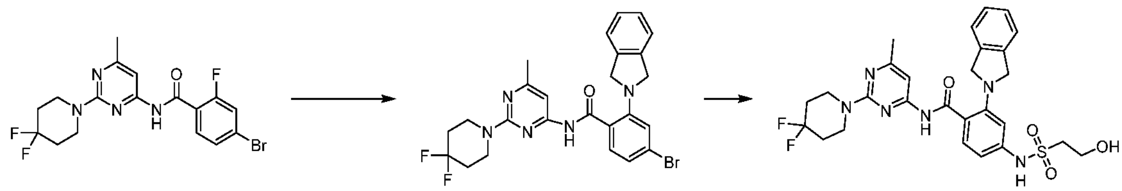

3A 44A 44

**Step 1: synthesis of compound 44A**

**Compound 3A** (500 mg, 1.17 mmol) was dissolved in N-methyl-2-pyrrolidone (6 mL), and then potassium carbonate (325 mg, 2.34 mmol) and isoindoline (220 mg, 1.4 mmol) were added. Then, the mixture was stirred at 120 °C for 16 h under a nitrogen atmosphere. After the reaction was completed as detected by TLC, water (30 mL) was added to the reaction liquid at 0 °C, and then the mixture was extracted with ethyl acetate (20 mL × 3). The organic phases were combined, washed with saturated brine (30 mL), dried over anhydrous sodium sulfate, then filtered to remove the drying agent, and concentrated under reduced pressure to remove the solvent, and the residue was purified by silica gel column chromatography to give a crude product of **compound 44A** (330 mg, 0.62 mmol, brownish-yellow oil). MS (ESI): 264.6 265.6 $[M/2+1]^+$

**Step 2: synthesis of compound 44**

**Compound 44A** (330 mg, 0.62 mmol) and 2-hydroxyethane-1-sulfonamide (99 mg, 0.81 mmol) were dissolved in N,N-dimethylformamide (6 mL), and then cuprous iodide (71 mg, 0.4 mmol), potassium phosphate (385 mg, 1.8 mmol), and (1*R*,2*R*)-(-)-*N,N'*-dimethyl-1,2-cyclohexanediamine (77 mg, 0.53 mmol) were sequentially added. Then, the mixture was stirred at 120 °C for 2 h under a nitrogen atmosphere. After the reaction was completed as detected by TLC, the reaction liquid was diluted with water (10 mL) and extracted with ethyl acetate (15 mL × 3). The organic phases were combined, washed with saturated brine (10 mL × 3), dried over anhydrous sodium sulfate, filtered, and concentrated under reduced pressure to remove the solvent, and the residue was purified by preparative HPLC to give **compound 44** (25 mg, yellow solid). MS (ESI): $m/z$ 573.1 $[M+1]^+$ $^1H$ NMR (300 MHz, DMSO-$d_6$) $\delta$ (ppm) 11.15 (s, 1H), 9.93 (s, 1H), 7.51 - 7.19 (m, 6H), 6.90 (s, 1H), 6.72 (d, $J$ = 8.5 Hz, 1H), 4.96 (t, $J$ = 5.6 Hz, 1H), 4.59 (s, 4H), 3.83 - 3.67 (m, 6H), 3.33 (t, $J$ = 5.6 Hz, 2H), 2.30 (s, 3H), 1.98 - 1.83 (m, 4H).

**Example 44: Preparation of Compound 45**

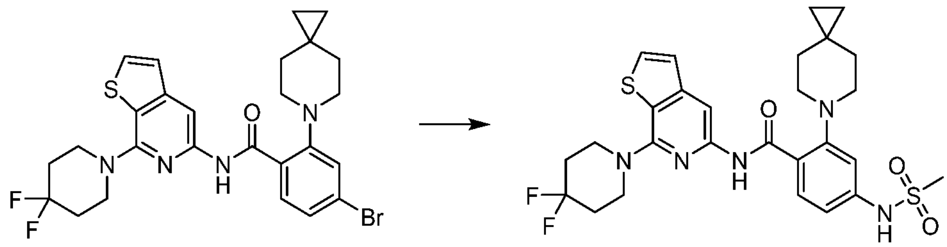

15G 45

**Preparation method:**

**Step 1: synthesis of compound 45**

**Compound 15G** (400 mg, 0.71 mmol) and methanesulfonamide (79 mg, 0.85 mmol) were dissolved in N,N-dimethylformamide (8 mL), and then cuprous iodide (13 mg, 0.07 mmol), potassium phosphate (295 mg, 1.42 mmol), and (1R,2R)-(-)-N,N'-dimethyl-1,2-cyclohexanediamine (20 mg, 0.14 mmol) were sequentially added. Then, the mixture was stirred at 120 °C for 2 h under a nitrogen atmosphere. After the reaction was completed as detected by TLC, the reaction liquid was diluted with water (30 mL) and extracted with ethyl acetate (30 mL × 3). The organic phases were combined, washed with saturated brine (20 mL × 2), dried over anhydrous sodium sulfate, filtered, and concentrated under reduced pressure to remove the solvent, and the residue was purified by preparative HPLC to give **compound 45** (150 mg, off-white solid). MS (ESI): *m/z* 288.5 $[M/2+1]^+$。$^1$H NMR (300 MHz, DMSO-$d_6$) δ(ppm) 12.92 (s, 1H), 10.25 (s, 1H), 8.30 (s, 1H), 8.10 (d, *J* = 8.6 Hz, 1H), 8.01 (d, *J* = 5.4 Hz, 1H), 7.51 (d, *J* = 5.5 Hz, 1H), 7.26 (d, *J* = 2.1 Hz, 1H), 7.13 (dd, *J* = 8.6, 2.1 Hz, 1H), 3.86 - 3.76 (m, 4H), 3.12 (s, 3H), 3.04 - 2.95 (m, 4H), 2.27 - 2.05 (m, 5H), 1.89 - 1.64 (m, 3H), 0.39 (s, 4H).

**Example 45: Preparation of Compound 46**

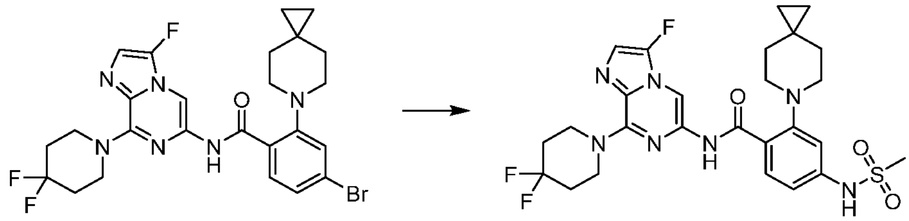

34E 46

**Step 5: synthesis of compound 46**

**Compound 34E** (600 mg, 1.07 mmol) and methanesulfonamide (132 mg, 1.4 mmol) were dissolved in N,N-dimethylformamide (6 mL), and then cuprous iodide (120 mg, 0.64 mmol), potassium phosphate (654 mg, 3.1 mmol), and (1*R*,2*R*)-(-)-*N,N'*-dimethyl-1,2-cyclohexanediamine (132 mg, 0.92 mmol) were sequentially added. Then, the mixture was stirred at 120 °C for 2 h under a nitrogen atmosphere. After the reaction was completed as detected by TLC, the reaction liquid was diluted with water (10 mL) and extracted with ethyl acetate (20 mL × 3). The organic phases were combined, washed with saturated brine (8 mL × 3), dried over anhydrous sodium sulfate, filtered, and concentrated under reduced pressure to remove the solvent, and the residue was purified by preparative HPLC to give **compound 46** (101 mg, off-white solid). MS (ESI): *m/z* 289.7 $[M/2+1]^+$。$^1$H NMR (300 MHz, DMSO-$d_6$) δ 13.03 (s, 1H), 10.27 (s, 1H), 8.43 (s, 1H), 8.09 (d, *J* = 8.6 Hz, 1H), 7.40 (d, *J* = *7.2* Hz, 1H), 7.27 (d, *J* = *2.2* Hz, 1H), 7.14 (dd, *J* = 8.6, 2.1 Hz, 1H), 4.41 (s, 4H), 3.13 (s, 3H), 2.99 (s, 4H), 2.23 - 1.99 (m, 5H), 1.73 (s, 3H), 0.41 (s, 4H).

**Example 46: Preparation of Compound 47**

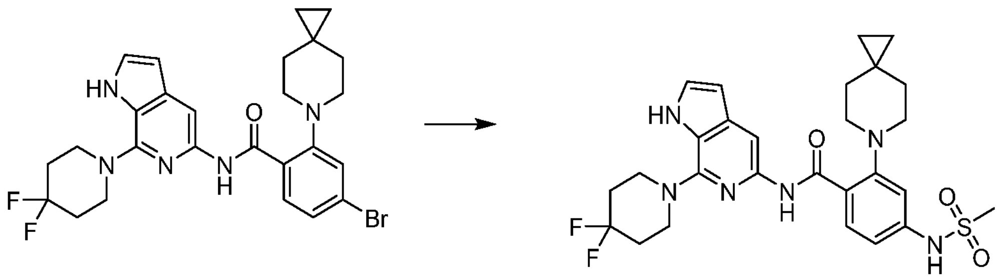

22F 47

**Step 1: synthesis of compound 47**

**Compound 22F** (200 mg, 0.4 mmol) and methanesulfonamide (45 mg, 0.52 mmol) were dissolved in *N,N*-dimethylformamide (4 mL), and then cuprous iodide (42 mg, 0.24 mmol), potassium phosphate (226 mg, 1.16 mmol), and (1R,2R)-(-)-N,N'-dimethyl-1,2-cyclohexanediamine (45 mg, 0.34 mmol) were sequentially added. Then, the mixture was stirred at 120 °C for 2 h under a nitrogen atmosphere. After the reaction was completed as detected by TLC, the reaction liquid was diluted with water (10 mL) and extracted with ethyl acetate (10 mL × 3). The organic phases were combined,

washed with saturated brine (8 mL), dried over anhydrous sodium sulfate, filtered, and concentrated under reduced pressure to remove the solvent, and the residue was purified by preparative HPLC to give **compound 47** (81 mg, yellow solid). MS (ESI):*m/z* 559.1 $[M+1]^+$。 $^1$H NMR (300 MHz, DMSO-$d_6$) δ(ppm) 12.69 (s, 1H), 11.34 (s, 1H), 10.19 (s, 1H), 8.15 - 8.04 (m, 2H), 7.46 (t, *J* = 2.8 Hz, 1H), 7.24 (d, *J* = 2.1 Hz, 1H), 7.11 (dd, *J* = 8.6, 2.1 Hz, 1H), 6.50 - 6.43 (m, 1H), 3.59 - 3.51 (m, 4H), 3.11 (s, 3H), 3.04 - 2.95 (m, 4H), 2.29 - 2.10 (m, 5H), 1.94 - 1.59 (m, 3H), 0.39 (s, 4H).

**Example 47: Preparation of Compound 48**

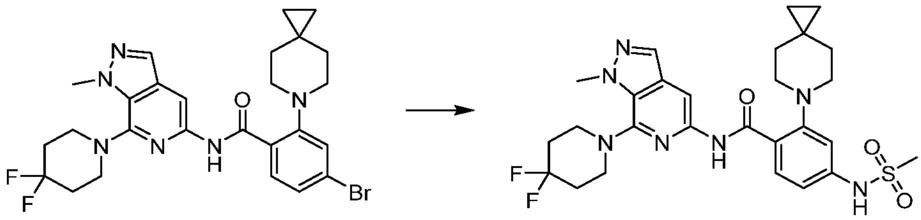

25H 48

**Preparation method:**

**Step 1: synthesis of compound 48**

**Compound 25H** (400 mg, 0.71 mmol) and methanesulfonamide (82 mg, 0.85 mmol) were dissolved in N,N-dimethylformamide (8 mL), and then cuprous iodide (14 mg, 0.07 mmol), potassium phosphate (304 mg, 1.42 mmol), and (1R,2R)-(-)-N,N'-dimethyl-1,2-cyclohexanediamine (20 mg, 0.14 mmol) were sequentially added. Then, the mixture was stirred at 120 °C for 2 h under a nitrogen atmosphere. After the reaction was completed as detected by TLC, the reaction liquid was diluted with water (50 mL) and extracted with ethyl acetate (50 mL × 3). The organic phases were combined, washed with saturated brine (40 mL × 2), dried over anhydrous sodium sulfate, filtered, and concentrated under reduced pressure to remove the solvent, and the residue was purified by preparative HPLC to give **compound 48** (180 mg, off-white solid). MS (ESI): *m/z* 287.7 $[M/2+1]^+$。 $^1$H NMR (300 MHz, DMSO-$d_6$) δ(ppm) 12.52 (s, 1H), 10.24 (br.s, 1H), 8.24 (s, 1H), 8.08 (d, *J* = 8.6 Hz, 1H), 7.82 (s, 1H), 7.23 (d, *J* = 2.2 Hz, 1H), 7.10 (dd, *J* = 8.6, 2.1 Hz, 1H), 4.34 - 4.23 (m, 4H), 4.14 (s, 3H), 3.10 (s, 3H), 3.03 - 2.96 (m, 4H), 2.19 - 1.98 (m, 5H), 1.89 - 1.58 (m, 3H), 0.40 (s, 4H).

**Example 48: Preparation of Compound 49**

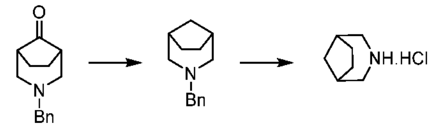

49-1 49-2 49-3

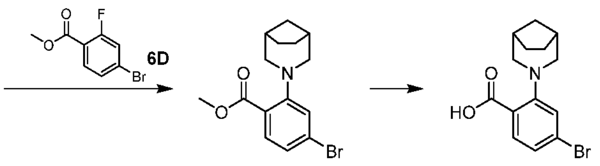


49-4 49A

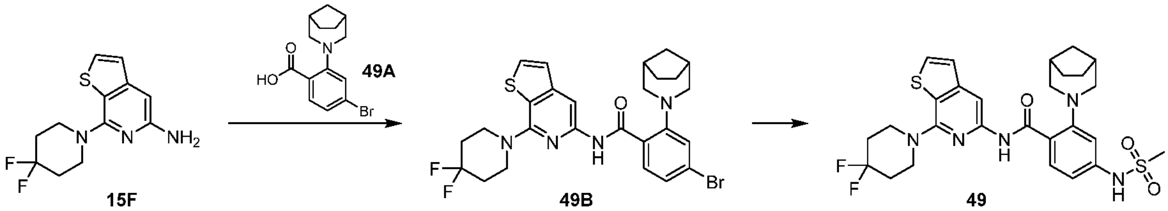


15F 49B 49

**Step 1: synthesis of compound 49-2**

**Compound 49-1** (50 g, 232.2 mmol) was dissolved in diethylene glycol (600 mL), and then potassium hydroxide (59.8 g, 1.07 mol) and hydrazine hydrate (62.9 g, 1.07 mol) were added under an ice bath. Then, the mixture was stirred at 180 °C for 5 h. After the reaction was completed as detected by LCMS, the reaction liquid was cooled to room temperature, diluted with water (500 mL), and extracted with methyl *tert-butyl* ether (500 mL × 3). The organic phases were combined, washed with saturated brine (300 mL), dried over anhydrous sodium sulfate, filtered, and concentrated under reduced pressure to remove the solvent to give a crude product of **compound 49-2** (50 g, pale yellow oil). MS (ESI): *m/z* 202.2 $[M+1]^+$

**Step 2: synthesis of compound 49-3**

A solution of hydrochloric acid in ethyl acetate (200 mL, 4 mol/L) was added to the crude product of **compound 49-2** (50 g, 248.4 mmol) under an ice bath, and then methanol (200 mL) was added. Then, the mixture was stirred at 45 °C for 96 h under a hydrogen atmosphere. After the reaction was completed as detected by LCMS, the reaction liquid was filtered through celite, and then the filtrate was concentrated under reduced pressure to remove the solvent to give a crude product of **compound 49-3** (20 g, pale yellow solid).

**Step 3: synthesis of compound 49-4**

**Compound 6D** (20 g, 85.8 mmol) was dissolved in N-methylpyrrolidone (200 mL), and then **compound 49-3** (40 g, 257 mmol) and potassium carbonate (71 g, 515 mmol) were added. Then, the mixture was stirred at 120 °C for 16 h. After the reaction was completed as detected by TLC, the reaction liquid was cooled to room temperature, diluted with water (1000 mL), and extracted with ethyl acetate (300 mL × 3). The organic phases were combined, washed with saturated brine (100 mL × 3), dried over anhydrous sodium sulfate, filtered, and concentrated under reduced pressure to remove the solvent, and the residue was purified by silica gel column chromatography to give **compound 49-4** (20 g, pale yellow oil, purity: 80.2%, yield: 57.6%). MS (ESI): *m/z* 324.0 326.0 $[M+1]^+$

**Step 4: synthesis of compound 49A**

**Compound 49-4** (20 g, 61.7 mmol) was dissolved in methanol (150 mL), water (150 mL), and tetrahydrofuran (150 mL), and then lithium hydroxide monohydrate (5.2 g, 123 mmol) was added under an ice bath. Then, the mixture was stirred at 50 °C for 16 h. After the reaction was completed as detected by TLC, the reaction liquid was concentrated under reduced pressure to remove the methanol and tetrahydrofuran, and then adjusted to pH = 5-6 by adding 1 N hydrochloric acid. A white solid was precipitated, and the mixture was filtered. The filter cake was washed with water (200 mL), collected, and dried to give **compound 49A** (17.5 g, white solid, purity: 90.8%, yield: 83.0%). MS (ESI): *m/z* 310.0 312.0

[M+1]+。

$^1H$ NMR (300 MHz, Chloroform-d) δ(ppm) 8.16 (d, *J* = 8.4 Hz, 1H), 7.61 (d, J=1.9 Hz, 1H), 7.54 (dd, *J* = 8.4, 1.8 Hz, 1H), 3.08 - 3.01 (m, 2H), 2.95 - 2.89 (m, 2H), 2.44 - 2.37 (m, 2H), 1.99 - 1.80 (m, 5H), 1.71 - 1.69 (m, 1H).

**Step 5: synthesis of compound 49B**

**Compound 49A** (1.0 g, 3.27 mmol) was dissolved in dichloromethane (10 mL), and then oxalyl chloride (1.0 g, 8.18 mmol) was added dropwise under an ice bath. Then, the mixture was stirred at room temperature for 30 min. After the reaction was completed as detected by TLC, the reaction liquid was concentrated to dryness by rotary evaporation, and then the residue was dissolved in anhydrous tetrahydrofuran (10 mL). A mixture of **compound 15F** (880 mg, 1.80 mmol) and N,N-diisopropylethylamine (1.7 g, 13.1 mmol) was added at room temperature, and then the reaction liquid was stirred at 50 °C for 1 h. After the reaction was completed as detected by TLC, the reaction liquid was diluted with water (80 mL) and extracted with ethyl acetate (60 mL × 3). The organic phases were combined, washed with saturated brine (30 mL), dried over anhydrous sodium sulfate, filtered, and concentrated under reduced pressure to remove the solvent, and the residue was purified by a silica gel column to give **compound 49B** (1.2 g, yellow solid, purity: 70.2%, yield: 45.9%). MS (ESI): *m/z* 281.0 282.3 [M/2+1]+

**Step 6: synthesis of compound 49**

**Compound 49B** (100 mg, 0.18 mmol) and methanesulfonamide (20 mg, 0.22 mmol) were dissolved in N,N-dimethylformamide (2 mL), and then cuprous iodide (3 mg, 0.02 mmol), potassium phosphate (76 mg, 0.36 mmol), and (1R,2R)-(-)-N,N-dimethyl-1,2-cyclohexanediamine (5 mg, 0.04 mmol) were sequentially added. Then, the mixture was stirred at 120 °C for 2 h under a nitrogen atmosphere. After the reaction was completed as detected by TLC, the reaction liquid was diluted with water (20 mL) and extracted with ethyl acetate (15 mL × 3). The organic phases were combined, washed with saturated brine (10 mL), dried over anhydrous sodium sulfate, filtered, and concentrated under reduced pressure to remove the solvent, and the residue was purified by preparative HPLC to give **compound 49** (30 mg, off-white solid). MS (ESI): *m/z* 288.7 [M/2+1]+。 $^1H$ NMR (300 MHz, DMSO-$d_6$) δ(ppm) 10.57 (s, 1H), 10.04 (s, 1H), 8.32 (s, 1H), 8.00 (d, *J* = 5.4 Hz, 1H), 7.68 (d, *J* = 8.4 Hz, 1H), 7.51 (d, *J* = 5.4 Hz, 1H), 7.12 (d, *J* = 2.1 Hz, 1H), 6.99 (dd, *J* = 8.4, 2.0 Hz, 1H), 3.81 - 3.70 (m, 4H), 3.09 - 2.96 (m, 5H), 2.80 (d, *J* = 10.6 Hz, 2H), 2.29 - 2.02 (m, 6H), 1.98 - 1.88 (m, 2H), 1.62 - 1.43 (m, 4H).

**Example 49: Preparation of Compound 50**

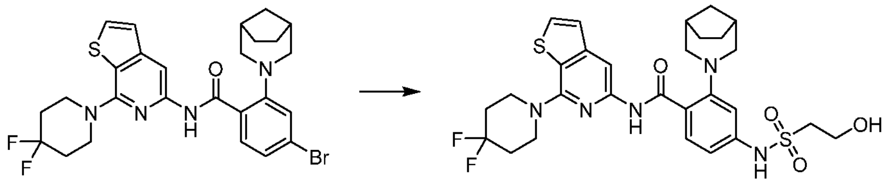

49B 50

**Preparation method:**

**Step 1: synthesis of compound 50**

**Compound 49B** (100 mg, 0.18 mmol) and 2-hydroxyethane-1-sulfonamide (27 mg, 0.22 mmol) were dissolved in N,N-dimethylformamide (4 mL), and then cuprous iodide (3 mg, 0.02 mmol), potassium phosphate (76 mg, 0.36 mmol), and (1R,2R)-(-)-N,N'-dimethyl-1,2-cyclohexanediamine (5 mg, 0.04 mmol) were sequentially added. Then, the mixture was stirred at 120 °C for 2 h under a nitrogen atmosphere. After the reaction was completed as detected by TLC, the reaction liquid was diluted with water (10 mL) and extracted with ethyl acetate (20 mL × 3). The organic phases were combined, washed with saturated brine (10 mL × 3), dried over anhydrous sodium sulfate, filtered, and concentrated under reduced pressure to remove the solvent, and the residue was purified by preparative HPLC to give **compound 50** (30 mg, off-white solid). MS (ESI): *m/z* 303.6 [M/2+1]+. $^1H$ NMR (300 MHz, DMSO-$d_6$) δ(ppm) 10.58 (s, 1H), 10.02 (br.s, 1H), 8.32 (s, 1H), 8.00 (d, *J* = 5.4 Hz, 1H), 7.67 (d, *J* = 8.4 Hz, 1H), 7.51 (d, *J* = 5.4 Hz, 1H), 7.13 (d, *J* = 2.0 Hz, 1H), 6.98 (dd, *J* = 8.4, 1.9 Hz, 1H), 4.95 (br.s, 1H), 3.78 - 3.73 (m, 6H), 3.31 (*t, J* = 6.7 Hz, 2H), 3.06 - 2.98 (m, 2H), 2.79 (d, *J* = 10.6 Hz, 2H), 2.25 - 2.06 (m, 6H), 1.98 - 1.89 (m, 2H), 1.58 - 1.45 (m, 4H).

**Example 50: Preparation of Compound 51**

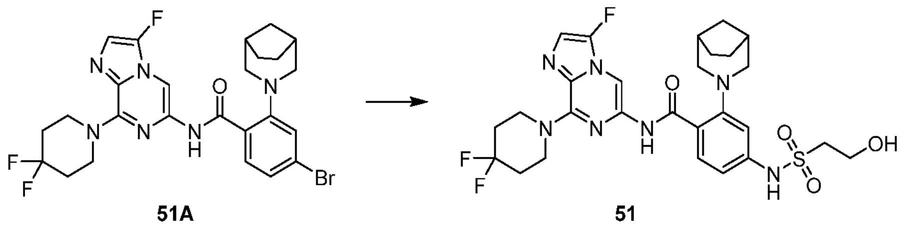

51A 51

**Step 1: synthesis of compound 51**

**Compound 51A** (180 mg, 0.3 mmol) and 2-hydroxyethane-1-sulfonamide (54 mg, 0.4 mmol) were dissolved in N,N-dimethylformamide (5 mL), and then cuprous iodide (36 mg, 0.18 mmol), potassium phosphate (198 mg, 0.87 mmol), and (1R,2R)-(-)-N,N'-dimethyl-1,2-cyclohexanediamine (42 mg, 0.26 mmol) were sequentially added. Then, the mixture was stirred at 120 °C for 2 h under a nitrogen atmosphere. After the reaction was completed as detected by LCMS, the reaction liquid was cooled to room temperature, diluted with water (10 mL), and extracted with ethyl acetate (8 mL × 3). The organic phases were combined, washed with saturated brine (8 mL), dried over anhydrous sodium sulfate, filtered, and concentrated under reduced pressure to remove the solvent, and the residue was purified by preparative HPLC to give **compound 51** (50 mg, yellow solid). MS (ESI): *m/z* 304.7 $[M/2+1]^+$. $^1H$ NMR (300 MHz, DMSO-$d_6$) δ(ppm) 10.65 (s, 1H), 10.05 (br.s, 1H), 8.51 (s, 1H), 7.70 (d, *J* = *8.5* Hz, 1H), 7.40 (d, *J* = 7.2 Hz, 1H), 7.17 (d, *J* = 2.1 Hz, 1H), 7.00 (dd, *J* = 8.5, 2.0 Hz, 1H), 4.95 (br.s, 1H), 4.44 - 4.31 (m, 4H), 3.75 (t, *J* = 6.6 Hz, 2H), 3.32 (t, *J* = 6.6 Hz, 2H), 3.03 - 2.94 (m, 2H), 2.80 (d, *J* = 10.5 Hz, 2H), 2.31 - 2.22 (m, 2H), 2.16 - 1.95 (m, 6H), 1.66 - 1.47 (m, 4H).

**Example 51: Preparation of Compound 52**

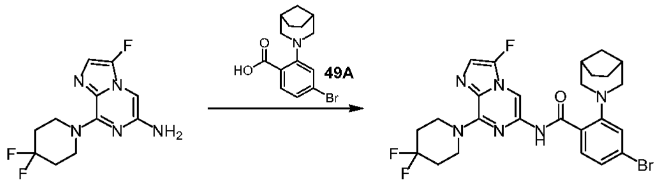


34D 51A

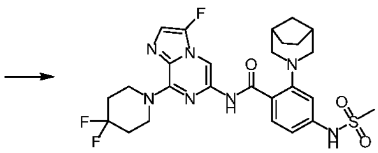

52

**Step 1: synthesis of compound 51A**

Compound 49A (456 mg, 1.5 mmol) was dissolved in dichloromethane (5 mL), and then oxalyl chloride (468 mg, 3.8 mmol) was added dropwise under an ice bath. Then, the mixture was stirred at room temperature for 30 min. After the reaction was completed as detected by TLC, the reaction liquid was concentrated to dryness by rotary evaporation, and the residue was dissolved in anhydrous tetrahydrofuran (5 mL). **Compound 34D** (400 mg, 1.5 mmol) and N,N-diisopropylethylamine (761 mg, 6.0 mmol) were added at room temperature, and then the reaction liquid was stirred at 25 °C for 2 h. After the reaction was completed as detected by TLC, the reaction liquid was diluted with water (20 mL) and extracted with ethyl acetate (15 mL × 3). The organic phases were combined, washed with saturated brine (10 mL), dried over anhydrous sodium sulfate, filtered, and concentrated under reduced pressure to remove the solvent, and the residue was purified by a silica gel column to give **compound 51A** (725 mg, yellow solid, yield: 86.6%). MS (ESI): *m/z* 563.1 565.1 $[M+1]^+$

**Step 2: synthesis of compound 52**

**Compound 51A** (180 mg, 0.3 mmol) and methanesulfonamide (42 mg, 0.4 mmol) were dissolved in N,N-dimethylformamide (5 mL), and then cuprous iodide (36 mg, 0.18 mmol), potassium phosphate (198 mg, 0.87 mmol), and (1R,2R)-(-)-N,N'-dimethyl-1,2-cyclohexanediamine (42 mg, 0.26 mmol) were sequentially added. Then, the mixture was stirred at 120 °C for 2 h under a nitrogen atmosphere. After the reaction was completed as detected by TLC, the reaction liquid was diluted with water (10 mL) and extracted with ethyl acetate (8 mL × 3). The organic phases were combined, washed with saturated brine (8 mL), dried over anhydrous sodium sulfate, filtered, and concentrated under reduced pressure to remove the solvent, and the residue was purified by preparative HPLC to give **compound 52** (60 mg, yellow solid). MS (ESI): *m/z* 289.5 $[M/2+1]^+$. $^1$H NMR (300 MHz, DMSO-$d_6$) δ(ppm) 10.64 (s, 1H), 10.08 (s, 1H), 8.51 (s, 1H), 7.71 (d, *J* = 8.5 Hz, 1H), 7.40 (d, *J* = 7.2 Hz, 1H), 7.15 (d, *J* = 2.1 Hz, 1H), 7.01 (dd, *J* = 8.5, 2.0 Hz, 1H), 4.43 - 4.32 (m, 4H), 3.08 (s, 3H), 2.99 (dd, *J* = 10.5, 3.6 Hz, 2H), 2.86 - 2.76 (m, 2H), 2.30 - 2.21 (m, 2H), 2.14 - 1.95 (m, 6H), 1.65 - 1.45 (m, 4H).

**Example 52: Preparation of Compound 53**

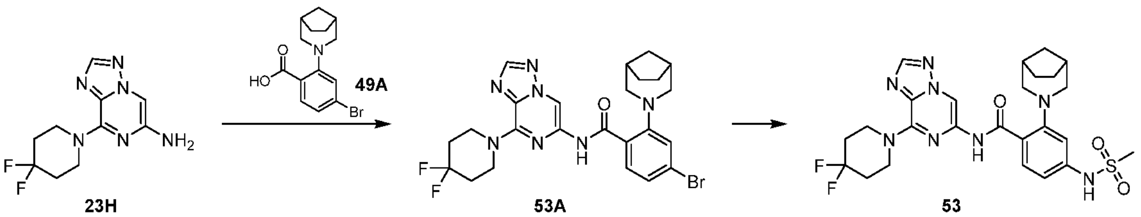


23H 53A 53

**Step 1: synthesis of compound 53A**

**Compound 49A** (438 mg, 1.42 mmol) was dissolved in dichloromethane (5 mL), and then oxalyl chloride (450 mg, 3.55 mmol) was added dropwise under an ice bath. Then, the mixture was stirred at room temperature for 30 min. After the reaction was completed as detected by TLC, the reaction liquid was concentrated to dryness by rotary evaporation, and then the residue was dissolved in anhydrous tetrahydrofuran (10 mL). A mixture of **compound 23H** (360 mg, 1.42 mmol) and N,N-diisopropylethylamine (731 mg, 5.68 mmol) was added at room temperature, and then the reaction liquid was stirred at 50 °C for 1 h. After the reaction was completed as detected by TLC, the reaction liquid was diluted with water (40 mL) and extracted with ethyl acetate (30 mL × 3). The organic phases were combined, washed with saturated brine (20 mL), dried over anhydrous sodium sulfate, filtered, and concentrated under reduced pressure to remove the solvent, and the residue was purified by a silica gel column to give **compound 53A** (350 mg, yellow solid, yield: 45.2%). MS (ESI): *m/z* 546.1 548.1 $[M+1]^+$

**Step 2: synthesis of compound 53**

**Compound 53A** (150 mg, 0.27 mmol) and methanesulfonamide (31 mg, 0.32 mmol) were dissolved in N,N-dimethylformamide (4 mL), and then cuprous iodide (5 mg, 0.03 mmol), potassium phosphate (116 mg, 0.54 mmol), and (1R,2R)-(-)-N,N'-dimethyl-1,2-cyclohexanediamine (8 mg, 0.05 mmol) were sequentially added. Then, the mixture was stirred at 120 °C for 2 h under a nitrogen atmosphere. After the reaction was completed as detected by TLC, the reaction liquid was diluted with water (20 mL) and extracted with ethyl acetate (15 mL × 3). The organic phases were combined, washed with saturated brine (10 mL), dried over anhydrous sodium sulfate, filtered, and concentrated under reduced pressure to remove the solvent, and the residue was purified by preparative HPLC to give **compound 53** (30 mg, off-white solid). MS (ESI): *m/z* 561.2 $[M+1]^+$。 $^1$H NMR (300 MHz, DMSO-$d_6$) δ(ppm) 10.92 (s, 1H), 10.09 (br.s, 1H), 9.04 (s, 1H), 8.50 (s, 1H), 7.74 (d, *J* = *8.5* Hz, 1H), 7.17 (d, *J* = 2.0 Hz, 1H), 7.06 - 6.96 (m, 1H), 4.39 - 4.32 (m, 4H), 3.08 (s, 3H), 3.02 - 2.96 (m, 2H), 2.82 (d, *J* = 10.7 Hz, 2H), 2.29 - 2.22 (m, 2H), 2.20 - 1.94 (m, 6H), 1.63 - 1.50 (m, 4H).

**Example 53: Preparation of Compound 54**

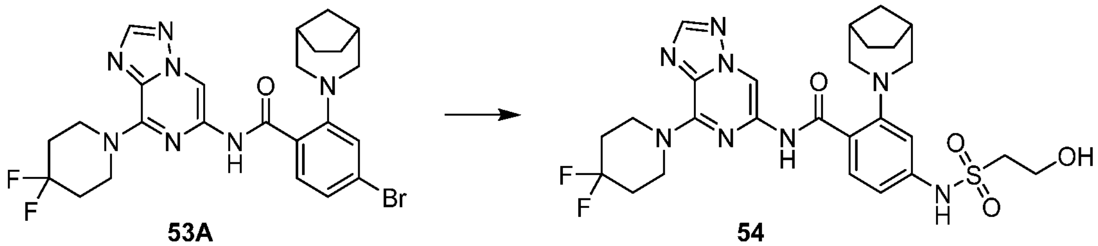

53A 54

Step 1: synthesis of compound 54

**Compound 53A** (150 mg, 0.27 mmol) and 2-hydroxyethane-1-sulfonamide (41 mg, 0.32 mmol) were dissolved in N,N-dimethylformamide (4 mL), and then cuprous iodide (5 mg, 0.03 mmol), potassium phosphate (116 mg, 0.54 mmol), and (1R,2R)-(-)-N,N'-dimethyl-1,2-cyclohexanediamine (8 mg, 0.05 mmol) were sequentially added. Then, the mixture was stirred at 120 °C for 2 h under a nitrogen atmosphere. After the reaction was completed as detected by TLC, the reaction liquid was diluted with water (20 mL) and extracted with ethyl acetate (15 mL × 3). The organic phases were combined, washed with saturated brine (10 mL × 3), dried over anhydrous sodium sulfate, filtered, and concentrated under reduced pressure to remove the solvent, and the residue was purified by preparative HPLC to give **compound 54** (30 mg, off-white solid). MS (ESI): *m/z* 591.2 $[M+1]^+$。 $^1H$ NMR (300 MHz, DMSO-$d_6$) δ(ppm) 10.92 (s, 1H), 10.06 (br.s, 1H), 9.04 (s, 1H), 8.50 (s, 1H), 7.73 (d, *J* = 8.5 Hz, 1H), 7.19 (d, *J* = 2.0 Hz, 1H), 7.01 (dd, *J* = 8.5, 2.0 Hz, 1H), 4.95 (br.s, 1H), 4.42 - 4.28 (m, 4H), 3.75 (t, *J* = 6.5 Hz, 2H), 3.32 (t, *J* = 6.5 Hz, 2H), 3.03 - 2.92 (m, 2H), 2.82 (d, *J* = 10.6 Hz, 2H), 2.29 - 2.21 (m, 2H), 2.20 - 1.92 (m, 6H), 1.66 - 1.46 (m, 4H).

**Example 54: Preparation of Compound 55**

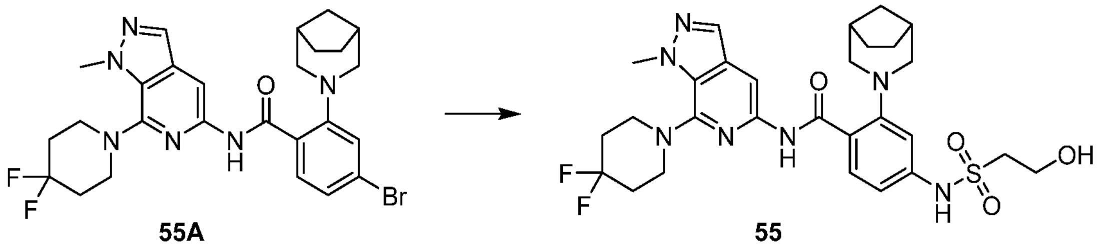

55A 55

Step 1: synthesis of compound 55

**Compound 55A** (150 mg, 0.27 mmol) and 2-hydroxyethane-1-sulfonamide (40 mg, 0.32 mmol) were dissolved in N,N-dimethylformamide (4 mL), and then cuprous iodide (4 mg, 0.03 mmol), potassium phosphate (114 mg, 0.54 mmol), and (1R,2R)-(-)-N,N'-dimethyl-1,2-cyclohexanediamine (8 mg, 0.05 mmol) were sequentially added. Then, the mixture was stirred at 120 °C for 2 h under a nitrogen atmosphere. After the reaction was completed as detected by TLC, the reaction liquid was diluted with water (20 mL) and extracted with ethyl acetate (20 mL × 3). The organic phases were combined, washed with saturated brine (10 mL × 3), dried over anhydrous sodium sulfate, filtered, and concentrated under reduced pressure to remove the solvent, and the residue was purified by preparative HPLC to give **compound 55** (30 mg, off-white solid). MS (ESI): *m/z* 302.7 $[M/2+1]^+$. $^1H$ NMR (300 MHz, DMSO-$d_6$) δ(ppm) 10.04 (s, 1H), 9.94 (br.s, 1H), 8.20 (s, 1H), 7.78 (s, 1H), 7.61 (d, *J* = 8.4 Hz, 1H), 7.07 (d, *J* = 2.0 Hz, 1H), 6.93 (dd, *J* = 8.4, 2.0 Hz, 1H), 4.91 (br.s, 1H), 4.25 - 4.17 (m, 4H), 4.11 (s, 3H), 3.72 (t, *J* = 6.6 Hz, 2H), 3.27 (t, *J* = 6.7 Hz, 2H), 3.04 - 2.95 (m, 2H), 2.74 (d, *J* = 10.6 Hz, 2H), 2.22 - 2.16 (m, 2H), 2.09 - 1.89 (m, 6H), 1.53 - 1.41 (m, 4H).

**Example 55: Preparation of Compound 56**

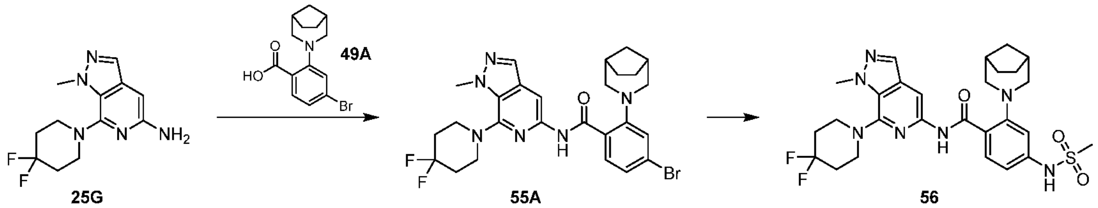


25G 55A 56

**Step 1: synthesis of compound 55A**

**Compound 49A** (556 mg, 1.80 mmol) was dissolved in dichloromethane (10 mL), and then oxalyl chloride (570 mg, 4.50 mmol) was added dropwise under an ice bath. Then, the mixture was stirred at room temperature for 30 min. After the reaction was completed as detected by TLC, the reaction liquid was concentrated to dryness by rotary evaporation, and then the residue was dissolved in anhydrous tetrahydrofuran (10 mL). A mixture of **compound 25G** (480 mg, 1.80 mmol) and N,N-diisopropylethylamine (928 mg, 7.20 mmol) was added at room temperature, and then the reaction liquid was stirred at 50 °C for 1 h. After the reaction was completed as detected by TLC, the reaction liquid was diluted with water (50 mL) and extracted with ethyl acetate (40 mL × 3). The organic phases were combined, washed with saturated brine (30 mL), dried over anhydrous sodium sulfate, filtered, and concentrated under reduced pressure to remove the solvent, and the residue was purified by a silica gel column to give **compound 55A** (350 mg, yellow solid, yield: 34.8%). MS (ESI): *m/z* 559.2 561.2 $[M+1]^+$

**Step 2: synthesis of compound 56**

**Compound 55A** (150 mg, 0.27 mmol) and methanesulfonamide (31 mg, 0.32 mmol) were dissolved in N,N-dimethylformamide (4 mL), and then cuprous iodide (4 mg, 0.03 mmol), potassium phosphate (114 mg, 0.54 mmol), and (1R,2R)-(-)-N,N'-dimethyl-1,2-cyclohexanediamine (8 mg, 0.05 mmol) were sequentially added. Then, the mixture was stirred at 120 °C for 2 h under a nitrogen atmosphere. After the reaction was completed as detected by TLC, the reaction liquid was diluted with water (20 mL) and extracted with ethyl acetate (15 mL × 3). The organic phases were combined, washed with saturated brine (10 mL × 3), dried over anhydrous sodium sulfate, filtered, and concentrated under reduced pressure to remove the solvent, and the residue was purified by preparative HPLC to give **compound 56** (30 mg, off-white solid). MS (ESI): *m/z* 287.6 $[M/2+1]^+$. $^1$H NMR (300 MHz, DMSO-$d_6$) δ(ppm) 10.03 (s, 1H), 9.97 (br.s, 1H), 8.20 (s, 1H), 7.78 (s, 1H), 7.62 (d, *J = 8.5* Hz, 1H), 7.06 (d, *J = 2.0* Hz, 1H), 6.93 (dd, *J* = 8.4, 2.0 Hz, 1H), 4.24 - 4.18 (m, 4H), 4.11 (s, 3H), 3.04 - 2.96 (m, 5H), 2.75 (d, *J* = 10.5 Hz, 2H), 2.22 - 2.15 (m, 2H), 2.07 - 1.89 (m, 6H), 1.56 - 1.42 (m, 4H).

**Example 56: Preparation of Compound 57**

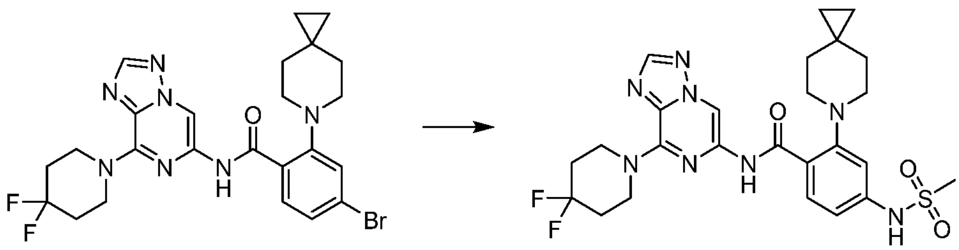

23I 57

**Step 1: synthesis of compound 57**

**Compound 23I** (100 mg, 0.18 mmol) and methanesulfonamide (21 mg, 0.22 mmol) were dissolved in N,N-dimethylformamide (2 mL), and then cuprous iodide (4 mg, 0.02 mmol), potassium phosphate (78 mg, 0.36 mmol), and (1R,2R)-(-)-N,N'-dimethyl-1,2-cyclohexanediamine (5 mg, 0.36 mmol) were sequentially added. Then, the mixture was stirred at 120 °C for 2 h under a nitrogen atmosphere. After the reaction was completed as detected by TLC, the reaction liquid was diluted with water (10 mL) and extracted with ethyl acetate (20 mL × 3). The organic phases were combined, washed with saturated brine (10 mL × 2), dried over anhydrous sodium sulfate, filtered, and concentrated under reduced pressure to remove the solvent, and the residue was purified by preparative HPLC to give **compound 57** (30 mg, off-white

solid). MS (ESI): *m/z* 281.2 [M/2+1]$^+$. $^1$H NMR (300 MHz, DMSO-$d_6$) δ(ppm) 13.28 (s, 1H), 10.29 (s, 1H), 8.95 (s, 1H), 8.49 (s, 1H), 8.10 (d, *J* = 8.7 Hz, 1H), 7.29 (d, *J* = 2.2 Hz, 1H), 7.15 (dd, *J* = 8.6, 2.1 Hz, 1H), 4.43 - 4.32 (m, 4H), 3.13 (s, 3H), 3.06 - 2.93 (m, 4H), 2.35 - 1.43 (m, 8H), 0.41 (s, 4H).

**Example 57: Preparation of Compound 58**

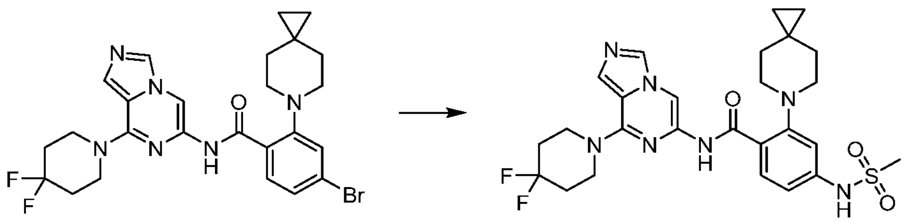

27H 58

**Step 1: synthesis of compound 58**

**Compound 27H** (200 mg, 0.37 mmol) and methanesulfonamide (42 mg, 0.44 mmol) were dissolved in N,N-dimethylformamide (4 mL), and then cuprous iodide (7 mg, 0.04 mmol), potassium phosphate (156 mg, 0.74 mmol), and (1*R*,2*R*)-(-)-*N,N'*-dimethyl-1,2-cyclohexanediamine (10 mg, 0.07 mmol) were sequentially added. Then, the mixture was stirred at 120 °C for 2 h under a nitrogen atmosphere. After the reaction was completed as detected by TLC, the reaction liquid was diluted with water (20 mL) and extracted with ethyl acetate (20 mL × 3). The organic phases were combined, washed with saturated brine (10 mL × 2), dried over anhydrous sodium sulfate, filtered, and concentrated under reduced pressure to remove the solvent, and the residue was purified by preparative HPLC to give **compound 58** (30 mg, off-white solid). MS (ESI): *m/z* 560.2 [M+1]$^+$。$^1$H NMR (300 MHz, DMSO-$d_6$) δ(ppm) 12.81 (s, 1H), 10.24 (s, 1H), 8.71 (d, *J* = 0.8 Hz, 1H), 8.53 (s, 1H), 8.08 (d, *J* = 8.6 Hz, 1H), 7.87 (s, 1H), 7.26 (d, *J* = 2.2 Hz, 1H), 7.14 (dd, *J* = 8.6, 2.1 Hz, 1H), 4.03 - 3.92 (m, 4H), 3.12 (s, 3H), 3.03 - 2.94 (m, 4H), 2.28 - 1.53 (m, 8H), 0.40 (s, 4H).

**Example 58: Preparation of Compound 59**

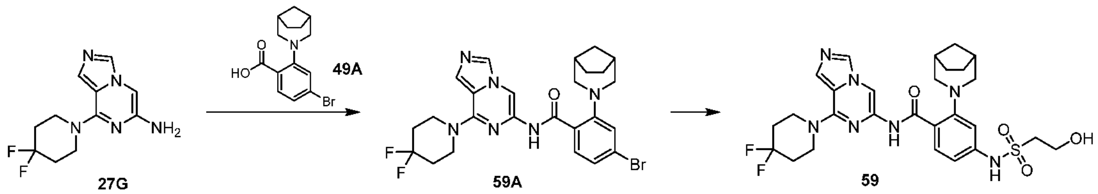

27G 49A 59A 59

**Step 1: synthesis of compound 59A**

**Compound 49A** (525 mg, 1.70 mmol) was dissolved in dichloromethane (10 mL), and then oxalyl chloride (540 mg, 4.25 mmol) was added dropwise under an ice bath. Then, the mixture was stirred at room temperature for 30 min. After the reaction was completed as detected by TLC, the reaction liquid was concentrated to dryness by rotary evaporation, and then the residue was dissolved in anhydrous tetrahydrofuran (10 mL). A mixture of **compound 27G** (430 mg, 1.70 mmol) and N,N-diisopropylethylamine (877 mg, 6.80 mmol) was added at room temperature, and then the reaction liquid was stirred at 50 °C for 1 h. After the reaction was completed as detected by TLC, the reaction liquid was diluted with water (80 mL) and extracted with ethyl acetate (60 mL × 3). The organic phases were combined, washed with saturated brine (30 mL), dried over anhydrous sodium sulfate, filtered, and concentrated under reduced pressure to remove the solvent, and the residue was purified by a silica gel column to give compound 59A (450 mg, yellow solid, purity: 75.1%, yield: 36.5%). MS (ESI): *m/z* 273.1 273.9 [M/2+1]$^+$

**Step 2: synthesis of compound 59**

**Compound 59A** (200 mg, 0.37 mmol) and 2-hydroxyethane-1-sulfonamide (55 mg, 0.44 mmol) were dissolved

in N,N-dimethylformamide (6 mL), and then cuprous iodide (7 mg, 0.04 mmol), potassium phosphate (156 mg, 0.74 mmol), and (1*R*,2*R*)-(-)-*N,N'*-dimethyl-1,2-cyclohexanediamine (10 mg, 0.07 mmol) were sequentially added. Then, the mixture was stirred at 120 °C for 2 h under a nitrogen atmosphere. After the reaction was completed as detected by TLC, the reaction liquid was diluted with water (20 mL) and extracted with ethyl acetate (30 mL × 3). The organic phases were combined, washed with saturated brine (20 mL × 3), dried over anhydrous sodium sulfate, filtered, and concentrated under reduced pressure to remove the solvent, and the residue was purified by preparative HPLC to give compound 59 (30 mg, off-white solid). MS (ESI): *m/z* 590.0 $[M+1]^+$。 $^1H$ NMR (300 MHz, DMSO-$d_6$) δ(ppm) 10.45 (s, 1H), 10.02 (br.s, 1H), 8.77 (s, 1H), 8.53 (s, 1H), 7.85 (s, 1H), 7.69 (d, *J* = 8.5 Hz, 1H), 7.15 (d, *J* = 2.1 Hz, 1H), 6.99 (dd, *J* = 8.4, 2.0 Hz, 1H), 4.94 (br.s, 1H), 3.98 - 3.87 (m, 4H), 3.75 (t, *J* = 6.6 Hz, 2H), 3.31 (t, *J* = 6.6 Hz, 2H), 3.04 - 2.94 (m, 2H), 2.79 (d, *J* = 10.6 Hz, 2H), 2.28 - 2.20 (m, 2H), 2.16 - 1.93 (m, 6H), 1.64 - 1.47 (m, 4H).

**Example 59: Preparation of Compound 60**

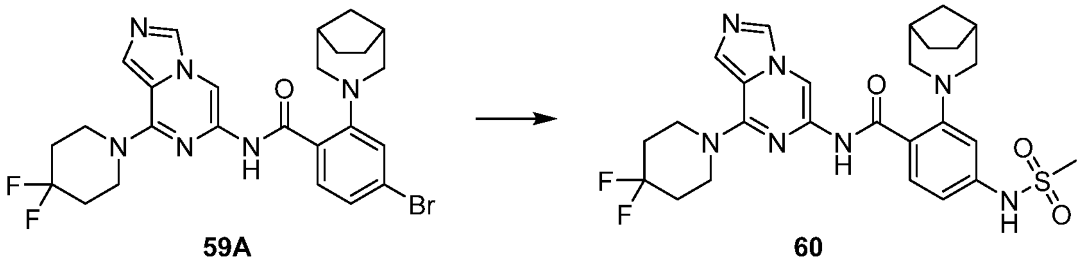

**59A** **60**

**Step 1: synthesis of compound 60**

**Compound 59A** (200 mg, 0.37 mmol) and methanesulfonamide (27 mg, 0.22 mmol) were dissolved in N,N-dimethylformamide (6 mL), and then cuprous iodide (7 mg, 0.04 mmol), potassium phosphate (156 mg, 0.74 mmol), and (1R,2R)-(-)-N,N'-dimethyl-1,2-cyclohexanediamine (10 mg, 0.07 mmol) were sequentially added. Then, the mixture was stirred at 120 °C for 2 h under a nitrogen atmosphere. After the reaction was completed as detected by TLC, the reaction liquid was diluted with water (20 mL) and extracted with ethyl acetate (30 mL × 3). The organic phases were combined, washed with saturated brine (20 mL × 3), dried over anhydrous sodium sulfate, filtered, and concentrated under reduced pressure to remove the solvent, and the residue was purified by preparative HPLC to give **compound 60** (20 mg, off-white solid). MS (ESI): *m/z* 560.3 $[M+1]^+$。 $^1H$ NMR (300 MHz, DMSO-$d_6$) δ(ppm) 10.45 (s, 1H), 10.05 (br.s, 1H), 8.77 (s, 1H), 8.53 (s, 1H), 7.85 (s, 1H), 7.70 (d, *J* = 8.4 Hz, 1H), 7.14 (d, *J* = 2.1 Hz, 1H), 7.00 (dd, *J* = 8.5, 2.0 Hz, 1H), 3.98 - 3.88 (m, 4H), 3.07 (s, 3H), 2.99 (dd, *J* = 10.7, 3.7 Hz, 2H), 2.80 (d, *J* = 10.6 Hz, 2H), 2.28 - 2.21 (m, 2H), 2.18 - 1.90 (m, 6H), 1.66 - 1.44 (m, 4H).

**Example 60: Preparation of Compound 61**

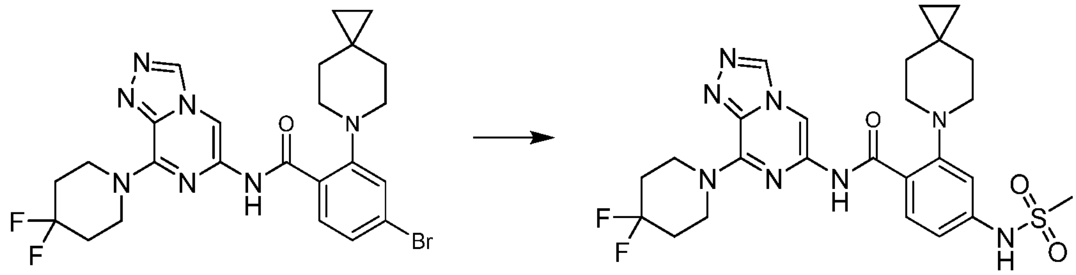

**24K** **61**

**Step 1: synthesis of compound 61**

**Compound 24K** (150 mg, 0.28 mmol) and methanesulfonamide (35 mg, 0.36 mmol) were dissolved in N,N-dimethylformamide (4 mL), and then cuprous iodide (32 mg, 0.17 mmol), potassium phosphate (170 mg, 0.81 mmol), and (1*R*,2*R*)-(-)-*N,N'*-dimethyl-1,2-cyclohexanediamine (34 mg, 0.24 mmol) were sequentially added. Then, the mixture was

stirred at 120 °C for 2 h under a nitrogen atmosphere. After the reaction was completed as detected by TLC, the reaction liquid was diluted with water (10 mL) and extracted with ethyl acetate (15 mL × 3). The organic phases were combined, washed with saturated brine (8 mL × 2), dried over anhydrous sodium sulfate, filtered, and concentrated under reduced pressure to remove the solvent, and the residue was purified by preparative HPLC to give **compound 61** (30 mg, yellow solid). MS (ESI): *m/z* 561.2 [M+1]$^+$。$^1$H NMR (300 MHz, DMSO-$d_6$) δ(ppm) 13.08 (s, 1H), 10.27 (s, 1H), 9.37 (s, 1H), 8.79 (s, 1H), 8.09 (d, *J* = 8.6 Hz, 1H), 7.28 (d, *J = 2.2* Hz, 1H), 7.15 (dd, *J* = 8.7, 2.1 Hz, 1H), 4.54 - 4.38 (m, 4H), 3.13 (s, 3H), 3.04 - 2.94 (m, 4H), 2.27 - 2.06 (m, 5H), 1.87 - 1.62 (m, 3H), 0.41 (s, 4H).

**Example 61: Preparation of Compound 62**

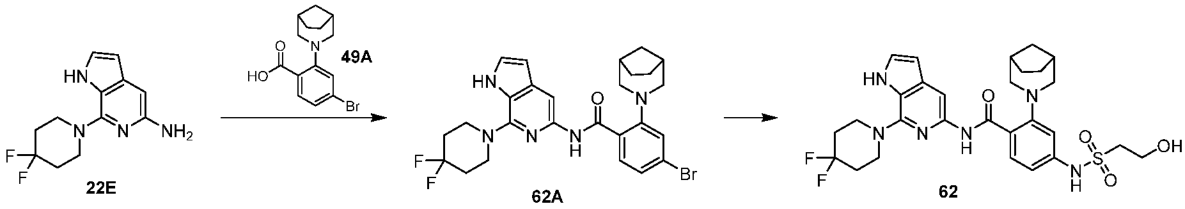


22E 62A 62

**Step 1: synthesis of compound 62A**

**Compound 49A** (800 mg, 2.6 mmol) was dissolved in dichloromethane (10 mL), and then oxalyl chloride (821 mg, 6.5 mmol) was added dropwise under an ice bath. Then, the mixture was stirred at room temperature for 30 min. After the reaction was completed as detected by TLC, the reaction liquid was concentrated to dryness by rotary evaporation, and then the residue was dissolved in anhydrous tetrahydrofuran (10 mL). **Compound 22E** (652 mg, 2.6 mmol) and N,N-diisopropylethylamine (1.33 g, 10.4 mmol) were added at room temperature, and then the mixture was stirred at 25 °C for 1 h. After the reaction was completed as detected by TLC, the reaction liquid was diluted with water (20 mL) and extracted with ethyl acetate (15 mL × 3). The organic phases were combined, dried over anhydrous sodium sulfate, filtered, and concentrated under reduced pressure to remove the solvent, and the residue was purified by a silica gel column to give compound **62A** (980 mg, 1.8 mmol, yellow solid, yield: 69%). MS (ESI): *m/z* 272.6 273.4[M/2+1]$^+$

**Step 2: synthesis of compound 62**

**Compound 62A** (200 mg, 0.36 mmol) and 2-hydroxyethane-1-sulfonamide (60 mg, 0.43 mmol) were dissolved in N,N-dimethylformamide (4 mL), and then cuprous iodide (42 mg, 0.22 mmol), potassium phosphate (226 mg, 1.01 mmol), and (1*R,*2*R*)-(-)-*N,N'*-dimethyl-1,2-cyclohexanediamine (44 mg, 0.31 mmol) were sequentially added. Then, the mixture was stirred at 120 °C for 2 h under a nitrogen atmosphere. After the reaction was completed as detected by TLC, the reaction liquid was diluted with water (10 mL) and extracted with ethyl acetate (15 mL × 3). The organic phases were combined, washed with saturated brine (8 mL × 3), dried over anhydrous sodium sulfate, filtered, and concentrated under reduced pressure to remove the solvent, and the residue was purified by preparative HPLC to give **compound 62** (52.7 mg, yellow solid). MS (ESI): *m/z* 589.1 [M+1]$^+$。$^1$H NMR (300 MHz, DMSO-$d_6$) δ(ppm) 11.32 (s, 1H), 10.37 (s, 1H), 10.01 (s, 1H), 8.09 (s, 1H), 7.72 (dd, *J = 8.4,* 1.4 Hz, 1H), 7.46 (t, *J* = 2.8 Hz, 1H), 7.15 (d, *J* = 2.2 Hz, 1H), 6.99 (dd, *J = 8.5,* 2.0 Hz, 1H), 6.46 (dd, *J* = 3.0, 1.6 Hz, 1H), 4.94 (br.s, 1H), 3.74 (t, *J* = 6.6 Hz, 2H), 3.52 - 3.44 (m, 4H), 3.31 (t, *J* = 6.7 Hz, 2H), 3.05 - 2.96 (m, 2H), 2.79 (d, *J* = 10.5 Hz, 2H), 2.26 - 2.08 (m, 6H), 2.02 - 1.94 (m, 2H), 1.59 - 1.44 (m, 4H).

**Example 62: Preparation of Compound 63**

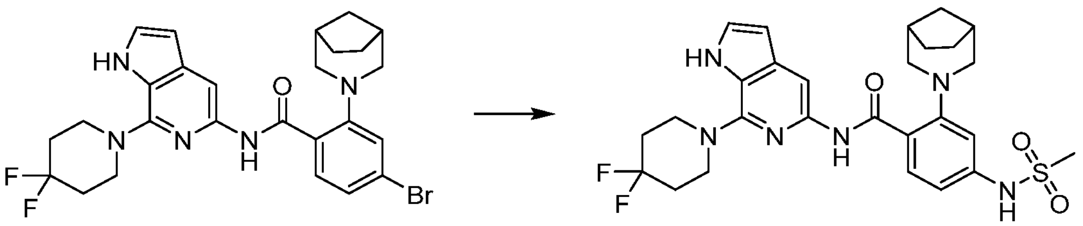

62A 63

**Step 1: synthesis of compound 63**

**Compound 62A** (200 mg, 0.36 mmol) and methanesulfonamide (46 mg, 0.43 mmol) were dissolved in N,N-dimethylformamide (4 mL), and then cuprous iodide (42 mg, 0.22 mmol), potassium phosphate (226 mg, 1.01 mmol), and (1*R,2R*)-(-)-*N,N'*-dimethyl-1,2-cyclohexanediamine (44 mg, 0.31 mmol) were sequentially added. Then, the mixture was stirred at 120 °C for 2 h under a nitrogen atmosphere. After the reaction was completed as detected by TLC, the reaction liquid was diluted with water (10 mL) and extracted with ethyl acetate (15 mL × 3). The organic phases were combined, washed with saturated brine (8 mL × 3), dried over anhydrous sodium sulfate, filtered, and concentrated under reduced pressure to remove the solvent, and the residue was purified by preparative HPLC to give **compound 63** (61.3 mg, yellow solid). MS (ESI): *m/z* 559.2 $[M+1]^+$.$^1$H NMR (300 MHz, DMSO-$d_6$) δ(ppm) 11.32 (s, 1H), 10.37 (s, 1H), 10.03 (s, 1H), 8.10 (d, *J* = 1.8 Hz, 1H), 7.73 (d, *J* = 8.5 Hz, 1H), 7.46 (t, *J* = 2.8 Hz, 1H), 7.14 (d, *J* = 2.1 Hz, 1H), 6.99 (dd, *J* = 8.4, 2.0 Hz, 1H), 6.46 (s, 1H), 3.52 - 3.48 (m, 4H), 3.07 (s, *J* = 1.8 Hz, 3H), 3.06 - 2.97 (m, 2H), 2.80 (d, *J* = 10.5 Hz, 2H), 2.25 - 2.10 (m, 6H), 2.03 - 1.93 (m, 2H), 1.59 - 1.44 (m, 4H).

**Example 63: Preparation of Compound 64**

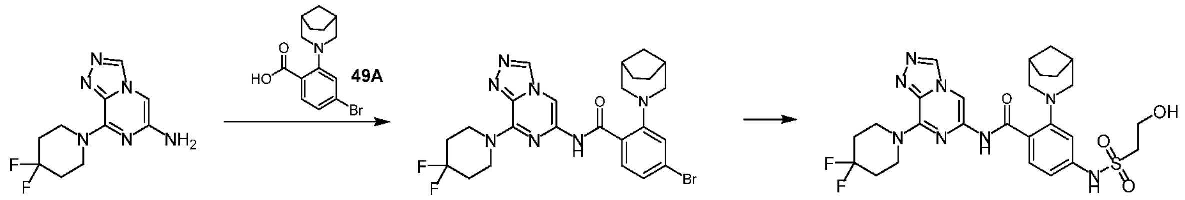


24J 64A 64

**Step 1: synthesis of compound 64A**

**Compound 49A** (729 mg, 2.4 mmol) was dissolved in dichloromethane (10 mL), and then oxalyl chloride (750 mg, 6 mmol) was added dropwise under an ice bath. Then, the mixture was stirred at room temperature for 30 min. After the reaction was completed as detected by TLC, the reaction liquid was concentrated to dryness by rotary evaporation, and then the residue was dissolved in anhydrous tetrahydrofuran (10 mL). **Compound 24J** (600 mg, 2.4 mmol) and N,N-diisopropylethylamine (1.2 g, 9.6 mmol) were added at room temperature, and then the mixture was stirred at room temperature for 1 h. After the reaction was completed as detected by TLC, the reaction liquid was diluted with water (20 mL) and extracted with ethyl acetate (15 mL × 3). The organic phases were combined, dried over anhydrous sodium sulfate, filtered, and concentrated under reduced pressure to remove the solvent, and the residue was purified by a silica gel column to give **compound 64A** (573 mg, yellow solid, yield: 45.8%). MS (ESI): *m/z* 273.6 274.5 $[M/2+1]^+$

**Step 2: synthesis of compound 64**

**Compound 64A** (180 mg, 0.33 mmol) and 2-hydroxyethane-1-sulfonamide (54 mg, 0.43 mmol) were dissolved in N,N-dimethylformamide (4 mL), and then cuprous iodide (376 mg, 0.20 mmol), potassium phosphate (203 mg, 0.96 mmol), and (1*R*,2*R*)-(-)-*N,N'*-dimethyl-1,2-cyclohexanediamine (40 mg, 0.28 mmol) were sequentially added. Then, the mixture was stirred at 120 °C for 2 h under a nitrogen atmosphere. After the reaction was completed as detected by TLC, the reaction liquid was diluted with water (10 mL) and extracted with ethyl acetate (10 mL × 3). The organic phases were combined, washed with saturated brine (8 mL × 3), dried over anhydrous sodium sulfate, filtered, and concentrated under reduced pressure to remove the solvent, and the residue was purified by preparative HPLC to give **compound 64** (30.1 mg, yellow solid). MS (ESI): *m/z* 591.3 $[M+1]^+$。$^1$H NMR (300 MHz, DMSO-$d_6$) δ(ppm) 10.67 (s, 1H), 10.05 (br.s, 1H), 9.37

(d, *J* = 1.2 Hz, 1H), 8.86 (d, *J* = 1.1 Hz, 1H), 7.68 (d, *J* = *8.4* Hz, 1H), 7.16 (d, *J* = *2.2* Hz, 1H), 6.99 (d, *J* = 8.5 Hz, 1H), 4.95 (br.s, 1H), 4.50 - 4.31 (m, 4H), 3.75 (t, *J* = 6.6 Hz, 2H), 3.31 (t, *J* = 7.1 Hz, 2H), 2.98 (d, *J* = 10.5 Hz, 2H), 2.80 (d, *J* = 10.5 Hz, 2H), 2.28 - 2.06 (m, 6H), 1.99 - 1.90 (m, 2H), 1.65 - 1.44 (m, 4H).

**Example 64: Preparation of Compound 65**

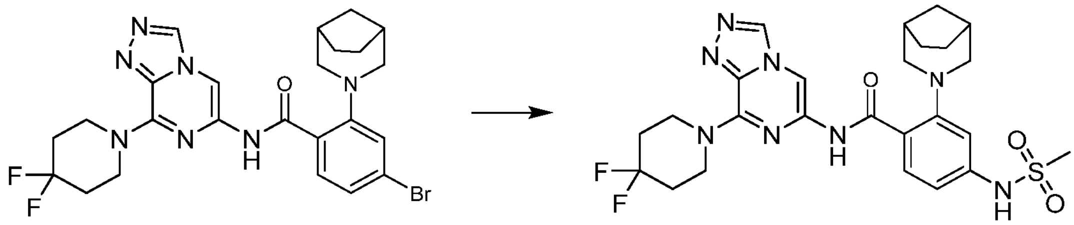

**64A** **65**

**Step 1: synthesis of compound 65**

**Compound 64A** (180 mg, 0.33 mmol) and methanesulfonamide (41 mg, 0.43 mmol) were dissolved in N,N-dimethylformamide (4 mL), and then cuprous iodide (376 mg, 0.20 mmol), potassium phosphate (203 mg, 0.96 mmol), and (1*R*,2*R*)-(-)-*N,N'*-dimethyl-1,2-cyclohexanediamine (40 mg, 0.28 mmol) were sequentially added. Then, the mixture was stirred at 120 °C for 2 h under a nitrogen atmosphere. After the reaction was completed as detected by TLC, the reaction liquid was diluted with water (10 mL) and extracted with ethyl acetate (15 mL × 3). The organic phases were combined, washed with saturated brine (8 mL × 3), dried over anhydrous sodium sulfate, filtered, and concentrated under reduced pressure to remove the solvent, and the residue was purified by preparative HPLC to give **compound 65** (37.7 mg, yellow solid). MS (ESI): *m/z* 561.2 $[M+1]^+$。$^1H$ NMR (300 MHz, DMSO-$d_6$) δ(ppm) 10.66 (s, 1H), 10.08 (br.s, 1H), 9.37 (d, *J* = 1.3 Hz, 1H), 8.86 (d, *J* = 1.4 Hz, 1H), 7.70 (d, *J* = 8.5 Hz, 1H), 7.14 (d, *J* = 2.1 Hz, 1H), 7.00 (dd, *J* = *8.4,* 2.0 Hz, 1H), 4.50 - 4.33 (m, 4H), 3.07 (s, 3H), 2.99 (d, *J* = 10.6 Hz, 2H), 2.81 (d, *J* = 10.6 Hz, 2H), 2.27 - 2.06 (m, 6H), 2.00 - 1.91 (m, 2H), 1.65 - 1.44 (m, 4H).

**Example 65: Preparation of Compound 66**

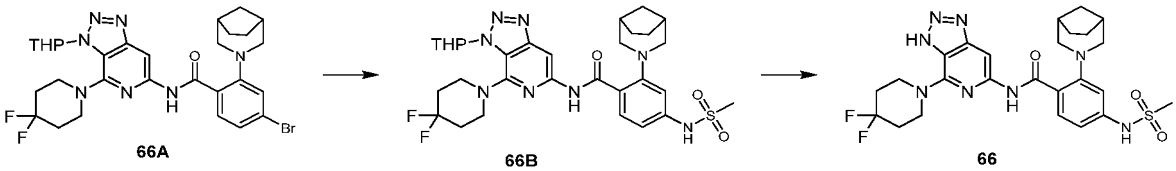

**66A** **66B** **66**

**Preparation method:**

**Step 1: synthesis of compound 66B**

**Compound 66A** (180 mg, 0.29 mmol) and methanesulfonamide (35 mg, 0.09 mmol) were dissolved in N,N-dimethylformamide (4 mL), and then cuprous iodide (33 mg, 0.17 mmol), potassium phosphate (176 mg, 0.84 mmol), and (1R,2R)-(-)-N,N'-dimethyl-1,2-cyclohexanediamine (34 mg, 0.25 mmol) were sequentially added. Then, the mixture was stirred at 120 °C for 2 h under a nitrogen atmosphere. After the reaction was completed as detected by TLC, the reaction liquid was diluted with water (10 mL) and extracted with ethyl acetate (20 mL × 3). The organic phases were combined, washed with saturated brine (8 mL × 3), dried over anhydrous sodium sulfate, filtered, and concentrated under reduced pressure to remove the solvent, and the residue was purified by a silica gel column to give **compound 66B** (120 mg, yellow solid, yield: 65%). MS (ESI): *m/z* 281.1$[M-THP/2+1]^+$

**Step 2: synthesis of compound 66**

**Compound 66B** (120 mg, 0.19 mmol) was dissolved in a solution of hydrochloric acid in ethyl acetate (3 mL), and then the mixture was stirred at room temperature for 0.5 h. After the reaction was completed as detected by TLC, the mixture was concentrated under reduced pressure to remove the solvent, and the residue was purified by preparative HPLC to give **compound 66** (42.1 mg, white solid). MS (ESI): *m/z* 281.1 $[M/2+1]^+$. $^1H$ NMR (300 MHz, DMSO-$d_6$) δ(ppm) 15.73 (s, 1H), 10.47 (s, 1H), 10.07 (br.s, 1H), 7.86 (d, *J* = 1.6 Hz, 1H), 7.66 (d, *J* = 8.4 Hz, 1H), 7.10 (d, *J* = 2.3 Hz, 1H), 6.98 (dd, *J* = 8.4, 2.1 Hz, 1H), 4.42 - 4.27 (m, 4H), 3.10 - 2.97 (m, 5H), 2.79 (d, *J* = 10.6 Hz, 2H), 2.27 - 2.17 (m, 2H), 2.14 - 2.00 (m, 4H), 1.97 - 1.90 (m, 2H), 1.61 - 1.43 (m, 4H).

**Example 66: Preparation of Compound 67**

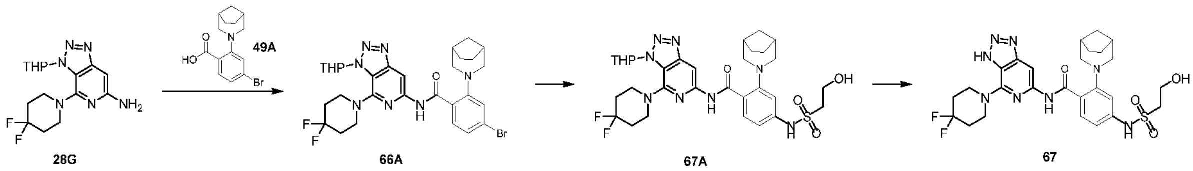


28G 66A 67A 67

**Step 1: synthesis of compound 66A**

**Compound 49A** (457 mg, 1.5 mmol) and **compound 28G** (625 mg, 1.8 mmol) were dissolved in pyridine (10 mL), and then phosphorus oxychloride (1.4 mg, 9 mmol) was added dropwise under an ice bath. Then, the mixture was stirred at room temperature for 3 h. After the reaction was completed as detected by TLC, the reaction liquid was adjusted to pH = 6 by adding a 1 N hydrochloric acid solution under an ice bath and then extracted with ethyl acetate (15 mL × 3). The organic phases were combined, dried over anhydrous sodium sulfate, filtered, and concentrated under reduced pressure to remove the solvent, and the residue was purified by a silica gel column to give **compound 66A** (360 mg, yellow solid, yield: 38%). MS (ESI): *m/z* 273.6 274.3$[M-THP/2+1]^+$

**Step 2: synthesis of compound 67A**

**Compound 66A** (180 mg, 0.29 mmol) and 2-hydroxyethane-1-sulfonamide (46 mg, 0.09 mmol) were dissolved in N,N-dimethylformamide (4 mL), and then cuprous iodide (33 mg, 0.17 mmol), potassium phosphate (176 mg, 0.84 mmol), and (1*R*,2*R*)-(-)-*N,N'*-dimethyl-1,2-cyclohexanediamine (34 mg, 0.25 mmol) were sequentially added. Then, the mixture was stirred at 120 °C for 2 h under a nitrogen atmosphere. After the reaction was completed as detected by TLC, the reaction liquid was diluted with water (10 mL) and extracted with ethyl acetate (10 mL × 3). The organic phases were combined, washed with saturated brine (8 mL × 3), dried over anhydrous sodium sulfate, filtered, and concentrated under reduced pressure to remove the solvent, and the residue was purified by a silica gel column to give **compound 67A** (180 mg, yellow solid, yield: 89.6%). MS (ESI): *m/z* 296.2$[M-THP/2+1]^+$

**Step 3: synthesis of compound 67**

**Compound 67A** (180 mg, 0.26 mmol) was dissolved in a solution of hydrochloric acid in ethanol (3 mL), and then the mixture was stirred at room temperature for 0.5 h. After the reaction was completed as detected by TLC, the mixture was concentrated under reduced pressure to remove the solvent, and the residue was purified by preparative HPLC to give **compound 67** (47.5 mg, white solid). MS (ESI): *m/z* 296.2 $[M/2+1]^+$. $^1H$ NMR (300 MHz, DMSO-$d_6$) δ(ppm) 15.59 (br.s, 1H), 10.48 (s, 1H), 10.03 (s, 1H), 7.86 (s, 1H), 7.65 (d, *J* = 8.3 Hz, 1H), 7.12 (s, 1H), 7.02 - 6.90 (m, 1H), 4.95 (br.s, 1H), 4.42 - 4.31 (m, 4H), 3.74 (d, *J* = 6.7 Hz, 2H), 3.31 (t, *J* = 6.6 Hz, 2H), 3.01 (d, *J* = 9.9 Hz, 2H), 2.78 (d, *J* = 10.5 Hz, 2H), 2.29 - 2.18 (m, 2H), 2.15 - 2.01 (m, 4H), 1.98 - 1.89 (m, 2H), 1.61 - 1.40 (m, 4H).

**Example 67: Preparation of Compound 68**

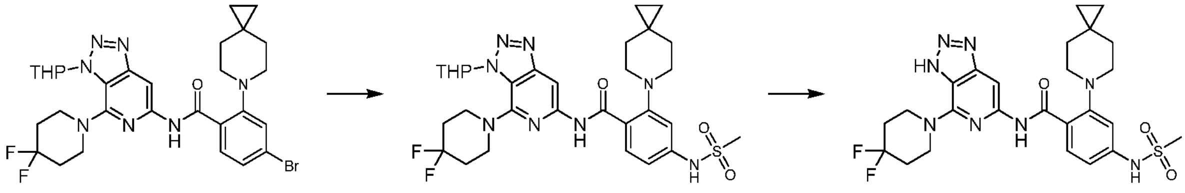

28H 68A 68

### Step 1: synthesis of compound 68A

**Compound 28H** (200 mg, 0.32 mmol) and methanesulfonamide (39 mg, 0.42 mmol) were dissolved in *N,N*-dimethylformamide (4 mL), and then cuprous iodide (36 mg, 0.19 mmol), potassium phosphate (195 mg, 0.93 mmol), and (1*R*,2*R*)-(-)-*N,N'*-dimethyl-1,2-cyclohexanediamine (39 mg, 0.28 mmol) were sequentially added. Then, the mixture was stirred at 120 °C for 2 h under a nitrogen atmosphere. After the reaction was completed as detected by TLC, the reaction liquid was diluted with water (10 mL) and extracted with ethyl acetate (15 mL × 3). The organic phases were combined, washed with saturated brine (8 mL × 3), dried over anhydrous sodium sulfate, filtered, and concentrated under reduced pressure to remove the solvent, and the residue was purified by a silica gel column to give **compound 68A** (144 mg, yellow solid, yield: 68.7%). MS (ESI): *m/z* 281.2 [M-THP/2+1]$^+$

### Step 2: synthesis of compound 68

**Compound 68A** (144 mg, 0.22 mmol) was dissolved in a solution of hydrochloric acid in ethyl acetate (3 mL). Then, the mixture was stirred at room temperature for 0.5 h. After the reaction was completed as detected by TLC, the mixture was concentrated under reduced pressure to remove the solvent, and the residue was purified by preparative HPLC to give compound **68** (50.6 mg, white solid). MS (ESI): *m/z* 281.2 [M/2+1]$^+$. $^1$H NMR (300 MHz, DMSO-$d_6$) δ(ppm) 15.60 (br.s, 1H), 12.99 (s, 1H), 10.27 (br.s, 1H), 8.10 (dd, *J* = 8.7, 1.4 Hz, 1H), 7.85 (d, *J* = 1.4 Hz, 1H), 7.26 (d, *J* = 2.2 Hz, 1H), 7.13 (dd, *J* = 8.7, 2.0 Hz, 1H), 4.49 - 4.32 (m, 4H), 3.12 (s, 3H), 3.05 - 2.94 (m, 4H), 2.29 - 2.02 (m, 5H), 1.91 - 1.55 (m, 3H), 0.40 (s, 4H).

### Example 68: Preparation of Compound 69

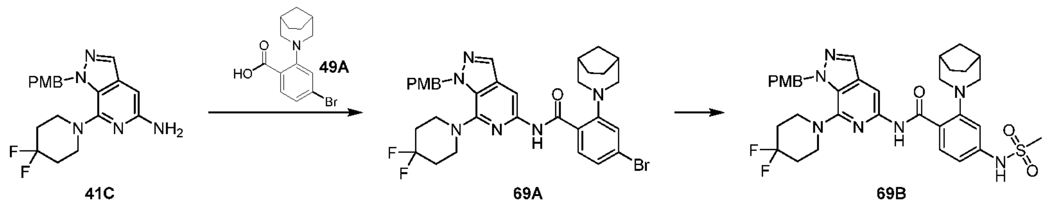


41C 69A 69B

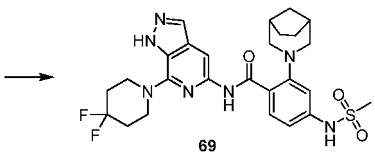

69

### Step 1: synthesis of compound 69A

**Compound 49A** (646 mg, 2.09 mmol) was dissolved in dichloromethane (10 mL), and then oxalyl chloride (664 mg, 5.23 mmol) was added dropwise under an ice bath. Then, the mixture was stirred at room temperature for 30 min. After the reaction was completed as detected by TLC, the reaction liquid was concentrated to dryness by rotary evaporation, and then the residue was dissolved in anhydrous tetrahydrofuran (10 mL). A mixture of **compound 41C** (780 mg, 2.09 mmol) and N,N-diisopropylethylamine (1.1 g, 8.36 mmol) was added at room temperature, and then the reaction liquid was stirred at 50 °C for 1 h. After the reaction was completed as detected by TLC, the reaction liquid was diluted with water (80 mL) and extracted with ethyl acetate (50 mL × 3). The organic phases were combined, washed with saturated brine (30 mL), dried

over anhydrous sodium sulfate, filtered, and concentrated under reduced pressure to remove the solvent, and the residue was purified by a silica gel column to give **compound 69A** (800 mg, yellow solid, purity: 81.2%, yield: 46.7%). MS (ESI): *m*/*z* 332.9 334.2 [M/2+1]$^+$

**Step 2: synthesis of compound 69B**

**Compound 69A** (200 mg, 0.30 mmol) and methanesulfonamide (34 mg, 0.36 mmol) were dissolved in N,N-dimethylformamide (6 mL), and then cuprous iodide (6 mg, 0.03 mmol), potassium phosphate (128 mg, 0.60 mmol), and (1R,2R)-(-)-N,N'-dimethyl-1,2-cyclohexanediamine (9 mg, 0.06 mmol) were sequentially added. Then, the mixture was stirred at 120 °C for 2 h under a nitrogen atmosphere. After the reaction was completed as detected by TLC, the reaction liquid was diluted with water (50 mL) and extracted with ethyl acetate (30 mL × 3). The organic phases were combined washed with saturated brine (20 mL), dried over anhydrous sodium sulfate, filtered, and concentrated under reduced pressure to remove the solvent, and the residue was purified by a silica gel column to give **compound 69B** (100 mg, yellow oil, purity: 93.7%, yield: 45.9%). MS (ESI): *m*/*z* 340.7[M/2+1]$^+$

**Step 3: synthesis of compound 69**

**Compound 69B** (100 mg, 0.14 mmol) was dissolved in trifluoroacetic acid (2 mL), and then trifluoromethanesulfonic acid (0.2 mL) was added dropwise under an ice bath. Then, the mixture was stirred at room temperature for 30 min. After the reaction was completed as detected by TLC, the reaction liquid was diluted with water (20 mL), then adjusted to pH = 8 with a sodium bicarbonate solid, and extracted with ethyl acetate (20 mL × 3). The organic phases were combined, washed with saturated brine (30 mL), dried over anhydrous sodium sulfate, filtered, and concentrated under reduced pressure to remove the solvent, and the residue was purified by preparative HPLC to give **compound 69** (20 mg, off-white solid). MS (ESI): *m*/*z* 560.2 [M+1]$^+$。$^1$H NMR (300 MHz, DMSO-$d_6$) δ(ppm) 13.94 (s, 1H), 10.31 - 9.98 (m, 2H), 8.22 (s, 1H), 7.96 (s, 1H), 7.67 (d, *J* = 8.4 Hz, 1H), 7.10 (d, *J* = *2.1* Hz, 1H), 6.97 (dd, *J* = *8.4,* 1.9 Hz, 1H), 4.32 - 3.79 (m, 4H), 3.08 - 2.98 (m, 5H), 2.79 (d, *J* = 10.5 Hz, 2H), 2.27 - 2.19 (m, 2H), 2.17 - 1.93 (m, 6H), 1.58 - 1.45 (m, 4H).

**Example 69: Preparation of Compound 70**

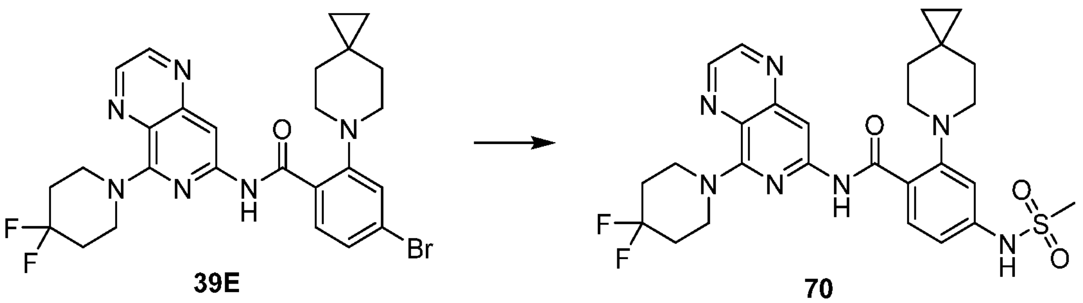

39E 70

**Step 1: synthesis of compound 70**

**Compound 39E** (100 mg, 0.18 mmol) and methanesulfonamide (21 mg, 0.22 mmol) were dissolved in N,N-dimethylformamide (2 mL), and then cuprous iodide (3 mg, 0.02 mmol), potassium phosphate (76 mg, 0.36 mmol), and (1R,2R)-(-)-N,N'-dimethyl-1,2-cyclohexanediamine (5 mg, 0.04 mmol) were sequentially added. Then, the mixture was stirred at 120 °C for 2 h under a nitrogen atmosphere. After the reaction was completed as detected by TLC, the reaction liquid was diluted with water (20 mL) and extracted with ethyl acetate (10 mL × 3). The organic phases were combined, washed with saturated brine (10 mL), dried over anhydrous sodium sulfate, filtered, and concentrated under reduced pressure to remove the solvent, and the residue was purified by preparative HPLC to give **compound 70** (8 mg, yellow solid). MS (ESI): *m*/*z* 286.6 [M/2+1]$^+$. $^1$H NMR (300 MHz, DMSO-$d_6$) δ(ppm) 13.09 (s, 1H), 10.30 (s, 1H), 8.93 (d, *J* = 1.8 Hz, 1H), 8.69 (d, *J* = 1.8 Hz, 1H), 8.22 - 8.06 (m, 2H), 7.27 (d, *J* = 2.1 Hz, 1H), 7.20 - 7.05 (m, 1H), 4.28 - 4.16 (m, 4H), 3.13 (s, 3H), 3.06 - 2.98 (m, 4H), 2.33 - 1.56 (m, 8H), 0.41 (s, 4H).

**Example 70: Preparation of Compound 71**

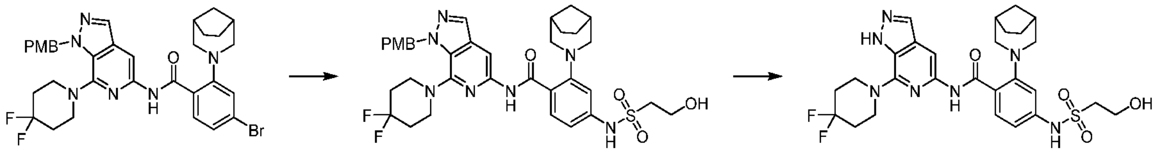

69A 71A 71

**Step 1: synthesis of compound 71A**

**Compound 69A** (200 mg, 0.30 mmol) and 2-hydroxyethane-1-sulfonamide (45 mg, 0.36 mmol) were dissolved in N,N-dimethylformamide (6 mL), and then cuprous iodide (6 mg, 0.03 mmol), potassium phosphate (128 mg, 0.60 mmol), and (1R,2R)-(-)-N,N'-dimethyl-1,2-cyclohexanediamine (9 mg, 0.06 mmol) were sequentially added. Then, the mixture was stirred at 120 °C for 2 h under a nitrogen atmosphere. After the reaction was completed as detected by TLC, the reaction liquid was diluted with water (50 mL) and extracted with ethyl acetate (30 mL × 3). The organic phases were combined, washed with saturated brine (20 mL), dried over anhydrous sodium sulfate, filtered, and concentrated under reduced pressure to remove the solvent, and the residue was purified by a silica gel column to give **compound 71A** (100 mg, yellow oil, purity: 88.5%, yield: 41.5%). MS (ESI): *m/z* 355.7 $[M/2+1]^+$

**Step 3: synthesis of compound 71**

**Compound 71A** (100 mg, 0.14 mmol) was dissolved in trifluoroacetic acid (2 mL), and then trifluoromethanesulfonic acid (0.2 mL) was added dropwise under an ice bath. Then, the mixture was stirred at room temperature for 30 min. After the reaction was completed as detected by TLC, the reaction liquid was diluted with water (20 mL), then adjusted to pH = 8 with a sodium bicarbonate solid, and extracted with ethyl acetate (20 mL × 3). The organic phases were combined, washed with saturated brine (30 mL), dried over anhydrous sodium sulfate, filtered, and concentrated under reduced pressure to remove the solvent, and the residue was purified by a silica gel column to give a crude product of the compound, which was then purified by preparative thin layer chromatography to give **compound 71** (30 mg, off-white solid). MS (ESI): *m/z* 590.3 $[M+1]^+$。 $^1$H NMR (300 MHz, DMSO-$d_6$) δ(ppm) 14.20 - 13.23 (m, 1H), 10.51 - 9.92 (m, 2H), 8.32 - 7.79 (m, 2H), 7.72 - 7.61 (m, 1H), 7.11 (s, 1H), 6.96 (d, *J* = 8.3 Hz, 1H), 4.96 (t, *J* = 5.7 Hz, 1H), 4.35 - 4.19 (m, 2H), 3.83 - 3.60 (m, 4H), 3.31 (t, *J* = 5.7 Hz, 2H), 3.08 - 2.96 (m, 2H), 2.82 - 2.73 (m, 2H), 2.26 - 1.93 (m, 8H), 1.61 - 1.44 (m, 4H).

**Example 71: Preparation of Compound 72**

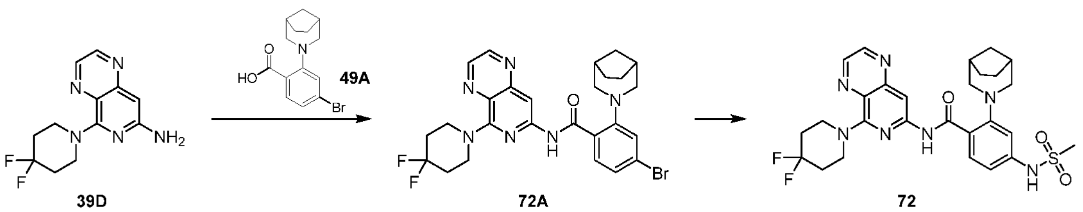


39D 72A 72

**Step 1: synthesis of compound 72A**

Compound **49A** (408 mg, 1.32 mmol) was dissolved in dichloromethane (5 mL), and then oxalyl chloride (420 mg, 3.30 mmol) was added dropwise under an ice bath. Then, the mixture was stirred at room temperature for 30 min. After the reaction was completed as detected by TLC, the reaction liquid was concentrated to dryness by rotary evaporation, and then the residue was dissolved in anhydrous tetrahydrofuran (10 mL). Compound **39D** (350 mg, 1.32 mmol) and N,N-diisopropylethylamine (512 mg, 3.96 mmol) were sequentially added at room temperature, and then the reaction liquid was stirred at 50 °C for 16 h. After the reaction was completed as detected by TLC, the reaction liquid was diluted with water (40 mL) and extracted with ethyl acetate (40 mL × 3). The organic phases were combined, dried over anhydrous sodium sulfate, filtered, and concentrated under reduced pressure to remove the solvent, and the residue was purified by column chromatography to give compound **72A** (200 mg, brownish-red solid, yield: 27.2%).

MS (ESI): *m/z* 279.2 280.2 $[M/2+1]^+$

**Step 2: synthesis of compound 72**

Compound **72A** (100 mg, 0.18 mmol) and methanesulfonamide (21 mg, 0.22 mmol) were dissolved in N,N-dimethylformamide (3 mL), and then cuprous iodide (3 mg, 0.04 mmol), potassium phosphate (76 mg, 0.36 mmol), and (1R,2R)-(-)-N,N'-dimethyl-1,2-cyclohexanediamine (5 mg, 0.04 mmol) were sequentially added. Then, the mixture was stirred at 120 °C for 2 h under a nitrogen atmosphere. After the reaction was completed as detected by TLC, the reaction liquid was diluted with water (20 mL) and extracted with ethyl acetate (10 mL × 3). The organic phases were combined, washed with saturated brine (10 mL), dried over anhydrous sodium sulfate, filtered, and concentrated under reduced pressure to remove the solvent, and the residue was purified by preparative high-performance liquid chromatography to give compound **72** (8 mg, orange-yellow solid).

MS (ESI): *m/z* 286.6 $[M/2+1]^+$.

$^1$H NMR (300 MHz, DMSO-$d_6$) δ(ppm) 10.65 (s, 1H), 10.07 (br.s, 1H), 8.93 (d, *J* = 1.8 Hz, 1H), 8.69 (d, *J* = 1.8 Hz, 1H), 8.17 (s, 1H), 7.65 (d, *J* = 8.3 Hz, 1H), 7.09 (s, 1H), 6.97 (d, *J* = 8.4 Hz, 1H), 4.22 - 4.14 (m, 4H), 3.08 - 2.99 (m, 5H), 2.85 - 2.77 (m, 2H), 2.26 - 2.05 (m, 6H), 1.97 - 1.86 (m, 2H), 1.58 - 1.43 (m, 4H).

**Example 72: Preparation of Compound 73**

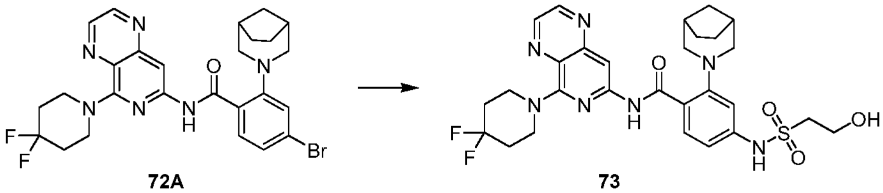

72A 73

Compound **72A** (200 mg, 0.36 mmol) and 2-hydroxyethane-1-sulfonamide (54 mg, 0.44 mmol) were dissolved in N,N-dimethylformamide (6 mL), and then cuprous iodide (7 mg, 0.04 mmol), potassium phosphate (153 mg, 0.72 mmol), and (1*R*,2*R*)-(-)-*N,N'*-dimethyl-1,2-cyclohexanediamine (10 mg, 0.08 mmol) were sequentially added. Then, the mixture was stirred at 120 °C for 2 h under a nitrogen atmosphere. After the reaction was completed as detected by TLC, the reaction liquid was diluted with water (20 mL) and extracted with ethyl acetate (10 mL × 3). The organic phases were combined, washed with saturated brine (10 mL), dried over anhydrous sodium sulfate, filtered, and concentrated under reduced pressure to remove the solvent, and the residue was purified by preparative chromatography to give compound **73** (55 mg, orange-yellow solid).

MS (ESI): *m/z* 301.7 $[M/2+1]^+$.

$^1$H NMR (300 MHz, DMSO-$d_6$) δ(ppm) 10.64 (s, 1H), 10.03 (br.s, 1H), 8.93 (t, *J* = 1.5 Hz, 1H), 8.68 (t, *J* = 1.6 Hz, 1H), 8.17 (s, 1H), 7.64 (d, *J* = 8.3 Hz, 1H), 7.11 (d, *J* = 2.1 Hz, 1H), 6.97 (dd, *J* = 8.4, 1.8 Hz, 1H), 4.96 (br.s, 1H), 4.24 - 4.11 (m, 4H), 3.76 (t, *J* = 6.6 Hz, 2H), 3.31 (t, *J* = 6.6 Hz, 2H), 3.07 - 2.98 (m, 2H), 2.84 - 2.75 (m, 2H), 2.25 - 2.06 (m, 6H), 1.95 - 1.86 (m, 2H), 1.57 - 1.44 (m, 4H).

**Example 73: Preparation of Compound 74**

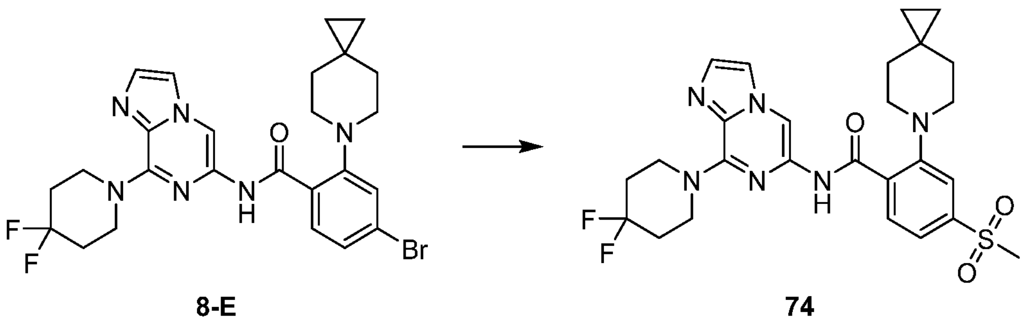

**8-E** **74**

Compound **8-E** (200 mg, 0.37 mmol) and sodium methanesulfinate (56 mg, 0.56 mmol) were dissolved in dimethyl sulfoxide (5 mL), and then sodium hydroxide (3 mg, 0.074 mmol), cuprous iodide (7 mg, 0.037 mmol), and L-proline (9 mg, 0.074 mmol) were sequentially added. Then, the mixture was stirred at 95 °C for 16 h under a nitrogen atmosphere. After the reaction was completed as detected by LCMS, the reaction liquid was diluted with water (20 mL) and extracted with ethyl acetate (20 mL × 3). The organic phases were combined, washed with saturated brine (10 mL × 3), dried over anhydrous sodium sulfate, filtered, and concentrated under reduced pressure to remove the solvent, and the residue was purified by silica gel column chromatography, and then purified by preparative chromatography to give compound **74** (5 mg, pale yellow solid).

MS (ESI): *m/z* 273.0 $[M/2+1]^+$.

$^1H$ NMR (400 MHz, DMSO-$d_6$) δ(ppm) 12.80 (br.s, 1H), 8.85 (s, 1H), 8.30 (d, *J* = 8.2 Hz, 1H), 8.11 (d, *J = 1.1* Hz, 1H), 7.95 (d, *J* = 1.8 Hz, 1H), 7.85 (dd, *J* = 8.2, 1.7 Hz, 1H), 7.57 (d, *J* = 1.0 Hz, 1H), 4.48 - 4.39 (m, 4H), 3.31 (s, 3H), 3.14 - 3.08 (m, 4H), 2.18 - 2.07 (m, 4H), 1.78 - 1.66 (m, 4H), 0.40 (s, 4H).

**Example** 74: **Preparation of Compound 75**

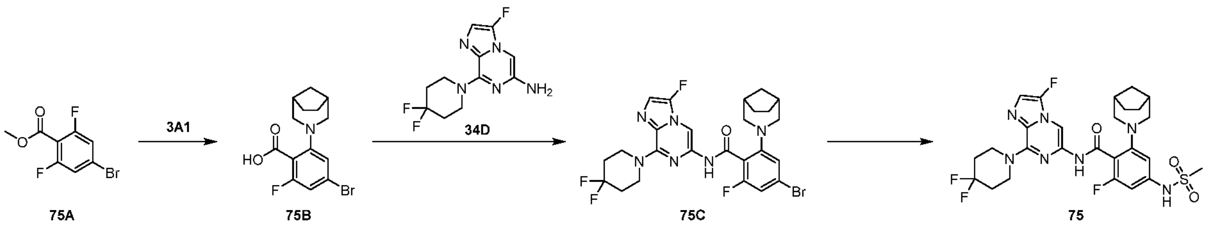


75A 75B 75C 75

Step 1: synthesis of compound **75B**

Compound **75A** (1 g, 3.98 mmol) was dissolved in methylpyrrolidone (10 mL), and then compound 3A1 (1.77 g, 15.92 mmol) and potassium carbonate (4.42 g, 31.84 mmol) were added. Then, the mixture was stirred at 120 °C for 16 h under a nitrogen atmosphere. After the reaction was completed as detected by LCMS, the reaction liquid was cooled to room temperature, diluted with water (50 mL), and extracted with ethyl acetate (50 mL × 3). The organic phases were combined, washed with saturated brine (30 mL ×3), dried over anhydrous sodium sulfate, filtered, and concentrated under reduced pressure to remove the solvent. The residue was purified by silica gel column chromatography to give compound **75B** (310 mg, yellow solid).

MS (ESI): m/z 328.0$[M+1]^+$

Step 2: synthesis of compound **75C**

Compound **75B** (217.78 mg, 0.664 mmol) was dissolved in dichloromethane (5 mL), and then oxalyl chloride (210.58 mg, 1.66 mmol) was added dropwise at 0 °C. The mixture was stirred at room temperature for 30 min. After the reaction was completed as detected by TLC, the mixture was concentrated to dryness by rotary evaporation to remove the solvent, and the residue was dissolved in tetrahydrofuran (3 mL). Then, compound **34D** (180 mg, 0.664 mmol) and a solution of N,N-diisopropylethylamine in tetrahydrofuran (3 mL) were added to the reaction liquid, and the mixture was stirred at 50 °C for one hour. After the reaction was completed as detected by LCMS, the reaction liquid was diluted with water (15 mL) and extracted with dichloromethane (20 mL × 3). The organic phases were combined, washed with saturated brine (10 mL), dried over anhydrous sodium sulfate, filtered, and concentrated under reduced pressure to remove the solvent, and the residue was purified by silica gel column chromatography to give compound 75C (101 mg, yellow solid).

Step 3: synthesis of compound 75

Compound 75C (119 mg, 0.205 mmol) was dissolved in anhydrous N,N-dimethylformamide (5 mL), and then methanesulfonamide (38.4 mg, 0.404 mmol), (1*S*,2*S*)-(+)-*N,N'*-dimethyl-1,2-cyclohexanediamine (5.83 mg, 0.041 mmol), cuprous iodide (3.89 mg, 0.0205 mmol), and anhydrous potassium phosphate (86.8 mg, 0.41 mmol) were added. Then, the mixture was stirred at 120 °C for 4 h under a nitrogen atmosphere. After the reaction was completed as detected by LCMS, the reaction liquid was diluted with water (15 mL) and extracted with ethyl acetate (20 mL × 3). The organic phases were combined, washed with saturated brine (10 mL), dried over anhydrous sodium sulfate, filtered, and concentrated under reduced pressure to remove the solvent, and the residue was purified by chromatography to give compound **75** (71 mg, pale yellow solid).

MS (ESI): m/z 298.6 $[M/2+1]^+$.

$^1H$ NMR (400 MHz, DMSO-*d6*) δ(ppm) 10.59 (s, 1H), 10.08 (s, 1H), 8.38 (s, 1H), 7.40 (d, *J* = 7.2 Hz, 1H), 6.72 - 6.60 (m, 2H), 4.43 - 4.29 (m, 4H), 3.12 - 3.02 (m, 5H), 2.74 (d, *J* = 10.4 Hz, 2H), 2.19 - 1.98 (m, 6H), 1.64 (d, *J* = 7.3 Hz, 2H), 1.49 - 1.32 (m, 4H).

**Example 75: Preparation of Compound 76**

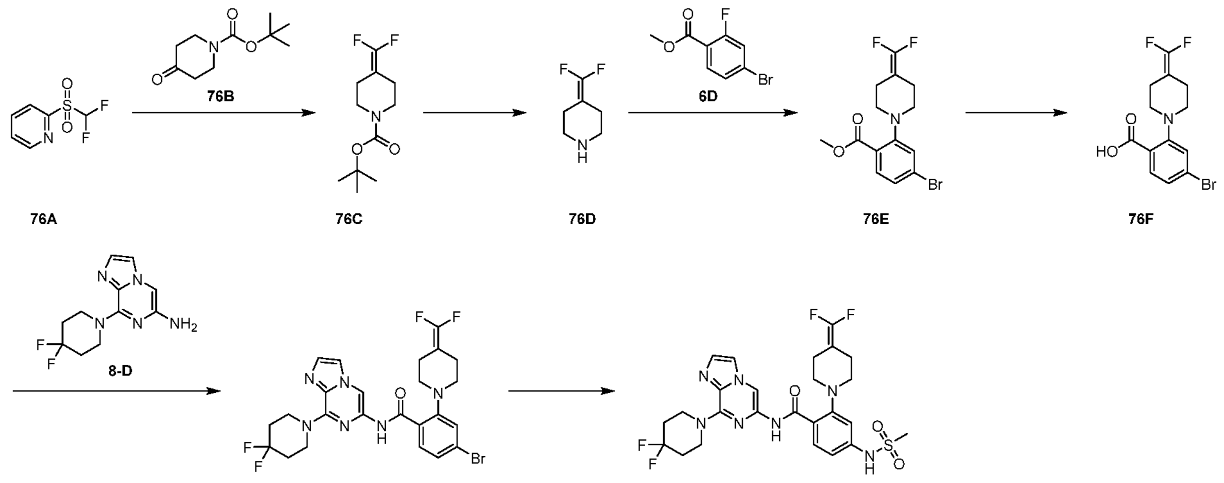

76B, 6D, 8-D

76A 76C 76D 76E 76F

76G 76

**Step 1: synthesis of compound 76C**

A solution of compound **76A** (9.2 g, 47.6 mmol) and compound **76B** (11.3 g, 57.1 mmol) in anhydrous N,N-dimethylformamide (50 mL) was slowly added to a solution of potassium tert-butoxide (9.6 g, 85.7 mmol) in anhydrous N,N-dimethylformamide (40 mL) at -60 °C, and then the mixture was stirred at -60 °C for 0.5 h under a nitrogen atmosphere. After the reaction was completed as detected by TLC, the reaction was quenched with a saturated sodium bicarbonate solution (50 mL) at -60 °C, and the mixture was stirred for 0.5 h. Then, a 3 N hydrochloric acid solution (100 mL) was slowly added, and the mixture was stirred for 0.5 h. Then, the mixture was warmed to room temperature and extracted with ethyl acetate (30 mL × 3). The organic phases were combined, washed with saturated brine (30 mL), dried over anhydrous sodium sulfate, then filtered to remove the drying agent, and concentrated under reduced pressure to remove the solvent, and the residue was purified by silica gel column chromatography to give compound **76C** (11.0 g, yellow oil).

MS (ESI): *m*/*z* 234.1 $[M+1]^+$

**Step 2: synthesis of compound 76D**

Compound **76C** (700 mg, 3.0 mmol) was dissolved in a solution of hydrochloric acid in ethyl acetate (10 mL), and then the mixture was stirred at 25 °C for 4 h. After the reaction was completed as detected by TLC, the reaction liquid was concentrated under reduced pressure to remove the solvent to give a crude product of compound **76D** (500 mg, white solid).

MS (ESI): *m*/*z* 134.1 $[M+1]^+$

**Step 3: synthesis of compound 76E**

Compound **76D** (500 mg, 2.9 mmol) was dissolved in N-methyl-2-pyrrolidone (5 mL), and then potassium carbonate (814 mg, 5.8 mmol) and compound **6D** (827 mg, 3.5 mmol) were added. After the addition was completed, the mixture was stirred at 120 °C for 24 h in a sealed bottle. After the reaction was completed as detected by TLC, the reaction liquid was cooled to room temperature and added to ice water (30 mL), and then the mixture was extracted with ethyl acetate (10 mL × 3). The organic phases were combined, washed with saturated brine (10 mL × 3), dried over anhydrous sodium sulfate, then filtered to remove the drying agent, and concentrated under reduced pressure to remove the solvent, and the residue was purified by silica gel column chromatography to give compound **76E** (800 mg, 2.3 mmol, yellow oil).

MS (ESI): *m*/*z* 346.1 $[M+1]^+$

**Step 4: synthesis of compound 76F**

Compound **76E** (800 mg, 2.3 mmol) was dissolved in methanol (8 mL) and water (8 mL), and then lithium hydroxide monohydrate (486 mg, 11.5 mmol) was added. Then, the mixture was stirred at room temperature for 5 h. After the reaction was completed as detected by TLC, the reaction liquid was diluted with water (20 mL) and extracted with ethyl acetate (15 mL). The aqueous phase was collected, adjusted to acidic with 1 N diluted hydrochloric acid, extracted with ethyl acetate (15 mL × 2), dried over anhydrous sodium sulfate, filtered, and concentrated under reduced pressure to remove the solvent, and the residue was purified by a silica gel column to give compound **76F** (250 mg, yellow solid).

MS (ESI): *m/z* 332.0 $[M+1]^+$

**Step 5: synthesis of compound 76G**

Compound **76F** (220 mg, 0.65 mmol) was dissolved in dichloromethane (3 mL), and then oxalyl chloride (211 mg, 1.63 mmol) was added dropwise under an ice bath. Then, the mixture was stirred at room temperature for 30 min. After the reaction was completed as detected by TLC, the reaction liquid was concentrated to dryness by rotary evaporation, and then the residue was dissolved in anhydrous tetrahydrofuran (3 mL). Compound **8-D** (168 mg, 0.65 mmol) and N,N-diisopropylethylamine (342 mg, 2.6 mmol) were added under an ice bath, and then the reaction liquid was stirred at 25 °C for 1 h. After the reaction was completed as detected by TLC, the reaction liquid was diluted with water (20 mL) and extracted with ethyl acetate (15 mL × 3). The organic phases were combined, dried over anhydrous sodium sulfate, filtered, and concentrated under reduced pressure to remove the solvent, and the residue was purified by a silica gel column to give **compound 76G** (220 mg, yellow solid).

MS (ESI): *m/z* 284.1 $[M/2+1]^+$

**Step 6: synthesis of compound 76**

Compound **76G** (220 mg, 0.39 mmol) and methanesulfonamide (44 mg, 0.51 mmol) were dissolved in N,N-dimethylformamide (4 mL), and then cuprous iodide (43 mg, 0.23 mmol), potassium phosphate (217 mg, 1.13 mmol), and (1*R*,2*R*)-(-)-*N,N'*-dimethyl-1,2-cyclohexanediamine (43 mg, 0.34 mmol) were sequentially added. Then, the mixture was stirred at 120 °C for 2 h under a nitrogen atmosphere in a sealed bottle. After the reaction was completed as detected by TLC, the reaction liquid was diluted with water (10 mL) and extracted with ethyl acetate (10 mL × 3). The organic phases were combined, washed with saturated brine (8 mL × 3), dried over anhydrous sodium sulfate, filtered, and concentrated under reduced pressure to remove the solvent, and the residue was purified by preparative chromatography to give compound **76** (20.4 mg, pale yellow solid).MS (ESI): *m/z* 582.0 $[M+1]^+$.

$^1H$ NMR (400 MHz, DMSO-$d_6$) δ(ppm) 12.47 (s, 1H), 10.29 (s, 1H), 8.82 (s, 1H), 8.15 - 8.00 (m, 2H), 7.56 (s, 1H), 7.22 - 7.06 (m, 2H), 4.42 - 4.31 (m, 4H), 3.12 (s, 3H), 3.05 - 2.96 (m, 4H), 2.58 - 2.53 (m, 4H), 2.09 - 1.96 (m, 4H).

**Efficacy Experimental Example 1: Experiment on Anti-Proliferative Activity Against Cells**

This assay can be used to examine the potency of compounds in inhibiting the proliferation of tumor cells (OVCAR3, HT-29, BT-549, HCT-116). A lower $IC_{50}$ value indicates high potency of the compound as a KIF18A inhibitor in the following assay setup.

a) Detection of OVCAR3 cell proliferation activity by CTG method

i.Experimental materials for detection by CTG method:

1640 medium; fetal bovine serum; penicillin/streptomycin antibiotics purchased from Wisent; CellTiter-Glo (chemiluminescence detection reagent for cell viability) reagent purchased from Promega; OVCAR3 cell line purchased from Nanjing Cobioer Biosciences Co., Ltd.; Nivo multimode microplate reader (PerkinElmer).

ii.Experimental method for detection by CTG:

OVCAR3 cells were seeded into a white 96-well plate, with 80 μL of cell suspension containing 2000 OVCAR3 cells added to each well. The cell plate was incubated in a $CO_2$ incubator overnight.

A test compound was 5-fold diluted to the 8th concentration (i.e., from 2 mM to 5.12 nM) using a multichannel pipette, with duplicate wells set up. To an intermediate plate was added 78 μL of medium, and the serially diluted compound was transferred to the corresponding wells of the intermediate plate at 2 μL/well. The mixture was mixed well and then transferred to the cell plate at 20 μL/well. The concentration of the compound transferred to the cell plate ranged

from 10 μM to 0.026 nM. The cell plate was incubated in a $CO_2$ incubator for 7 days. Another cell plate was prepared and read for signal values on the day of compound addition, and these values were used as the maximum values (the Max value in the equation below) in data analysis. To this cell plate was added the chemiluminescence detection reagent for cell viability at 25 μL/well, and the luminescence signals were stabilized by incubation at room temperature for 10 min. Readings were taken using the multimode microplate reader.

To the cell plate was added the chemiluminescence detection reagent for cell viability at 25 μL/well, and the luminescence signals were stabilized by incubation at room temperature for 10 min. Readings were taken using the multimode microplate reader.

ii.Data analysis for detection by CTG method:

The original data were converted to inhibition rates using the equation (Sample - Min)/(Max - Min) × 100%, and the $IC_{50}$ values were then obtained by curve fitting using a four-parameter method (obtained from the "log(inhibitor) vs. response -- Variable slope" model in GraphPad Prism).

b) Detection of cell proliferation activity by CCK8 method

i.Experimental materials for detection by CCK8 method:

McCoy's 5A medium purchased from Sangon Biotech; CCK8 kit purchased from Beyotime; OVCAR3, HT-29, HCT-116, and BT-549 cells purchased from Shanghai Fuheng Biotechnology Co., Ltd.; multimode microplate reader (Thermo).

ii.Experimental method for detection by CCK8:

Cells at the logarithmic growth phase were taken, digested with a cell lysis buffer (trypsin), centrifuged, counted, and seeded into a 96-well plate at an appropriate cell density (BT549: 1500 cells/well in 1640 medium; HT-29 and HCT-116: 2000 cells/well in McCoy's 5A medium), with 180 μL per well, for a total of 60 wells. The surrounding wells were filled with an equal amount of PBS to prevent water evaporation from the edge wells. The next day after cell plating, the test compounds at different concentration gradients (with an initial final concentration of 10 μM, diluted in a 1:5 gradient to obtain 9 concentration gradients) were added to the wells. Three replicate wells were set up for each concentration point, and a corresponding DMSO negative control group was also set up. Detection: After 96 h of drug treatment, the test cell culture plate was removed from the incubator. All the liquid in the 96-well plate was aspirated, and 100 μL of medium containing 10% CCK8 solution was added to each well. The plate was then incubated at 37 °C for 2 h (the OD450 of the DMSO group reached 1.5 or more), and the absorbance at 450 nm was measured using a microplate reader.

ii.Data analysis for detection by CCK8 method:

After the background value was subtracted from the measurement for each well, the inhibition rate was calculated. Inhibition Rate (%) = (1 - Sample/Vehicle) × 100. Sample is the luminescence value of the drug treatment group, and Vehicle is the luminescence value of the DMSO control group. A sigmoidal dose-survival rate curve was plotted using a non-linear regression model in the GraphPad Prism 7.0 software, and the $IC_{50}$ values were calculated.

Table 1. $IC_{50}$ data for the inhibitory activity of the compounds of the present disclosure against the proliferation of OVCAR3, HT-29, BT-549, and HCT-116 cells

| Compound No. | OVCAR3 CTG method $IC_{50}$ (nM) | OVCAR3 CCK8 method $IC_{50}$ (nM) | HT-29 CCK8 method $IC_{50}$ (nM) | BT-549 CCK8 method $IC_{50}$ (nM) | HCT-116 CCK8 method $IC_{50}$ (nM) |
|---|---|---|---|---|---|
| 1 | 167 | - | - | - | - |
| 2 | 5225 | - | - | - | - |
| 3 | 85 | 145 | 115 | 145 | > 10000 |
| 4 | 186 | - | - | - | - |
| 6 | 118 | 57 | 144 | 146 | > 10000 |
| 7 | 2007 | - | - | - | - |
| 8 | 5.8 | 7.2 | 19 | 7.2 | > 10000 |

(continued)

| Compound No. | OVCAR3 CTG method $IC_{50}$ (nM) | OVCAR3 CCK8 method $IC_{50}$ (nM) | HT-29 CCK8 method $IC_{50}$ (nM) | BT-549 CCK8 method $IC_{50}$ (nM) | HCT-116 CCK8 method $IC_{50}$ (nM) |
|---|---|---|---|---|---|
| 9 | 122 | - | - | - | - |
| 10 | 16 | - | 61 | 52 | > 10000 |
| 11 | 36 | - | 146 | 109 | > 10000 |
| 12 | 15 | - | 73 | 34 | > 10000 |
| 13 | 33 | - | 131 | 110 | > 10000 |
| 14 | 5.6 | - | 30 | 25 | > 10000 |
| 15 | 24 | - | 107 | 86 | > 10000 |
| 16 | 53 | - | 338 | 192 | > 10000 |
| 17 | 6.3 | - | 27 | 33 | > 10000 |
| 18 | 93 | - | 336 | 247 | > 10000 |
| 19 | - | 23.1 | 44 | 69.6 | > 10000 |
| 20 | - | 116.8 | 518 | 735.9 | > 10000 |
| 21 | - | 3.4 | 5.1 | 8.2 | > 10000 |
| 22 | 11.2 | 3.4 | 20.6 | 22.1 | > 10000 |
| 23 | 6.4 | 5.7 | 22.5 | 22.6 | > 10000 |
| 24 | 17.8 | 12.3 | 141.8 | 52.8 | > 10000 |
| 25 | 6.1 | 4.4 | 13.5 | 16.2 | > 10000 |
| 26 | 29.3 | 19.3 | 38 | 73.6 | > 10000 |
| 27 | 38.6 | 27.2 | 141 | 81.9 | > 10000 |
| 28 | 27.4 | 23.4 | 320.6 | 116.7 | > 10000 |
| 29 | 219.7 | 206.3 | 334.7 | 1035.4 | > 10000 |
| 30 | 64.2 | 30.4 | 73.9 | 104.6 | > 10000 |
| 31 | 67.6 | 41.8 | 93.8 | 110.6 | > 10000 |
| 32 | 29.9 | 22.4 | 45.3 | 46.1 | > 10000 |
| 33 | 75.0 | 53.3 | 84.2 | 117.9 | > 10000 |
| 34 | - | 10.6 | 28.8 | 33.3 | > 10000 |
| 35 | - | 1847 | - | - | > 10000 |
| 36 | - | 283.2 | 520.2 | 1605 | > 10000 |
| 37 | - | 234.2 | - | 610.7 | > 10000 |
| 38 | - | 11.9 | 132.5 | 31.9 | > 10000 |
| 39 | - | 10.1 | 28.15 | 26.6 | > 10000 |
| 40 | - | 68.3 | 177.3 | 218.8 | > 10000 |
| 42 | - | 83.2 | 229 | 304 | > 10000 |
| 43 | - | 23.4 | 94.6 | 65.2 | > 10000 |
| 44 | - | 733.4 | 1642 | 1900.5 | > 10000 |
| 45 | - | 54.3 | 94 | 169.9 | > 10000 |
| 46 | - | 34.8 | 55.6 | 75.8 | > 10000 |
| 47 | - | 8 | 10.5 | 18.9 | > 10000 |

(continued)

| Compound No. | OVCAR3 CTG method $IC_{50}$ (nM) | OVCAR3 CCK8 method $IC_{50}$ (nM) | HT-29 CCK8 method $IC_{50}$ (nM) | BT-549 CCK8 method $IC_{50}$ (nM) | HCT-116 CCK8 method $IC_{50}$ (nM) |
|---|---|---|---|---|---|
| 48 | - | 12.2 | 17.1 | 26.3 | > 10000 |
| 49 | - | 132.1 | 150 | 292.8 | > 10000 |
| 50 | - | 51.9 | 55.5 | 174.6 | > 10000 |
| 51 | - | 26.1 | 35.7 | 54.7 | > 10000 |
| 52 | - | 52.9 | 75.2 | 208.9 | > 10000 |
| 53 | - | 32.8 | 44.7 | 169.6 | > 10000 |
| 54 | - | 11.7 | 26.7 | 54.7 | > 10000 |
| 55 | - | 6.8 | 12.7 | 54.4 | > 10000 |
| 56 | - | 16.2 | 25.4 | 121 | > 10000 |
| 57 | - | 14.6 | 33.8 | 31.9 | > 10000 |
| 58 | - | 37.9 | 97.1 | 90.4 | > 10000 |
| 59 | - | 27.5 | 81.6 | 83 | > 10000 |
| 60 | - | 63.2 | 143.7 | 240 | > 10000 |
| 61 | - | 23.2 | 59.6 | 30.9 | > 10000 |
| 62 | - | 6 | 15.1 | 23.1 | > 10000 |
| 63 | - | 10.1 | 15.6 | 33.7 | > 10000 |
| 64 | - | 10.2 | 55.2 | 42.4 | > 10000 |
| 65 | - | 14.6 | 40.4 | 53.2 | > 10000 |
| 66 | - | 18.6 | 71.5 | 90.1 | > 10000 |
| 67 | - | 13.9 | 88.1 | 61.8 | > 10000 |
| 68 | - | 43.4 | 156.8 | 90.9 | > 10000 |
| 69 | - | 6.5 | 11.5 | 39.6 | > 10000 |
| 70 | - | 31.5 | 68.9 | 91 | > 10000 |
| 71 | - | 5.0 | 17.4 | 39.7 | > 10000 |
| 72 | - | 66.6 | 141.1 | 330.5 | > 10000 |
| 73 | - | 20.2 | 41.8 | 77.9 | > 10000 |
| 74 | - | 47.9 | 82.4 | 156.3 | - |
| 75 | - | - | 97.3 | - | >5000 |
| 76 | - | - | 19.4 | - | >5000 |

As can be seen from Table 1, the compounds of the present disclosure exhibited relatively good anti-proliferative effects on OVCAR3, HT-29 and BT-549 aneuploid cancer cells with chromosomal instability (CIN) characteristics, and some of the compounds had inhibitory effects of less than 100 nM, indicating good clinical application prospects; in contrast, the compounds showed no inhibitory effect on the proliferation of HCT116 tumor cells without chromosomal instability (CIN) characteristics, demonstrating good selectivity.

**Efficacy Experimental Example 2: Activity Detection Experiment at Molecular Level**

In this experiment, an ADP-Glo kit was used to detect the inhibitory activity of the compounds against KIF18A. Each compound was prepared into a 10 mM stock solution with DMSO, which was then diluted at 1:5 to obtain 9 gradients with the initial final concentration set at 10 μM. Meanwhile, a control group and a blank group were also set up. The reaction

buffer consisted of 15 mM Tris (pH 7.5), 10 mM MgCl2, and 0.01% Pluronic F-68. 4 nM KIF18A (1-467) and the test compound were added to a 384-well plate and incubated at 37 °C for 20 min. Then, 30 μM ATP was added to initiate the enzymatic reaction, and the plate was incubated at room temperature for 15 min. Finally, the ADP-Glo reagent was added to detect the luminescence intensity, where the luminescence intensity is directly proportional to the ADP content. Inhibition rate (%) = (1 - Sample signal)/DMSO signal × 100. Sample signal is the value of the compound group, and DMSO signal is the value of the DMSO control group. Then, the inhibition rate at each concentration was calculated, and the $IC_{50}$ was determined by fitting using the GraphPadPrism 7.0 software.

Table 2. $IC_{50}$ data for the inhibition of KIF18A enzymatic activity by the compounds of the present disclosure

| Compound No. | $IC_{50}$ (nM) |
|---|---|
| 3 | 19.8 |
| 6 | 95.6 |
| 8 | 21.3 |
| 10 | 25.7 |
| 11 | 15.2 |
| 12 | 10.2 |
| 13 | 187.2 |
| 14 | 28.7 |
| 15 | 126.4 |
| 16 | 11.3 |
| 17 | 16.4 |
| 18 | 93.2 |
| 19 | 26.8 |
| 20 | 113.0 |
| 21 | 10.7 |
| 22 | 26.4 |
| 23 | 3.6 |
| 24 | 3.7 |
| 25 | 20.0 |
| 26 | 49.7 |
| 27 | 23.1 |
| 28 | 33.4 |
| 29 | 63.4 |
| 30 | 103.2 |
| 31 | 55.3 |
| 32 | 117.2 |
| 33 | 110.9 |
| 34 | 44.3 |
| 37 | 11.3 |
| 38 | 7.6 |
| 39 | 32.2 |
| 40 | 62.9 |
| 51 | 10.0 |
| 52 | 22.0 |

(continued)

| Compound No. | $IC_{50}$ (nM) |
|---|---|
| 55 | 13.3 |
| 56 | 40.8 |
| 57 | 27.9 |
| 65 | 19.2 |
| 69 | 26.0 |
| 70 | 95.9 |
| 72 | 133.9 |
| 73 | 38.4 |

.

**Efficacy Experimental Example 3: mitotic index analysis**

HCT-116 and HT-29 cells at the logarithmic growth phase were taken, digested with a cell lysis buffer (trypsin), centrifuged, counted, and seeded into a 96-well plate at an appropriate cell density (20000 cells/well), with 100 μL per well. The surrounding wells were filled with an appropriate amount of PBS to prevent water evaporation. The next day, the cells were treated with the compounds at different concentrations (with a starting concentration of 10 μM, 3-fold diluted to obtain 9 concentration gradients). After 24 h, the medium was aspirated, and the cells were washed twice with 100 μL of PBS. The cells were then fixed with 4% paraformaldehyde and incubated at room temperature for 15 min. The fixative solution was discarded, and the cells were washed with PBS. Then, 150 μL of specialized blocking solution (containing 1% Triton-100) was added to each well for blocking at room temperature for 2 h, and the plate was washed with PBS to remove excess blocking solution. Anti-phistone H3 (pH3) was diluted at a ratio of 1:1000 with a blocking solution containing 0.033% Triton-100, and a blank control group was set up. Then, incubation was performed at 4 °C overnight. The next day, the plate was washed twice with 1× PBST. Anti-rabbit-IgG (H + L) (CST) and DRAQ5 (Thermofisher) were each diluted at an appropriate ratio with a blocking solution containing 0.033% Triton-100 (secondary antibody: 1:2000; DRAQ5: 1:10000). Then, incubation was performed at room temperature for 2 h. The plate was washed twice with 1× PBST and once with PBS. Fluorescence signals at 700 nm and 800 nm were detected using a Li-COR Odyssey two-color near-infrared laser imager. Calculation was performed as follows: pH3 expression (%) = (Cmpd dose/DMSO control) × 100. Cmpd dose is the value of the drug treatment group, and DMSO control is the value of the DMSO control group. The $EC_{50}$ values were determined by fitting using the GraphPad Prism 7.0 software.

Table 2. Detection of the effects of the compounds of the present disclosure on pH3 expression in HCT-116 and HT-29 cells $EC_{50}$

| Compound No. | HT-29 $EC_{50}$ (nM) | HCT-116 $EC_{50}$ (nM) |
|---|---|---|
| 3 | 495 | > 10000 |
| 6 | 175 | > 10000 |
| 8 | 6.4 | > 10000 |
| 10 | 183 | > 10000 |
| 11 | 139 | > 10000 |
| 12 | 51 | > 10000 |
| 13 | 235 | > 10000 |
| 14 | 22 | > 10000 |
| 15 | 82 | > 10000 |
| 16 | 94 | > 10000 |
| 17 | 57 | > 10000 |
| 18 | 87 | > 10000 |

(continued)

| Compound No. | HT-29 $EC_{50}$ (nM) | HCT-116 $EC_{50}$ (nM) |
|---|---|---|
| 19 | 34 | > 10000 |
| 20 | 434 | > 10000 |
| 21 | 6.0 | > 10000 |
| 22 | 8.9 | > 10000 |
| 23 | 9.8 | > 10000 |
| 24 | 65.6 | > 10000 |
| 25 | 9.6 | > 10000 |
| 26 | 12.3 | > 10000 |
| 27 | 34.6 | > 10000 |
| 28 | 77.8 | > 10000 |
| 29 | 108.2 | > 10000 |
| 30 | 47.6 | > 10000 |
| 31 | 64.0 | > 10000 |
| 32 | 61.8 | > 10000 |
| 33 | 58.5 | > 10000 |
| 34 | 14.5 | > 10000 |
| 35 | - | > 10000 |
| 36 | - | > 10000 |
| 37 | - | > 10000 |
| 38 | - | > 10000 |
| 39 | - | > 10000 |
| 40 | - | > 10000 |
| 42 | 254.7 | > 10000 |
| 43 | 30.0 | > 10000 |
| 44 | | > 10000 |
| 45 | 80.8 | > 10000 |
| 46 | 53.0 | > 10000 |
| 47 | 6.4 | > 10000 |
| 48 | 12.2 | > 10000 |
| 49 | 92.9 | > 10000 |
| 50 | 31.5 | > 10000 |
| 51 | 13.7 | > 10000 |
| 52 | 42.9 | > 10000 |
| 53 | 26.7 | > 10000 |
| 54 | 10.8 | > 10000 |
| 55 | 4.6 | > 10000 |
| 56 | 13.4 | > 10000 |
| 57 | 26.7 | > 10000 |
| 58 | 119.0 | > 10000 |

(continued)

| Compound No. | HT-29 $EC_{50}$ (nM) | HCT-116 $EC_{50}$ (nM) |
|---|---|---|
| 59 | 49.1 | > 10000 |
| 60 | 124.0 | > 10000 |
| 61 | 52.6 | > 10000 |
| 62 | 7.7 | > 10000 |
| 63 | 10.3 | > 10000 |
| 64 | 26.8 | > 10000 |
| 65 | 20.0 | > 10000 |
| 66 | 34.5 | > 10000 |
| 67 | 36.1 | > 10000 |
| 68 | 90.1 | > 10000 |
| 69 | 4.8 | > 10000 |
| 70 | 33.6 | > 10000 |
| 71 | 7.8 | > 10000 |
| 72 | 72.4 | > 10000 |
| 73 | 24.8 | > 10000 |

As can be seen from Table 2, the compounds of the present disclosure exhibited relatively good inhibitory effects on mitosis in HT-29 aneuploid cancer cells with chromosomal instability (CIN) characteristics, and some of the compounds had inhibitory effects of less than 100 nM, indicating good clinical application prospects; in contrast, the compounds showed no inhibitory effect on mitosis in HCT116 tumor cells without chromosomal instability (CIN) characteristics, demonstrating good selectivity.

**Efficacy Experimental Example 4: Stability Test in Liver Microsomes**

The phase I metabolic stability of the compounds in liver microsomes of CD-1 mice, Sprague-Dawley rats, beagle dogs, cynomolgus monkeys, and humans was assessed.

**Experimental system:**

The animal and human liver microsomes used in the test system were purchased from Xenotech, Corning, or other qualified suppliers and stored in a freezer at a temperature below -60 °C prior to use.

**Brief introduction of the experiment:**

The test samples and control compounds were separately incubated with animal and human liver microsomes at 37±1 °C for a period of incubation time up to 60 min. The samples were removed at the indicated time points, and the reaction was stopped with acetonitrile or another organic solvent containing an internal standard. After centrifugation, the resulting supernatants were assayed by the liquid chromatography-tandem mass spectrometry (LC-MS/MS) method.

**Experimental method:**

1. Preparation of buffer

73.21 g of dipotassium hydrogen phosphate trihydrate and 10.78 g of potassium dihydrogen phosphate were dissolved in 4000 mL of ultrapure water. The solution was adjusted to pH 7.40±0.10 with 10% phosphoric acid or 1 M potassium hydroxide to make a final concentration of 100 mM.

2. Preparation of working solution

The test sample powder was prepared into a stock solution at a certain concentration with DMSO or another organic solvent, which was then further diluted with a suitable organic solvent.

Control compounds testosterone, diclofenac, and propafenone were each prepared into a 10 mM stock solution with DMSO, which was then further diluted with a suitable organic solvent.

3. Preparation of liver microsome solution

Microsomes of each species were diluted into a 2× working solution with a 100 mM potassium phosphate buffer. The final concentration of the microsomes in the reaction system was 0.5 mg/mL.

4. Preparation of reduced nicotinamide adenine dinucleotide phosphate (NADPH) regeneration system

An appropriate amount of nicotinamide adenine dinucleotide phosphate (NADP) and isocitric acid (ISO) powder was weighed out, dissolved in a magnesium chloride solution, and shaken and mixed well. An appropriate amount of isocitrate dehydrogenase (IDH) was added, and the mixture was mixed well by gently turning upside down. The final concentrations in the reaction system were: 1 mM NADP, 1 mM magnesium chloride, 6 mM ISO, and 1 unit/mL IDH.

5. Preparation of stop solution

The stop solution was prepared with acetonitrile or another organic solvent containing an internal standard (tolbutamide or another suitable compound). The prepared stop solution was stored in a freezer at 2-8 °C.

6. Incubation process

Incubation was performed in a 96-well plate. Eight incubation plates were prepared and designated T0, T5, T15, T30, T45, T60, Blank60, and NCF60, respectively. The first 6 plates corresponded to reaction time points of 0, 5, 15, 30, 45, and 60 min, respectively. No test sample or control compound was added to the Blank60 plate, and samples were taken after 60 min of incubation. In the NCF60 plate, incubation was performed for 60 min using the potassium phosphate buffer instead of the NADPH regeneration system solution. Three replicates were made for samples in all conditions.

The microsomes and the test sample or control compound were mixed, and then the incubation plates Blank60, T5, T15, T30, T45, and T60 except for T0 and NCF60 were placed in a 37 °C water bath for pre-incubation for about 10 min. In the incubation plate T0, a stop solution was first added, followed by addition of the NADPH regeneration system working solution, and 98 μL of the potassium phosphate buffer was added to each sample well of the incubation plate NCF60 to initiate the reaction. After the pre-incubation of the incubation plates Blank60, T5, T15, T30, T45, and T60 was completed, 98 μL of the NADPH regeneration system working solution was added to each sample well to initiate the reaction. The reaction temperature was 37±1 °C, the final volume of the reaction was 200 μL, and the reaction system included 0.5 mg/mL microsomes, 1.0 μM substrate, 1 mM NADP, 6 mM ISO, and 1 unit/mL IDH.

At 5 min, 15 min, 30 min, 45 min, and 60 min, a cold stop solution containing an internal standard was added to the reaction plate to stop the reaction.

All the reaction plates after the reaction was stopped were shaken well and centrifuged at 3220× g at 4 °C for 20 min. After the supernatant was diluted at a certain ratio, LC-MS/MS analysis was performed.

**Sample analysis**

Sample analysis was performed by the liquid chromatography-tandem mass spectrometry (LC-MS/MS) method without standard curves and quality control samples. Semi-quantitative determination was performed according to the ratio of the analyte peak area to the internal standard peak area. The retention times of the analyte and internal standard, the chromatogram acquisition, and chromatogram integration were processed using the Analyst software (Sciex, Framingham, Massachusetts, USA).

The CV for the internal standard peak area in each matrix in each analysis batch should be within 20%.

**Data analysis**

The *in vitro* elimination rate constant ke of the compound was obtained by converting the ratio of the peak area of the compound to the internal standard peak area in the following formula into the remaining rate:

$$\text{Remaining rate (\%)} = \frac{\text{the peak area ratio of the compound to the internal standard at any time point}}{\text{the peak area ratio of the compound to the internal standard at 0 min}} \times 100$$

$$C_t = C_0 \bullet e^{-k_e \bullet t}$$

*when*

$$C_t = \frac{1}{2} C_0 \ ,$$

i. $$T_{1/2} = \frac{Ln\ 2}{k_e} = \frac{0.693}{k_e}$$

$CL_{int\ (mic)}$ = 0.693/$T_{1/2}$/microsome protein content (microsome concentration during incubation in mg/mL)
$CL_{int\ (liver)}$ = $CL_{int\ (mic)}$ × microsome protein amount in liver (mg/g) × liver-to-body weight ratio

According to the well stir model, the hepatic intrinsic clearance and hepatic clearance can be converted through the following formula.

$$CL_{(liver)} = (CL_{int(liver)} \times Q_h)/(CL_{int(liver)} + Q_h)$$

Parameters in the formula are shown in Table 4.

| Species | Liver-to-body weight ratio (g/kg body weight) [1-2] | Liver blood flow volume ($Q_h$) (mL/min/kg)[1-2] | Microsome protein amount (mg/g liver weight) |
|---|---|---|---|
| Mouse | 88 | 90.0 | |
| Rat | 40 | 55.2 | |
| Dog | 32 | 30.9 | 45 |
| Monkey | 30 | 43.6 | |
| Human | 20 | 20.7 | |

[1] Brian Davies and Tim Morris, Physiological Parameters in Laboratory Animals and Human. Pharmaceutical Research, Vol. 10 No.7, 1993;
[2] Journal of Pharmacology and Experimental Therapeutics, 1997, 283(1): 46-58.

Table 5. Liver microsome stability test results for some of the compounds in the present disclosure

| Compound No. | HLM 0.5 | | RLM 0.5 | | MLM 0.5 | |
|---|---|---|---|---|---|---|
| | T1/2 (min) | CLint(liver) (mL/min/kg) | T1/2 (min) | CLint(liver) (mL/min/kg) | T1/2 (min) | CLint(liver) (mL/min/kg) |
| 6 | >145 | <8.6 | >145 | <17.3 | >145 | <38.0 |
| 8 | >145 | <8.6 | >145 | <17.3 | 25.1 | 218.8 |
| 10 | >145 | <8.6 | 129.4 | 19.3 | 30.1 | 182.3 |
| 14 | 56 | 22.3 | 72.8 | 34.3 | 101.5 | 54.1 |
| 15 | >145 | <8.6 | 144.9 | 17.2 | 108.5 | 50.6 |

(continued)

| Compound No. | HLM 0.5 | | RLM 0.5 | | MLM 0.5 | |
|---|---|---|---|---|---|---|
| | T1/2 (min) | CLint(liver) (mL/min/kg) | T1/2 (min) | CLint(liver) (mL/min/kg) | T1/2 (min) | CLint(liver) (mL/min/kg) |
| 19 | 107.6 | 11.6 | 91.5 | 27.3 | 105 | 52.3 |
| 21 | >145 | <8.6 | >145 | <17.3 | >145 | <38.0 |
| 23 | >145 | <8.6 | >145 | <17.3 | >145 | <38.0 |
| 24 | 133.2 | 9.4 | 84.6 | 29.5 | 84.8 | 64.7 |
| 25 | >145 | <8.6 | >145 | <17.3 | >145 | <38.0 |
| 34 | 920.5 | 1.9 | 559.6 | 4.4 | 380.6 | 14.3 |
| 47 | 121.3 | 14.3 | 275.5 | 9.0 | 2419.6 | 2.3 |

It can be seen that the compounds of the present application had relatively good stability in human, rat and mouse liver microsomes.

**Efficacy Experimental Example 5:** Pharmacokinetic Test in Mice

In this example, the pharmacokinetic behavior of the compounds after intravenous injection (IV) and intragastric (PO) administration in BALB/c mice was tested.

On the day of administration, mice were weighed for actual body weight and the administration volume was calculated. Each compound was tested in two groups with 9 mice in each group; one group was subjected to administration by single intravenous injection, and the other group of mice was subjected to single intragastric administration. Whole blood samples were collected via the orbit at prescribed time points (0.25 h, 0.5 h, 1 h, 2 h, 4 h, 8 h, and 24 h after administration). After blood sample collection, the samples were immediately transferred to labeled commercial sample tubes containing K2-EDTA (0.85-1.15 mg), followed by centrifugation (3200× g, 4 °C, 10 min) and plasma collection. The plasma was transferred to a pre-cooled centrifuge tube, frozen in dry ice, and then stored in an ultra-low temperature freezer at -60 °C or lower until the LC-MS/MS analysis.

Plasma concentrations were determined by the LC-MS/MS method. Plasma drug concentration data for the compounds were processed using a non-compartmental model in the WinNonlin Version 6.3 (Pharsight, Mountain View, CA) pharmacokinetic software. The relevant pharmacokinetic parameters were calculated by a linear-log trapezoidal method.

Table 6. Pharmacokinetic parameters of some of the compounds of the present disclosure in mice

| Compound No. | **AUC (nM*h)** | **t1/2 (h)** | **Cmax (nM)** | **F (%)** | **PO/Dose** |
|---|---|---|---|---|---|
| 3 | 74846 | 44.5 | 4247 | 87 | @5mpk |
| 6 | 13505 | 4.7 | 1550 | 44 | @5mpk |
| 8 | 6872 | 1.2 | 1607 | 97 | @5mpk |
| 15 | 17788 | 3.4 | 1767 | 79 | @5mpk |
| 17 | 13269 | 3.3 | 2690 | 116 | @5mpk |
| 19 | 58302 | 14.9 | 3922 | 101 | @5mpk |
| | 319667 | 16.5 | 15333 | 92 | @30mpk |
| 21 | 60899 | 4 | 4172 | 107 | @5mpk |
| | 207169 | 5.4 | 15338 | 61 | @30mpk |
| 22 | 79792 | 10 | 14642 | 218 | @30mpk |
| 25 | 16331 | 2.8 | 1731 | 135 | @5mpk |
| 26 | 537225 | 19.5 | 28440 | 136 | @30mpk |
| 33 | 126422 | 134 | 6311 | 108 | @30mpk |

(continued)

| Compound No. | AUC (nM*h) | t1/2 (h) | Cmax (nM) | F (%) | PO/Dose |
|---|---|---|---|---|---|
| 34 | 70061 | 3.7 | 6387 | 257 | @5mpk |
| | 219598 | 9.6 | 15176 | 147 | @30mpk |
| 51 | 272210 | 62.3 | 14220 | 67 | @30mpk |
| 52 | 269031 | 39 | 14079 | 107 | @30mpk |
| 53 | 78305 | 13 | 4556 | 16 | @30mpk |
| 55 | 379581 | 7.8 | 25756 | 109 | @30mpk |
| 56 | 436122 | 80.8 | 23603 | 142 | @30mpk |
| 57 | 153690 | 145.7 | 6981 | 44 | @30mpk |
| 65 | 19945 | 3.9 | 3562 | 82 | @5mpk |
| 69 | 220526 | 8.2 | 20481 | 385 | @30mpk |
| 72 | 66328 | 6.0 | 5336 | 175 | @5mpk |
| 73 | 69129 | 3.8 | 4657 | 72 | @30mpk |

It can be seen that the compounds of the present application exhibited relatively good pharmacokinetic performance in mice.

**Efficacy Experimental Example 6:** Pharmacokinetic Test in Rats

In this example, the pharmacokinetic behavior of the compounds after intravenous injection (IV) and intragastric (PO) administration in SD rats was tested.

On the day of administration, rats were weighed for actual body weight and the administration volume was calculated. Each compound was tested in two groups with 3 rats in each group; one group was subjected to administration by single intravenous injection, and the other group was subjected to single intragastric administration. Whole blood samples were collected via the jugular vein at prescribed time points (0.25 h, 0.5 h, 1 h, 2 h, 4 h, 8 h, and 24 h after administration). After blood sample collection, the samples were immediately transferred to labeled commercial sample tubes containing K2-EDTA (0.85-1.15 mg), followed by centrifugation (3200× g, 4 °C, 10 min) and plasma collection. The plasma was transferred to a pre-cooled centrifuge tube, frozen in dry ice, and then stored in an ultra-low temperature freezer at -60 °C or lower until the LC-MS/MS analysis.

Plasma concentrations were determined by the LC-MS/MS method. Plasma drug concentration data for the compounds were processed using a non-compartmental model in the WinNonlin Version 6.3 (Pharsight, Mountain View, CA) pharmacokinetic software. The relevant pharmacokinetic parameters were calculated by a linear-log trapezoidal method.

Table 7. Pharmacokinetic parameters of some of the compounds of the present disclosure in rats

| Compound No. | AUC (nM*h) | t1/2 (h) | Cmax (nM) | F (%) | PO/Dose |
|---|---|---|---|---|---|
| 3 | 16184 | 7 | 1334 | 57 | @5mpk |
| 6 | 8917 | 55.8 / | 509 / | 23 | @5mpk |
| 8 | 1564 | 5.9 | 303 | 16 | @5mpk |
| 11 | 604 | 2.4 | 217 | 25 | @5mpk |
| 12 | 2409 | 2.6 | 526 | 18.3 | @5mpk |
| 14 | 1915 | 6.4 | 351 | 25 | @5mpk |
| 15 | 9665 | 22.8 | 540 | 27 | @5mpk |
| 17 | 3201 | 4.9 | 582 | 39 | @5mpk |
| 19 | 20505 | 12.2 | 1244 | 63 | @5mpk |
| 21 | 33903 | 10.6 | 2086 | 24.7 | @5mpk |

(continued)

| Compound No. | AUC (nM*h) | t1/2 (h) | Cmax (nM) | F (%) | PO/Dose |
|---|---|---|---|---|---|
| 26 | 5787 | 8.1 | 390 | 54 | @5mpk |
| 34 | 62486 | 66.3 | 3428.1 | 25.7 | @30mpk |

It can be seen that the compounds of the present application exhibited relatively good pharmacokinetic performance in rats.

**Efficacy Experimental Example 7: hERG**

HEK293 cells were cultured in a DMEM medium containing 10% fetal bovine serum and 0.8 mg/mL G418 at 37 °C with 5% $CO_2$. The cells were digested by TrypLE™ Express and then centrifuged. The cell density was adjusted to $2 \times 10^6$ cells/mL. The cells were then gently mixed for 15-20 min using a shaker equilibrated at room temperature, and subjected to patch clamping on a machine. The medium of the prepared cells was replaced with extracellular fluid. The intracellular fluid and the extracellular fluid were taken from the fluid pool, and added to the intracellular fluid pool and the cell and test substance pool of the QPlate chip, respectively. The voltage stimulation of the whole-cell hERG potassium current was recorded by the whole-cell patch clamp, and the experimental data was collected and stored by Qpatch. The test compound was 3-fold diluted starting from 30 μM to obtain 6 concentration points, each for two administrations over a period of at least 5 min. The current detected in compound-free extracellular fluid for each cell served as its own control group, and the detection was repeated at least twice independently using two cells per concentration. All electrophysiological experiments were performed at room temperature.

For data analysis, the current after the action of each drug concentration and the current of the blank control were standardized (

$$\frac{\text{Peak tail current}_{\text{compound}}}{\text{Peak tail current}_{\text{vehicle}}}$$

), and then the inhibition rate corresponding to each drug concentration was calculated (

$$1 - \frac{\text{Peak tail current}_{\text{compound}}}{\text{Peak tail current}_{\text{vehicle}}}$$

).The mean and standard error were calculated for each concentration, and the half maximal inhibitory concentration of each compound was calculated:

$$Y = \text{Bottom} + \frac{\text{Top} - \text{Bottom}}{1 + 10\text{^}((\text{LogIC}_{50} - C) \times \text{HillSlope})}$$

The dose-dependent effect was subjected to non-linear fitting using the above equation, where Y represents the inhibition rate, C represents the concentration of the test substance, $IC_{50}$ is the half maximal inhibitory concentration, and HillSlope represents the Hill coefficient. Curve fitting and calculation of $IC_{50}$ were performed using the Graphpad software.

Table 8. Effects of some of the compounds of the present disclosure on the hERG potassium channel

| Compound No. | hERG $IC_{50}$(μM) |
|---|---|
| 6 | >30 |
| 8 | >30 |
| 19 | >30 |
| 21 | >30 |

It can be seen that the compounds of the present application have a low risk of cardiotoxicity.

**Efficacy Experimental Example 8: Inhibition of Cytochrome Oxidase P450**

1) Preparation of buffer:

100 mM K-Buffer: 9.5 mL of stock solution A was mixed with 40.5 mL of stock solution B, the total volume was adjusted to 500 mL with ultrapure water, and the buffer was titrated to pH 7.4 with KOH or $H_3PO_4$.

Starting material A (1 M potassium dihydrogen phosphate): 136.5 g of potassium dihydrogen phosphate in 1 L of water.

Stock B (1 M potassium dihydrogen phosphate): 174.2 g of potassium dihydrogen phosphate in 1 L of water.

2) Preparation of test substance

The test compound powder was prepared into a stock solution at a certain concentration with DMSO or another organic solvent, which was then further diluted with a proper organic solvent.

3) *In vitro* incubation

The liver microsomal *in vitro* incubation system for the CYP450 enzyme metabolism phenotype study was a biochemical reaction of prepared liver microsomes supplemented with a redox coenzyme and an enzyme-specific selective inhibitor under simulated physiological temperature and environment conditions.

4) Detection of parent drug or metabolite

The concentration of the parent drug or a metabolite thereof in the incubation solution was determined by LC-MS/MS.

Table 9. Inhibitory effects of some of the compounds of the present disclosure on cytochrome oxidase P450

| Compound No. | 1A2/2C9/2C19/2D6/3A4 (Midazolam) $IC_{50}$($\mu$M) |
|---|---|
| 6 | >10/5.28/>10/>10/>10 |
| 8 | >10/2.66/>10/>10/>10 |
| 19 | >10/9.38/>10/>10/>10 |
| 21 | >10/>10/>10/>10/>10 |
| 34 | >10/4.46/>10/>10/>10 |
| 47 | >10/0.18/0.98/2.24/0.24 |

It can be seen that the compounds of the present application have a low risk of drug-drug interactions.

**Efficacy Experimental Example 9: Pharmacodynamic Experiment in Mice**

Human colon cancer HT-29 cells were cultured in monolayer *in vitro* under the following conditions: DMEM medium containing 10% fetal bovine serum and 1% penicillin and streptomycin, at 37 °C with 5% CO2. The cells were digested with trypsin three times a week for passaging. When the saturation degree of the cells was 80%-90%, the cells were collected, counted, and inoculated. 0.1 mL ($1 \times 10^6$ cells) of HT-29 cells were subcutaneously inoculated on the right back of each mouse. On day 11 after inoculation of the cells, the mean tumor volume reached 325 $mm^3$. The mice were randomly grouped for administration (the animals were divided into an administration group and a control group, with 6 animals in each group). The day of grouping was defined as d0, and intragastric administration was started on d1. The administration was performed twice daily at an administration volume of 10 mL/kg. Vehicle control was administered to the control group. The tumor volume was measured 2-3 times per week, and meanwhile, the mice were weighed and the data were recorded; daily observations were made to record the body surface signs of the mice. After the experiment was completed, the tumors were removed, weighed, and photographed for recording.

Calculation formula for tumor volume:

Tumor volume ($mm^3$) = $1/2 \times (a \times b^2)$ (note: a represents long diameter and b represents short diameter)

Tumor growth inhibition rate (TGI%) = (1 - tumor weight in treatment group/tumor weight in control group) × 100%

Table 9. TGI values of compounds after 16 days of administration

| Compound No. | TGI |
|---|---|
| Compound 19 | 20mpk BID TGI = 111.82% |
| Compound 34 | 20mpk BID TGI = 105.10% |
| Compound 47 | 20mpk BID TGI = 108.84% |

Although specific embodiments of the present disclosure have been described above, it will be appreciated by those skilled in the art that these embodiments are merely illustrative and that many changes or modifications can be made to these embodiments without departing from the principles and spirit of the present disclosure. The protection scope of the present disclosure is therefore defined by the appended claims.

## Claims

**1.** A nitrogen-containing compound represented by formula I, a pharmaceutically acceptable salt thereof, a stereoisomer thereof, a tautomer thereof, a metabolite thereof, a prodrug thereof, an isotopically labeled derivative thereof, a solvate thereof, or a solvate of the pharmaceutically acceptable salt thereof:

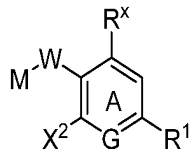

I

wherein $R^x$ is 3- to 6-membered monocyclic cycloalkyl, 5- to 6-membered monocyclic heterocycloalkyl, 6-to 9-membered bridged heterocycloalkyl, 7- to 9-membered spiro heterocycloalkyl, 5- to 6-membered monocyclic heterocycloalkyl substituted with one or more $R^{x-1}$, 6- to 9-membered bridged heterocycloalkyl substituted with one or more $R^{x-2}$, 7- to 9-membered spiro heterocycloalkyl substituted with one or more $R^{x-3}$, or 3- to 6-membered monocyclic cycloalkyl substituted with one or more $R^{x-4}$; in the "5- to 6-membered monocyclic heterocycloalkyl", the "6- to 9-membered bridged heterocycloalkyl", the "7- to 9-membered spiro heterocycloalkyl", the "5- to 6-membered monocyclic heterocycloalkyl substituted with one or more $R^{x-1}$", the "6- to 9-membered bridged heterocycloalkyl substituted with one or more $R^{x-2}$", and the "7- to 9-membered spiro heterocycloalkyl substituted with one or more $R^{x-3}$", the heteroatom(s) independently consist(s) of 1 N atom and 0, 1, or 2 X atoms, the X atom being independently selected from 1 or 2 of N, O, and S; the "5- to 6-membered monocyclic heterocycloalkyl", the "6- to 9-membered bridged heterocycloalkyl", the "7- to 9-membered spiro heterocycloalkyl", the "5- to 6-membered monocyclic heterocycloalkyl substituted with one or more $R^{x-1}$", the "6- to 9-membered bridged heterocycloalkyl substituted with one or more $R^{x-2}$", and the "7- to 9-membered spiro heterocycloalkyl substituted with one or more $R^{x-3}$" are connected to ring A through the N atom;

each of $R^{x-1}$, $R^{x-2}$, $R^{x-3}$, and $R^{x-4}$ is independently halogen, $R^{N-10k}$, $R^{N-10l}$, $C_{1-6}$ alkyl, $C_{1-6}$ alkyl substituted with one or more halogens, $-OR^{N-a}$, $C_{1-6}$ alkoxy substituted with one or more halogens, CN, $-NR^{N-a}R^{N-a}$, or oxo; $R^1$ is $-L-R^{1-1}$;

L is $*-NHSO_2-$, -NH-, *-NHC(O)-, -NHC(O)NH-, $*-SO_2NH-$, or $-SO_2-$; * represents the end connected to ring A;

$R^{1-1}$ is $C_{1-6}$ alkyl, 3- to 6-membered monocyclic cycloalkyl, 4- to 8-membered monocyclic heterocycloalkyl, $C_{1-6}$ alkyl substituted with one or more $R^a$, or 3- to 6-membered monocyclic cycloalkyl substituted with one or more $R^b$; in the 4- to 8-membered monocyclic heterocycloalkyl, the heteroatom is independently selected from 1, 2, or 3 of N, O, and S, and the number of the heteroatom is independently 1, 2, 3, or 4;

each $R^a$ is independently hydroxyl, halogen, or $-NH(R^{a-1})$;

$R^{a-1}$ is $C_{1-6}$ alkyl;

each $R^b$ is independently $C_{1-6}$ alkyl or $C_{1-6}$ alkyl substituted with one or more hydroxyl;

G is $C(R^{10})$ or N; $R^{10}$ is H, halogen, $C_{1-6}$ alkyl, $C_{1-6}$ alkyl substituted with one or more halogens, -OH, $-OR^{N-8a}$, or

$-O-R^{N-8b}$;

M is phenyl, naphthyl, 5-membered heteroaryl, 6-membered heteroaryl, 9- to 10-membered heteroaryl, 6-membered heterocycloalkenyl, 6-membered heteroaryl substituted with one or more $R^2$, 9- to 10-membered heteroaryl substituted with one or more $R^3$, 6-membered heterocycloalkenyl substituted with one or more $R^4$, phenyl substituted with one or more $R^5$, naphthyl substituted with one or more $R^5$, or 5-membered heteroaryl substituted with one or more $R^6$; in the "5-membered heteroaryl", "6-membered heteroaryl", "9-to 10-membered heteroaryl", "6-membered heterocycloalkenyl", "6-membered heteroaryl substituted with one or more $R^2$", "9- to 10-membered heteroaryl substituted with one or more $R^3$", "6-membered heterocycloalkenyl substituted with one or more $R^4$", and "5-membered heteroaryl substituted with one or more $R^6$", the heteroatom is independently selected from 1, 2, or 3 of N, O, and S, and the number of the heteroatom is independently 1, 2, 3, or 4;

each of $R^2$, $R^3$, $R^4$, $R^5$, and $R^6$ is independently oxo, halogen, $R^{N-4a}$, $R^{N-4b}$, $C_{1-6}$ alkyl, $C_{1-6}$ alkyl substituted with one or more halogens, cyano, $C_{1-6}$ alkoxy, $-O-R^{N-6a}$, or $-Y-R^{N-13}$;

$R^{N-6a}$ is a saturated or partially saturated 3-, 4-, 5-, or 6-membered monocyclic ring comprising 0, 1, 2, or 3 N atoms and 0, 1, or 2 atoms selected from O and S;

Y is $-C_{1-6}$ alkyl-, $-N(C_{1-6}$ alkyl$)-C_{1-6}$ alkyl-, $-C(=O)NR^{N-a}R^{N-a}(C_{1-6}$ alkyl), -O-, $-O-C_{1-6}$ alkyl-, S, S=O, $S(=O)_2$, $-S(=O)(=N-R^{N-13})-$, or -S(=O)(=NH)-;

RN-13 is $R^{N-13a}$ or $R^{N-13b}$;

each of $R^{N-4a}$, $R^{N-8a}$, $R^{N-10k}$, and $R^{N-13a}$ is independently selected from the group consisting of: a saturated, partially saturated, or unsaturated 3-, 4-, 5-, 6-, or 7-membered monocyclic ring or 4-, 5-, 6-, 7-, 8-, 9-, 10-, 11-, or 12-membered bicyclic ring comprising 0, 1, 2, or 3 N atoms and 0, 1, or 2 atoms selected from O and S, wherein the monocyclic or bicyclic ring is substituted with 0, 1, 2, or 3 groups selected from: halogen, $C_{1-6}$ alkyl, $C_{1-6}$ alkyl substituted with one or more halogens, $-OR^{N-a}$, $C_{1-6}$ alkoxy substituted with one or more halogens, CN, $-C(=O)R^{N-b}$, $-C(=O)OR^{N-a}$, $-C(=O)NR^{N-a}R^{N-a}$, $-C(=NR^{N-a})NR^{N-a}R^{N-a}$, $-OC(=O)R^{N-b}$, $-OC(=O)NR^{N-a}R^{N-a}$, $-OC_{1-6}$ alkyl $NR^{N-a}R^{N-a}$, $-OC_{1-6}$ alkyl $OR^{N-a}$, $-SR^{N-a}$, $-S(=O)R^{N-b}$, $-S(=O)_2R^{N-b}$, $-S(=O)_2NR^{N-a}R^{N-a}$, $-NR^{N-a}R^{N-a}$, $-N(R^{N-a})C(=O)R^{N-b}$, $-N(R^{N-a})C(=O)OR^{N-b}$, $-N(R^{N-a})C(=O)NR^{N-a}R^{N-a}$, $-N(R^{N-a})C(=NR^{N-a})NR^{N-a}R^{N-a}$, $-N(R^{N-a})S(=O)_2R^{N-b}$, $-N(R^{N-a})S(=O)_2NR^{N-a}R^{N-a}$, $-NR^{N-a}C_{1-6}$ alkyl $NR^{N-a}R^{N-a}$, $-NR^{N-a}C_{1-6}$ alkyl $OR^{N-a}$, $-C_{1-6}$ alkyl $NR^{N-a}R^{N-a}$, $-C_{1-6}$ alkyl $OR^{N-a}$, $-C_{1-6}$ alkyl $N(R^{N-a})C(=O)R^{N-b}$, $-C_{1-6}$ alkyl $OC(=O)R^{N-b}$, $-C_{1-6}$ alkyl $C(=O)NR^{N-a}R^{N-a}$, $-C_{1-6}$ alkyl $C(=O)OR^{N-a}$, $R^{N-14}$, and oxo;

each of $R^{N-4b}$, $R^{N-8b}$, $R^{N-10l}$, and $R^{N-13b}$ is independently $C_{1-6}$ alkyl substituted with 0, 1, 2, 3, 4, or 5 groups selected from halogen, $-OR^{N-a}$, $C_{1-6}$ alkoxy substituted with one or more halogens, and CN;

each $R^{N-14}$ is independently selected from the group consisting of: a saturated, partially saturated, or unsaturated 3-, 4-, 5-, 6-, or 7-membered monocyclic ring or 4-, 5-, 6-, 7-, 8-, 9-, 10-, 11-, or 12-membered bicyclic ring comprising 0, 1, 2, or 3 N atoms and 0 or 1 atom selected from O and S, wherein the monocyclic or bicyclic ring is substituted with 0, 1, 2, or 3 groups selected from: halogen, $C_{1-6}$ alkyl, $C_{1-6}$ alkyl substituted with one or more halogens, $-OR^{N-a}$, $C_{1-6}$ alkoxy substituted with one or more halogens, CN, $-C(=O)R^{N-b}$, $-C(=O)OR^{N-a}$, $-C(=O)NR^{N-a}R^{N-a}$, $-C(=NR^{N-a})NR^{N-a}R^{N-a}$, $-OC(=O)R^{N-b}$, $-OC(=O)NR^{N-a}R^{N-a}$, $-OC_{1-6}$ alkyl $NR^{N-a}R^{N-a}$, $-OC_{1-6}$ alkyl $OR^{N-a}$, $-SR^{N-a}$, $-S(=O)R^{N-b}$, $-S(=O)_2R^{N-b}$, $-S(=O)_2NR^{N-a}R^{N-a}$, $-NR^{N-a}R^{N-a}$, $-N(R^{N-a})C(=O)R^{N-b}$, $-N(R^{N-a})C(=O)OR^{N-b}$, $-N(R^{N-a})C(=O)NR^{N-a}R^{N-a}$, $-N(R^{N-a})C(=NR^{N-a})NR^{N-a}R^{N-a}$, $-N(R^{N-a})S(=O)2R^{N-b}$, $-N(R^{N-a})S(=O)_2NR^{N-a}R^{N-a}$, $-NR^{N-a}C_{1-6}$ alkyl $NR^{N-a}R^{N-a}$, $-NR^{N-a}C_{1-6}$ alkyl $OR^{N-a}$, $-C_{1-6}$ alkyl $NR^{N-a}R^{N-a}$, $-C_{1-6}$ alkyl $OR^{N-a}$, $-C_{1-6}$ alkyl $N(R^{N-a})C(=O)R^{N-b}$, $-C_{1-6}$ alkyl $OC(=O)R^{N-b}$, $-C_{1-6}$ alkyl $C(=O)NR^{N-a}R^{N-a}$, $-C_{1-6}$ alkyl $C(=O)OR^{N-a}$, and oxo;

each $R^{N-a}$ is independently H or $R^{N-b}$;

each $R^{N-b}$ is independently $C_{1-6}$ alkyl, phenyl, or benzyl, wherein the $C_{1-6}$ alkyl is substituted with 0, 1, 2, or 3 substituents selected from: halogen, -OH, $-OC_{1-6}$ alkyl, $-NH_2$, $-NHC_{1-6}$ alkyl, $-OC(=O)C_{1-6}$ alkyl, or $-N(C_{1-6}$ alkyl$)C_{1-6}$ alkyl; and the phenyl or benzyl is substituted with 0, 1, 2, or 3 substituents selected from: halogen, $C_{1-6}$ alkyl, $C_{1-6}$ alkyl substituted with one or more halogens, -OH, $-OC_{1-6}$ alkyl, $-NH_2$, $-NHC_{1-6}$ alkyl, $-OC(=O)C_{1-6}$ alkyl, or $-N(C_{1-6}$ alkyl$)C_{1-6}$ alkyl;

W and $X^2$ are as defined in any one of the following schemes:

scheme 1: W is $*-CONR^W-$; $X^2$ is $R^{W-1}$; * represents the end connected to ring A;

scheme 2: W is $*-NR^WCO-$; $X^2$ is $R^{W-1}$; * represents the end connected to ring A; and

scheme 3: W is $*-C(=X^1)NR^W-$; $X^1$, $X^2$, and the C atoms therebetween together form 6-membered heteroaryl; in the 6-membered heteroaryl, the heteroatom is selected from 1, 2, or 3 of N, O, and S, and the number of the heteroatom is 1, 2, or 3; * represents the end connected to ring A;

$R^W$ is H, $C_{1-6}$ alkyl, or $C_{1-6}$ alkyl substituted with one or more halogens;

$R^{W-1}$ is H, halogen, $C_{1-6}$ alkyl, or $C_{1-6}$ alkyl substituted with one or more halogens; and

the compound represented by formula I satisfies one, two, or three of the following conditions:

condition 1: $R^x$ is 6- to 9-membered bridged heterocycloalkyl or 6- to 9-membered bridged heterocycloalkyl substituted with one or more $R^{x-2}$;

condition 2: M is naphthyl, 5-membered heteroaryl, 9- to 10-membered heteroaryl, 6-membered heterocycloalkenyl, naphthyl substituted with one or more $R^5$, 5-membered heteroaryl substituted with one or more $R^6$, 9- to 10-membered heteroaryl substituted with one or more $R^3$, or 6-membered heterocycloalkenyl substituted with one or more $R^4$; and

condition 3: W is *-C(=$X^1$)$NR^W$-; $X^1$, $X^2$, and the C atoms therebetween together form 6-membered heteroaryl.

**2.** A nitrogen-containing compound represented by formula II, a pharmaceutically acceptable salt thereof, a stereoisomer thereof, a tautomer thereof, a metabolite thereof, a prodrug thereof, an isotopically labeled derivative thereof, a solvate thereof, or a solvate of the pharmaceutically acceptable salt thereof:

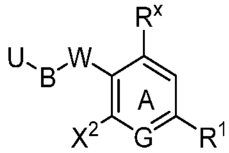

II

wherein $R^x$ is 3- to 6-membered monocyclic cycloalkyl, 5- to 6-membered monocyclic heterocycloalkyl, 6-to 9-membered bridged heterocycloalkyl, 7- to 9-membered spiro heterocycloalkyl, 5- to 6-membered monocyclic heterocycloalkyl substituted with one or more $R^{x-1}$, 6- to 9-membered bridged heterocycloalkyl substituted with one or more $R^{x-2}$, 7- to 9-membered spiro heterocycloalkyl substituted with one or more $R^{x-3}$, or 3- to 6-membered monocyclic cycloalkyl substituted with one or more $R^{x-4}$; in the "5- to 6-membered monocyclic heterocycloalkyl", the "6- to 9-membered bridged heterocycloalkyl", the "7- to 9-membered spiro heterocycloalkyl", the "5- to 6-membered monocyclic heterocycloalkyl substituted with one or more $R^{x-1}$", the "6- to 9-membered bridged heterocycloalkyl substituted with one or more $R^{x-2}$", and the "7- to 9-membered spiro heterocycloalkyl substituted with one or more $R^{x-3}$", the heteroatom(s) independently consist(s) of 1 N atom and 0, 1, or 2 X atoms, the X atom being independently selected from 1 or 2 of N, O, and S; the "5- to 6-membered monocyclic heterocycloalkyl", the "6- to 9-membered bridged heterocycloalkyl", the "7- to 9-membered spiro heterocycloalkyl", the "5- to 6-membered monocyclic heterocycloalkyl substituted with one or more $R^{x-1}$", the "6- to 9-membered bridged heterocycloalkyl substituted with one or more $R^{x-2}$", and the "7- to 9-membered spiro heterocycloalkyl substituted with one or more $R^{x-3}$" are connected to ring A through the N atom;

each of $R^{x-1}$, $R^{x-2}$, $R^{x-3}$, and $R^{x-4}$ is independently halogen, $C_{1-6}$ alkyl, $C_{1-6}$ alkoxy, $C_{1-6}$ alkyl substituted with one or more halogens, or $C_{1-6}$ alkoxy substituted with one or more halogens;

$R^1$ is -L-$R^{1-1}$;

L is *-$NHSO_2$-, -NH-, *-NHC(O)-, -NHC(O)NH-, *-$SO_2$NH-, or -$SO_2$-; * represents the end connected to ring A;

$R^{1-1}$ is $C_{1-6}$ alkyl, 3- to 6-membered monocyclic cycloalkyl, 4- to 8-membered monocyclic heterocycloalkyl, $C_{1-6}$ alkyl substituted with one or more $R^a$, or 3- to 6-membered monocyclic cycloalkyl substituted with one or more $R^b$; in the 4- to 8-membered monocyclic heterocycloalkyl, the heteroatom is independently selected from 1, 2, or 3 of N, O, and S, and the number of the heteroatom is independently 1, 2, 3, or 4;

each $R^a$ is independently hydroxyl, halogen, or -NH($R^{a-1}$);

$R^{a-1}$ is $C_{1-6}$ alkyl;

each $R^b$ is independently $C_{1-6}$ alkyl or $C_{1-6}$ alkyl substituted with one or more hydroxyl;

B is phenyl, naphthyl, 5-membered heteroaryl, 6-membered heteroaryl, 9- to 10-membered heteroaryl, 6-membered heterocycloalkenyl, 6-membered heteroaryl substituted with one or more $R^2$, 9- to 10-membered heteroaryl substituted with one or more $R^3$, 6-membered heterocycloalkenyl substituted with one or more $R^4$, phenyl substituted with one or more $R^5$, naphthyl substituted with one or more $R^5$, or 5-membered heteroaryl substituted with one or more $R^6$; in the "5-membered heteroaryl", "6-membered heteroaryl", "9-to 10-membered heteroaryl", "6-membered heterocycloalkenyl", "6-membered heteroaryl substituted with one or more $R^2$", "9- to 10-membered heteroaryl substituted with one or more $R^3$", "6-membered heterocycloalkenyl substituted with one or more $R^4$", and "5-membered heteroaryl substituted with one or more $R^6$", the heteroatom is independently selected from 1, 2, or 3 of N, O, and S, and the number of the heteroatom is independently 1, 2, 3, or 4;

each of $R^2$, $R^3$, $R^4$, $R^5$, and $R^6$ is independently halogen, cyano, oxo (=O), $C_{1-6}$ alkyl, or $C_{1-6}$ alkyl substituted with one or more halogens;

U is 3- to 10-membered cycloalkyl, 4- to 10-membered heterocycloalkyl, 6- to 10-membered aryl, 5- to 10-membered heteroaryl, 3- to 10-membered cycloalkyl substituted with one or more $R^{2-1}$, 4- to 10-membered heterocycloalkyl substituted with one or more $R^{2-2}$, or 5- to 10-membered heteroaryl substituted with one or more

$R^{2-3}$; in the "5- to 10-membered heteroaryl", "4- to 10-membered heterocycloalkyl", "4- to 10-membered heterocycloalkyl substituted with one or more $R^{2-2}$", and "5- to 10-membered heteroaryl substituted with one or more $R^{2-3}$", the heteroatom is independently selected from 1, 2, or 3 of N, O, and S, and the number of the heteroatom is independently 1, 2, 3, or 4;
each of $R^{2-1}$, $R^{2-2}$, and $R^{2-3}$ is independently halogen, $C_{1-6}$ alkyl, oxo, CN, hydroxyl, 3- to 6-membered monocyclic cycloalkyl, $C_{1-6}$ alkyl substituted with one or more halogens, $C_{1-6}$ alkoxy substituted with one or more halogens, 3- to 6-membered monocyclic cycloalkyl substituted with one or more halogens, or $C_{1-6}$ alkoxy;
W and $X^2$ are as defined in any one of the following schemes:

(1) W is *-CONR$^W$-; $X^2$ is $R^{W-1}$; * represents the end connected to ring A;
(2) W is *-NR$^W$CO-; $X^2$ is $R^{W-1}$; * represents the end connected to ring A; and
(3) W is *-C(=$X^1$)NR$^W$-; $X^1$, $X^2$, and the C atoms therebetween together form 6-membered heteroaryl; in the 6-membered heteroaryl, the heteroatom is selected from 1, 2, or 3 of N, O, and S, and the number of the heteroatom is 1, 2, or 3; * represents the end connected to ring A;

$R^W$ is H, $C_{1-6}$ alkyl, or $C_{1-6}$ alkyl substituted with one or more halogens;
$R^{W-1}$ is H, halogen, $C_{1-6}$ alkyl, or $C_{1-6}$ alkyl substituted with one or more halogens;
G is C($R^{10}$) or N;
$R^{10}$ is H, halogen, $C_{1-6}$ alkyl, hydroxyl, or $C_{1-6}$ alkyl substituted with one or more halogens; and
the compound represented by formula II satisfies one, two, or three of the following conditions:

(1) $R^x$ is 6- to 9-membered bridged heterocycloalkyl or 6- to 9-membered bridged heterocycloalkyl substituted with one or more $R^{x-2}$;
(2) B is naphthyl, 5-membered heteroaryl, 9- to 10-membered heteroaryl, 6-membered heterocycloalkenyl, naphthyl substituted with one or more $R^5$, 5-membered heteroaryl substituted with one or more $R^6$, 9- to 10-membered heteroaryl substituted with one or more $R^3$, or 6-membered heterocycloalkenyl substituted with one or more $R^4$; and
(3) W is *-C(=$X^1$)NR$^W$-; $X^1$, $X^2$, and the C atoms therebetween together form 6-membered heteroaryl.

**3.** The nitrogen-containing compound represented by formula II, the pharmaceutically acceptable salt thereof, the stereoisomer thereof, the tautomer thereof, the metabolite thereof, the prodrug thereof, the isotopically labeled derivative thereof, the solvate thereof, or the solvate of the pharmaceutically acceptable salt thereof according to claim 2, wherein the nitrogen-containing compound represented by formula II is any one of the following schemes:

scheme 1:

$R^x$ is 6- to 9-membered bridged heterocycloalkyl, 7- to 9-membered spiro heterocycloalkyl, or 6- to 9-membered bridged heterocycloalkyl substituted with one or more $R^{x-2}$; in the "6- to 9-membered bridged heterocycloalkyl", the "7- to 9-membered spiro heterocycloalkyl", and the "6- to 9-membered bridged heterocycloalkyl substituted with one or more $R^{x-2}$", the heteroatom(s) independently consist(s) of 1 N atom and 0, 1, or 2 X atoms, the X atom being independently selected from 1 or 2 of N, O, and S; the "6- to 9-membered bridged heterocycloalkyl", the "7- to 9-membered spiro heterocycloalkyl", and the "6- to 9-membered bridged heterocycloalkyl substituted with one or more $R^{x-2}$" are connected to ring A through the N atom;
each $R^{x-2}$ is independently $C_{1-6}$ alkyl;
$R^1$ is -L-$R^{1-1}$;
L is *-NHSO$_2$-; * represents the end connected to ring A;
$R^{1-1}$ is $C_{1-6}$ alkyl, $C_{1-6}$ alkyl substituted with one or more $R^a$, or 3- to 6-membered monocyclic cycloalkyl substituted with one or more $R^b$;
each $R^a$ is independently hydroxyl;
each $R^b$ is independently $C_{1-6}$ alkyl;
B is 5-membered heteroaryl, 9- to 10-membered heteroaryl, 6-membered heteroaryl substituted with one or more $R^2$, or 9- to 10-membered heteroaryl substituted with one or more $R^3$; in the "5-membered heteroaryl", "9- to 10-membered heteroaryl", "6-membered heteroaryl substituted with one or more $R^2$", and "9- to 10-membered heteroaryl substituted with one or more $R^3$", the heteroatom is independently selected from 1, 2, or 3 of N, O, and S, and the number of the heteroatom is independently 1, 2, 3, or 4;
each $R^2$ is independently $C_{1-6}$ alkyl;
each $R^3$ is independently halogen, $C_{1-6}$ alkyl, or $C_{1-6}$ alkyl substituted with one or more halogens;

U is 3- to 10-membered cycloalkyl, 4- to 10-membered heterocycloalkyl, 3- to 10-membered cycloalkyl substituted with one or more $R^{2-1}$, or 4- to 10-membered heterocycloalkyl substituted with one or more $R^{2-2}$; in the "4- to 10-membered heterocycloalkyl" or "4- to 10-membered heterocycloalkyl substituted with one or more $R^{2-2}$", the heteroatom is independently selected from 1, 2, or 3 of N, O, and S, and the number of the heteroatom is independently 1, 2, 3, or 4;

each of $R^{2-1}$, $R^{2-2}$, and $R^{2-3}$ is independently halogen;

W and $X^2$ are as defined in any one of the following schemes:

(1) W is *-CONR$^{W}$-; $X^2$ is $R^{W-1}$; * represents the end connected to ring A; and

(2) W is *-C(=$X^1$)NR$^{W}$-; $X^1$, $X^2$, and the C atoms therebetween together form 6-membered heteroaryl; in the 6-membered heteroaryl, the heteroatom is selected from 1, 2, or 3 of N, O, and S, and the number of the heteroatom is 1, 2, or 3; * represents the end connected to ring A;

$R^W$ is H; $R^{W-1}$ is H;

G is C($R^{10}$) or N;

$R^{10}$ is H; and

the compound represented by formula II satisfies one, two, or three of the following conditions:

(1) $R^x$ is 6- to 9-membered bridged heterocycloalkyl or 6- to 9-membered bridged heterocycloalkyl substituted with one or more $R^{x-2}$;

(2) B is naphthyl, 5-membered heteroaryl, 9- to 10-membered heteroaryl, 6-membered heterocycloalkenyl, naphthyl substituted with one or more $R^5$, 5-membered heteroaryl substituted with one or more $R^6$, 9- to 10-membered heteroaryl substituted with one or more $R^3$, or 6-membered heterocycloalkenyl substituted with one or more $R^4$; and

(3) W is *-C(=$X^1$)NR$^{W}$-; $X^1$, $X^2$, and the C atoms therebetween together form 6-membered heteroaryl;

scheme 2:

$R^x$ is 6- to 9-membered bridged heterocycloalkyl, 7- to 9-membered spiro heterocycloalkyl, or 6- to 9-membered bridged heterocycloalkyl substituted with one or more $R^{x-2}$; in the "6- to 9-membered bridged heterocycloalkyl", the "7- to 9-membered spiro heterocycloalkyl", and the "6- to 9-membered bridged heterocycloalkyl substituted with one or more $R^{x-2}$", the heteroatom(s) independently consist(s) of 1 N atom and 0, 1, or 2 X atoms, the X atom being independently selected from 1 or 2 of N, O, and S; the "6- to 9-membered bridged heterocycloalkyl", the "7- to 9-membered spiro heterocycloalkyl", and the "6- to 9-membered bridged heterocycloalkyl substituted with one or more $R^{x-2}$" are connected to ring A through the N atom;

each $R^{x-2}$ is independently $C_{1-6}$ alkyl;

$R^1$ is -L-$R^{1-1}$;

L is *-$NHSO_2$-; * represents the end connected to ring A;

$R^{1-1}$ is $C_{1-6}$ alkyl, $C_{1-6}$ alkyl substituted with one or more $R^a$, or 3- to 6-membered monocyclic cycloalkyl substituted with one or more $R^b$;

each $R^a$ is independently hydroxyl;

each $R^b$ is independently $C_{1-6}$ alkyl;

B is 9- to 10-membered heteroaryl, 6-membered heteroaryl substituted with one or more $R^2$, or 9- to 10-membered heteroaryl substituted with one or more $R^3$; in the "5-membered heteroaryl", "9- to 10-membered heteroaryl", "6-membered heteroaryl substituted with one or more $R^2$", and "9- to 10-membered heteroaryl substituted with one or more $R^3$", the heteroatom is independently selected from 1, 2, or 3 of N, O, and S, and the number of the heteroatom is independently 1, 2, 3, or 4; when the 9- to 10-membered heteroaryl is 9-membered heteroaryl with 2 heteroatoms, the heteroatoms are independently selected from 1 or 2 of N and O; when the 9- to 10-membered heteroaryl is 9-membered heteroaryl with 3 heteroatoms, the heteroatoms are N; when the 9- to 10-membered heteroaryl is 10-membered heteroaryl with 2 heteroatoms of N, the heteroatoms are located on different rings;

when B is 6-membered heteroaryl substituted with one or more $R^2$, $R^x$ is 8- to 9-membered bridged heterocycloalkyl; in the "8- to 9-membered bridged heterocycloalkyl", the heteroatom(s) independently consist(s) of 1 N atom and 0, 1, or 2 X atoms, the X atom being independently selected from 1 or 2 of N, O, and S; the "8- to 9-membered bridged heterocycloalkyl" is connected to ring A through the N atom;

each $R^2$ is independently $C_{1-6}$ alkyl;

each $R^3$ is independently halogen or $C_{1-6}$ alkyl;

U is 4- to 10-membered heterocycloalkyl or 4- to 10-membered heterocycloalkyl substituted with one or more $R^{2-2}$; in the "4- to 10-membered heterocycloalkyl" or "4- to 10-membered heterocycloalkyl substituted with one or more $R^{2-2}$", the heteroatoms are independently selected from 1, 2, or 3 of N, O, and S, and the number of the heteroatoms is independently 2, 3, or 4;
each $R^{2-2}$ is independently halogen;
W is *-CONR$^{W}$-; $X^2$ is $R^{W-1}$; * represents the end connected to ring A;
$R^W$ is H; $R^{W-1}$ is H;
G is C($R^{10}$) or N;
$R^{10}$ is H; and
the compound represented by formula II satisfies one or two of the following conditions:

(1) $R^x$ is 6- to 9-membered bridged heterocycloalkyl or 6- to 9-membered bridged heterocycloalkyl substituted with one or more $R^{x-2}$; and
(2) B is 9- to 10-membered heteroaryl or 9- to 10-membered heteroaryl substituted with one or more $R^3$; and

scheme 3:

$R^x$ is 6- to 9-membered bridged heterocycloalkyl, 6- to 9-membered bridged heterocycloalkyl substituted with one or more $R^{x-2}$, or 7- to 9-membered spiro heterocycloalkyl;
each $R^{x-2}$ is independently $C_{1-6}$ alkyl;
$R^1$ is -L-$R^{1-1}$;
L is *-$NHSO_2$-;
$R^{1-1}$ is $C_{1-6}$ alkyl, $C_{1-6}$ alkyl substituted with one or more $R^a$, or 3- to 6-membered monocyclic cycloalkyl substituted with one or more $R^b$;
$R^a$ is hydroxyl;
each $R^b$ is independently $C_{1-6}$ alkyl;
B is 5-membered heteroaryl, 9- to 10-membered heteroaryl, 6-membered heteroaryl substituted with one or more $R^2$, or 9- to 10-membered heteroaryl substituted with one or more $R^3$;
each $R^2$ is independently $C_{1-6}$ alkyl;
each $R^3$ is independently $C_{1-6}$ alkyl;
U is 4- to 10-membered heterocycloalkyl, 3- to 10-membered cycloalkyl substituted with one or more $R^{2-1}$, or 4- to 10-membered heterocycloalkyl substituted with one or more $R^{2-2}$;
each $R^{2-1}$ is independently halogen;
each $R^{2-2}$ is independently halogen;
W is *-CONH-; $X^2$ is H; and
the compound represented by formula II satisfies one or two of the following conditions:

(1) $R^x$ is 6- to 9-membered bridged heterocycloalkyl; and
(2) B is 5-membered heteroaryl, naphthyl, 9- to 10-membered heteroaryl, naphthyl substituted with one or more $R^5$, or 9- to 10-membered heteroaryl substituted with one or more $R^3$.

**4.** The nitrogen-containing compound represented by formula II, the pharmaceutically acceptable salt thereof, the stereoisomer thereof, the tautomer thereof, the metabolite thereof, the prodrug thereof, the isotopically labeled derivative thereof, the solvate thereof, or the solvate of the pharmaceutically acceptable salt thereof according to claim 2, wherein

$R^x$ is 6- to 9-membered bridged heterocycloalkyl, 6- to 9-membered bridged heterocycloalkyl substituted with one or more $R^{x-2}$, or 7- to 9-membered spiro heterocycloalkyl;
each $R^{x-2}$ is independently $C_{1-6}$ alkyl;
$R^1$ is -L-$R^{1-1}$;
L is *-$NHSO_2$-;
$R^{1-1}$ is $C_{1-6}$ alkyl, $C_{1-6}$ alkyl substituted with one or more $R^a$, or 3- to 6-membered monocyclic cycloalkyl substituted with one or more $R^b$;
$R^a$ is hydroxyl;
each $R^b$ is independently $C_{1-6}$ alkyl;
B is naphthyl, 5-membered heteroaryl, 9- to 10-membered heteroaryl, naphthyl substituted with one or more $R^5$, 6-membered heteroaryl substituted with one or more $R^2$, or 9- to 10-membered heteroaryl substituted with one or

more $R^3$;
each $R^2$ is independently $C_{1-6}$ alkyl;
each $R^3$ is independently $C_{1-6}$ alkyl;
U is 4- to 10-membered heterocycloalkyl, 3- to 10-membered cycloalkyl substituted with one or more $R^{2-1}$, or 4- to 10-membered heterocycloalkyl substituted with one or more $R^{2-2}$;
each $R^{2-1}$ is independently halogen;
each $R^{2-2}$ is independently halogen;
W is *-CONH-; $X^2$ is H; and
the compound represented by formula II satisfies one or two of the following conditions:

(1) $R^x$ is 6- to 9-membered bridged heterocycloalkyl; and
(2) B is 5-membered heteroaryl, naphthyl, 9- to 10-membered heteroaryl, naphthyl substituted with one or more $R^5$, or 9- to 10-membered heteroaryl substituted with one or more $R^3$.

**5.** The nitrogen-containing compound represented by formula II, the pharmaceutically acceptable salt thereof, the stereoisomer thereof, the tautomer thereof, the metabolite thereof, the prodrug thereof, the isotopically labeled derivative thereof, the solvate thereof, or the solvate of the pharmaceutically acceptable salt thereof according to claim 2, wherein one or more of the following conditions are satisfied:

(1) $R^x$ is 6- to 9-membered bridged heterocycloalkyl, 7- to 9-membered spiro heterocycloalkyl, 6- to 9-membered bridged heterocycloalkyl substituted with one or more $R^{x-2}$, or 7- to 9-membered spiro heterocycloalkyl substituted with one or more $R^{x-3}$;
(2) each of $R^{x-1}$, $R^{x-2}$, $R^{x-3}$, and $R^{x-4}$ is independently halogen, $C_{1-6}$ alkyl, or $C_{1-6}$ alkyl substituted with one or more halogens;
(3) L is *-NHSO$_2$-;
(4) $R^{1-1}$ is $C_{1-6}$ alkyl, 3- to 6-membered monocyclic cycloalkyl, 3- to 6-membered monocyclic cycloalkyl substituted with one or more $R^b$, or $C_{1-6}$ alkyl substituted with one or more $R^a$;
(5) $R^a$ is hydroxyl;
(6) each $R^b$ is independently $C_{1-6}$ alkyl;
(7) B is phenyl, naphthyl, 5-membered heteroaryl, 6-membered heteroaryl, 9- to 10-membered heteroaryl, 6-membered heterocycloalkenyl, naphthyl substituted with one or more $R^5$, 6-membered heteroaryl substituted with one or more $R^2$, 9- to 10-membered heteroaryl substituted with one or more $R^3$, or 6-membered heterocycloalkenyl substituted with one or more $R^4$;
(8) each of $R^2$, $R^3$, $R^4$, $R^5$, and $R^6$ is independently halogen, oxo, or $C_{1-6}$ alkyl;
(9) U is 3- to 10-membered cycloalkyl, 4- to 10-membered heterocycloalkyl, 3- to 10-membered cycloalkyl substituted with one or more $R^{2-1}$, or 4- to 10-membered heterocycloalkyl substituted with one or more $R^{2-2}$;
(10) each of $R^{2-1}$, $R^{2-2}$, and $R^{2-3}$ is independently halogen, $C_{1-6}$ alkyl, hydroxyl, $C_{1-6}$ alkoxy, or $C_{1-6}$ alkyl substituted with one or more halogens;
(11) G is C($R^{10}$);
(12) $R^{10}$ is H, halogen, $C_{1-6}$ alkyl, or $C_{1-6}$ alkyl substituted with one or more halogens; and
(13) W is *-CONR$^W$-; $X^2$ is $R^{W-1}$; $R^{W-1}$ is H or halogen; $R^W$ is H.

**6.** The nitrogen-containing compound represented by formula II, the pharmaceutically acceptable salt thereof, the stereoisomer thereof, the tautomer thereof, the metabolite thereof, the prodrug thereof, the isotopically labeled derivative thereof, the solvate thereof, or the solvate of the pharmaceutically acceptable salt thereof according to claim 2, wherein one or more of the following conditions are satisfied:

(1) $R^x$ is 6- to 9-membered bridged heterocycloalkyl, 7- to 9-membered spiro heterocycloalkyl, or 6- to 9-membered bridged heterocycloalkyl substituted with one or more $R^{-2}$;
each $R^{x-2}$ is independently $C_{1-6}$ alkyl;
(2) each of $R^{x-1}$, $R^{x-2}$, $R^{x-3}$, and $R^{x-4}$ is independently $C_{1-6}$ alkyl;
(3) L is *-NHSO$_2$- or -NH-;
(4) B is 5-membered heteroaryl, 9- to 10-membered heteroaryl, 6-membered heteroaryl substituted with one or more $R^2$, or 9- to 10-membered heteroaryl substituted with one or more $R^3$;
preferably, B is 9- to 10-membered heteroaryl or 9- to 10-membered heteroaryl substituted with one or more $R^3$;
(5) when B is 6-membered heteroaryl substituted with one or more $R^2$, $R^x$ is 8- to 9-membered bridged heterocycloalkyl;
(6) each of $R^2$, $R^3$, $R^4$, $R^5$, and $R^6$ is independently halogen, $C_{1-6}$ alkyl, or $C_{1-6}$ alkyl substituted with one or more

halogens;
each $R^2$ is independently preferably $C_{1-6}$ alkyl; each $R^3$ is independently preferably halogen or $C_{1-6}$ alkyl;
(7) U is 4- to 10-membered heterocycloalkyl or 4- to 10-membered heterocycloalkyl substituted with one or more $R^{2-2}$;
each $R^{2-2}$ is independently halogen;
(8) G is $C(R^{10})$ or N; $R^{10}$ is H;
(9) $R^W$ is H; and
(10) $R^{W-1}$ is H.

**7.** The nitrogen-containing compound represented by formula II, the pharmaceutically acceptable salt thereof, the stereoisomer thereof, the tautomer thereof, the metabolite thereof, the prodrug thereof, the isotopically labeled derivative thereof, the solvate thereof, or the solvate of the pharmaceutically acceptable salt thereof according to claim 2, wherein one or more of the following conditions are satisfied:

(1) in B, the 9- to 10-membered heteroaryl is 9-membered heteroaryl with 2 heteroatoms independently selected from 1 or 2 of N and O; or

in B, the 9- to 10-membered heteroaryl is 9-membered heteroaryl with 3 heteroatoms of N; or
in B, the 9- to 10-membered heteroaryl is 10-membered heteroaryl with 2 heteroatoms of N, and the heteroatoms are located on different rings;

(2) B is 9- to 10-membered heteroaryl, 6-membered heteroaryl substituted with one or more $R^2$, or 9- to 10-membered heteroaryl substituted with one or more $R^3$;

each $R^2$ is independently $C_{1-6}$ alkyl;
each $R^3$ is independently halogen or $C_{1-6}$ alkyl;

(3) in U, the 4- to 10-membered heterocycloalkyl is 4- to 6-membered monocyclic heterocycloalkyl, 6- to 9-membered bridged heterocycloalkyl, or 7- to 9-membered spiro heterocycloalkyl;
(4) $R^x$ is 6- to 9-membered bridged heterocycloalkyl or 6- to 9-membered bridged heterocycloalkyl substituted with one or more $R^{x-2}$; and
(5) in $R^{1-1}$, the $C_{1-6}$ alkyl is ethyl or isopropyl.

**8.** The nitrogen-containing compound represented by formula II, the pharmaceutically acceptable salt thereof, the stereoisomer thereof, the tautomer thereof, the metabolite thereof, the prodrug thereof, the isotopically labeled derivative thereof, the solvate thereof, or the solvate of the pharmaceutically acceptable salt thereof according to claim 2, wherein one or more of the following conditions are satisfied:

(1) $R^x$ is 6- to 9-membered bridged heterocycloalkyl substituted with one or more $R^{x-2}$, 6- to 9-membered bridged heterocycloalkyl, or 7- to 9-membered spiro heterocycloalkyl, preferably 6- to 9-membered bridged heterocycloalkyl or 7- to 9-membered spiro heterocycloalkyl;
(2) each of $R^{x-1}$, $R^{x-2}$, $R^{x-3}$, and $R^{x-4}$ is independently halogen or $C_{1-6}$ alkyl, preferably $C_{1-3}$ alkyl, F, Cl, or Br;
(3) $R^{1-1}$ is $C_{1-3}$ alkyl, $C_{1-3}$ alkyl substituted with one or more $R^a$, or 3- to 6-membered monocyclic cycloalkyl substituted with one or more $R^b$;
(4) B is 5-membered heteroaryl, 9- to 10-membered heteroaryl, 6-membered heteroaryl substituted with one or more $R^2$, or 9- to 10-membered heteroaryl substituted with one or more $R^3$, preferably 9- to 10-membered heteroaryl or 9- to 10-membered heteroaryl substituted with one or more $R^3$;
(5) each of $R^2$, $R^3$, $R^4$, $R^5$, and $R^6$ is independently $C_{1-3}$ alkyl;
(6) U is 4- to 9-membered heterocycloalkyl, 3- to 8-membered cycloalkyl substituted with one or more $R^{2-1}$, or 4- to 9-membered heterocycloalkyl substituted with one or more $R^{2-2}$, preferably 4- to 9-membered heterocycloalkyl or 4- to 9-membered heterocycloalkyl substituted with one or more $R^{2-2}$;
(7) each of $R^{2-1}$, $R^{2-2}$, and $R^{2-3}$ is independently halogen, preferably F, Cl, or Br; and
(8) W is *-$CONR^W$-; $X^2$ is $R^{W-1}$; $R^{W-1}$ is H; $R^W$ is H.

**9.** The nitrogen-containing compound represented by formula II, the pharmaceutically acceptable salt thereof, the stereoisomer thereof, the tautomer thereof, the metabolite thereof, the prodrug thereof, the isotopically labeled derivative thereof, the solvate thereof, or the solvate of the pharmaceutically acceptable salt thereof according to claim 2, wherein one or more of the following conditions are satisfied:

(1) $R^x$ is 6- to 9-membered bridged heterocycloalkyl, 7- to 9-membered spiro heterocycloalkyl, 6- to 9-membered bridged heterocycloalkyl substituted with one or more $R^{x-2}$, or 7- to 9-membered spiro heterocycloalkyl substituted with one or more $R^{x-3}$;
each of $R^{x-1}$, $R^{x-2}$, $R^{x-3}$, and $R^{x-4}$ is independently halogen, $C_{1-6}$ alkyl, or $C_{1-6}$ alkyl substituted with one or more halogens, preferably halogen or $C_{1-6}$ alkyl, and more preferably $C_{1-3}$ alkyl, F, Cl, or Br;
(2) $R^{1-1}$ is $C_{1-6}$ alkyl, 3- to 6-membered monocyclic cycloalkyl substituted with one or more $R^b$, or $C_{1-6}$ alkyl substituted with one or more $R^a$;

$R^a$ is hydroxyl;
each $R^b$ is independently $C_{1-6}$ alkyl;

(3) B is 5-membered heteroaryl, 9- to 10-membered heteroaryl, 6-membered heteroaryl substituted with one or more $R^2$, or 9- to 10-membered heteroaryl substituted with one or more $R^3$;
each of $R^2$ and $R^3$ is independently halogen or $C_{1-6}$ alkyl;
(4) U is 3- to 10-membered cycloalkyl, 4- to 10-membered heterocycloalkyl, 3- to 10-membered cycloalkyl substituted with one or more $R^{2-1}$, or 4- to 10-membered heterocycloalkyl substituted with one or more $R^{2-2}$;
each of $R^{2-1}$ and $R^{2-2}$ is independently halogen, $C_{1-6}$ alkyl, hydroxyl, $C_{1-6}$ alkoxy, or $C_{1-6}$ alkyl substituted with one or more halogens, preferably F, Cl, or Br;
(5) G is $C(R^{10})$; $R^{10}$ is H; and
(6) the compound represented by formula II satisfies 1 or 2 of the following conditions: (a) $R^x$ is 6- to 9-membered bridged heterocycloalkyl; and (b) B is 5-membered heteroaryl, 9- to 10-membered heteroaryl, 6-membered heterocycloalkenyl, 6-membered heterocycloalkenyl substituted with one or more $R^4$, or 9- to 10-membered heteroaryl substituted with one or more $R^3$, preferably 9- to 10-membered heteroaryl or 9- to 10-membered heteroaryl substituted with one or more $R^3$.

**10.** The nitrogen-containing compound represented by formula II, the pharmaceutically acceptable salt thereof, the stereoisomer thereof, the tautomer thereof, the metabolite thereof, the prodrug thereof, the isotopically labeled derivative thereof, the solvate thereof, or the solvate of the pharmaceutically acceptable salt thereof according to claim 2, wherein

the nitrogen-containing compound represented by formula II is the following compound:

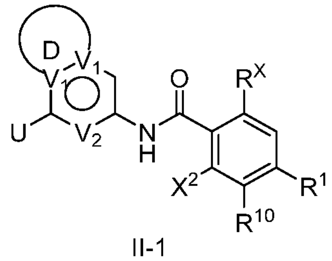

II-1

;

each $V_1$ is independently C or N;
$V_2$ is N or C-$R^{11}$, preferably N;
D is partially saturated or unsaturated 5- to 6-membered carbocycle, or partially saturated or unsaturated 5-to 6-membered heterocycle, the 5- to 6-membered carbocycle or 5- to 6-membered heterocycle being optionally substituted with 0, 1, 2, or 3 $R^{11}$; in the "5- to 6-membered heterocycle", the heteroatom is N, O, or S, and the number of the heteroatom is independently 1, 2, 3, or 4;
$R^{11}$ is hydrogen, halogen, cyano, $C_{1-6}$ alkyl, $C_{1-6}$ alkoxy, or $C_{1-6}$ alkyl substituted with one or more halogens.

**11.** The nitrogen-containing compound represented by formula II, the pharmaceutically acceptable salt thereof, the stereoisomer thereof, the tautomer thereof, the metabolite thereof, the prodrug thereof, the isotopically labeled derivative thereof, the solvate thereof, or the solvate of the pharmaceutically acceptable salt thereof according to claim 2 or 10, wherein one or more of the following conditions are satisfied:

(1) each "6- to 9-membered bridged heterocycloalkyl" is independently 6- to 9-membered bridged heterocycloalkyl comprising 1 or 2 heteroatoms, preferably any one of the following fragments:

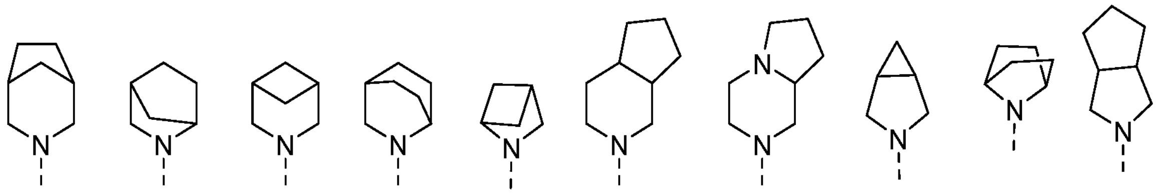

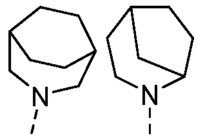 ,

and more preferably

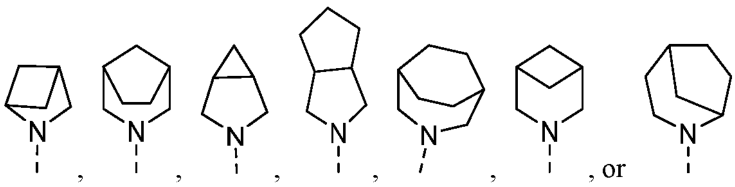 , , , , , , or ;

(2) in a certain scheme, each "9- to 10-membered heteroaryl" is independently any one of the following fragments:

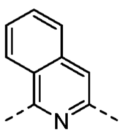 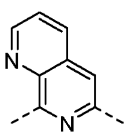 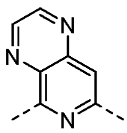 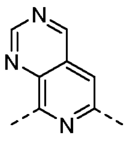 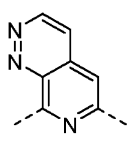 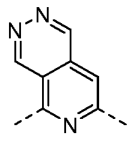 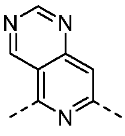 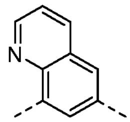

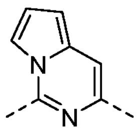 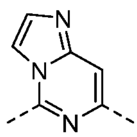 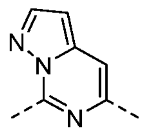 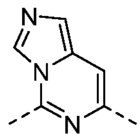 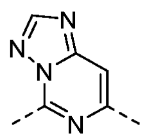 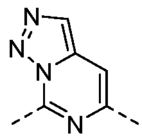 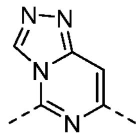

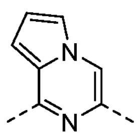 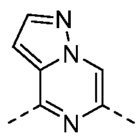 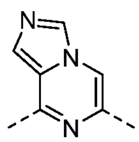 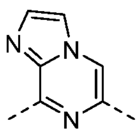 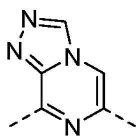 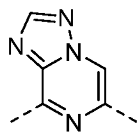 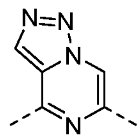

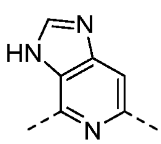 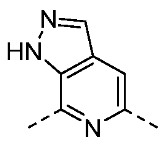 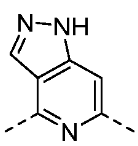 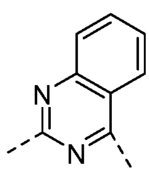 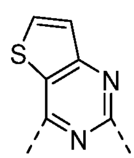 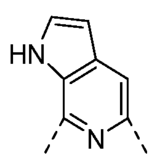 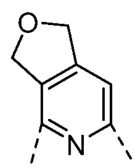

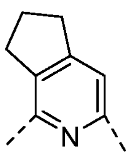 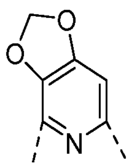 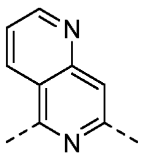 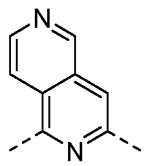 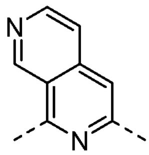 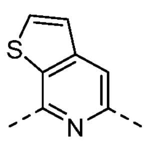 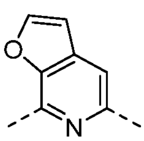

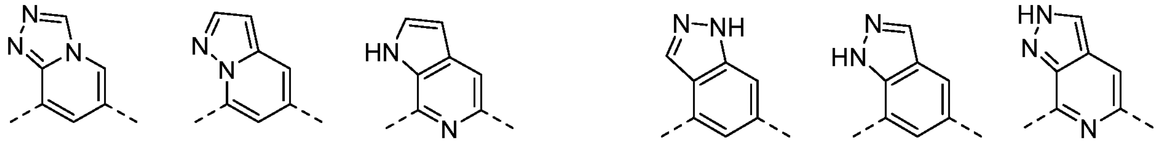

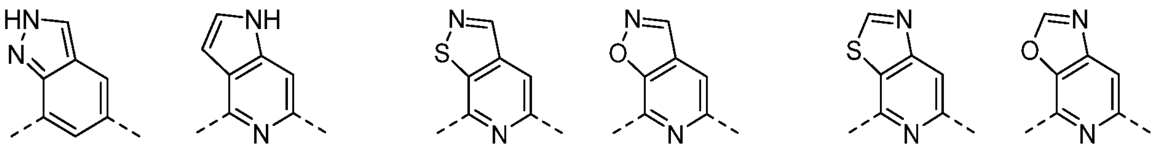

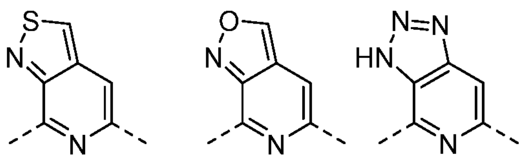
,

preferably

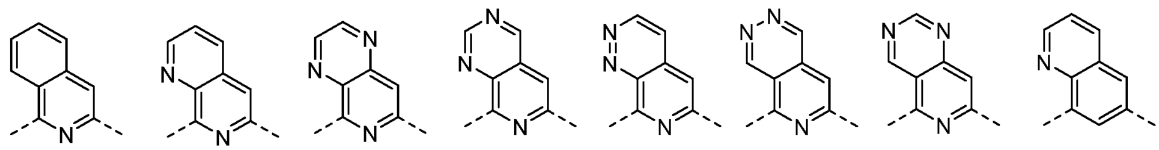

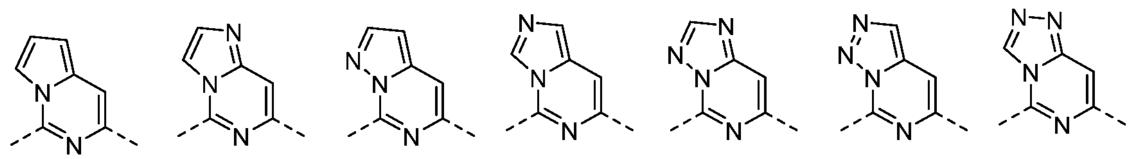

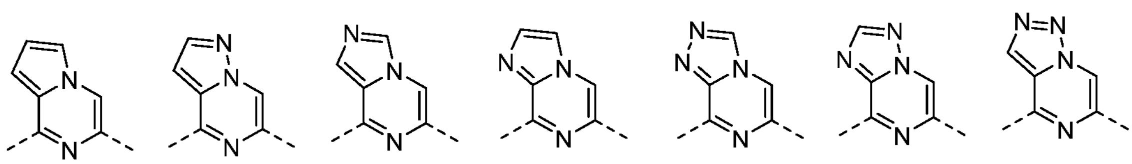

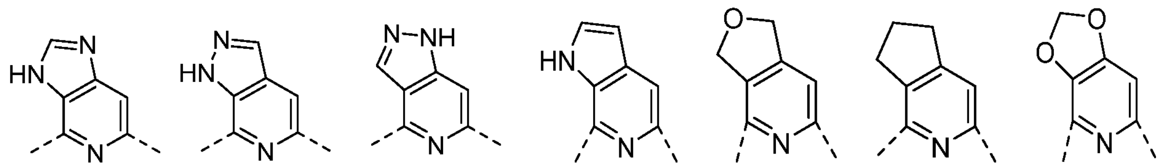

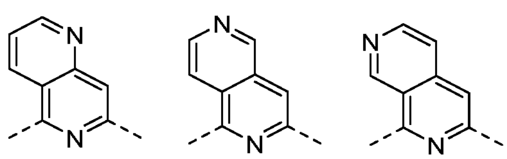

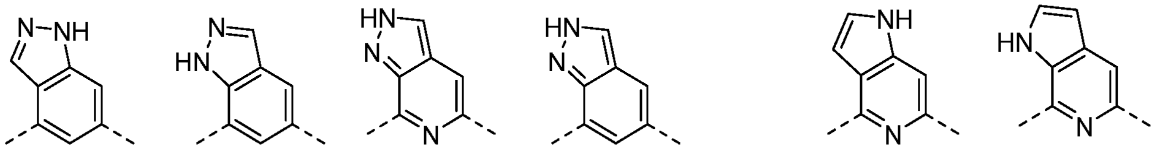

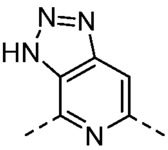

connected to U through the left dashed line, and more preferably

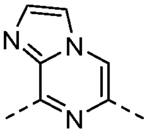

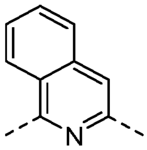 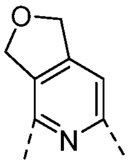 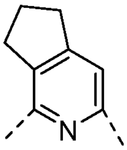 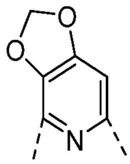 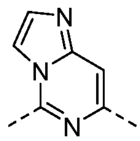 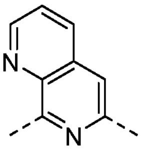 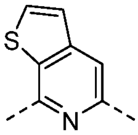

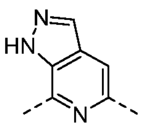 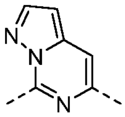 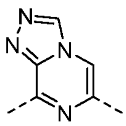 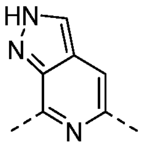 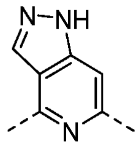 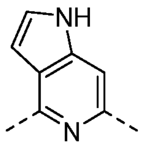 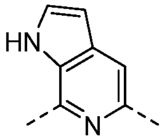

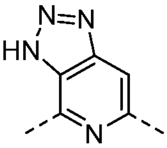 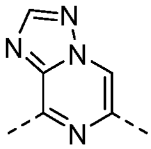

connected to U through the left dashed line;

(3) each "4- to 10-membered heterocycloalkyl" is independently 4- to 6-membered monocyclic heterocycloalkyl, 6- to 8-membered spiro heterocycloalkyl, or 7- to 9-membered bridged heterocycloalkyl, preferably

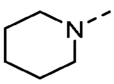, 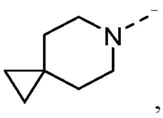, 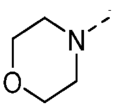, 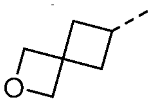, 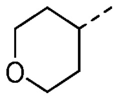, 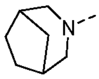, or 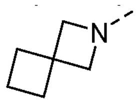,

and more preferably

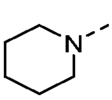, 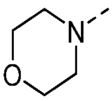, 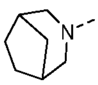, or 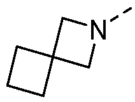;

(4) $R^x$ is any one of the following fragments:

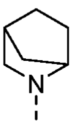 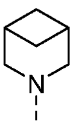 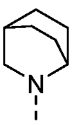 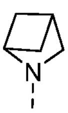 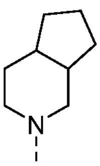 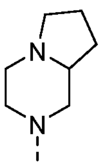 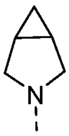 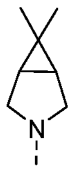 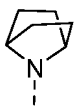

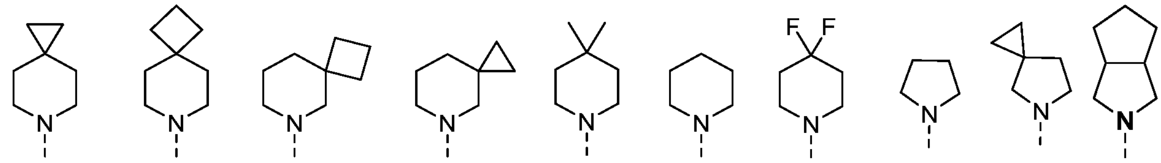

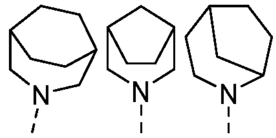
,

preferably

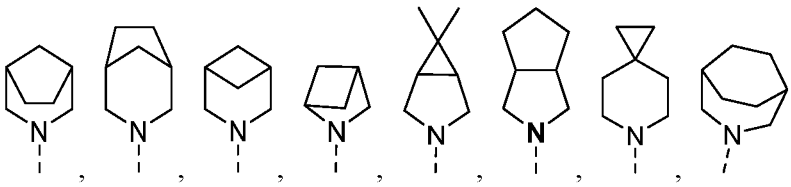
, , , , , , , ,

or

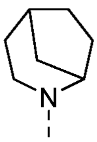
;

(5) in a certain scheme, B is any one of the following fragments (the left dashed line in the following fragments represents the bond connected to U):

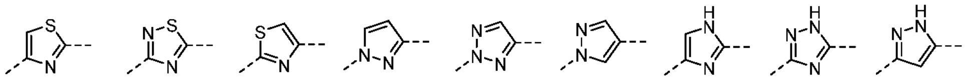

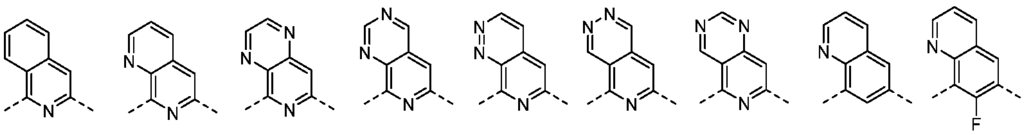

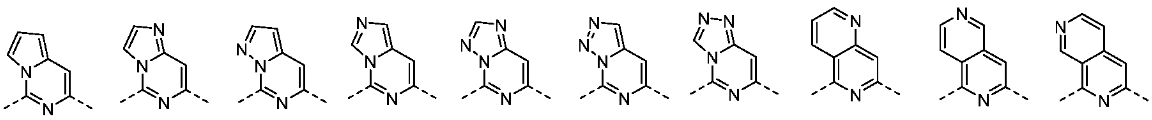

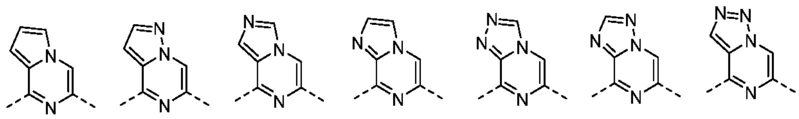

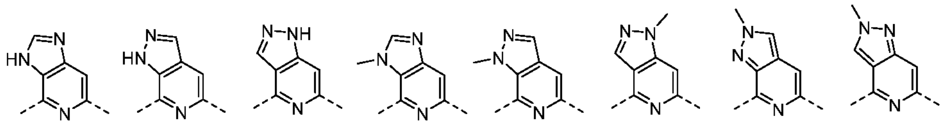

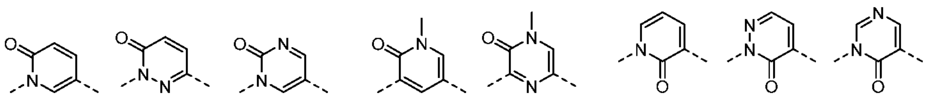

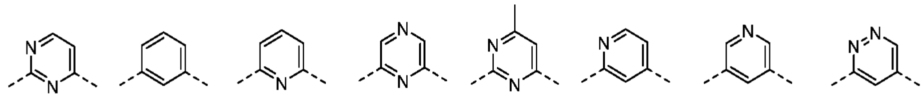

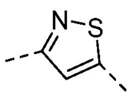 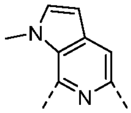 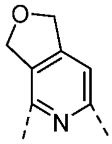 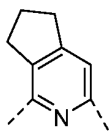 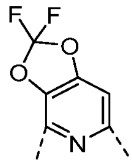 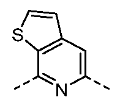 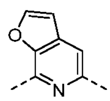

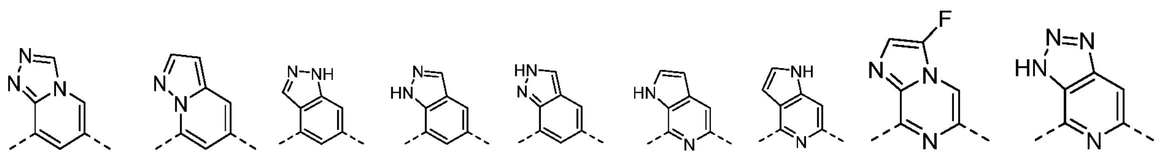

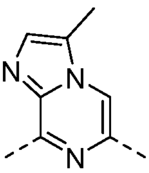 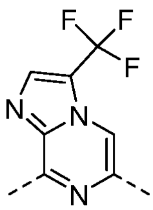 or 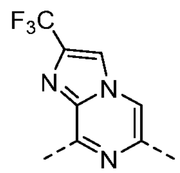 ,

preferably

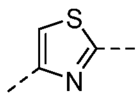 , 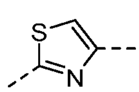 , 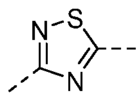 ,

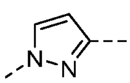 , 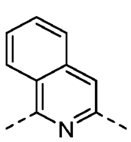 , 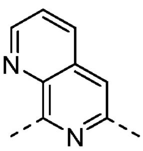 , 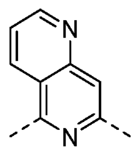 , 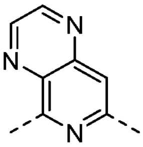 , 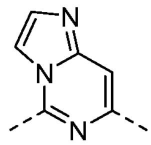 , 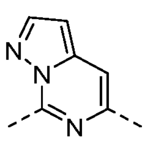 ,

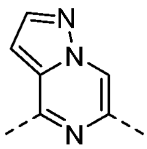 , 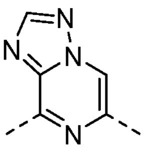 , 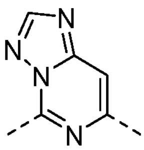 , 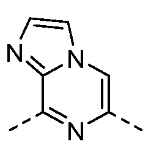 , 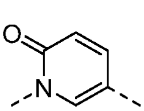 , 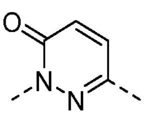 ,

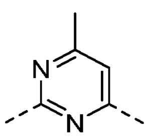 , 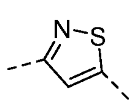 , 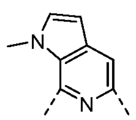 , 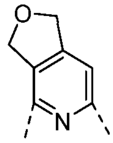 , 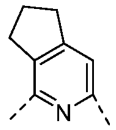 , 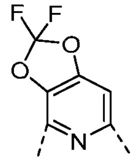 , 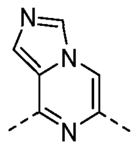 ,

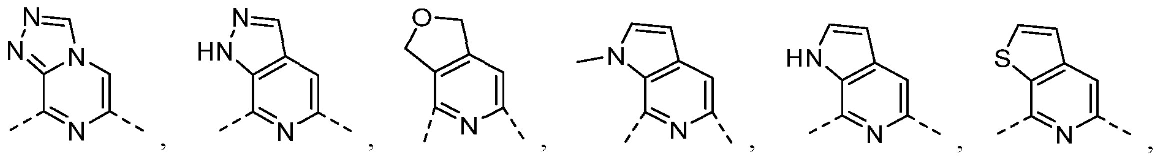

, , , , , ,

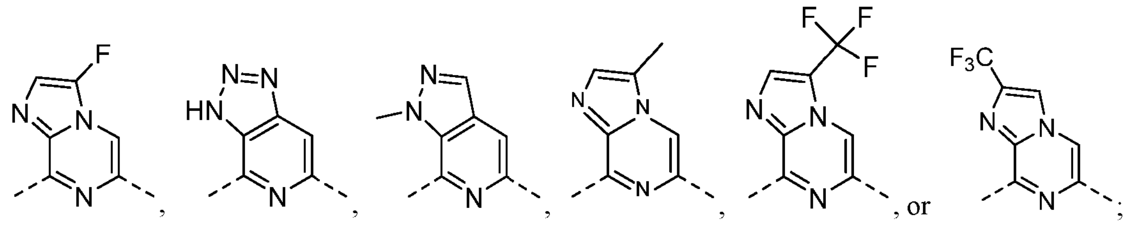

, , , , , or ;

and

(6) U is any one of the following fragments:

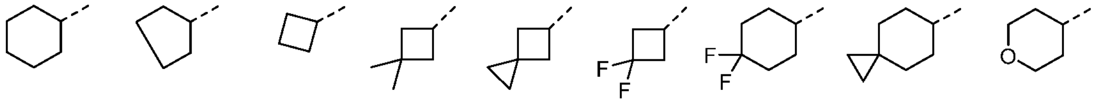

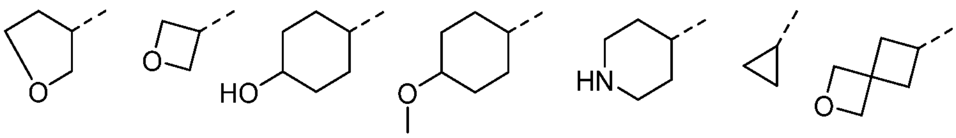

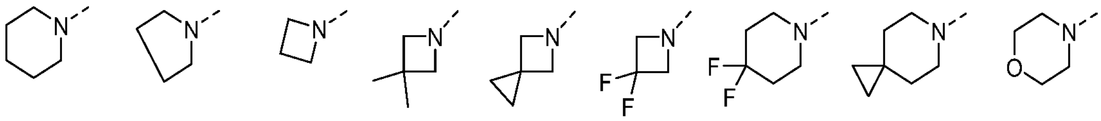

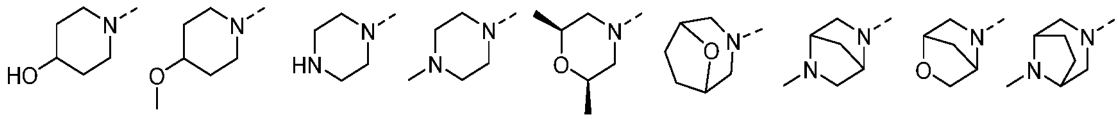

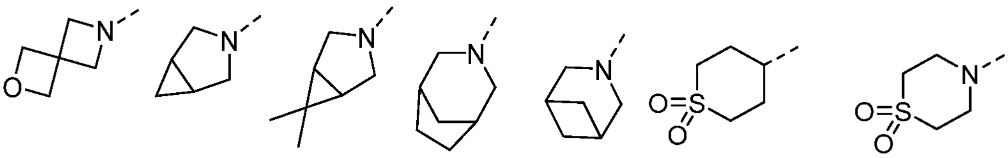

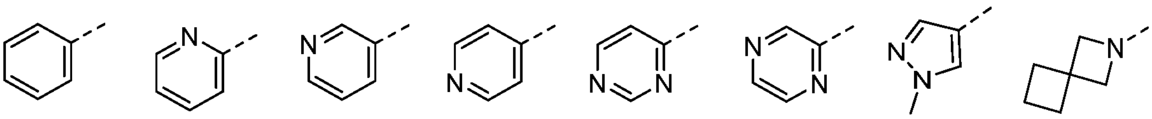

or

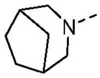

,

preferably

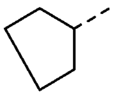, 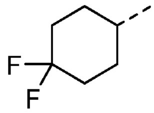, 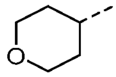, 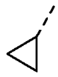, 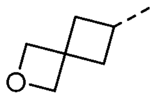, 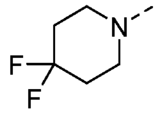,

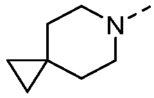, 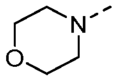, 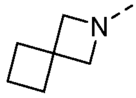, or 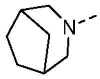.

**12.** The nitrogen-containing compound represented by formula II, the pharmaceutically acceptable salt thereof, the stereoisomer thereof, the tautomer thereof, the metabolite thereof, the prodrug thereof, the isotopically labeled derivative thereof, the solvate thereof, or the solvate of the pharmaceutically acceptable salt thereof according to claim 2 or 10, wherein one or more of the following conditions are satisfied:

(1) each "5- to 6-membered monocyclic heterocycloalkyl" is independently 5- to 6-membered heterocycloalkyl comprising 1 or 2 heteroatoms;
(2) each "6- to 9-membered bridged heterocycloalkyl" is independently 6- to 9-membered bridged heterocycloalkyl comprising 1 or 2 heteroatoms;
(3) each "7- to 9-membered spiro heterocycloalkyl" is independently 7- to 9-membered spiro heterocycloalkyl comprising 1 or 2 heteroatoms;
(4) each "halogen" is independently fluorine, chlorine, bromine, or iodine;
(5) each "$C_{1-6}$ alkyl" is independently methyl, ethyl, n-propyl, isopropyl, n-butyl, isobutyl, or *tert-butyl;*
(6) each "3- to 6-membered monocyclic cycloalkyl" is independently cyclopropyl, cyclobutyl, cyclopentyl, or cyclohexyl;
(7) each "$C_{1-6}$ alkoxy" is independently methoxy, ethoxy, n-propoxy, isopropoxy, n-butoxy, isobutoxy, or tert-butoxy;
(8) the "4- to 8-membered monocyclic heterocycloalkyl" is "4- to 8-membered monocyclic heterocycloalkyl" comprising one O atom;
(9) each 5-membered heteroaryl is independently 5-membered heteroaryl with "heteroatoms selected from 1 or 2 of N and S";
(10) each "6-membered heteroaryl" is independently any one of the following fragments:

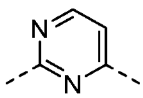 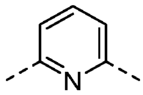 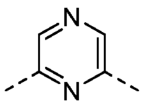 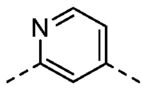 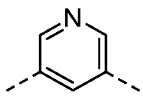 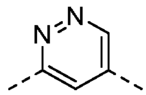;

(11) each "9- to 10-membered heteroaryl" is independently any one of the following fragments:

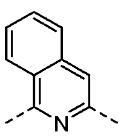 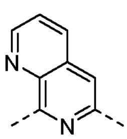 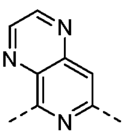 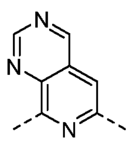 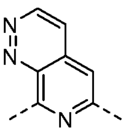 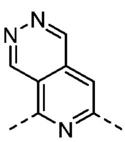 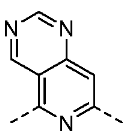 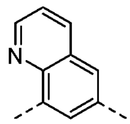

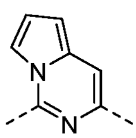 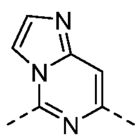 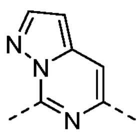 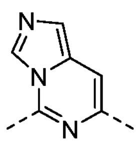 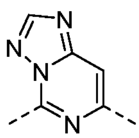 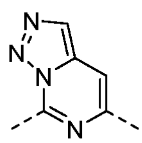 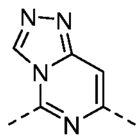

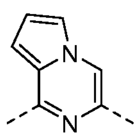 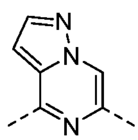 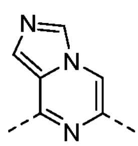 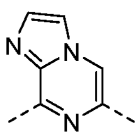 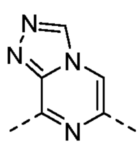 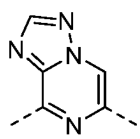 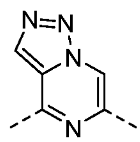

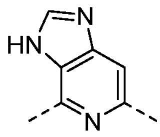 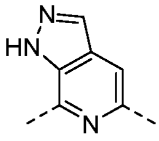 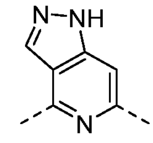 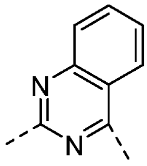 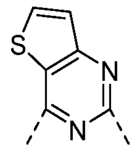 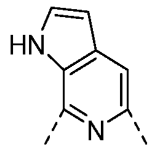 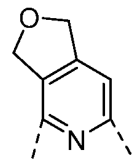

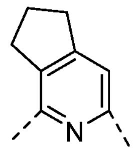 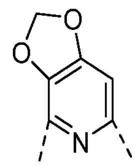 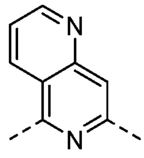 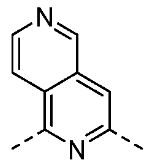 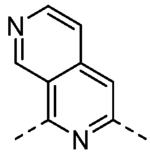 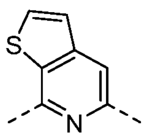 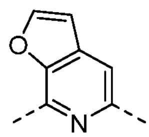

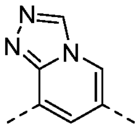 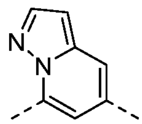 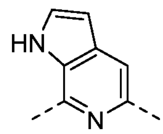 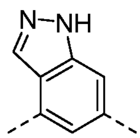 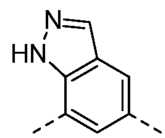 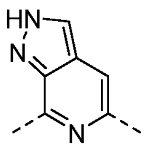

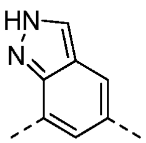 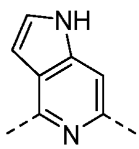 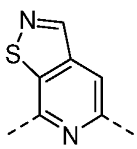 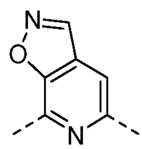 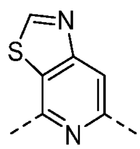 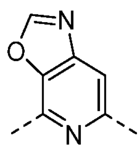

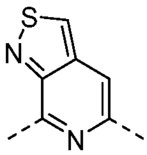 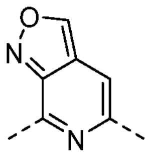 ;

(12) each "6-membered heterocycloalkenyl" is independently 6-membered heterocycloalkenyl with 1 or 2 heteroatoms of N;
(13) the "3- to 10-membered cycloalkyl" is 3- to 6-membered monocyclic cycloalkyl or 6- to 8-membered spiro cycloalkyl;
(14) the "4- to 10-membered heterocycloalkyl" is 4- to 6-membered monocyclic heterocycloalkyl, 6- to 8-membered spiro heterocycloalkyl, or 7- to 9-membered bridged heterocycloalkyl;
(15) the "6- to 10-membered aryl" is phenyl;
(16) the "5- to 10-membered heteroaryl" is 5- to 6-membered heteroaryl with 1 or 2 heteroatoms of N; and
(17) when W is *-C($X^1$)$NR^W$-, and $X^1$, $X^2$, and the C atoms therebetween together form 6-membered heteroaryl, in the "6-membered heteroaryl", the heteroatom is N, and the number of the heteroatom is 1 or 2.

**13.** The nitrogen-containing compound represented by formula II, the pharmaceutically acceptable salt thereof, the stereoisomer thereof, the tautomer thereof, the metabolite thereof, the prodrug thereof, the isotopically labeled derivative thereof, the solvate thereof, or the solvate of the pharmaceutically acceptable salt thereof according to claim 2 or 10, wherein one or more of the following conditions are satisfied:

(1) each "5- to 6-membered monocyclic heterocycloalkyl" is independently

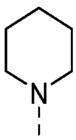 or 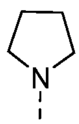 ;

(2) each "6- to 9-membered bridged heterocycloalkyl" is independently

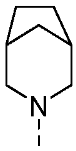 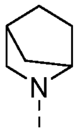 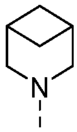 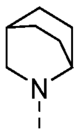 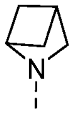 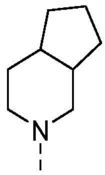 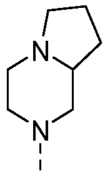 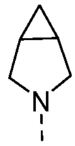 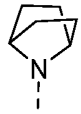 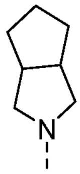 ;

(3) each "7- to 9-membered spiro heterocycloalkyl" is independently any one of the following fragments:

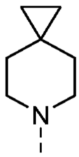 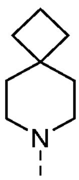 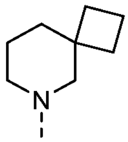 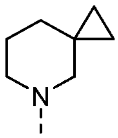 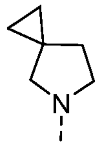 ;

(4) the "halogen" is fluorine;
(5) each "$C_{1-6}$ alkyl" is independently methyl or ethyl;
(6) the "3- to 6-membered monocyclic cycloalkyl" is cyclopropyl;
(7) each "$C_{1-6}$ alkoxy" is independently methoxy or ethoxy;
(8) the "4- to 8-membered monocyclic heterocycloalkyl" is

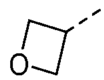 ;

(9) each "5-membered heteroaryl" is independently any one of the following fragments:

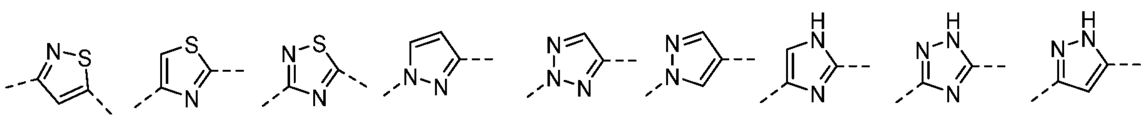 ;

(10) each "6-membered heteroaryl" is independently

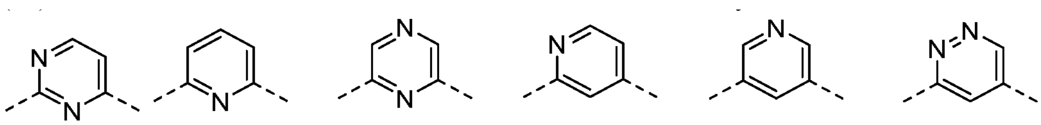

connected to U through the left dashed line;
(11) each "9- to 10-membered heteroaryl" is independently

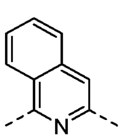 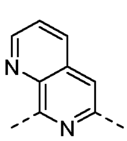 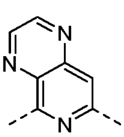 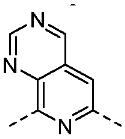 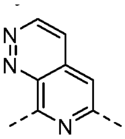 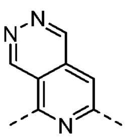 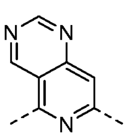 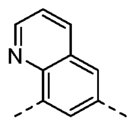

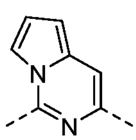 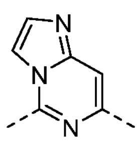 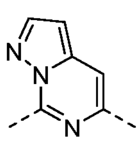 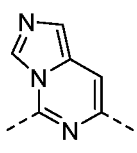 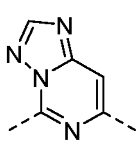 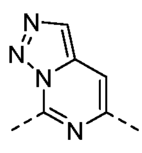 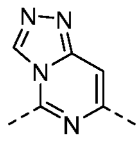

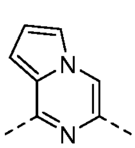 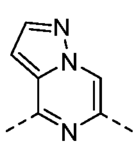 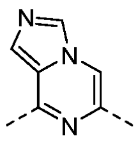 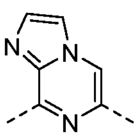 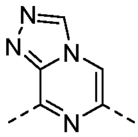 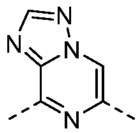 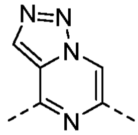

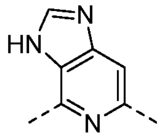 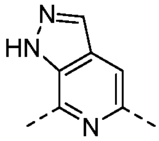 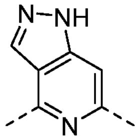 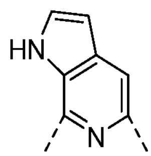 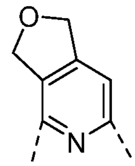 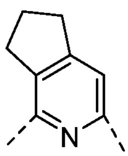 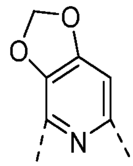

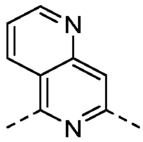 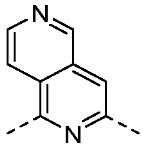 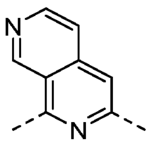

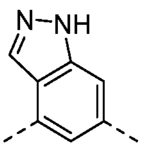 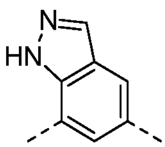 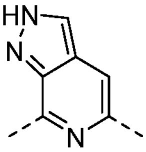 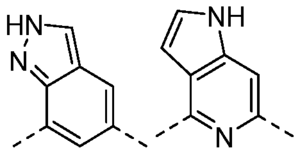 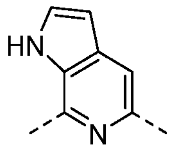

connected to U through the left dashed line;

(12) each "6-membered heterocycloalkenyl" is independently any one of the following fragments:

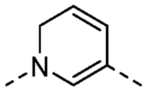 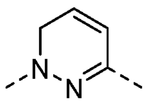 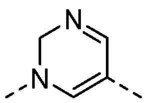 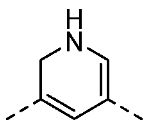 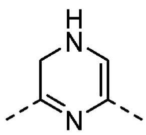 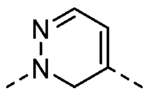 ;

(13) each "3- to 10-membered cycloalkyl" is independently cyclopropyl, cyclobutyl, cyclopentyl,

cyclohexyl, 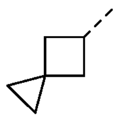 , or 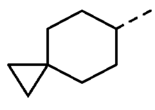 ;

(14) each "4- to 10-membered heterocycloalkyl" is independently 4- to 6-membered monocyclic heterocycloalkyl, 6- to 8-membered spiro heterocycloalkyl, or 7- to 9-membered bridged heterocycloalkyl, preferably

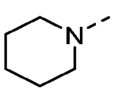 , 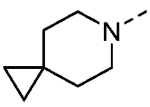 , 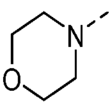 , 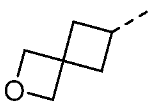 , or 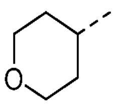 ;

and

(15) the "5- to 10-membered heteroaryl" is

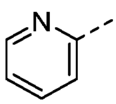 .

**14.** The nitrogen-containing compound represented by formula II, the pharmaceutically acceptable salt thereof, the stereoisomer thereof, the tautomer thereof, the metabolite thereof, the prodrug thereof, the isotopically labeled

derivative thereof, the solvate thereof, or the solvate of the pharmaceutically acceptable salt thereof according to claim 2 or 10, wherein one or more of the following conditions are satisfied:

(1) each "6- to 9-membered bridged heterocycloalkyl" is independently

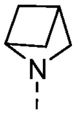 , 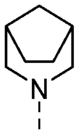 , 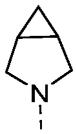 , 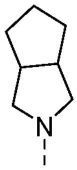 ,

or

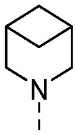 ;

(2) the "7- to 9-membered spiro heterocycloalkyl" is

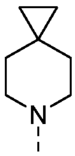 ;

(3) the "5-membered heteroaryl" is

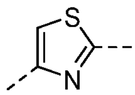 ,

such as

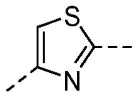

connected to U through the right dashed line;
(4) the "6-membered heteroaryl" is

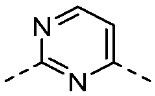

connected to U through the left dashed line;
(5) each "9- to 10-membered heteroaryl" is independently

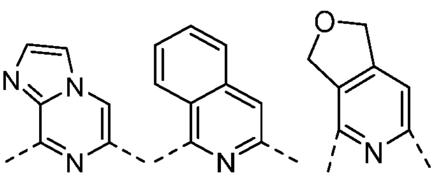

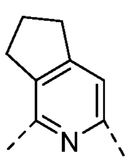 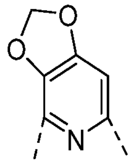 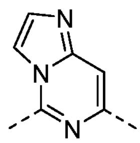 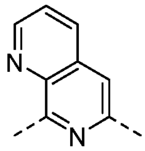 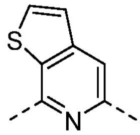

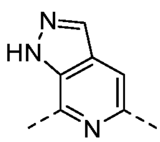 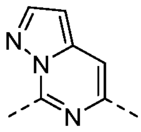 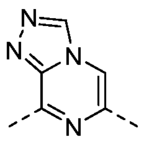 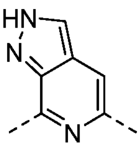 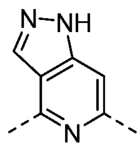 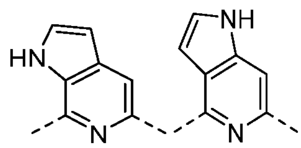

connected to U through the left dashed line;
(6) the "6-membered heterocycloalkenyl" is

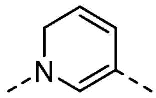 or 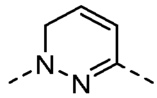

connected to U through the left dashed line;
(7) the "3- to 10-membered cycloalkyl" is

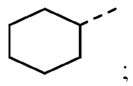 ;

and
(8) each "4- to 10-membered heterocycloalkyl" is independently

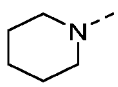 or 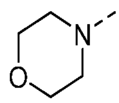 .

**15.** The nitrogen-containing compound represented by formula II, the pharmaceutically acceptable salt thereof, the stereoisomer thereof, the tautomer thereof, the metabolite thereof, the prodrug thereof, the isotopically labeled derivative thereof, the solvate thereof, or the solvate of the pharmaceutically acceptable salt thereof according to claim 2 or 10, wherein one or more of the following conditions are satisfied:

(1) $R^x$ is any one of the following fragments:

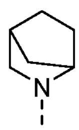 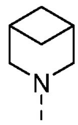 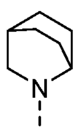 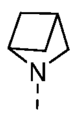 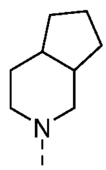 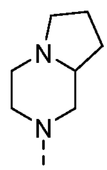 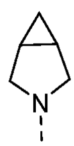 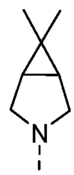 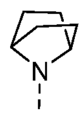

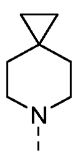 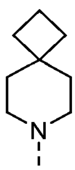 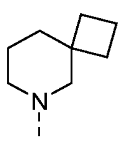 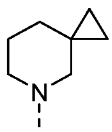 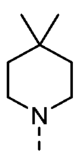 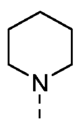 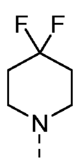 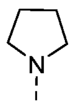 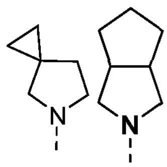

or

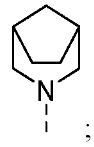;

(2) $R_1$ is any one of the following fragments:

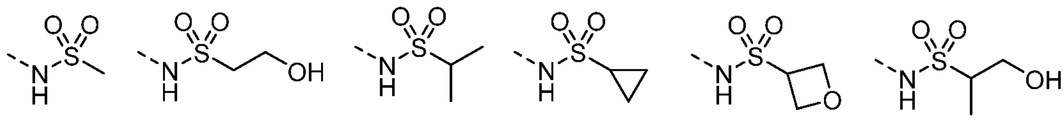

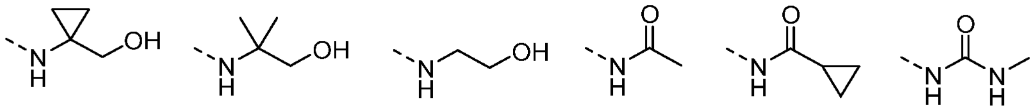

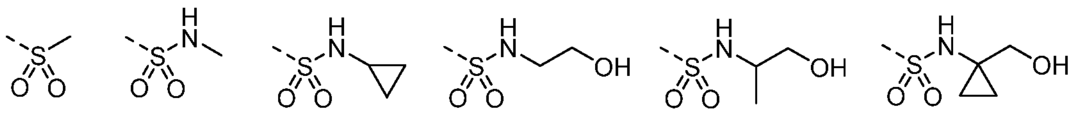

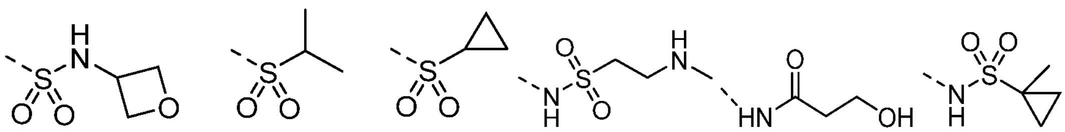 or

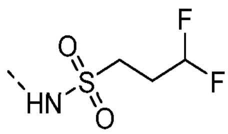;

(3) B is any one of the following fragments:

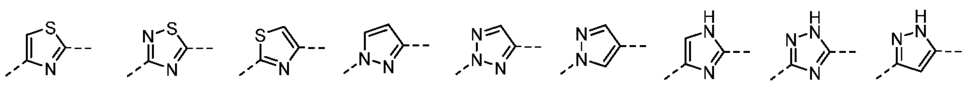

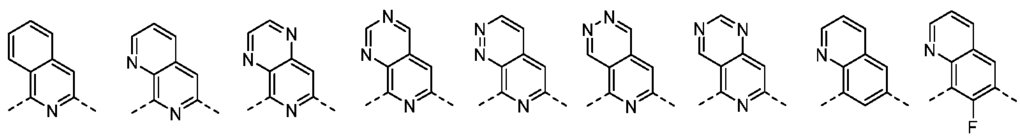

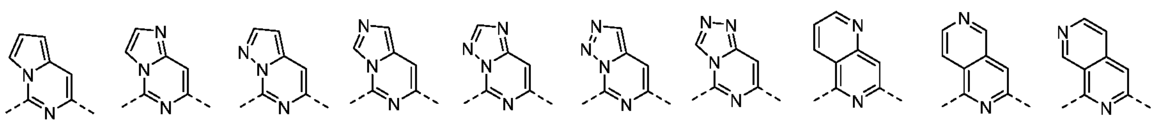

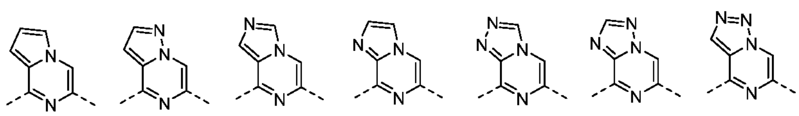

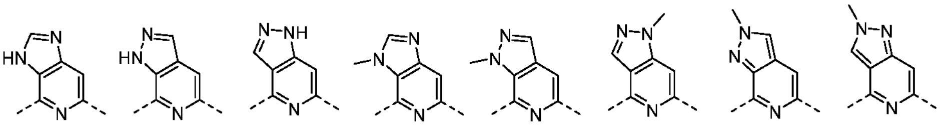

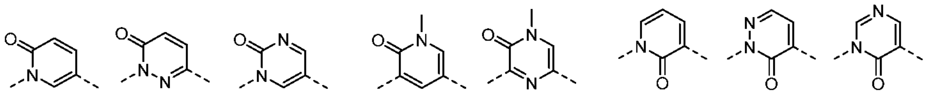

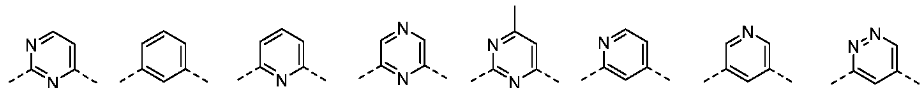

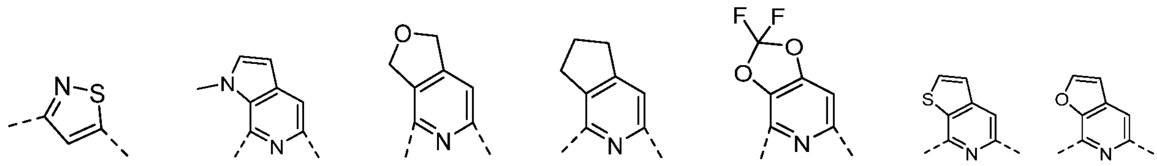

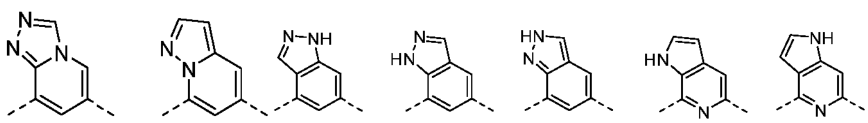;

and

(4) U is any one of the following fragments:

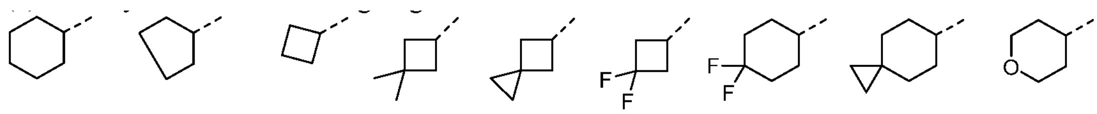

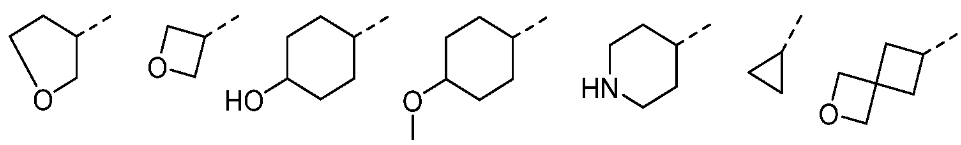

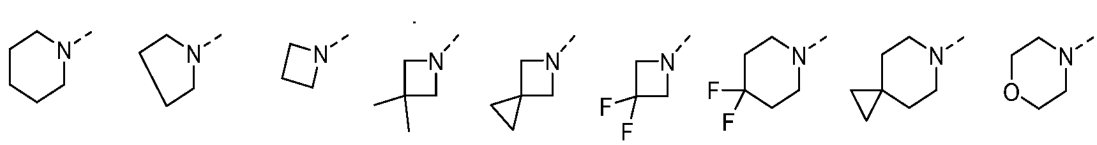

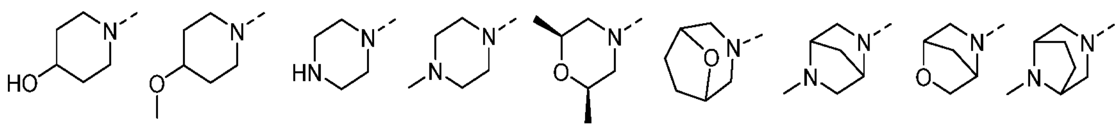

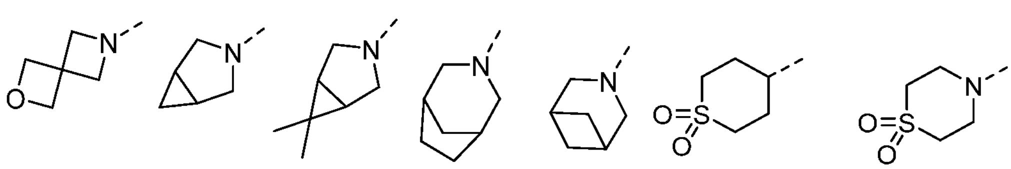

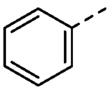 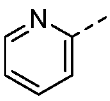 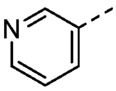 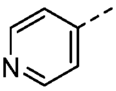 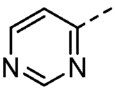 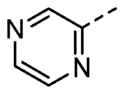 or 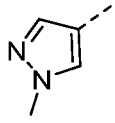 .

**16.** The nitrogen-containing compound represented by formula II, the pharmaceutically acceptable salt thereof, the stereoisomer thereof, the tautomer thereof, the metabolite thereof, the prodrug thereof, the isotopically labeled derivative thereof, the solvate thereof, or the solvate of the pharmaceutically acceptable salt thereof according to claim 2 or 10, wherein one or more of the following conditions are satisfied:

(1) $R^x$ is

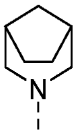 , 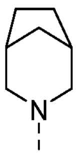 , 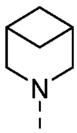 , 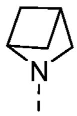 , 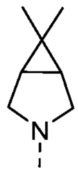 , 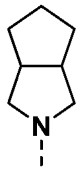 , or 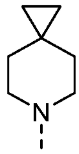 ;

(2) $R_1$ is

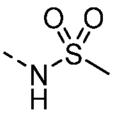 , 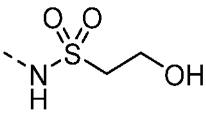 , 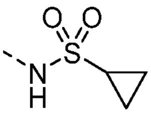 , 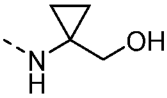 , 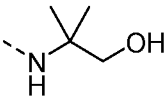 , 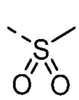 ,

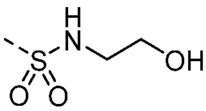 , 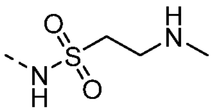 , 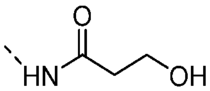 , 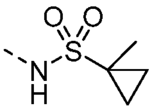 , or 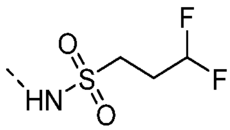 ;

(3) B is

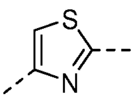 , 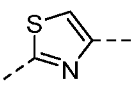 , 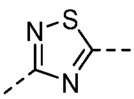 , 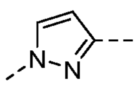 , 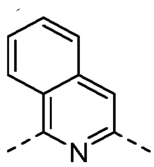 , 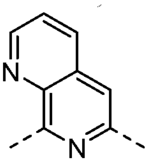 ,

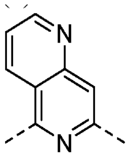 , 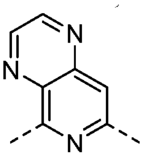 , 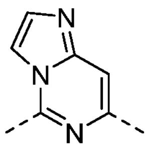 , 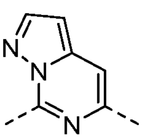 , 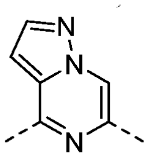 , 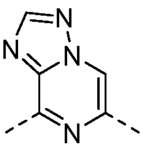 , 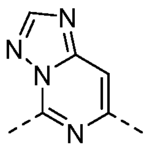 ,

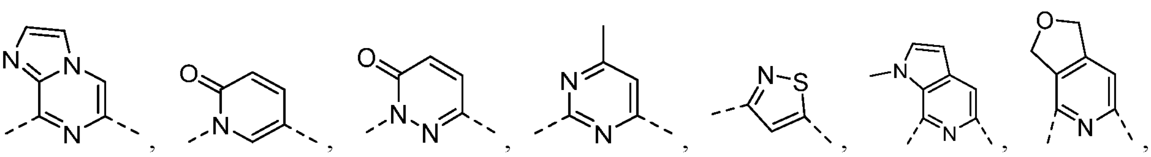 , , , , , , ,

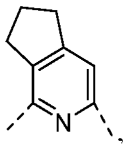 , or 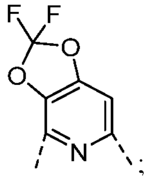 ;

(4) U is

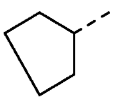 , 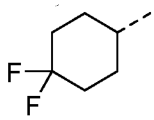 , 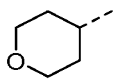 , 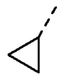 , 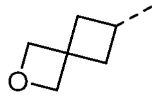 , 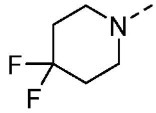 , 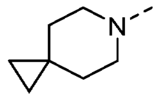 ,

or

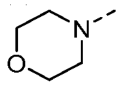 .

**17.** A nitrogen-containing compound, a pharmaceutically acceptable salt thereof, a stereoisomer thereof, a tautomer thereof, a metabolite thereof, a prodrug thereof, an isotopically labeled derivative thereof, a solvate thereof, or a solvate of the pharmaceutically acceptable salt thereof, wherein the nitrogen-containing compound is any one of following compounds:

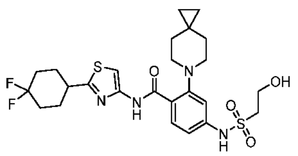 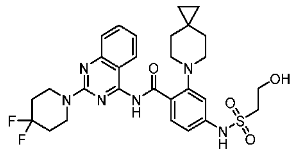 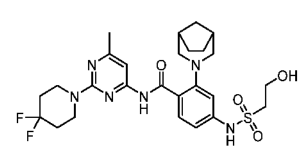 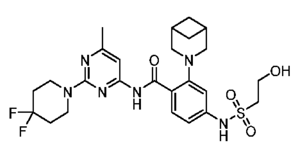

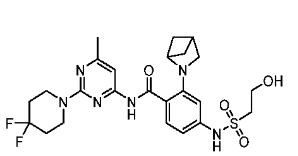 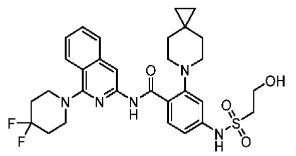 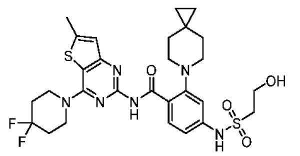 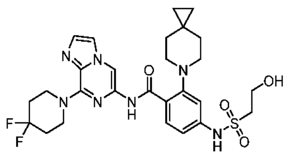

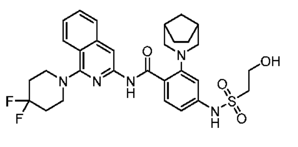 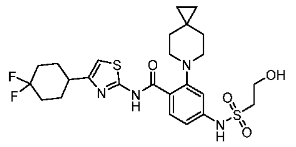 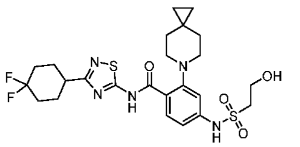 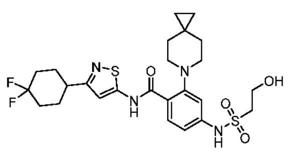

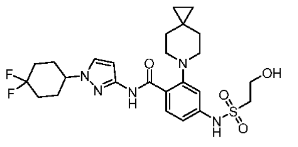 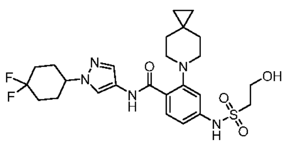 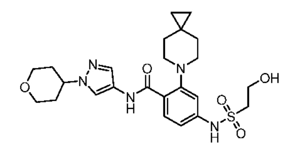 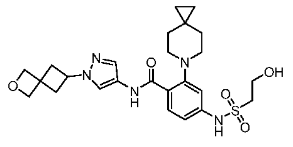

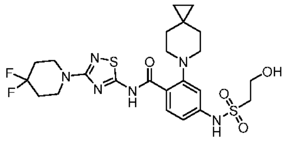 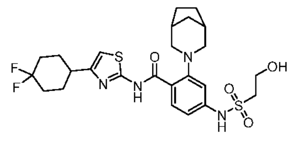 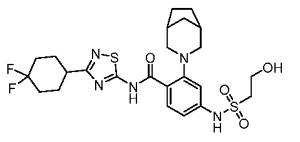 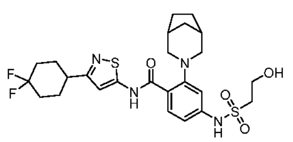

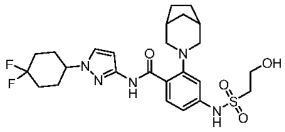
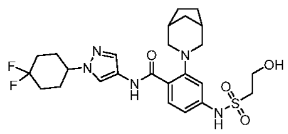
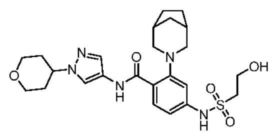
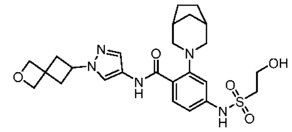

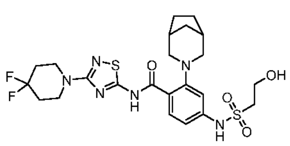
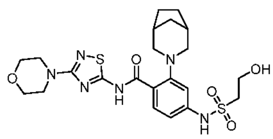
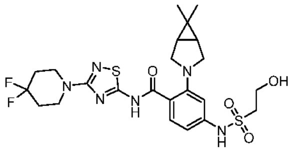
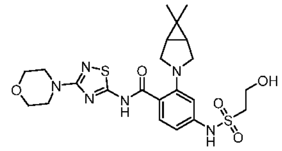

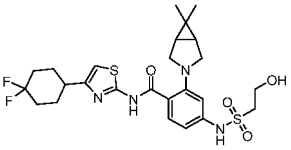
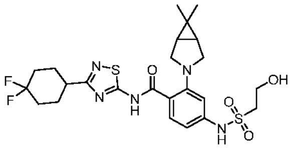
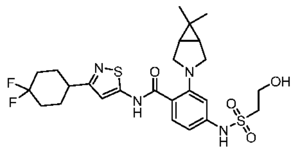

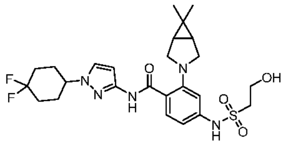
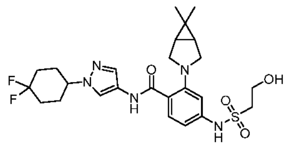
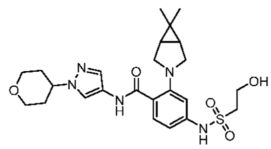
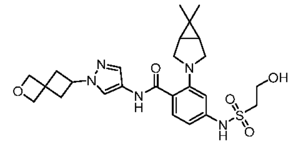

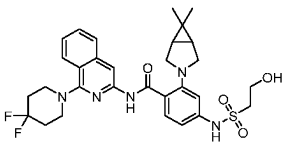
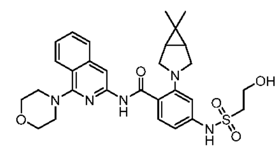
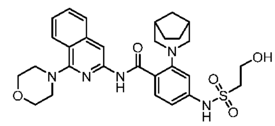

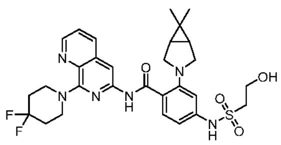
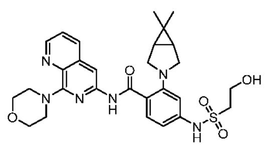
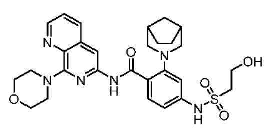
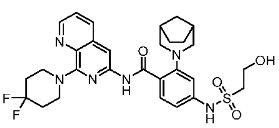

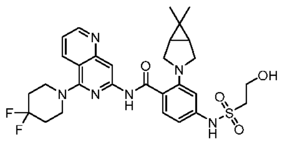
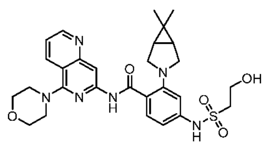
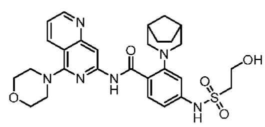
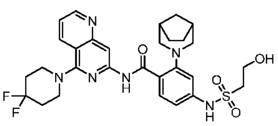

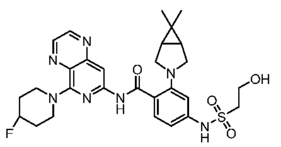
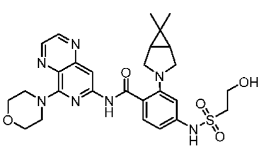
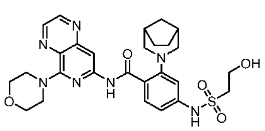
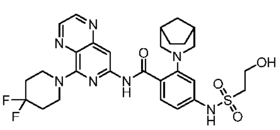

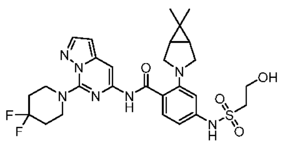
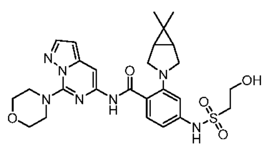
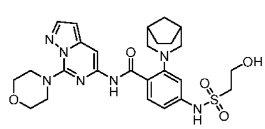
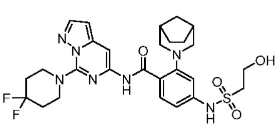

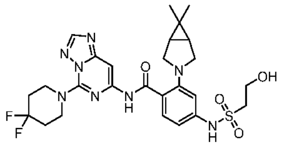
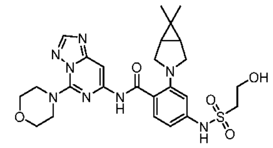
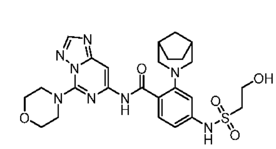
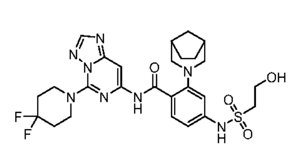
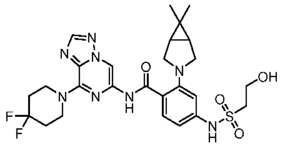
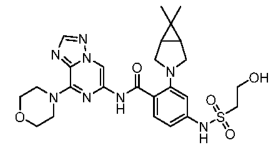
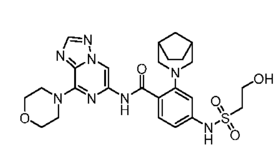
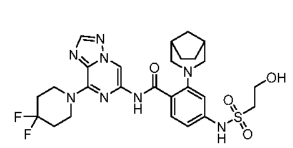
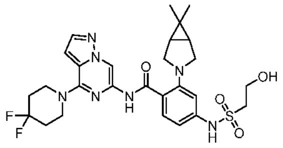
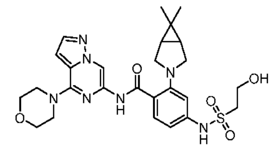
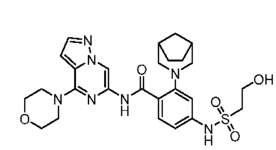
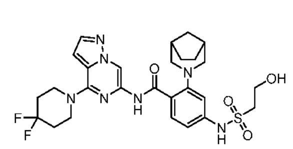
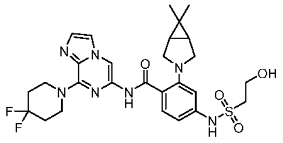
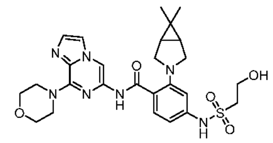
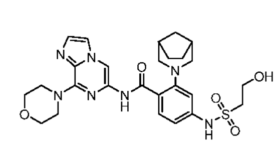
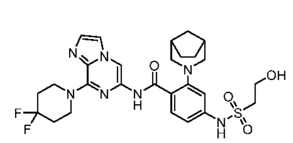
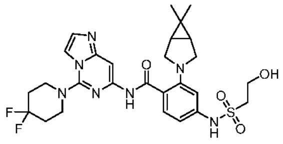
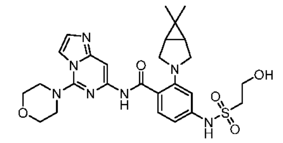
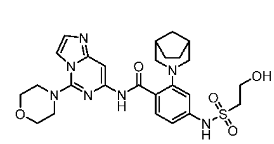
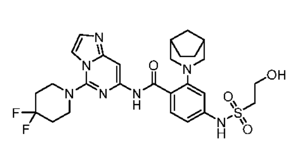
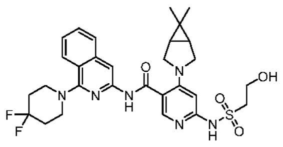
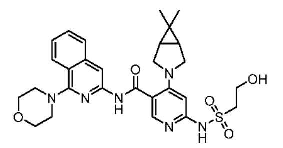
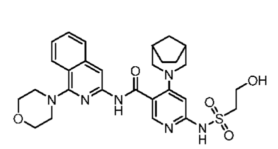
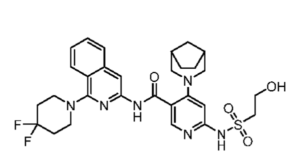
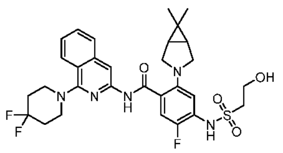
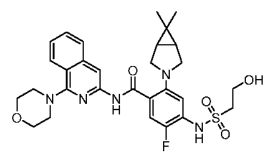
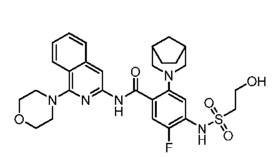
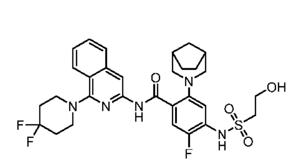
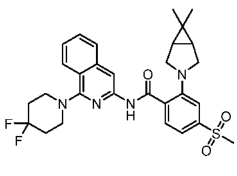
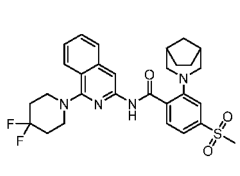
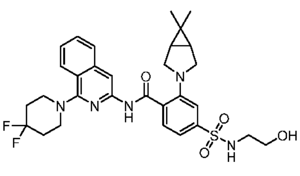
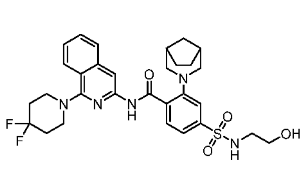
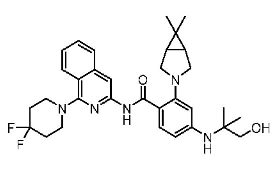
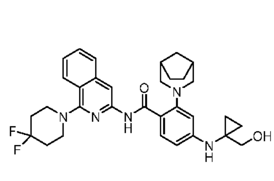
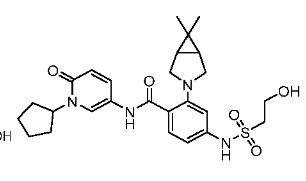
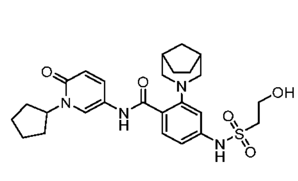

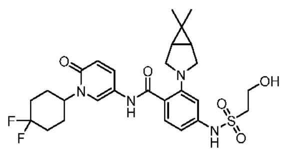 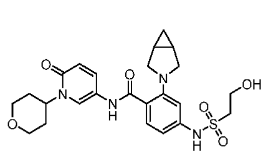 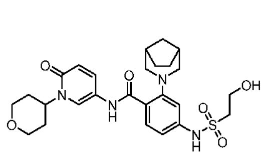 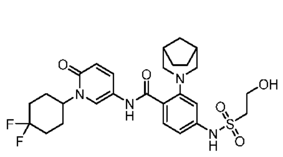

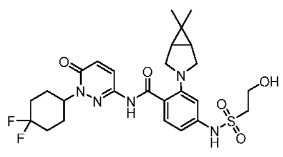 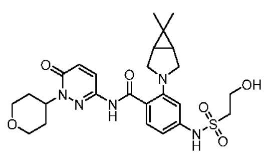 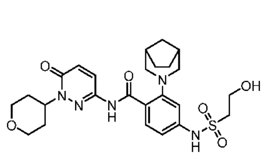 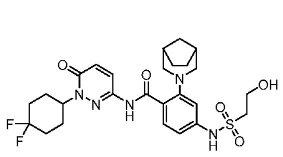

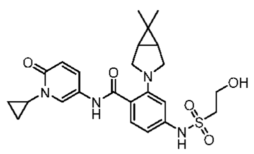 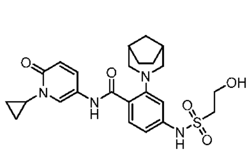

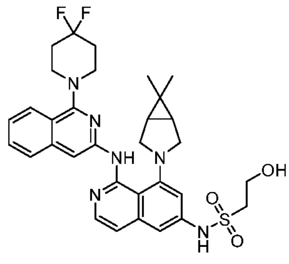 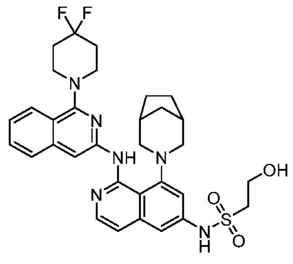 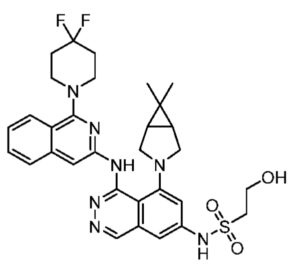 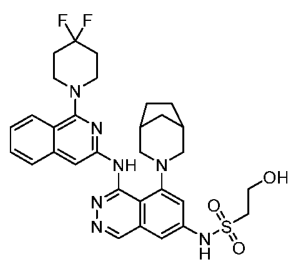

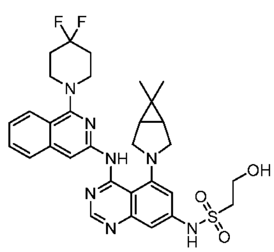 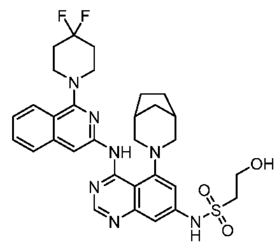

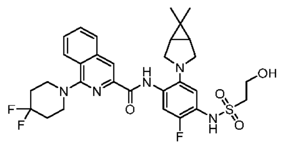 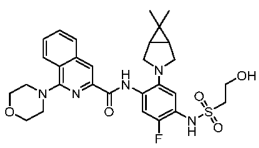 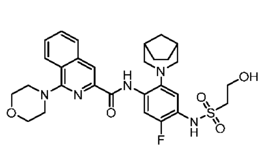 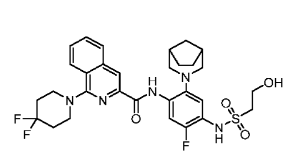

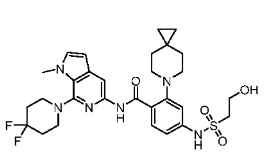 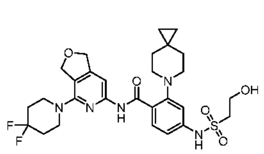 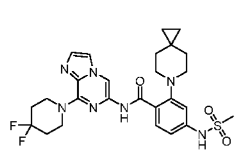 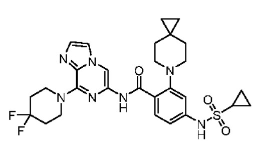

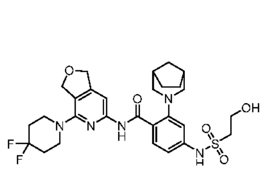 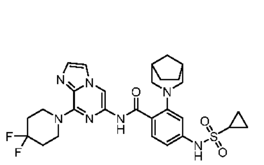 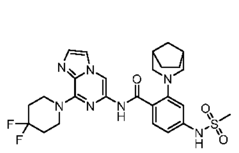 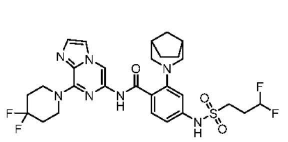

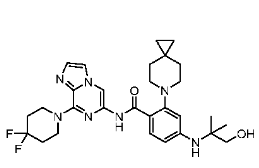
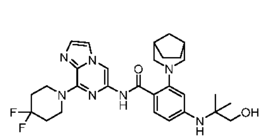
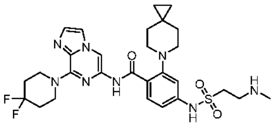
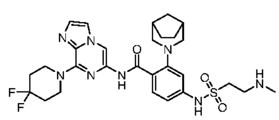

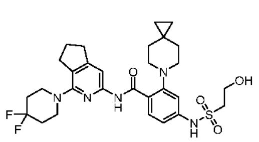
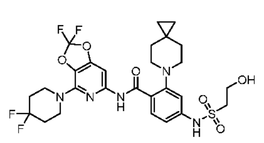
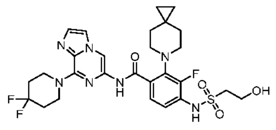
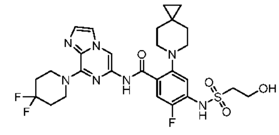

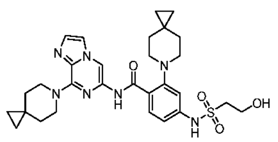
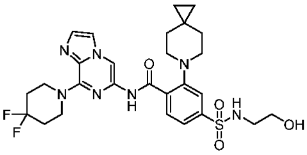
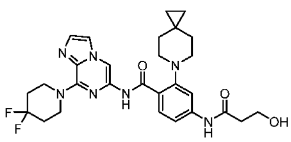
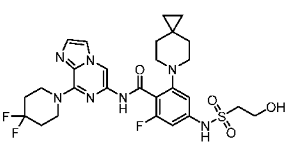

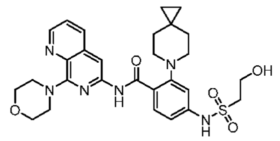
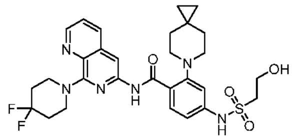
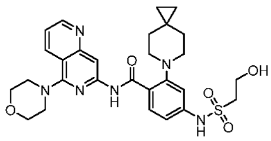
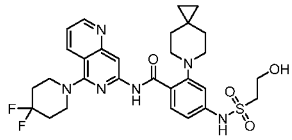

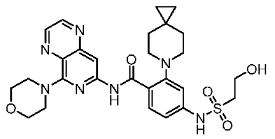
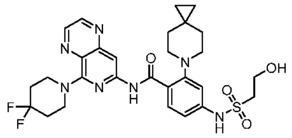
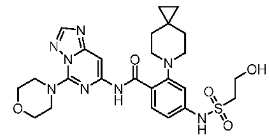
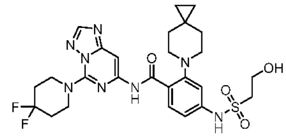

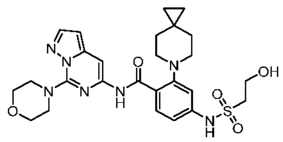
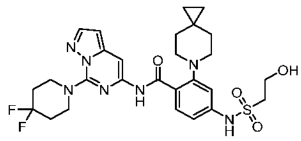
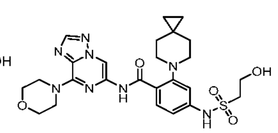
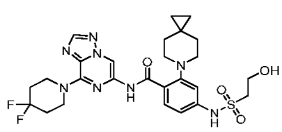

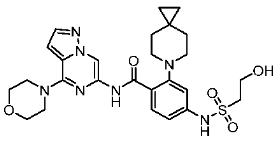
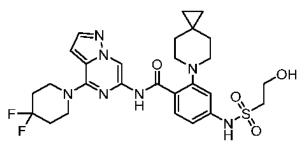
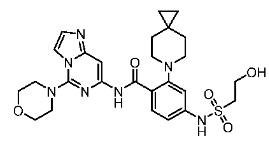
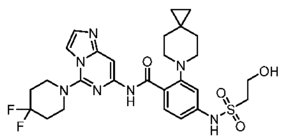

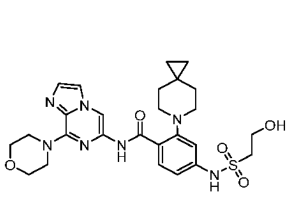
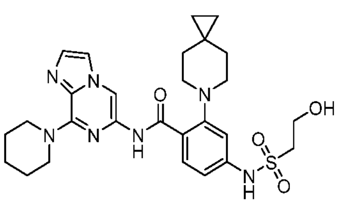
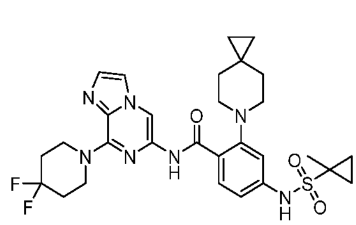

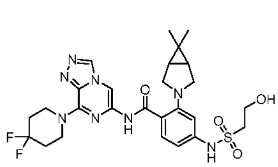
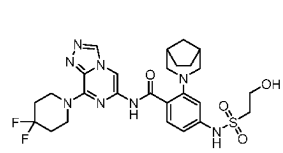
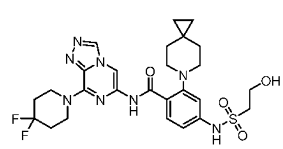

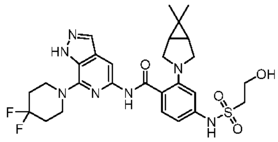
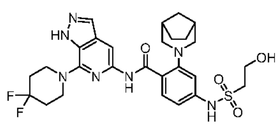
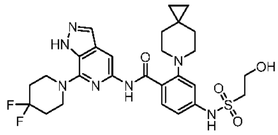

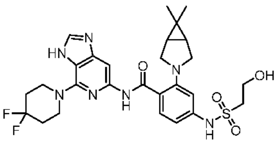
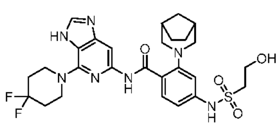
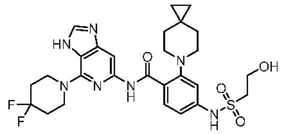

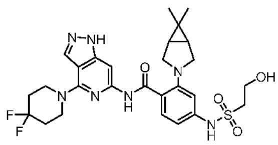
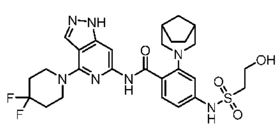
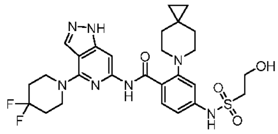

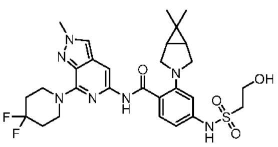
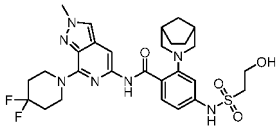
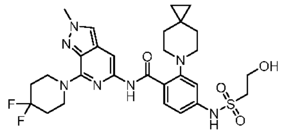

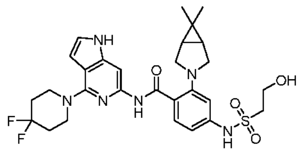
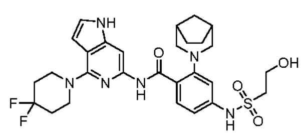
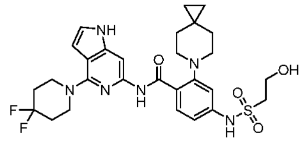

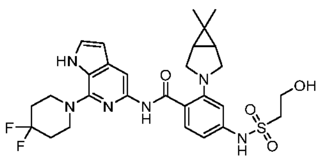
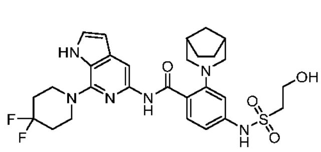
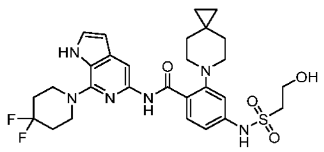

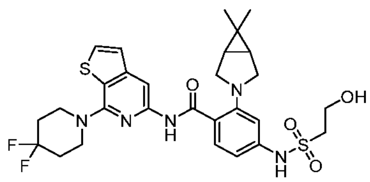
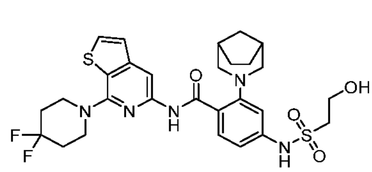
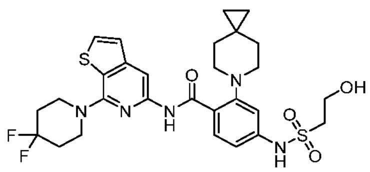

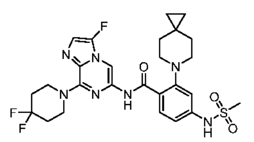
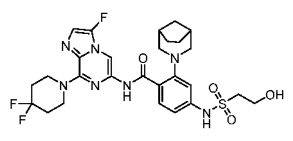
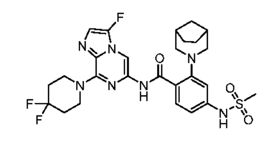
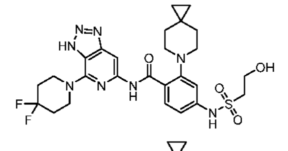
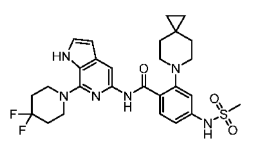
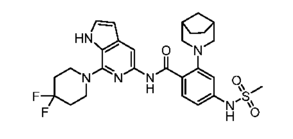
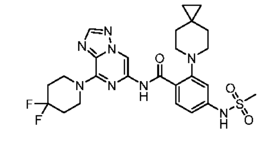
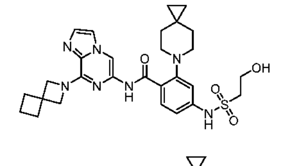
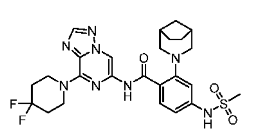
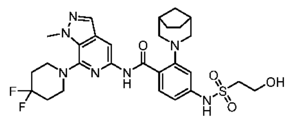
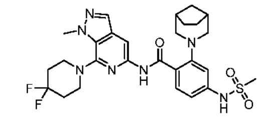
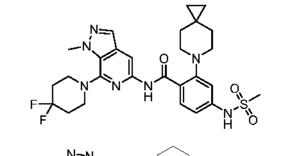
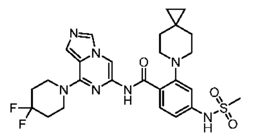
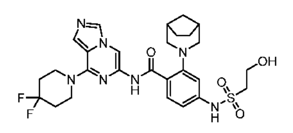
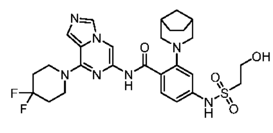
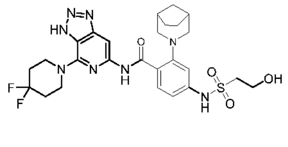
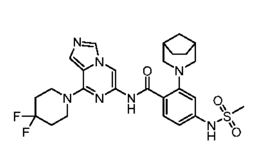
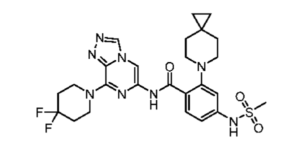
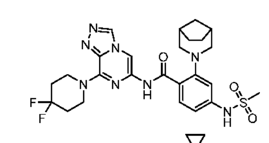
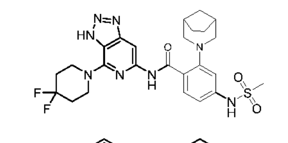
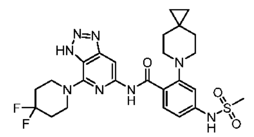
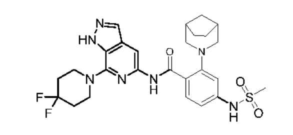
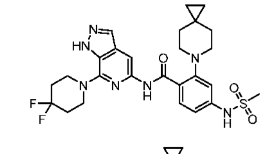
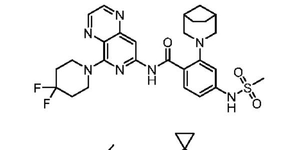
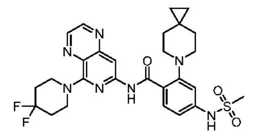
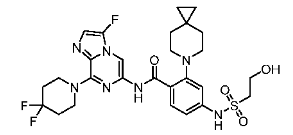
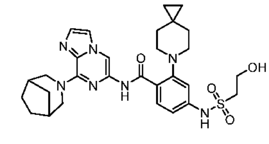
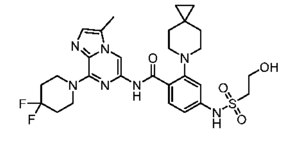
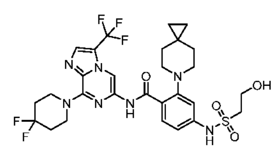
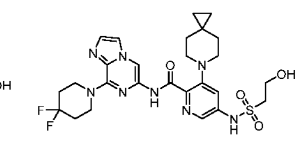
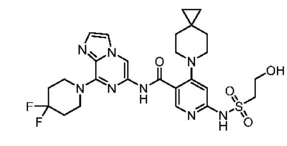
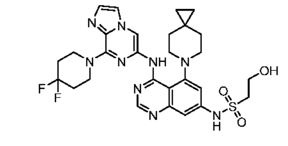
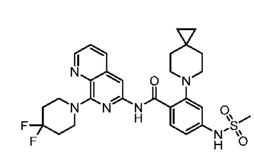
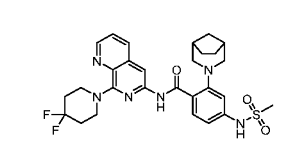
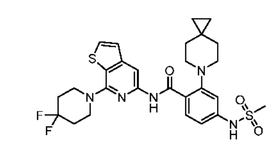
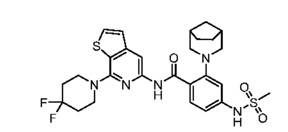
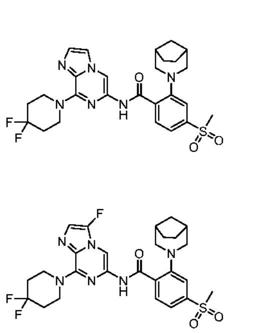
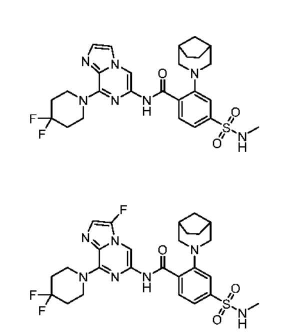
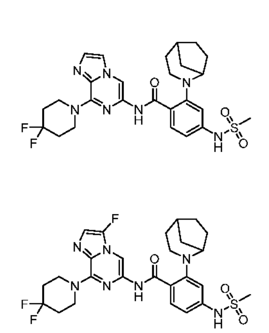
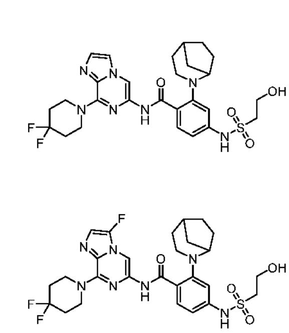

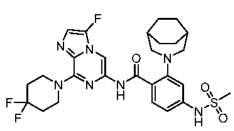 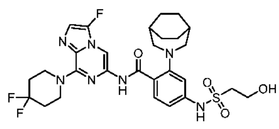 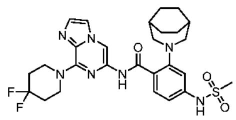 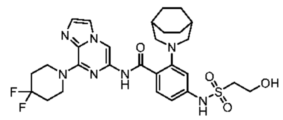

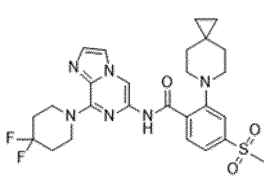 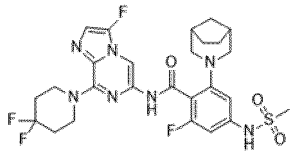 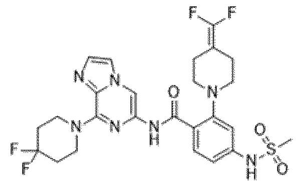 .

**18.** A pharmaceutical composition, comprising:

(1) the nitrogen-containing compound represented by formula I, the pharmaceutically acceptable salt thereof, the stereoisomer thereof, the tautomer thereof, the metabolite thereof, the prodrug thereof, the isotopically labeled derivative thereof, the solvate thereof, or the solvate of the pharmaceutically acceptable salt thereof according to claim 1, the nitrogen-containing compound represented by formula II, the pharmaceutically acceptable salt thereof, the stereoisomer thereof, the tautomer thereof, the metabolite thereof, the prodrug thereof, the isotopically labeled derivative thereof, the solvate thereof, or the solvate of the pharmaceutically acceptable salt thereof according to claim 2 or 10, or the nitrogen-containing compound, the pharmaceutically acceptable salt thereof, the stereoisomer thereof, the tautomer thereof, the metabolite thereof, the prodrug thereof, the isotopically labeled derivative thereof, the solvate thereof, or the solvate of the pharmaceutically acceptable salt thereof according to claim 17, and
(2) a pharmaceutically acceptable auxiliary material.

**19.** Use of the nitrogen-containing compound represented by formula I, the pharmaceutically acceptable salt thereof, the stereoisomer thereof, the tautomer thereof, the metabolite thereof, the prodrug thereof, the isotopically labeled derivative thereof, the solvate thereof, or the solvate of the pharmaceutically acceptable salt thereof according to claim 1, the nitrogen-containing compound represented by formula II, the pharmaceutically acceptable salt thereof, the stereoisomer thereof, the tautomer thereof, the metabolite thereof, the prodrug thereof, the isotopically labeled derivative thereof, the solvate thereof, or the solvate of the pharmaceutically acceptable salt thereof according to claim 2 or 10, or the nitrogen-containing compound, the pharmaceutically acceptable salt thereof, the stereoisomer thereof, the tautomer thereof, the metabolite thereof, the prodrug thereof, the isotopically labeled derivative thereof, the solvate thereof, or the solvate of the pharmaceutically acceptable salt thereof according to claim 17 in the preparation of a KIF18A inhibitor.

**20.** Use of the nitrogen-containing compound represented by formula I, the pharmaceutically acceptable salt thereof, the stereoisomer thereof, the tautomer thereof, the metabolite thereof, the prodrug thereof, the isotopically labeled derivative thereof, the solvate thereof, or the solvate of the pharmaceutically acceptable salt thereof according to claim 1, the nitrogen-containing compound represented by formula II, the pharmaceutically acceptable salt thereof, the stereoisomer thereof, the tautomer thereof, the metabolite thereof, the prodrug thereof, the isotopically labeled derivative thereof, the solvate thereof, or the solvate of the pharmaceutically acceptable salt thereof according to claim 2 or 10, or the nitrogen-containing compound, the pharmaceutically acceptable salt thereof, the stereoisomer thereof, the tautomer thereof, the metabolite thereof, the prodrug thereof, the isotopically labeled derivative thereof, the solvate thereof, or the solvate of the pharmaceutically acceptable salt thereof according to claim 17 in the preparation of a medicament, wherein the medicament is used for treating a disease related to the mechanism of action of a KIF18A protein, for example, a cancer, and for another example, a cancer related to the mechanism of action of the KIF18A protein; preferably, the cancer is ovarian cancer, colon cancer, or ductal breast cancer.

**21.** Use of the nitrogen-containing compound represented by formula I, the pharmaceutically acceptable salt thereof, the stereoisomer thereof, the tautomer thereof, the metabolite thereof, the prodrug thereof, the isotopically labeled derivative thereof, the solvate thereof, or the solvate of the pharmaceutically acceptable salt thereof according to claim 1, the nitrogen-containing compound represented by formula II, the pharmaceutically acceptable salt thereof, the stereoisomer thereof, the tautomer thereof, the metabolite thereof, the prodrug thereof, the isotopically labeled derivative thereof, the solvate thereof, or the solvate of the pharmaceutically acceptable salt thereof according to claim 2 or 10, or the nitrogen-containing compound, the pharmaceutically acceptable salt thereof, the stereoisomer thereof,

the tautomer thereof, the metabolite thereof, the prodrug thereof, the isotopically labeled derivative thereof, the solvate thereof, or the solvate of the pharmaceutically acceptable salt thereof according to claim 17 in the preparation of a medicament, wherein the medicament is used for treating a cancer; preferably, the cancer is ovarian cancer, colon cancer, or ductal breast cancer.

**INTERNATIONAL SEARCH REPORT**

International application No.
**PCT/CN2023/104529**

**A. CLASSIFICATION OF SUBJECT MATTER**

C07D417/12(2006.01)i; C07D401/04(2006.01)i; C07D401/14(2006.01)i; C07D413/14(2006.01)i; A61K31/517(2006.01)i; A61K31/5377(2006.01)i; A61P35/00(2006.01)i

According to International Patent Classification (IPC) or to both national classification and IPC

**B. FIELDS SEARCHED**

Minimum documentation searched (classification system followed by classification symbols)

IPC: C07D,A61K,A61P

Documentation searched other than minimum documentation to the extent that such documents are included in the fields searched

Electronic data base consulted during the international search (name of data base and, where practicable, search terms used)

CNTXT, WPABS, CAPLUS (STN), REGISTRY (STN), CNKI: 勤浩医药, KIF18A, 磺酰, 氮杂螺, 哌啶, 肿瘤, 癌症, 癌, azaspiro?, sulfonamido, piperidin?, cancer, tumor, tumour, 结构式检索, structural formula search.

**C. DOCUMENTS CONSIDERED TO BE RELEVANT**

| Category* | Citation of document, with indication, where appropriate, of the relevant passages | Relevant to claim No. |
|---|---|---|
| X | CN 114269731 A (AMGEN INC.) 01 April 2022 (2022-04-01)<br>claims 1 and 44-49, and description, paragraphs [0104], [0106], [0111], [0113]-[0115], [0117]-[0120], [0124]-[0128], [0130], [0132], [0139]-[0141], [0143]-[0145] and [0147] | 1-3, 5-9, 11-21 |
| X | CN 114391012 A (AMGEN INC.) 22 April 2022 (2022-04-22)<br>claims 1, 40 and 42-47 | 1-21 |
| PX | WO 2022268230 A1 (HANGZHOU INNOGATE PHARMA CO., LTD.) 29 December 2022 (2022-12-29)<br>claims 1-15, and description, page 15, compound 15, page 17, compound 16, pages 21-22 | 1-9, 11-21 |
| A | WO 2020132649 A1 (AMGEN INC.) 25 June 2020 (2020-06-25)<br>claims 1-38 | 1-21 |
| A | WO 2020132651 A1 (AMGEN INC.) 25 June 2020 (2020-06-25)<br>claims 1-35 | 1-21 |
| A | WO 2020132653 A1 (AMGEN INC.) 25 June 2020 (2020-06-25)<br>claims 1-40 | 1-21 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **31 August 2023** | **07 September 2023** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**

International application No.
**PCT/CN2023/104529**

**C. DOCUMENTS CONSIDERED TO BE RELEVANT**

| Category* | Citation of document, with indication, where appropriate, of the relevant passages | Relevant to claim No. |
|---|---|---|
| A | WO 2021211549 A1 (AMGEN INC.) 21 October 2021 (2021-10-21)<br>description, pages 79-88 | 1-21 |
| A | CN 113226473 A (AMGEN INC.) 06 August 2021 (2021-08-06)<br>claims 1-42 | 1-21 |
| A | CN 114302880 A (AMGEN INC.) 08 April 2022 (2022-04-08)<br>claims 1-35 | 1-21 |

Form PCT/ISA/210 (second sheet) (July 2022)

**INTERNATIONAL SEARCH REPORT**
**Information on patent family members**

International application No.
**PCT/CN2023/104529**

| Patent document cited in search report | | | Publication date (day/month/year) | Patent family member(s) | | | Publication date (day/month/year) |
|---|---|---|---|---|---|---|---|
| CN | 114269731 | A | 01 April 2022 | US | 2022281843 | A1 | 08 September 2022 |
| | | | | JP | 2022542394 | A | 03 October 2022 |
| | | | | CA | 3147451 | A1 | 11 February 2021 |
| | | | | AU | 2020324963 | A1 | 24 February 2022 |
| | | | | WO | 2021026101 | A1 | 11 February 2021 |
| | | | | EP | 4007756 | A1 | 08 June 2022 |
| CN | 114391012 | A | 22 April 2022 | EP | 4007638 | A1 | 08 June 2022 |
| | | | | US | 2022372018 | A1 | 24 November 2022 |
| | | | | WO | 2021026100 | A1 | 11 February 2021 |
| | | | | JP | 2022542392 | A | 03 October 2022 |
| | | | | CA | 3147276 | A1 | 11 February 2021 |
| | | | | AU | 2020325115 | A1 | 17 March 2022 |
| WO | 2022268230 | A1 | 29 December 2022 | None | | | |
| WO | 2020132649 | A1 | 25 June 2020 | None | | | |
| WO | 2020132651 | A1 | 25 June 2020 | None | | | |
| WO | 2020132653 | A1 | 25 June 2020 | None | | | |
| WO | 2021211549 | A1 | 21 October 2021 | None | | | |
| CN | 113226473 | A | 06 August 2021 | CA | 3123871 | A1 | 25 June 2020 |
| | | | | UY | 38526 | A | 30 June 2020 |
| | | | | US | 2022106293 | A1 | 07 April 2022 |
| | | | | PE | 20211475 | A1 | 05 August 2021 |
| | | | | US | 2020239441 | A1 | 30 July 2020 |
| | | | | US | 11236069 | B2 | 01 February 2022 |
| | | | | CL | 2021001634 | A1 | 10 December 2021 |
| | | | | CR | 20210387 | A | 19 August 2021 |
| | | | | TW | 202034924 | A | 01 October 2020 |
| | | | | JP | 2022513972 | A | 09 February 2022 |
| | | | | WO | 2020132648 | A1 | 25 June 2020 |
| | | | | AU | 2019403486 | A1 | 24 June 2021 |
| | | | | IL | 283639 | A | 29 July 2021 |
| | | | | BR | 112021011989 | A2 | 08 September 2021 |
| | | | | KR | 20210106474 | A | 30 August 2021 |
| | | | | CO | 2021008224 | A2 | 30 July 2021 |
| | | | | MX | 2021007156 | A | 16 August 2021 |
| | | | | EP | 3897852 | A1 | 27 October 2021 |
| | | | | EA | 202191730 | A1 | 24 August 2021 |
| | | | | AR | 117490 | A1 | 11 August 2021 |
| | | | | MA | 54543 | A | 30 March 2022 |
| | | | | JOP | 20210154 | A1 | 30 January 2023 |
| | | | | SG | 11202106520 | VA | 29 July 2021 |
| CN | 114302880 | A | 08 April 2022 | CA | 3146693 | A1 | 11 February 2021 |
| | | | | US | 2022289724 | A1 | 15 September 2022 |
| | | | | EP | 4007752 | A1 | 08 June 2022 |
| | | | | JP | 2022542967 | A | 07 October 2022 |
| | | | | WO | 2021026098 | A1 | 11 February 2021 |
| | | | | AU | 2020326627 | A1 | 17 March 2022 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 2022107730353
- CN 202310066234
- CN 2023107394075
- WO 2020132648 A1


### Non-patent literature cited in the description


- **P. HEINRICH STAHL** ; **CAMILLE G. WERMUTH**. Handbook of Pharmaceutical Salts: Properties, Selection, and Use. 2011
- Pharmacopoeia of the People's Republic of China. 2020
- **PAUL J SHESKEY** ; **BRUNO C HANCOCK** ; **GARY P MOSS** ; **DAVID J GOLDFARB**. Handbook of Pharmaceutical Excipients. 2020
- **BRIAN DAVIES** ; **TIM MORRIS**. Physiological Parameters in Laboratory Animals and Human. *Pharmaceutical Research*, 1993, vol. 10 (7)
- *Journal of Pharmacology and Experimental Therapeutics*, 1997, vol. 283 (1), 46-58